# EUROPEAN PATENT APPLICATION

(11) **EP 4 218 773 A1**
(43) Date of publication of application: **02.08.2023**
(21) Application number: 23160003.2
(22) Date of filing: 14.02.2017
(51) Int. Cl.: A61K 35/00, C12N 5/079, G01N 33/00, A61P 27/00, A61P 27/02, A61K 35/30

(54) **HUMAN FUNCTIONAL CORNEAL ENDOTHELIAL CELL AND APPLICATION THEREOF**

(30) Priority: 15.02.2016 JP 2016026423; 15.02.2016 JP 2016026424; 15.02.2016 JP 2016026425; 15.02.2016 JP 2016026426; 07.04.2016 JP 2016077450
(62) Divisional of application: 17709487.7
(71) Applicant: Kyoto Prefectural Public University Corporation, Kyoto-shi, Kyoto 602-8566 (JP)
(72) Inventor: KINOSHITA, Shigeru, Kyoto-shi, Kyoto, 602-8566 (JP); HAMURO, Junji, Kyoto-shi, Kyoto, 602-8566 (JP); SOTOZONO, Chie, Kyoto-shi, Kyoto, 602-8566 (JP); UENO, Morio, Kyoto-shi, Kyoto, 602-8566 (JP)
(74) Representative: Engelhard, Markus

(57) **Abstract**

The present invention complete a technique of treating a corneal disorder or disease by infusion into an anterior chamber of human eyes. Specifically, the present invention based on the findings discovered that cultured human corneal endothelial cells are comprised of a plurality of subpopulations, most of them are not suitable for infusion into patients. The above-described subject was overcome by providing, as a medicament, functionally high grade quality of cells having the function of mature differentiated human corneal endothelial cells which is a specific subpopulation and characterized by their biochemical and functional phenotypes. The present invention provides such a functional mature differentiated corneal endothelial cells, medicament comprising the same, and manufacturing method, quality control and techniques related thereto.

## Description

### [Technical Field]

The present invention relates to a human functional corneal endothelial cell capable of eliciting a human corneal functional property when infused into an anterior chamber of a human eye, medicament comprising the cell, manufacturing method thereof, and application thereof in quality control of the manufactured cell and the processes of the manufacturing or the like.

### [Background Art]

Currently, the only therapeutic method for corneal endothelial disorders including bullous keratopathy is corneal transplantation surgery using a donor cornea, although the long-term clinical result of this surgery is poor. Furthermore, the visual acuity after corneal transplantation is not sufficient due to induced corneal irregular astigmatism. About 60% or more of corneal transplantation patients suffer from the corneal endothelial dysfunction (bullous keratopathy). The primary causes of bullous keratopathy are corneal endothelial disorders due to ophthalmic surgery such as cataract surgery, glaucoma surgery, vitreo-retinal surgery, or laser iridotomy, corneal trauma, pseudoexfoliation syndrome, and Fuchs endothelial corneal dystrophy. The potential prevalence rate of Fuchs endothelial corneal dystrophy involving a genetic factor in Europe and US is reportedly about 5% or higher. Corneal transplantation surgery requires one donor cornea for treating one diseased eye, such that transplantation surgery cannot be a means for solving the sustained shortage of donors. In view of the large number of latent patients, there is an intense worldwide demand, as an urgent issue to be solved, for the provision of innovative versatile medical treatment that can be applied at a wide range of medical institutions compared to corneal transplantation techniques. In addition, the cell infusion therapy reproduce the normal shape of the cornea without distortion, resulting in recovering a good visual function.

### [Summary of Invention]

### [Solution to Problem]

The inventors have achieved the present innovative invention by first findings in the world that the cultured human corneal endothelial cell is comprised of multiple subpopulations due to cell state transition (fibrosis, epithelial-mesenchymal transition, senescence, dedifferentiation or the like) in culture; and devising a technique for selectively propagating in cultures a subpopulation, which allows confirmation of a specific subpopulation, i.e., functional cell (herein also called effector cell) which sufficiently share with a function(s) of mature differentiated human corneal endothelial cell, is a mature differentiated endothelial cell, and is optimal for cell infusion therapy, and also form a small hexagonal cobble-stone shape and utilize an energy metabolizing system mainly by a mitochondrial function.

The inventors succeeded in the development of an in vitro culture technique of a functional human corneal endothelial cell, which had long been being considered impossible with conventional culture techniques, and established the methods for infusing high quality grade of functional cultured human corneal endothelial cell manufactured by this technique into the anterior chamber of a human eye. The concept of regenerating corneal endothelia by intra-anterior chamber infusion is (1) minimally invasive, (2) uses no artificial material as substrates, and (3) allows use of a high quality functional cultured human corneal endothelial cell from a young donor with little senescence as a master cell.

Thus, the present invention provides the following.

### (Cell invention)

In another aspect, the present invention also provides the following.
(Item 1) A human functional corneal endothelial cell capable of eliciting a human corneal endothelial functional property when infused into an anterior chamber of human eyes.
(Item 2) The cell of Item 1, wherein the cell expresses cell surface antigens comprising CD166 positive and CD133 negative phenotypes.
(Item 3) The cell of Item 2, wherein the cell surface antigens comprise CD166 positive, CD133 negative, and CD44 negative to intermediately positive phenotypes.
(Item 4) The cell of Item 2, wherein the cell surface antigens comprise CD166 positive, CD133 negative, and CD44 negative to CD44 weakly positive phenotypes.
(Item 5) The cell of Item 2, wherein the cell surface antigens comprise CD166 positive, CD133 negative, and CD200 negative phenotypes.
(Item 6) The cell of any one of Items 2-5, further comprising at least one expression property selected from the group consisting of CD90 negative to weakly positive, CD105 negative to weakly positive, CD24 negative, CD26 negative, LGR5 negative, SSEA3 negative, MHC1 weakly positive, MHC2 negative, PDL1 positive, ZO1 positive, Na⁺/K⁺ATPase positive and a cell surface antigen described in the following table:

**[Table 1-1A]**

| **Cell surface marker** | **Functional cell** |
|---|---|
| CD59 | Strongly positive |
| CD147 | Strongly positive |
| CD81 | Strongly positive |
| CD73 | Strongly positive |
| CD49c | Strongly positive |
| CD166 | Strongly positive |
| CD56 | Intermediately positive |
| CD54 | Intermediately positive |
| B2-uGlob | Intermediately positive |
| CD47 | Intermediately positive |
| CD46 | Intermediately positive |
| CD141 | Intermediately positive |
| CD151 | Intermediately positive |
| CD98 | Weakly positive |
| CD165 | Weakly positive |
| CD340 (Her2) | Weakly positive |
| CD58 | Weakly positive |
| CD201 | Weakly positive |
| CD140b | Weakly positive |
| EGF-r | Weakly positive |
| CD63 | Weakly positive |
| CD9 | Negative |
| CD49b | Negative |
| CD227 | Negative |
| CD90 | Negative |
| CD44 | Negative |

(Item 7) The cell of any one of Items 1-6, wherein the cell has at least one property selected from the group consisting of PDGF-BB high production, IL-8 low production, MCP-1 low production, TNF-alpha high production, IFNgamma high production, and IL-1R antagonist high production.
(Item 8) The cell of any one of Items 1-7, wherein the cell has at least one miRNA with a cell property of mature differentiated functional corneal endothelial cell a5, wherein a property of a cell surface antigen of the a5 is CD44 negative to weakly positive and CD24 negative CD26 negative.
(Item 9) The cell of Item 8, wherein the property of said miRNA comprises at least one miRNA selected from the group consisting of:
(A) functional mature differentiated corneal endothelial cell (a5): intermediately differentiated corneal endothelial cell (a1): corneal endothelial nonfunctional cell (a2) exhibits high expression: high expression: low expression:
   (intracellular) miR23a-3p, miR23b-3p, miR23c, miR27a-3p, miR27b-3p, miR181a-5p, miR181b-5p, miR181c-5p, miR181d-5p
   (cell-secreted) miR24-3p, miR1273e;
(B) a5:a1:a2 exhibits high expression: intermediate expression: low expression:
   (intracellular) miR30a-3p, miR30a-5p, miR30b-5p, miR30c-5p, miR30e-3p, miR30e-5p, miR130a-3p, miR130b-3p, miR378a-3p, miR378c, miR378d, miR378e, miR378f, miR378h, miR378i, miR184, miR148a-3p
   (cell-secreted) miR184;
(C) a5:a1:a2 exhibits high expression: low expression: low expression:
   (intracellular) miR34a-5p, miR34b-5p
   (cell-secreted) miR4419b, miR371b-5p, miR135a-3p, miR3131, miR296-3p, miR920, miR6501-3p;
(D) a5:a1:a2 exhibits low expression: low expression: intermediate to high expression:
   (intracellular) miR29a-3p, miR29b-3p, miR199a-3p, miR199a-5p, miR199b-5p, miR143-3p
   (cell-secreted) miR1915-3p, miR3130-3p, miR92a-2-5p, miR1260a;
(E) a5:a1:a2 exhibits low expression: intermediate expression: high expression:
   (intracellular) miR31-3p, miR31-5p, miR193a-3p, miR193b-3p, miR138-5p
(F) a5:a1:a2 exhibits high expression: low expression: high expression:
   (cell-secreted) miR92b-5p; and
(G) a5:a1:a2 exhibits low expression: high expression: low expression:
   (cell-secreted) miR1246, miR4732-5p, miR23b-3p, miR23a-3p, miR1285-3p, miR5096;
   wherein an expression level is relative intensity among 3 types of cells, expression of a cell surface antigen of the a1 being CD44 intermediately positive CD24 negative CD26 negative, and expression of a cell surface antigen of the a2 being CD44 strongly positive CD24 negative CD26 positive.
(Item 10) The cell of Item 9, wherein the miRNA marker comprises at least one selected from (B) or (C).
(Item 11) The cell of any one of Items 1-10, wherein a mean cell area of the cell is 250 .micro.m² or less. As used herein, "micro." signifies Greek letter and means 10⁻⁶.
(Item 12) The cell of any one of Items 1-11 having a cell function property homologous to a5 in at least one cell indicator selected from the group consisting of: a cell surface marker; a proteinaceous product or a related biological material of the product; a SASP related protein; miRNA; an exosome; a cellular metabolite comprising an amino acid and a related biological material of the metabolite; cell size; cell density and the presence of an autoantibody reactive cell.
(Item 13) The cell of any one of Items 1-12, wherein the cell does not have a karyotype abnormality.
(Item 14) A cell population comprising the cell of any one of Items 1-13.
(Item 15) The cell population of Item 14, wherein a mean cell density as of saturated cell culture (culture confluence) of the cell population is at least 1500 cells/mm² or greater.
(Item 16) The cell population of Item 14 or 15, wherein a mean cell density as of saturated cell culture (culture confluence) of the cell population is at least 2000 cells/mm² or greater.
(Item 17) The cell population of any one of Items 14-16, wherein a mean cell density of cells integrated into a human corneal endothelial surface after infusing the cell population is at least 1000 cells/mm² or greater.
(Item 18) The cell population of any one of Items 14-17, wherein a mean cell density of cells integrated into a human corneal endothelial surface after infusing the cell population is at least 2000 cells/mm² or greater.
(Item 19) The cell population of any one of Items A14-A18, wherein at least 70% of cells in the cell population have the characteristic of Item A2 or A3.
(Item 20) The cell population of any one of Items A14-A19, wherein at least 90% of cells in the cell population have the characteristic of Item A2 or A3.
(Item 21) The cell population of any one of Items 14-20, wherein at least 40% of cells in the cell population have the characteristic of Item 4.
(Item 22) The cell population of any one of Items 14-21, wherein at least 70% of cells in the cell population have the characteristic of Item 4.
(Item 23) The cell population of any one of Items 14-22, wherein at least 80% of cells in the cell population have the characteristic of Item 4.
(Item 24) The cell of any one of Items 1-13 or the cell population of any one of Items 14-23, which does not induce an allogeneic rejection upon infusion into an anterior chamber.
(Item 25) The cell of any one of Items 1-13 or the cell population of any one of Items 14-24, wherein the cell or the cell population does not substantially elicit an unintended biological response that is not associated with human corneal endothelial tissue reconstruction such as an increased amount of serum inflammatory cytokines after in vivo administration in a serum cytokine profile.
(Item 26) A product comprising the cell of any one of Items 1-13 or the cell population of any one of Items 14-25.
(Item 27) A method of preserving a cell or a cell population for maintaining and preserving a cell function property by exchanging a medium of the cell of any one of Items 1-13 or the cell population of any one of Items 14-25.
(Item 28) A method of delivering the cell of any one of Items 1-13 or the cell population of any one of Items 14-25, comprising implementing the method of preserving a cell or a cell population.

### (Medicaments and Pharmaceuticals)

(Item A1) A medicament comprising a human functional corneal endothelial cell capable of eliciting a human corneal functional property when infused into an anterior chamber of a human eye.

(Item A2) The medicament of Item A1, wherein the medicament is for treating a corneal endothelial dysfunction or disease.

(Item A3) The medicament of Item A2, wherein the corneal endothelial dysfunction or disease comprises at least one selected from the group consisting of corneal endothelial disorder Grade 3 and corneal endothelial disorder Grade 4

(bullous keratopathy) (e.g., Fuchs endothelial corneal dystrophy, PEX-BK (pseudoexfoliation bullous keratopathy; bullous keratopathy involving pseudoexfoliation syndrome), post-laser iridotomy bullous keratopathy, post-cataract surgery bullous keratopathy (including pseudophakic or aphakic bullous keratopathy), post-glaucoma surgery bullous keratopathy, and post-trauma bullous keratopathy, bullous keratopathy of unknown cause after multiple surgeries, post-corneal transplantation graft failure, congenital corneal endothelial dystrophy, and congenital anterior chamber angle hypoplasia syndrome. The grade system used herein is based upon the severity classification of corneal endothelial disorders, which is based on Japanese Journal of Ophthalmology 118: 81-83, 2014.

(Item A4) The medicament of any one of Items A1-A3, wherein the cell is administered into an anterior chamber.

(Item A5) The medicament of any one of Items A1-A4, wherein the cell is administered in conjunction with an additional agent.

(Item A6) The medicament of Item A5, wherein the additional agent comprises at least one agent selected from the group consisting of a steroid agent, antimicrobial, and NSAID.

(Item A7) The medicament of Item A5 or A6, wherein the additional agent comprises a ROCK inhibitor.

(Item A8) The medicament of any one of Item A5-A7, wherein the additional agent is contained in the medicament.

(Item A9) The medicament of any one of Items A1-A8, wherein the medicament comprises the cell at a density of 5 x 10⁴ cells/300 .micro.L to 2 × 10⁶ cells/300 .micro.L.

(Item A10) The medicament of any one of Items A1-A9, wherein the medicament further comprises a cell infusion vehicle.

(Item A11) The medicament of Item A10, wherein the cell infusion vehicle further comprises at least one of ROCK inhibitor, albumin, ascorbic acid, and lactic acid.

(Item A12) The medicament of Item A10 or A11, wherein the cell infusion vehicle further comprises albumin, ascorbic acid, and lactic acid.

(Item A13) The medicament of any one of Items A10-A12, wherein the cell infusion vehicle further comprises all of ROCK inhibitor, albumin, ascorbic acid and lactic acid.

(Item A14) The medicament of any one of Items A10-A13, wherein the cell infusion vehicle comprises OPEGUARD-MA(R).

(Item A15) A medicament, wherein a human functional corneal endothelial cell capable of eliciting a human corneal endothelial functional property when infused into an anterior chamber of a human eye, has one or more of the following characteristics (A15-2) to (A15-13):
(A15-2) the cell expresses a cell surface antigens comprising CD166 positive and CD133 negative phenotypes;
(A15-3) the cell surface antigen comprises CD166 positive, CD133 negative, and CD44 negative to intermediately positive phenotypes;
(A15-4) the cell surface antigen comprises CD166 positive, CD133 negative, and CD44 negative to CD44 weakly positive phenotypes;
(A15-5) the cell surface antigen comprises CD166 positive, CD133 negative, and CD200 negative phenotypes;
(A15-6) the cell surface antigen further comprises at least one expression property selected from the group consisting of CD90 negative to weakly positive, CD105 negative to weakly positive, CD24 negative, CD26 negative, LGR5 negative, SSEA3 negative, MHC1 weakly positive, MHC2 negative, PDL1 positive, ZO1 positive, Na⁺/K⁺ ATPase positive and a cell surface antigen described in the following table:

**[Table 1-1B]**

| **Cell surface marker** | **Functional cell** |
|---|---|
| CD59 | **Strongly positive** |
| CD147 | **Strongly positive** |
| CD81 | **Strongly positive** |
| CD73 | **Strongly positive** |
| CD49c | **Strongly positive** |
| CD166 | **Strongly positive** |
| CD56 | **Intermediately positive** |
| CD54 | **Intermediately positive** |
| B2-uGlob | **Intermediately positive** |
| CD47 | **Intermediately positive** |
| CD46 | **Intermediately positive** |
| CD141 | **Intermediately positive** |
| CD151 | **Intermediately positive** |
| CD98 | **Weakly positive** |
| CD165 | **Weakly positive** |
| CD340 (Her2) | **Weakly positive** |
| CD58 | **Weakly** positive |
| CD201 | **Weakly positive** |
| CD140b | **Weakly positive** |
| EGF-r | **Weakly positive** |
| CD63 | **Weakly positive** |
| CD9 | **Negative** |
| CD49b | **Negative** |
| CD227 | **Negative** |
| CD90 | **Negative** |
| CD44 | **Negative** |

;
(A15-7) the cell has at least one property selected from the group consisting of PDGF-BB high production, IL-8 low production, MCP-1 low production, TNF-alpha high production, IFNgamma high production, and IL-1R antagonist high production; (A15-8) the cell has at least one miRNA with a cell property of mature differentiated functional corneal endothelial cell a5, wherein a property of a cell surface antigen of the a5 is CD44 negative to weakly positive and CD24 negative CD26 negative; (A15-9) the cell of (A15-8), wherein the property of said miRNA comprises at least one miRNA selected from the group consisting of:
(A) functional mature differentiated corneal endothelial cell (a5): intermediately differentiated corneal endothelial cell (a1): corneal endothelial nonfunctional cell (a2) exhibits high expression: high expression: low expression:
   (intracellular) miR23a-3p, miR23b-3p, miR23c, miR27a-3p, miR27b-3p, miR181a-5p, miR181b-5p, miR181c-5p, miR181d-5p
   (cell-secreted) miR24-3p, miR1273e;
(B) a5:a1:a2 exhibits high expression: intermediate expression: low expression:
   (intracellular) miR30a-3p, miR30a-5p, miR30b-5p, miR30c-5p, miR30e-3p, miR30e-5p, miR130a-3p, miR130b-3p, miR378a-3p, miR378c, miR378d, miR378e, miR378f, miR378h, miR378i, miR184, miR148a-3p
   (cell-secreted) miR184;
(C) a5:a1:a2 exhibits high expression: low expression: low expression:
   (intracellular) miR34a-5p, miR34b-5p
   (cell-secreted) miR4419b, miR371b-5p, miR135a-3p, miR3131, miR296-3p, miR920, miR6501-3p;
(D) a5:a1:a2 exhibits low expression: low expression: intermediate to high expression:
   (intracellular) miR29a-3p, miR29b-3p, miR199a-3p, miR199a-5p, miR199b-5p, miR143-3p
   (cell-secreted) miR1915-3p, miR3130-3p, miR92a-2-5p, miR1260a;
(E) a5:a1:a2 exhibits low expression: intermediate expression: high expression:
   (intracellular) miR31-3p, miR31-5p, miR193a-3p, miR193b-3p, miR138-5p
(F) a5:a1:a2 exhibits high expression: low expression: high expression:
   (cell-secreted) miR92b-5p; and
(G) a5:a1:a2 exhibits low expression: high expression: low expression:
   (cell-secreted) miR1246, miR4732-5p, miR23b-3p, miR23a-3p, miR1285-3p, miR5096;
   wherein an expression level is relative intensity among 3 types of cells, expression of a cell surface antigen of the a1 being CD44 intermediately positive CD24 negative CD26 negative, and expression of a cell surface antigen of the a2 being CD44 strongly positive CD24 negative CD26 positive;
(A15-10) the miRNA marker comprises at least one selected from (B) or (C);
(A15-11) a mean cell area of the cell is 250 .micro.m² or less;
(A15-12) the cell has a cell function property homologous to a5 in at least one cell indicator selected from the group consisting of: a cell surface marker; a proteinaceous product or a related biological material of the product; a SASP related protein; miRNA; an exosome; a cellular metabolite comprising an amino acid and a related biological material of the metabolite; cell size; cell density and the presence of an autoantibody reactive cell;
(A15-13) the cell does not have a karyotype abnormality;
or is a cell population, wherein the cell population is
(A15-14) a cell population comprising the cell of any one of (A15-2) to A15-13);
(A15-15) the cell population of A15-14, wherein a mean cell density as of saturated cell culture (culture confluence) of the cell population is at least 1500 cells/mm² or greater;
(A15-16) the cell population of (A15-14) to (A15-15), wherein a mean cell density as of saturated cell culture (culture confluence) of the cell population is at least 2000 cells/mm² or greater;
(A15-17) the cell population of (A15-14) to (A15-16), wherein a mean cell density of cells integrated into a human corneal endothelial surface after infusing the cell population is at least 1000 cells/mm² or greater;
(A15-18) the cell population of (A15-14) to (A15-17), wherein a mean cell density of cells integrated into a human corneal endothelial surface after infusing the cell population is at least 2000 cells/mm² or greater;
(A15-19) the cell population of (A15-14) to (A15-18), wherein at least 70% of cells in the cell population have the characteristic of (A15-2) or (A15-3);
(A15-20) the cell population of (A15-14) to (A15-19), wherein at least 90% of cells in the cell population have the characteristic of (A15-2) or (A15-3);
(A15-21) the cell population of (A15-14) to (A15-20), wherein at least 40% of cells in the cell population have the characteristic of (A15-4);
(A15-22) the cell population of (A15-14) to (A15-21), wherein at least 70% of cells in the cell population have the characteristic of (A15-4);
(A15-23) the cell population of any one of (A15-14) to (A15-22), wherein at least 80% of cells in the cell population have the characteristic of (15-4);
(A15-24) the cell of any one of (A15-2) to (A15-13) or the cell population of any one of Items (A15-14) to (A15-22), which does not induce an allogeneic rejection upon infusion into an anterior chamber;
(A15-25) the cell of any one of (A15-2 to (A15-13) or the cell population of any one of (A15-14) to (A15-24), wherein the cell or the cell population does not substantially elicit an unintended biological response that is not associated with human corneal endothelial tissue reconstruction such as an increased amount of serum inflammatory cytokines after in vivo administration in a serum cytokine profile.

### (Manufacturing method)

The present invention also provides the following.
(Item B1) A method of manufacturing a human functional corneal endothelial cell capable of eliciting a human corneal functional property when infused into an anterior chamber of a human eye, comprising a step of maturing and differentiating a corneal endothelial tissue-derived cell or a corneal endothelial progenitor cell directly or indirectly via a step of dedifferentiation
(Item B2) A method of manufacturing a human functional corneal endothelial cell capable of eliciting a human corneal functional property when infused into an anterior chamber of a human eye, comprising a step of culturing to mature and differentiate a corneal endothelial tissue-derived cell or a corneal endothelial progenitor cell by a step comprising actin depolymerization.
(Item B3) The method of manufacturing of Item B1 or B2, wherein the actin depolymerization is accomplished by one or a plurality of agents selected from the group consisting of a ROCK inhibitor, HDAC inhibitor, actin depolymerization inhibitor, PPARgamma inhibitor, MMP2 inhibitor, p53 activator, and miRNA.
(Item B4) The method of manufacturing of Item B3, wherein the ROCK inhibitor is Y-27632.
(Item B5) The method of manufacturing of Item B3, wherein the actin depolymerization inhibitor is selected from the group consisting of latrunculin A and swinholide A.
(Item B6) The method of manufacturing of any one of Items B1-B5, further comprising a step of culturing the corneal endothelial tissue-derived cell or corneal endothelial progenitor cell under steps where a cell enters into epithelial-mesenchymal transition-like transformation, proliferation, maturation and differentiation.
(Item B7) The method of manufacturing of Item B6, wherein the condition for growing, maturing, and differentiating comprises culturing in the absence of a transforming growth factor beta (TGF-beta) signaling inhibitor.
(Item B8) The method of any one of Items B1-B7, further comprising a step of culturing the corneal endothelial tissue-derived cell or corneal endothelial progenitor cell under a condition where cellular senescence is suppressed.
(Item B9) The method of manufacturing of Item B8, wherein the condition where cellular senescence is suppressed comprises culturing in the presence of a p38 MAP kinase inhibitor.
(Item B10) The method of manufacturing of Item B9, wherein the p38 MAP kinase inhibitor comprises SB203580.
(Item B11) The method of manufacturing of any one of Items B1-B10, wherein the corneal endothelial tissue-derived cell or corneal endothelial progenitor cell is collected from a living body or differentiated from a stem cell or a progenitor cell.
(Item B12) The method of manufacturing of any one of Items B1-B11, wherein the culturing is carried out at a seeding density of 100-1000 cells/mm².
(Item B13) The method of manufacturing of any one of Items B1-B12, comprising a step of further culturing for cell function maturation after a cell density of cultured cells has reached a saturation density.
(Item B14) The method of manufacturing of Item B13, wherein after the cultured cell reaches saturated cell density and then the differentiation and maturation of cultured cells become complete with sufficient formation of tight junctions, culturing is further maintained for 1 week or more by only exchanging a medium to preserve the cultured cells.
(Item B15) The method of manufacturing of any one of Items B1-B14, further comprising a step of testing a cell function after the culturing by using at least one cell indicator for identifying the human functional corneal endothelial cell.
(Item B16) The method of manufacturing of Item B15, further comprising a step of selectively propagating in cultures a fraction determined to be the corneal endothelial functional effector cell after the testing.
(Item B17) The method of manufacturing of any one of Items B1-B16, further comprising a step of monitoring cell subpopulation composition during the culturing.
(Item B18) The method of manufacturing of Item B17, wherein the monitoring comprises tracking at least one Item selected from the group consisting of mitochondrial function, oxygen consumption and pH of a culture solution, amino acid composition, proteinaceous product, soluble miRNA, cell density with a noninvasive engineering approach, cell size, and cell homogeneity.
(Item B19) The method of manufacturing of any one of Items B1-B18, wherein the step of culturing comprises a step of subculturing.
(Item B20) The method of manufacturing of any one of Items B1-B19, wherein the step of culturing comprises a step of adding one or a plurality of agents selected from the group consisting of a ROCK inhibitor, HDAC inhibitor, actin depolymerization inhibitor, PPARgamma inhibitor and MMP2 inhibitor, p53 activator, and miRNA at the time of subculture.
(Item B21) The method of any one of Items B1-B20, comprising a step of culturing in the presence of a serum-free medium.
(Item B22) The method of manufacturing of any one of Items B1-B21, wherein the corneal endothelial tissue-derived cell or corneal endothelial progenitor cell is selected from the group consisting of pluripotent stem cell, mesenchymal stem cell, corneal endothelial progenitor cell collected from a corneal endothelium, cell collected from a corneal endothelium, and corneal endothelium progenitor cell and corneal endothelium-like cell made by a direct programming method.
(Item B23) A method of preserving a mature differentiated functional corneal endothelial cell comprising a step of continuously culturing the functional mature differentiated corneal endothelial cell of any one of Items B1-B22 after manufacture.

### (Quality control)

(Item C1) A method of quality control or process control of a cultured human functional corneal endothelial cell capable of eliciting a human corneal endothelial functional property when infused into an anterior chamber of a human eye, comprising the step of measuring at least one cell indicator selected from the group consisting of: a cell surface marker; a proteinaceous product and a related biological material of the product; a SASP related protein; miRNA; an exosome; a cellular metabolite comprising an amino acid and a related biological material of the metabolite; cell size; cell density and the presence of an autoantibody reactive cell.
(Item C2) The method of Item C1, wherein at least 3 of the cell indicators are used.
(Item C3) The method of Item C1 or C2, wherein the cell indicator comprises cell size, cell density or a combination thereof.
(Item C4) The method of any one of Items C1-C3, wherein the cell indicator comprises a combination of: at least one of cell surface marker, proteinaceous product and related biological material of the product; at least one of miRNA; and at least one of cellular metabolite and related biological material of the metabolite.
(Item C5) The method of any one of Items C1-C4, further comprising identifying a subpopulation of the cultured functional corneal endothelial cell by a corneal functional property.
(Item C6) The method of Item C5, wherein the corneal functional property is expression of a cell surface antigen comprising CD166 positive and CD133 negative on a cell surface.
(Item C7) The method of Item C5 or C6, wherein the cell surface antigen comprises CD166 positive, CD133 negative, and CD44 negative to intermediately positive.
(Item C8) The method of any one of Items C5-C7, wherein the cell surface antigen comprises CD166 positive, CD133 negative, and CD44 negative to CD44 weakly positive.
(Item C9) The method of any one of Items C5-C8, wherein the cell surface antigen comprises CD166 positive, CD133 negative, CD44 negative to CD44 weakly positive, and CD90 negative to weakly positive.
(Item C10) The method of any one of Items C5-C9, wherein the cell surface antigen comprises CD166 positive, CD133 negative, and CD200 negative.
(Item C11) The method of any one of Items C5-C10, wherein a plurality of indicators from each of proteinaceous product and related biological material of the product, secreted miRNA, and cellular metabolite comprising an amino acid and related biological material of the metabolite are selected to examine a variation in a profile of each indicator to determine homogeneity of cells having a cell indicator comprising CD166 positive, CD133 negative, CD44 negative to CD44 weakly positive and CD90 negative to weakly positive.
(Item C12) The method of any one of Items C1-C11, wherein the proteinaceous product and related biological material of the product is selected from the group consisting of:
   (A) COL4A1, COL4A2, COL8A1, COL8A2, CDH2, and TGF-beta2 whose expression increases in the human functional corneal endothelial cell capable of eliciting a human corneal functional property when infused into an anterior chamber of a human eye, and
   (B) MMP1, MMP2, TIMP1, BMP2, IL13RA2, TGF-beta1, CD44, COL3A1, IL6, IL8, HGF, THBS2, and IGFBP3 whose expression decreases in the human functional corneal endothelial cell capable of eliciting a human corneal functional property when infused into an anterior chamber of a human eye.
(Item C13) The method of any one of Items C1-C12, wherein the property of said miRNA comprises at least one miRNA selected from the group consisting of those the pattern of which are:
   (A) mature differentiated functional corneal endothelial cell (a5): mature differentiated corneal endothelial progenitor cell (a1): corneal endothelial nonfunctional cell (a2) exhibits high expression: high expression: low expression:
      (intracellular) miR23a-3p, miR23b-3p, miR23c, miR27a-3p, miR27b-3p, miR181a-5p, miR181b-5p, miR181c-5p, miR181d-5p
      (cell-secreted) miR24-3p, miR1273e;
   (B) a5:a1:a2 exhibits high expression: intermediate expression: low expression:
      (intracellular) miR30a-3p, miR30a-5p, miR30b-5p, miR30c-5p, miR30e-3p, miR30e-5p, miR130a-3p, miR130b-3p, miR378a-3p, miR378c, miR378d, miR378e, miR378f, miR378h, miR378i, miR184, miR148a-3p
      (cell-secreted) miR184;
   (C) a5:a1:a2 exhibits high expression: low expression: low expression:
      (intracellular) miR34a-5p, miR34b-5p
      (cell-secreted) miR4419b, miR371b-5p, miR135a-3p, miR3131, miR296-3p, miR920, miR6501-3p;
   (D) a5:a1:a2 exhibits low expression: low expression: intermediate to high expression:
      (intracellular) miR29a-3p, miR29b-3p, miR199a-3p, miR199a-5p, miR199b-5p, miR143-3p
      (cell-secreted) miR1915-3p, miR3130-3p, miR92a-2-5p, miR1260a;
   (E) a5:a1:a2 exhibits low expression: intermediate expression: high expression:
      (intracellular) miR31-3p, miR31-5p, miR193a-3p, miR193b-3p, miR138-5p
   (F) a5:a1:a2 exhibits high expression: low expression: high expression:
      (cell-secreted) miR92b-5p; and
   (G) a5:a1:a2 exhibits low expression: high expression: low expression:
      (cell-secreted) miR1246, miR4732-5p, miR23b-3p, miR23a-3p, miR1285-3p, miR5096;
      wherein an expression level is relative intensity among 3 types of cells, the expression intensity being defined to be weaker in the order of high expression > intermediate expression > low expression, and wherein a property of a cell surface antigen of the a5 is CD44 negative to weakly positive and CD24 negative CD26 negative, expression of a cell surface antigen of the a1 is CD44 intermediately positive CD24 negative CD26 negative, and expression of a cell surface antigen of the a2 is CD44 strongly positive CD24 negative CD26 positive.
(Item C14) The method of any one of Items C1-C13, wherein the exosome comprises at least one cell indicator selected from the group consisting of:
   (A) CD63, CD9, CD81, and HSP70 whose expression decreases in the human functional corneal endothelial cell capable of eliciting a human corneal functional property when infused into an anterior chamber of a human eye.
(Item C15) The method of any one of Items C1-C14, wherein the cellular metabolite and related biological material of the metabolite comprises at least one selected from the group consisting of succinic acid (succinate), Pro, Gly, glycerol3-phosphate, Glu, lactic acid (lactate), argininosuccinic acid (arginosuccinate), xanthine, N-carbamoyl aspartic acid (N-carbamoyl aspartate), isocitric acid (isocitrate), cis-aconitic acid (cis-aconitate), citric acid (citrate), Ala, 3-phosphoglyceric acid (3-phosphoglycerate), hydroxyproline, malic acid (malate), uric acid (urate), betaine, folic acid (folate), Gln, 2-oxoisovaleric acid (2-oxoisovalerate), pyruvic acid (pyruvate), Ser, hypoxanthine, Asn, Trp, Lys, choline, Tyr, urea, Phe, Met, carnosine, Asp, ornithine, Arg, creatine, 2-hydroxy glutaminic acid (2-hydroxy glutamate), beta-Ala, citrulline, Thr, Ile, Leu, Val, creatinine, His, N,N-dimethyl glycine, or a combination or relative ratio thereof.
(Item C16) The method of Item C15, wherein the cellular metabolite and related biological material of the metabolite comprises increase in serine, alanine, proline, glutamine or citric acid (citrate)/lactic acid (lactate) ratio in culture supernatant.
(Item C17) The method of any one of Items C1-C16, wherein the cell size is a mean cell area of 250 .micro.m² or less.
(Item C18) The method of any one of Items C1-C17, wherein a mean cell density as of saturated cell culture of the cell is at least 2000 cells/mm² or greater.
(Item C19) A method of detecting a corneal endothelial nonfunctional cell coexisting with a cultured human corneal endothelial cell comprising a step of measuring at least one cell indicator selected from the group consisting of cell size, cell density and the presence of an autoantibody reactive cell.
(Item C20) A quality evaluating agent, process controlling agent, or corneal endothelial nonfunctional cell detecting agent for a functional mature differentiated corneal endothelial cell, comprising a reagent or means for measuring a cell indicator of any one of Items 01-019.
(Item C21) The quality evaluating agent, process controlling agent, or detecting agent of Item C20, wherein the means for measuring is labeled.
(Item C22) A method of selectively propagating in cultures a human functional corneal endothelial cell, comprising the steps of:
   A) providing a sample that possibly comprises a human functional corneal endothelial cell capable of eliciting a human corneal functional property when infused into an anterior chamber of a human eye;
   B) determining whether the sample comprises the human functional corneal endothelial cell capable of eliciting a human corneal functional property when infused into an anterior chamber of a human eye by using the quality evaluating agent, process controlling agent, or corneal endothelial nonfunctional cell detecting agent of Item C20 or C21, wherein it is determined that the sample comprises the human functional corneal endothelial cell capable of eliciting a human corneal functional property when infused into an anterior chamber of a human eye when a result of evaluation with the quality evaluating agent, process controlling agent, or corneal endothelial nonfunctional cell detecting agent indicates that the cell is a human functional corneal endothelial cell capable of eliciting a human corneal functional property when infused into an anterior chamber of a human eye; and
   C) selectively propagating in cultures a cell determined to be a human functional corneal endothelial cell capable of eliciting a human corneal functional property when infused into an anterior chamber of a human eye.
(Item C23) A method of assaying quality of a human functional corneal endothelial cell, comprising the steps of:
   A) obtaining information related to a cell indicator of the functional corneal endothelial cell of cells provided as being human functional corneal endothelial cells capable of eliciting a human corneal functional property when infused into an anterior chamber of a human eye by using the quality evaluating agent, process controlling agent, or corneal endothelial nonfunctional cell detecting agent of Item C20 or C21; and
   B) determining that the provided cells are human functional corneal endothelial cells capable of eliciting a human corneal functional property when infused into an anterior chamber of a human eye based on the information.
(Item C24) A method of controlling quality in preparation of a human functional corneal endothelial cell capable of eliciting a human corneal functional property when infused into an anterior chamber of a human eye, comprising the steps of:
   A) obtaining information related to a cell indicator of a mature differentiated functional corneal endothelial cell of cells obtained in the preparation by using the quality evaluating agent, process controlling agent, or corneal endothelial nonfunctional cell detecting agent of Item C20 or C21; and
   B) determining that the preparation is suitable for preparation of a human functional corneal endothelial cell capable of eliciting a human corneal endothelial functional property when infused into an anterior chamber of a human eye based on the information.
(Item C25) A method of assaying purity of a human functional corneal endothelial cell capable of eliciting a human corneal functional property when infused into an anterior chamber of a human eye, comprising the steps of:
   A) providing a sample possibly comprising the human functional corneal endothelial cell capable of eliciting a human corneal functional property when infused into an anterior chamber of a human eye;
   B) obtaining information related to a cell indicator of a functional corneal endothelial cell of the cells by using the quality evaluating agent, process controlling agent, or corneal endothelial nonfunctional cell detecting agent of Item C20 or C21; and
   C) calculating the purity of human functional corneal endothelial cell capable of eliciting a human corneal functional property when infused into an anterior chamber of a human eye in the sample based on the information.
(Item C26) A method of assaying quality of a medium for a human functional corneal endothelial cell, comprising the steps of:
   A) culturing cells provided as being a functional mature differentiated corneal endothelial cell capable of eliciting a human corneal functional property when infused into an anterior chamber of a human eye in the medium to obtain information related to a cell indicator of the functional corneal endothelial cell of the cells by using the quality evaluating agent, process controlling agent, or corneal endothelial nonfunctional cell detecting agent of Item C20 or C21; and
   B) determining that the medium is suitable for manufacture of a human functional corneal endothelial cell capable of eliciting a human corneal functional property when infused into an anterior chamber of a human eye based on the information.
(Item C27) A method of assaying quality of a cell infusion vehicle for a human functional corneal endothelial cell, comprising the steps of:
   A) culturing cells provided as being a human functional corneal endothelial cell capable of eliciting a human corneal functional property when infused into an anterior chamber of a human eye in the cell infusion vehicle to obtain information related to a cell indicator of the functional corneal endothelial cell of the cells by using the quality evaluating agent, process controlling agent, or corneal endothelial nonfunctional cell detecting agent of Item C20 or C21; and
   B) determining that the cell infusion vehicle is suitable for cell infusion therapy based on the information.
(Item C28) A method of quality control or process control of a cultured human functional corneal endothelial cell capable of eliciting a human corneal functional property when infused into an anterior chamber of a human eye or a method of detecting a corneal endothelial nonfunctional cell coexisting with a cultured human corneal endothelial cell, comprising the step of examining one or a plurality of the following:
   (1) purity test by culture supernatant ELISA
      TIMP-1: 500 ng/mL or less
      IL-8: 500 pg/mL or less
      PDGF-BB: 30 pg/mL or greater
      MCP-1: 3000 pg/mL or less
   (2) purity test by cell FACS
      CD166 = 95% or greater
      CD133 = 5% or less
      CD105 low positive = 95% or greater
      CD44 low positive = 70% or greater
      CD44 high positive = 15% or less
      CD24 = 10% or less
      CD26 positive = 5% or less
      CD200 = 5% or less
   (3) barrier function (ZO-1) positive
   (4) pumping function (Na+/K+ ATPase) positive
   (5) cell survival
      70% or greater with trypan blue stain
   (6) cell form
      transformed cells cannot be found by visual inspection
   (7) Claudin10 positive
   (8) effector cell (E-ratio) > 50%
   (9) non-intended cell
   non-intended cell A (CD44 strongly positive cell) < 15%, non-intended cell B (CD26 positive cell) <5 %, non-intended cell C (CD24 positive cell) < 10% (10) karyotype abnormality negative.
(Item C29) The method of Item C28, comprising carrying out the examining three weeks to immediately prior to cell infusion therapy or during preserved culture only exchanging a medium.
(Item C30) The method of Item C28 or C29, comprising carrying out the examining about 7 day prior to or immediately prior to cell infusion therapy.
(Item C31) The method of any one of Items C22-C27, characterized by one or a plurality of the characteristics of Items C28-C30.
(Item C32) A method of quality control or process control of a human functional corneal endothelial cell capable of eliciting a human corneal functional property when infused into an anterior chamber of a human eye, comprising the step of determining one or a plurality of the following characteristics with respect to a target cell: (1) retention of endothelial pumping/barrier functions; (2) adhesion/attachment to a specific laminin; (3) secreted cytokine profile; (4) produced metabolite profile; (5) saturated cell density upon in vitro culture; (6) spatial size and distribution of cells obtained in culturing; and (8) cell retention in case of cell infusion after freeze damage by cryo treatment by liquid nitrogen on mouse cornea.
(Item C33) The method of Item C32, wherein determination of the retention of endothelial pumping/barrier functions is determined by using a pumping function measuring method or a barrier function measuring method commonly used for corneal endothelia.
(Item C34) The method of Item C32 or C33, wherein determination of the adhesion/attachment to a specific laminin is determined by adhesiveness to laminin 511 (composite of alpha5 chain, beta1 chain, and gamma1 chain), laminin 521 (composite of alpha5 chain, beta2 chain, and gamma1 chain), or a functional fragment thereof and/or increase in integrin expression with respect thereto as an indicator.
(Item C35)
   The method of any one of Items C32-C34, wherein determination of the secreted cytokine profile comprises measuring a production level of a cytokine profile of serum or aqueous humour.
(Item C36) The method of any one of Items C32-C35, wherein determination of the produced metabolite profile comprises measuring a production level of metabolite of the cell.
(Item C37) The method of any one of Items C32-C36, wherein determination of the produced micro RNA (miRNA) profile comprises obtaining total RNA to obtain a micro RNA expression profile thereof.
(Item C38) The method of any one of Items C32-C37, wherein determination of saturated cell density upon in vitro culture comprises counting cells in an image of the cells obtained by using an image capturing system.
(Item C39) The method of any one of Items C32-C38, wherein determination of the spatial size and distribution of cells obtained in culturing comprises counting cells in an image of the cells obtained by using an image capturing system.
(Item C40) The method of any one of Items C32-C39, wherein determination of the cell retention in case of cell infusion after freeze damage by cryo treatment by liquid nitrogen on mouse cornea comprises: infusing cells to be determined into an anterior chamber of a human eye of a model made by pre-treatment of a central region of a mouse cornea by freeze damage to remove endothelial cells; clinically observing a characteristic of the cornea; assessing the thickness of the cornea with a pachymeter; histopathologically testing HCEC adhesion with human nuclear staining; and examining whether the cell has a function.

### (Alternative Embodiments)

Thus, the present invention provides the following.

### (Cells)

### (Item X1)

A human functional corneal endothelial cell capable of eliciting a human corneal endothelial functional property when transplanted into an anterior chamber of a human eye.

### (Item X2)

The cell of Item X1, wherein the cell expresses cell surface antigens comprising CD166 positive and CD133 negative phenotypes.

### (Item X3)

The cell of Item X2, wherein the cell surface antigens comprise CD166 positive, CD133 negative, and CD44 negative to intermediate positive phenotypes.

### (Item X4)

The cell of Item X2 or X3, wherein the cell surface antigens comprise CD166 positive, CD133 negative, and CD44 negative to CD44 weak positive phenotypes.

### (Item X4A)

The cell of Item X1, wherein the cell expresses a cell surface antigen comprising CD44 negative to CD44 weak positive phenotype.

### (Item X4B)

The cell of Item X1, wherein the cell expresses a cell surface antigen comprising CD44 negative phenotype.

### (Item X5)

The cell of any one of Items X2-X4, X4A, and X4B, wherein the cell surface antigens comprise CD166 positive, CD133 negative, and CD200 negative phenotypes.

### (Item X6)

The cell of any one of Items X2-4, X4A, X4B and X5, wherein the cell surface antigens comprise CD166 positive, CD133 negative, and CD44 negative to intermediate positive and CD90 negative phenotypes.

### (Item X7)

The cell of any one of Items X2-X4, X4A, X4B and X5-X6, further comprising at least one surface antigen expression property selected from the group consisting of CD90 negative to weak positive, CD105 negative to weak positive, CD24 negative, CD26 negative, LGR5 negative, SSEA3 negative, MHC1 weak positive, MHC2 negative, PDL1 positive, ZO-1 positive, and Na⁺/K⁺ATPase positive.

### (Item X8)

The cell of any one of Items X1-X4, X4A, X4B and X5-X7, wherein the cell has at least one property selected from the group consisting of PDGF-BB high production, IL-8 low production, MCP-1 low production, TNF-alpha high production, IFNgamma high production, and IL-1R antagonist high production.

### (Item X9)

The cell of any one of Items X1-X4, X4A, X4B and X5-X8, wherein the cell has at least one miRNA with a cell property of mature differentiated corneal endothelial functional cell a5, wherein a property of a cell surface antigen of the a5 is CD44 negative to weak positive and CD24 negative CD26 negative.

### (Item X10)

The cell of Item X9, wherein the property of said miRNA comprises at least one miRNA selected from the group consisting of:
(A) miR23a-3p, miR23b-3p, miR23c, miR27a-3p, miR27b-3p, miR181a-5p, miR181b-5p, miR181c-5p, miR181d-5p, andmiR24-3p, miR1273e;
(B) miR30a-3p, miR30a-5p, miR30b-5p, miR30c-5p, miR30e-3p, miR30e-5p, miR130a-3p, miR130b-3p, miR378a-3p, miR378c, miR378d, miR378e, miR378f, miR378h, miR378i, miR184, miR148a-3p, and miR184;
(C) miR34a-5p, miR34b-5p, miR4419b, miR371b-5p, miR135a-3p, miR3131, miR296-3p, miR920, and miR6501-3p;
(D) miR29a-3p, miR29b-3p, miR199a-3p, miR199a-5p, miR199b-5p, miR143-3p, miR1915-3p, miR3130-3p, and miR92a-2-5p, miR1260a;
(E) miR31-3p, miR31-5p, miR193a-3p, miR193b-3p, and miR138-5p
(F) miR92b-5p; and
(G) miR1246, miR4732-5p, miR23b-3p, miR23a-3p, miR1285-3p, and miR5096. (Item X11)

The cell of Item X10, wherein the miRNA marker comprises at least one selected from (B) or (C).

### (Item X12)

The cell of any one of Items X1-X4, X4A, X4B and X5-X11, wherein a mean cell area of the cell is 250 .micro.m² or less.

### (Item X13)

The cell of any one of Items X1-X4, X4A, X4B and X5-X12 having a cell function property homologous to a5 in at least one cell indicator selected from the group consisting of: a cell surface marker; a proteinaceous product and a related biological material of the product; a SASP related protein; intracellular and secreted miRNA; an exosome; a cellular metabolite comprising an amino acid and a related biological material of the metabolite; cell size; cell density and the presence of an autoantibody reactive cell.

### (Item X14)

The cell of any one of Items X1-X4, X4A, X4B and X5-X13, wherein the cell does not have a karyotype abnormality.

### (Item X15)

A cell population comprising the cells of any one of Items X1-X4, X4A, X4B and X5-X14.

### (Item X16)

The cell population of Item X15, wherein a mean cell density as of saturated cell culture (culture confluence) of the cell population is at least 1500 cells/mm² or greater.

### (Item X17)

The cell population of Item X15 or X16, wherein a mean cell density as of saturated cell culture (culture confluence) of the cell population is at least 2000 cells/mm² or greater.

### (Item X18)

The cell population of any one of Items X15-X17, wherein a mean cell density of cells integrated into a human corneal endothelial surface after transplanting the cell population is at least 1000 cells/mm² or greater.

### (Item X19)

The cell population of any one of Items X15-X18, wherein a mean cell density of cells integrated into a human corneal endothelial surface after transplanting the cell population is at least 2000 cells/mm² or greater.

### (Item X20)

The cell population of any one of Items X15-X19, wherein at least 70% of cells in the cell population have the characteristic of any one of Items X2-4, X4A, X4B and X5-X6.

### (Item X21)

The cell population of any one of Items X15-X20, wherein at least 90% of cells in the cell population have the characteristic of any one of Items X2-X4, X4A, X4B and X5-X6.

### (Item X22)

The cell population of any one of Items X15-X21, wherein at least 40% of cells in the cell population have the characteristic of Item X4.

### (Item X23)

The cell population of any one of Items X15-X22, wherein at least 70% of cells in the cell population have the characteristic of Item X4.

### (Item X24)

The cell population of any one of Items X15-X23, wherein at least 80% of cells in the cell population have the characteristic of Item X4.

### (Item X25)

The cell of any one of Items X1-X4, X4A, X4B and X5-X14 or the cell population of any one of Items X 15-24, which does not induce an allogeneic rejection upon transplantation into an anterior chamber.

### (Item X26)

The cell of any one of Items X1-X4, X4A, X4B and X5-14 and X25 or the cell population of any one of Items X15-X25, wherein the cell or the cell population does not substantially elicit an unintended biological response that is not associated with human corneal endothelial tissue reconstruction such as an increased amount of serum inflammatory cytokines after in vivo administration in a serum cytokine profile.

### (Item X27)

A product comprising the cell of any one of Items X1-X4, X4A, X4B and X5-X14 and X25-X26 or the cell population of any one of Items X15-X26.

### (Item X28)

A method of preserving a cell or a cell population for maintaining and preserving a cell function property by exchanging a medium of the cell of any one of Items X1-X4, X4A, X4B and X5-X14 and X25-X26 or the cell population of any one of Items X15-X26.

### (Item X29)

A method of delivering the cell of any one of Items X1-X4, X4A, X4B and X5-X14 or the cell population of any one of Items X15-X26, comprising implementing the method of preserving a cell or a cell population.

### (Medicaments and pharmaceuticals)

### (Item XA1)

A medicament comprising a functional corneal endothelial cell capable of eliciting a human corneal functional property when transplanted into an anterior chamber of a human an eye.

### (Item XA2)

The medicament of Item XA1, wherein the medicament is for treating a corneal endothelial dysfunction or disease.

### (Item XA3)

The medicament of Item XA2, wherein the corneal endothelial dysfunction or disease comprises at least one selected from the group consisting of corneal endothelial disorder Grade 3 and corneal endothelial disorder Grade 4 (bullous keratopathy) (e.g., Fuchs endothelial corneal dystrophy, PEX-BK (pseudoexfoliation bullous keratopathy; bullous keratopathy involving pseudoexfoliation syndrome), post-laser iridotomy bullous keratopathy, post-cataract surgery bullous keratopathy (including pseudophakic or aphakic bullous keratopathy), post-glaucoma surgery bullous keratopathy, and post-trauma bullous keratopathy, bullous keratopathy of unknown cause after multiple surgeries, post-corneal transplantation graft failure, congenital corneal endothelial dystrophy, and congenital anterior chamber angle hypoplasia syndrome. The grade system used herein is based upon the severity classification of corneal endothelial disorders, which is based on Japanese Journal of Ophthalmology 118: 81-83, 2014.

### (Item XA4)

The medicament of any one of Items XA1-XA3, wherein the cells are administered into an anterior chamber.

### (Item XA5)

The medicament of any one of Items XA1-XA4, wherein the cell is administered in conjunction with an additional agent.

### (Item XA6)

The medicament of Item XA5, wherein the additional agent comprises at least one agent selected from the group consisting of a steroid agent, antimicrobial, and NSAID.

### (Item XA7)

The medicament of Item XA5 or XA6, wherein the additional agent comprises a ROCK inhibitor.

### (Item XA8)

The medicament of any one of Item XA5-XA7, wherein the additional agent is contained in the medicament.

### (Item XA9)

The medicament of any one of Items XA1-XA8, wherein the medicament comprises the cell at a density of 5 x 10⁴ cells/300 .micro.L to 2 × 10⁶ cells/300 .micro.L.

### (Item XA10)

The medicament of any one of Items XA1-XA9, wherein the medicament further comprises a cell transfer solution.

### (Item XA11)

The medicament of Item XA10, wherein the cell infusion vehicle further comprises at least one of ROCK inhibitor, albumin, ascorbic acid, and lactic acid.

### (Item XA12)

The medicament of Item XA10 or XA11, wherein the cell infusion vehicle further comprises albumin, ascorbic acid, and lactic acid.

### (Item XA13)

The medicament of any one of Items XA10-XA12, wherein the cell infusion vehicle further comprises all of ROCK inhibitor, albumin, ascorbic acid and lactic acid.

### (Item XA14)

The medicament of any one of Items XA10-XA13, wherein the cell infusion vehicle comprises OPEGUARD-MA(R).

### (Item XA15)

The medicament of any one of Items XA1-XA14, wherein said human functional corneal endothelial cell is the cell according to any one of Items X1-X4, X4A, X4B and X5-X14 and X25-X26 or the cell population according to any one of Items X15-X26.

### (Manufacturing Process)

### (Item XB1)

A method of manufacturing a human functional corneal endothelial cell capable of eliciting a human corneal endothelial functional property when transplanted into an anterior chamber of a human eye, comprising a step of proliferating, maturating and differentiating a human corneal endothelial tissue-derived cell or a corneal endothelial progenitor cell directly or indirectly via a step of dedifferentiation.

### (Item XB2)

A method of manufacturing human functional corneal endothelial cells capable of eliciting a human corneal endothelial functional property when transplanted into an anterior chamber of human eyes, comprising a step of culturing to mature and differentiate a corneal endothelial tissue-derived cell or a corneal endothelial progenitor cell by a step comprising actin depolymerization.

### (Item XB3)

The method of manufacturing of Item XB1 or XB2, wherein the actin depolymerization is accomplished by one or a plurality of agents selected from the group consisting of a ROCK inhibitor, HDAC inhibitor, actin depolymerization inhibitor, PPARgamma inhibitor, MMP2 inhibitor, p53 activator, and miRNA.

### (Item XB4)

The method of manufacturing of Item XB3, wherein the ROCK inhibitor is Y-27632.

### (Item XB5)

The method of manufacturing of Item XB3 or XB4, wherein the actin depolymerization inhibitor is selected from the group consisting of latrunculin A and swinholide A.

### (Item XB6)

The method of manufacturing of any one of Items XB1-XB5, further comprising a step of culturing the corneal endothelial tissue-derived cell or corneal endothelial progenitor cell under steps where a cell enter into epithelial-mesenchymal transition-like transformation, proliferation, maturation and differentiation.

### (Item XB7)

The method of manufacturing of Item XB6, wherein the condition for growing, maturing, and differentiating comprises culturing in the absence of a transforming growth factor beta (TGF-beta) signaling inhibitor.

### (Item XB8)

The method of any one of Items XB1-XB7, further comprising a step of culturing the corneal endothelial tissue-derived cell or corneal endothelial progenitor cell under a condition where cellular senescence is suppressed.

### (Item XB9)

The method of manufacturing of Item XB8, wherein the condition where cellular senescence is suppressed comprises culturing in the presence of a p38 MAP kinase inhibitor.

### (Item XB10)

The method of manufacturing of Item XB9, wherein the p38 MAP kinase inhibitor comprises SB203580.

### (Item XB11)

The method of manufacturing of any one of Items XB1-XB10, wherein the corneal endothelial tissue-derived cell or corneal endothelial progenitor cell is collected from a living body or differentiated from a stem cell or a precursor cell.

### (Item XB12)

The method of manufacturing of any one of Items XB1-XB11, wherein the culturing is carried out at a seeding density of 100-1000 cells/mm².

### (Item XB13)

The method of manufacturing of any one of Items XB1-XB12, comprising a step of further culturing for cell function maturation after a cell density of cultured cells has reached a saturation density.

### (Item XB14)

The method of manufacturing of Item XB13, wherein after the cultured cell reaches saturated cell density and then the differentiation and maturation of a cultured cell becomes complete with sufficient formation of tight junctions, culturing is further maintained for 1 week or more by only exchanging a medium to preserve the cultured cells.

### (Item XB15)

The method of manufacturing of any one of Items XB1-XB14, further comprising a step of testing a cell function after the culturing by using at least one cell indicator for identifying the human functional corneal endothelial cell.

### (Item XB16)

The method of manufacturing of Item XB15, further comprising a step of sorting out a fraction determined to be the human functional corneal endothelial cell after the testing.

### (Item XB17)

The method of manufacturing of any one of Items XB1-XB16, further comprising a step of monitoring cell subpopulation composition during the culturing.

### (Item XB18)

The method of manufacturing of Item XB17, wherein the monitoring comprises tracking at least one Item selected form the group consisting of mitochondrial function, oxygen consumption and pH of a culture solution, amino acid composition, proteinaceous product, soluble miRNA, cell density with a noninvasive engineering approach, cell size, and cell homogeneity.

### (Item XB19)

The method of manufacturing of any one of Items XB1-XB18, wherein the step of culturing comprises a step of subculturing.

### (Item XB20)

The method of manufacturing of any one of Items XB1-XB19, wherein the step of culturing comprises a step of adding one or a plurality of agents selected from the group consisting of a ROCK inhibitor, HDAC inhibitor, actin depolymerization inhibitor, PPARgamma inhibitor and MMP2 inhibitor, p53 activator, and miRNA at the time of subculture.

### (Item XB21)

The method of any one of Items XB1-XB20, comprising a step of culturing in the presence of a serum-free medium.

### (Item XB22)

The method of manufacturing of any one of Items XB1-XB21, wherein the corneal endothelial tissue-derived cell or corneal endothelial progenitor cell is selected from the group consisting of pluripotent stem cell, mesenchymal stem cell, corneal endothelial progenitor cell collected from a corneal endothelium, cell collected form a corneal endothelium, and corneal endothelium precursor cell and corneal endothelium-like cell made by a direct programming method.

### (Item XB23)

A method of preserving a mature differentiated human functional corneal endothelial cell comprising a step of continuously culturing the mature differentiated human functional corneal endothelial cell of any one of Items XB1-XB22 after manufacture.

### (Item XB24)

The method of any one of Items X B1-B23, wherein said human functional corneal endothelial cell is the cell according to any one of Items X1-X4, X4A, X4B and X5-X14 and X25-X26 or the cell population according to any one of Items X15-X26.

### (Quality control)

### (Item XC1)

A method of quality control or process control of a cultured human functional corneal endothelial cell capable of eliciting a human corneal endothelial functional property when transplanted into an anterior chamber of a human eye, comprising the step of measuring at least one cell function indicator selected from the group consisting of: a cell surface marker; a proteinaceous product and a related biological material of the product; a SASP related protein; intracellular and secreted miRNA; an exosome; a cellular metabolite comprising an amino acid and a related biological material of the metabolite; cell size; cell density and the presence of an autoantibody reactive cell.

### (Item XC2)

The method of Item XC1, wherein at least 3 of the cell indicators are used.

### (Item XC3)

The method of Item XC1 or XC2, wherein the cell indicator comprises cell size, cell density or a combination thereof.

### (Item XC4)

The method of any one of Items XC1-XC3, wherein the cell indicator comprises a combination of: at least one of cell surface marker, proteinaceous product and related biological material of the product; at least one of miRNA; and at least one of cellular metabolite and related biological material of the metabolite.

### (Item XC5)

The method of any one of Items XC1-XC4, further comprising identifying a subpopulation of the human functional cultured corneal endothelial cell by a corneal functional property.

### (Item XC6)

The method of any one of Item XC1-XC4, further comprising identifying a subpopulation of the cultured functional corneal endothelial cells by at least one of corneal functional properties according to any one of Items X1-X4, X4A, X4B and X5-X14 and X25-X26 and/or Items X15-X26.

### (Item XC7)

The method of any one of Items XC5-XC6, wherein a plurality of indicators from each of proteinaceous product and related biological material of the product, secreted miRNA, and cellular metabolite comprising an amino acid and related biological material of the metabolite are selected to examine a variation in a profile of each indicator to determine homogeneity of cells having a cell indicator comprising CD166 positive, CD133 negative, CD44 negative to CD44 weak positive and CD90 negative to weak positive.

### (Item XC8)

The method of any one of Items XC1-XC7, wherein the proteinaceous product and related biological material of the product is selected from the group consisting of:
(A) COL4A1, COL4A2, COL8A1, COL8A2, CDH2, and TGF-beta2 whose expression increases in the human functional corneal endothelial cell capable of eliciting a human corneal functional property when transplanted into an anterior chamber of an eye, and
(B) MMP1, MMP2, TIMP1, BMP2, IL13RA2, TGF-beta1, CD44, COL3A1, IL6, IL8, HGF, THBS2, and IGFBP3 whose expression decreases in the human functional corneal endothelial cell capable of eliciting a human corneal functional property when transplanted into an anterior chamber of an eye.

### (Item XC9)

The method of any one of Items XC1-XC8, wherein the exosome comprises at least one cell indicator selected from the group consisting of:
(A) CD63, CD9, CD81, and HSP70 whose expression decreases in the human functional corneal endothelial cell capable of elicitinga human corneal functional property when transplanted into an anterior chamber of an eye.

### (Item XC10)

The method of any one of Items XC1-XC9, wherein the cellular metabolite and related biological material of the metabolite comprises at least one selected from the group consisting of succinic acid (succinate), Pro, Gly, glycerol3-phosphate, Glu, lactic acid (lactate), argininosuccinic acid (arginosuccinate), xanthine, N-carbamoyl aspartic acid (N-carbamoyl aspartate), isocitric acid (isocitrate), cis-aconitic acid (cis-aconitate), citric acid (citrate), Ala, 3-phosphoglyceric acid (3-phosphoglycerate), hydroxyproline, malic acid (malate), uric acid (urate), betaine, folic acid (folate), Gln, 2-oxoisovaleric acid (2-oxoisovalerate), pyruvic acid (pyruvate), Ser, hypoxanthine, Asn, Trp, Lys, choline, Tyr, urea, Phe, Met, carnosine, Asp, ornithine, Arg, creatine, 2-hydroxy glutaminic acid (2-hydroxy glutamate), beta-Ala, citrulline, Thr, Ile, Leu, Val, creatinine, His, N,N-dimethyl glycine, or a combination or relative ratio thereof.

### (Item XC11)

The method of Item XC10, wherein the cellular metabolite and related biological material of the metabolite comprises increase in serine, alanine, proline, glutamine or citric acid (citrate)/lactic acid (lactate) ratio in culture supernatant.

### (Item XC12)

A method of detecting a corneal endothelial nonfunctional cell coexisting with a cultured human corneal endothelial cell comprising the step of measuring at least one cell function indicator selected from the group consisting of: a cell surface marker; a proteinaceous product and a related biological material of the product; a SASP related protein; intracellular and secreted miRNA; an exosome; a cellular metabolite comprising an amino acid and a related biological material of the metabolite; cell size; cell density and the presence of an autoantibody reactive cell.

### (Item XC13)

A quality evaluating agent, process controlling agent, or corneal endothelial nonfunctional cell detecting agent for a mature differentiated corneal endothelial functional cell, comprising a reagent or means for measuring a cell indicator of any one of Items XC1-XC12.

### (Item XC14)

The quality evaluating agent, process controlling agent, or detecting agent of Item XC13, wherein the means for measuring is labeled.

### (Item XC15)

A method of sorting out a human functional corneal endothelial cell, comprising the steps of:
A) providing a sample that possibly comprises a human functional corneal endothelial cell capable of eliciting a human corneal functional property when transplanted into an anterior chamber of an eye;
B) determining whether the sample comprises the human functional corneal endothelial cell capable of eliciting a human corneal functional property when transplanted into an anterior chamber of an eye by using the quality evaluating agent, process controlling agent, or corneal endothelial nonfunctional cell detecting agent of Item XC13 or XC14, wherein it is determined that the sample comprises the human functional corneal endothelial cell capable of eliciting a human corneal functional property when transplanted into an anterior chamber of an eye when a result of evaluation with the quality evaluating agent, process controlling agent, or corneal endothelial nonfunctional cell detecting agent indicates that the cell is a human functional corneal endothelial cell capable of eliciting a human corneal functional property when transplanted into an anterior chamber of an eye; and
C) sorting out a cell determined to be a human functional corneal endothelial cell capable of eliciting a human corneal functional property when transplanted into an anterior chamber of an eye.

### (Item XC16)

A method of assaying quality of a human functional corneal endothelial cell, comprising the steps of:
A) obtaining information related to a cell indicator of the human functional corneal endothelial cell of cells provided as being human functional corneal endothelial cells capable of eliciting a human corneal functional property when transplanted into an anterior chamber of an eye by using the quality evaluating agent, process controlling agent, or corneal endothelial nonfunctional cell detecting agent of Item XC13 or XC14; and
B) determining that the provided cells are human functional corneal endothelial cells capable of eliciting a human corneal functional property when transplanted into an anterior chamber of an eye based on the information.

### (Item XC17)

A method of controlling quality in preparation of a human functional corneal endothelial cell capable of eliciting a human corneal functional property when transplanted into an anterior chamber of an eye, comprising the steps of:
A) obtaining information related to a cell indicator of a mature differentiated human functional corneal endothelial cell of cells obtained in the preparation by using the quality evaluating agent, process controlling agent, or corneal endothelial nonfunctional cell detecting agent of Item XC13 or XC14; and
B) determining that the preparation is suitable for preparation of a human functional corneal endothelial cell capable of eliciting a human corneal endothelial functional property when transplanted into an anterior chamber of an eye based on the information.

### (Item XC18)

A method of testing purity of a human functional corneal endothelial cell capable of eliciting a human corneal functional property when transplanted into an anterior chamber of an eye, comprising the steps of:
A) providing a sample possibly comprising the human functional corneal endothelial cell capable of eliciting a human corneal functional property when transplanted into an anterior chamber of an eye;
B) obtaining information related to a cell indicator of a human functional corneal endothelial cell of the cells by using the quality evaluating agent, process controlling agent, or corneal endothelial nonfunctional cell detecting agent of Item XC13 or XC14; and
C) calculating the purity of human functional corneal endothelial cell capable of eliciting a human corneal functional property when transplanted into an anterior chamber of an eye in the sample based on the information.

### (Item XC19)

A method of assaying quality of a medium for a human functional corneal endothelial cell, comprising the steps of:
A) culturing cells provided as being a mature differentiated human functional corneal endothelial cell capable of eliciting a human corneal functional property when transplanted into an anterior chamber of an eye in the medium to obtain information related to a cell indicator of the human functional corneal endothelial cell of the cells by using the quality evaluating agent, process controlling agent, or corneal endothelial nonfunctional cell detecting agent of Item XC13 or XC14; and
B) determining that the medium is suitable for manufacture of a human functional corneal endothelial cell capable of eliciting a human corneal functional property when transplanted into an anterior chamber of an eye based on the information.

### (Item XC20)

A method of assaying quality of a cell infusion vehicle for a human functional corneal endothelial cell, comprising the steps of:
A) culturing cells provided as being a human functional corneal endothelial cell capable of eliciting a human corneal functional property when transplanted into an anterior chamber of an eye in the cell infusion vehicle to obtain information related to a cell indicator of the human functional corneal endothelial cell of the cells by using the quality evaluating agent, process controlling agent, or corneal endothelial nonfunctional cell detecting agent of Item XC13 or XC14; and
B) determining that the cell infusion vehicle is suitable for cell transfer therapy based on the information.

### (Item XC21)

A method of quality control or process control of a cultured human functional corneal endothelial cell capable of eliciting a human corneal functional property when transplanted into an anterior chamber of a human eye or a method of detecting a corneal endothelial nonfunctional cell coexisting with a cultured human corneal endothelial cell, comprising the step of examining one or a plurality of the following:
(1) purity test by culture supernatant ELISA
   TIMP-1: 500 ng/mL or less
   IL-8: 500 pg/mL or less
   PDGF-BB: 30 pg/mL or greater
   MCP-1: 3000 pg/mL or less
(2) purity test by cell FACS
   CD166 = 95% or greater
   CD133 = 5% or less
   CD105 negative-low positive = 95% or greater
   CD44 negative-low positive = 70% or greater
   CD44 medium-high positive = 15% or less
   CD24 = 5% or less
   CD26 positive = 5% or less
   CD200 = 5% or less
(3) barrier function (ZO-1) positive
(4) pump function (Na+/K+ ATPase) positive
(5) cell survival
   70% or greater with trypan blue stain
(6) cell form
   transformed cells cannot be found by visual inspection
(7) Claudin10 positive
(8) effector cell (E-ratio) > 50%
(9) non-intended cell
   non-intended cell A (CD44 strong positive cell) < 15%, non-intended cell B (CD26 positive cell) <5 %, non-intended cell C (CD24 positive cell) < 5% (10) karyotype abnormality negative.

### (Item XC22)

The method of Item XC21, comprising carrying out the examining three weeks to immediately prior to cell infusion therapy or during preserved culture only exchanging a medium.

### (Item XC23)

The method of Item XC1 or XC22, comprising carrying out the examining about 7 day prior to or immediately prior to cell infusion therapy.

### (Item XC24)

The method of any one of Items XC15-XC20, characterized by one or a plurality of the characteristics of Items X C19-C21.

### (Item XC25)

A method of quality control or process control of cultured human functional corneal endothelial cells capable of eliciting human corneal functional property when transplanted into an anterior chamber of human eyes, comprising the step of determining one or a plurality of the following characteristics with respect to a target cell: (1) retention of endothelial pumping/barrier functions; (2) adhesion/attachment to a specific laminin; (3) produced cytokine profile; (4) produced metabolite profile; (5) saturated cell density upon in vitro culturing; (6) spatial size and distribution of cells obtained in culturing; and (8) cell retention in case of cell transfer after freeze damage from liquid nitrogen on mouse cornea.

### (Item XC26)

The method of Item XC25, wherein determination of the retention of endothelial pumping/barrier functions is determined by using a pumping function measuring method or a barrier function measuring method commonly used for corneal endothelia.

### (Item XC27)

The method of Item XC25 or C26, wherein determination of the adhesion/attachment to a specific laminin is determined by adhesiveness to laminin 511 (composite of alpha5 chain, beta1 chain, and gamma1 chain), laminin 521 (composite of alpha5 chain, beta2 chain, and gamma1 chain), or a functional fragment thereof and/or increase in integrin expression with respect thereto as an indicator.

### (Item XC28)

The method of Item XC23, wherein determination of the produced cytokine profile comprises measuring a production level of a cytokine profile of serum or aqueous humour.

### (Item XC29)

The method of any one of Items XC25-XC28, wherein determination of the produced metabolite profile comprises measuring a production level of metabolite of the cell.

### (Item XC30)

The method of any one of Items XC25-XC29, wherein determination of the produced micro RNA (miRNA) profile comprises obtaining total RNA to obtain a micro RNA expression profile thereof.

### (Item XC31)

The method of any one of Items XC25-XC30, wherein determination of saturated cell density upon in vitro culturing comprises counting cells in an image of the cells obtained by using an image capturing system.

### (Item XC32)

The method of any one of Items XC25-XC31, wherein determination of the spatial size and distribution of cells obtained in culturing comprises counting cells in an image of the cells obtained by using an image capturing system.

### (Item XC33)

The method of any one of Items XC25-XC32, wherein determination of the cell retention in case of cell transfer after freeze damage from liquid nitrogen on mouse cornea comprises: infusing cells to be determined into an anterior chamber of an eye of a model made by pre-treatment of a central region of a mouse cornea by freeze damage to remove endothelial cells; clinically observing a characteristic of the cornea; assessing the thickness of the cornea with a pachymeter; histopathologically testing HCEC adhesion with human nuclear staining; and examining whether the cell has a function.

It is understood that one or more of the aforementioned features can further be provided as a combination thereof in addition to the explicitly shown combinations in the present invention. Additional embodiments and advantages of the present invention are recognized by those skilled in the art who read and understand the following detailed description as needed.

### [Advantageous Effects of Invention]

The present therapeutic method gives rise to a paradigm shift in the corneal endothelium regenerative medicine, which has a potential to expand application to over a million patients worldwide as an internationally deployable, versatile medicine. With respect to the following Brief Description of the Drawings, as used herein, -, +, ++, and +++, with regard to the intensity of expression of a cell surface marker, indicate negative, weakly positive, intermediately positive, and strongly positive, respectively. +/- is encompassed by - (negative) herein. Neg, low, med, and high indicate negative, weakly positive (herein also referred to as low), intermediately positive (herein also referred to as medium), and strongly positive (herein also referred to as high), respectively. Weakly positive, intermediately positive, and strongly positive are determined as follows: a PE-Cy7-conjugated anti-human CD44 antibody (BD Biosciences) is used, and Area Scaling Factor of Blue laser of FACS Canto II is set to 0.75 and the voltage of PE-Cy7 is set to 495; under these settings, the range of weak fluorescence intensity is less than about 3800, the range of medium fluorescence intensity is about 3800 or greater to less than 27500, and the range of strong fluorescence intensity is about 27500 or greater. It is determined to be negative if the negative control (isotype control) has the same staining intensity pattern, and positive if the pattern is shifted even by a small amount. The mean fluorescence intensity of the negative control (isotype control) with the above-described setting is about 50 (range of 55 +/- 25). The following setting was used for other fluorescent dyes: for Area Scaling Factor, FSC = 0.5, Blue laser = 0.75, and Red laser = 0.8; for voltage, FSC = 270, SSC = 400, FITC = 290, PE = 290, PerCP-Cy5.5 = 410, PE-Cy 7 = 495, and APC = 430. Leakage of each fluorescence into other fluorescence is corrected with BD comp Beads (BD Biosciences) and FACS DiVa soft. The mean fluorescence intensity of the negative control (isotype control) at this time is as follows: about 130 for FITC, about 120 for PE, about 120 for PerCP-Cy5.5, about 50 for PE-Cy7, and about 110 for APC. For detection using a Lyoplate experiment (see, e.g., Table 2), Alexa Fluor 647-labeled secondary antibody (attached to kit) is used for measurement. In such a case, the value of median fluorescence intensity of each marker/median fluorescence intensity of negative control (staining by isotype control antibody) of less than 5, 5 or greater and less than 10, 10 or greater and less than 30, and 30 or greater are defined as -, +, ++, and +++, respectively.

### [Brief Description of Drawings]

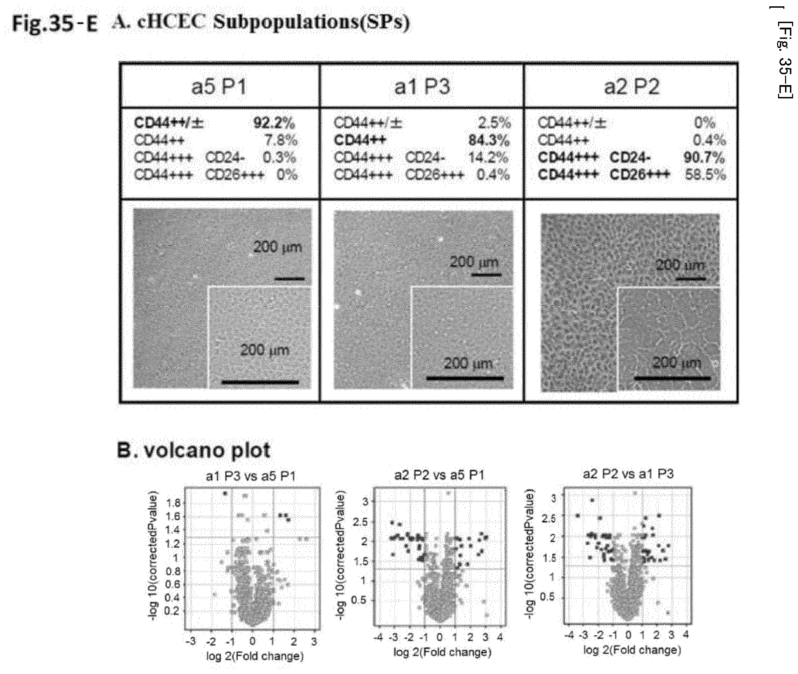

[Fig. 1-A]

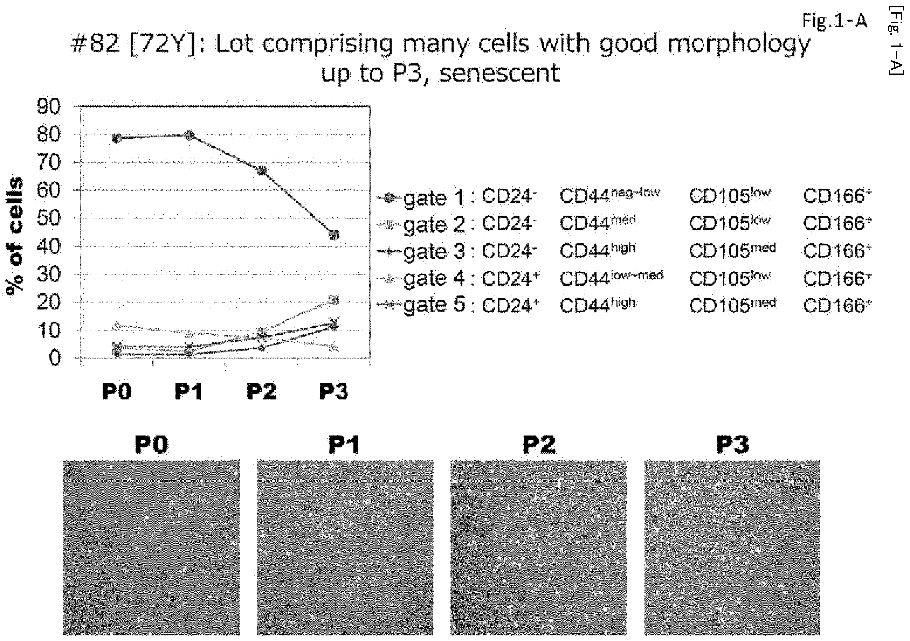

   Fig. 1-A shows the change in subpopulation (SP) compositions depending on the number of passages. The results of phase contrast microscope pictures and FACS analysis for primary culture and first-third passage of HCECs of #82 are shown. The vertical axis of the graph indicates the percentage of the number of cells selectively propagated in cultures at each gate to the total number of cells. The gate conditions are the following: gate 1: CD24-CD44- to +CD105+CD166+, gate 2: CD24-CD44++CD105+CD166+, gate 3: CD24-CD44+++CD105++CD166+, gate 4: CD24+CD44+ to ++CD105+CD166+, and gate 5: CD24+CD44+++CD105++CD166+.
[Fig. 1-B]

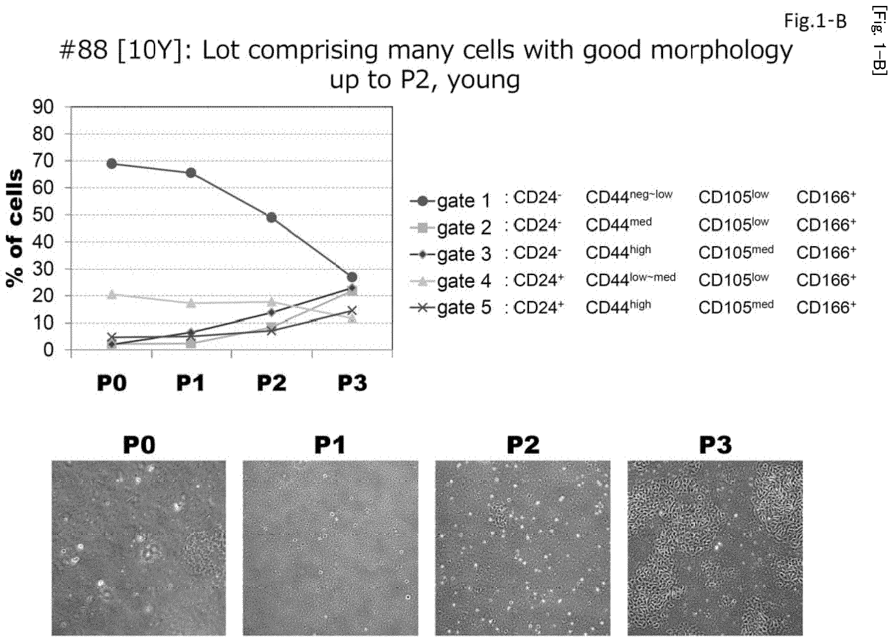

   Fig. 1-B shows the change in subpopulation (SP) compositions depending on the number of passages. The results of phase contrast microscope pictures and FACS analysis for primary culture and first-third passage of HCECs of #88 are shown. The vertical axis of the graph indicates the percentage of the number of cells selectively propagated in cultures at each gate to the total number of cells. The gate conditions are the same as in Fig. 1-A.
[Fig. 1-C]

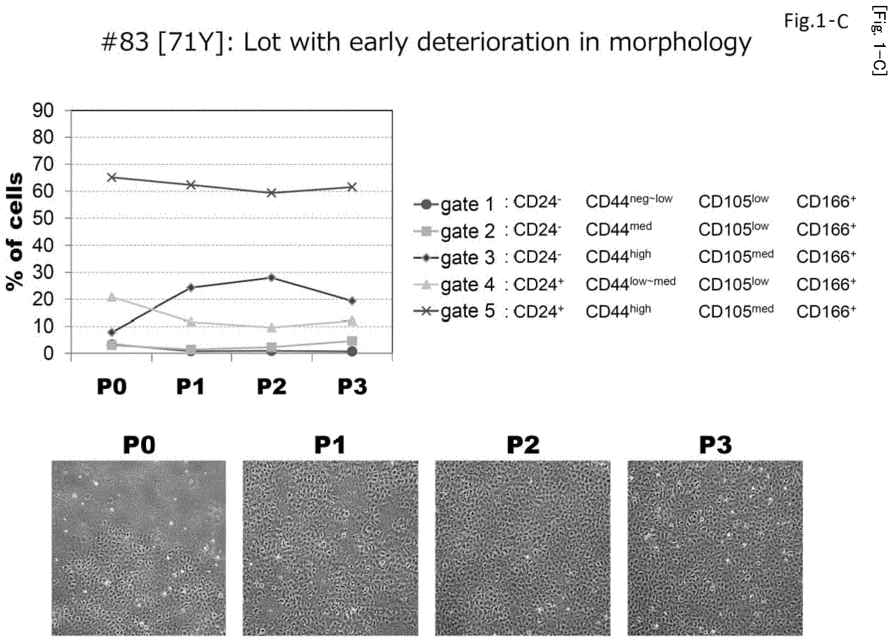

   Fig. 1-C shows the change in subpopulation (SP) compositions depending on the number of passages. The results of phase contrast microscope pictures and FACS analysis for primary culture and first-third passage of HCECs of #83 are shown. The vertical axis of the graph indicates the percentage of the number of cells selectively propagated in cultures at each gate to the total number of cells. The gate conditions are the same as in Fig. 1-A.
[Fig. 1-D]

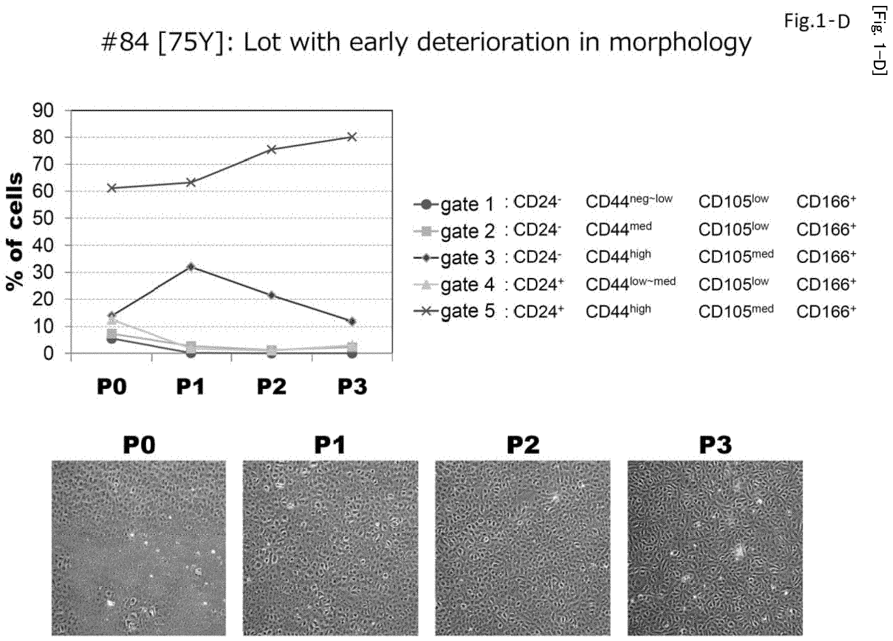

   Fig. 1-D shows the change in subpopulation (SP) compositions depending on the number of passages. The results of phase contrast microscope pictures and FACS analysis for primary culture and first-third passage of HCECs of #84 are shown. The vertical axis of the graph indicates the percentage of the number of cells selectively propagated in cultures at each gate to the total number of cells. The gate conditions are the same as in Fig. 1-A.
[Fig. 2-A]

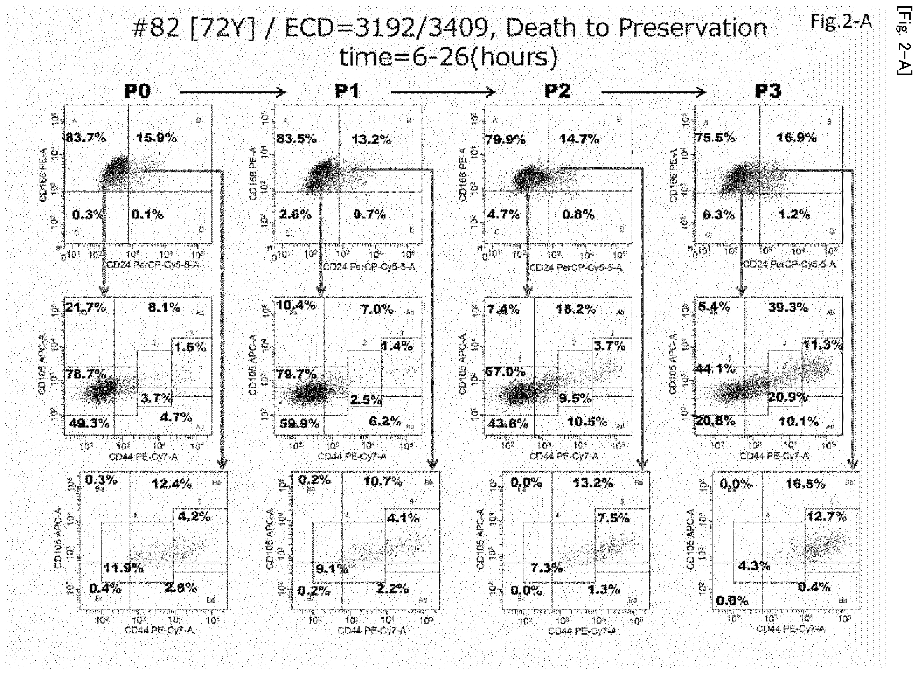

   Fig. 2-A shows typical FACS analysis showing the change in subpopulation compositions depending on the number of passages. The results of FACS analysis for CD44, CD166, CD24, and CD105 expression with respect to primary culture and first-third passage of HCECs of #82 are shown.
[Fig. 2-B]

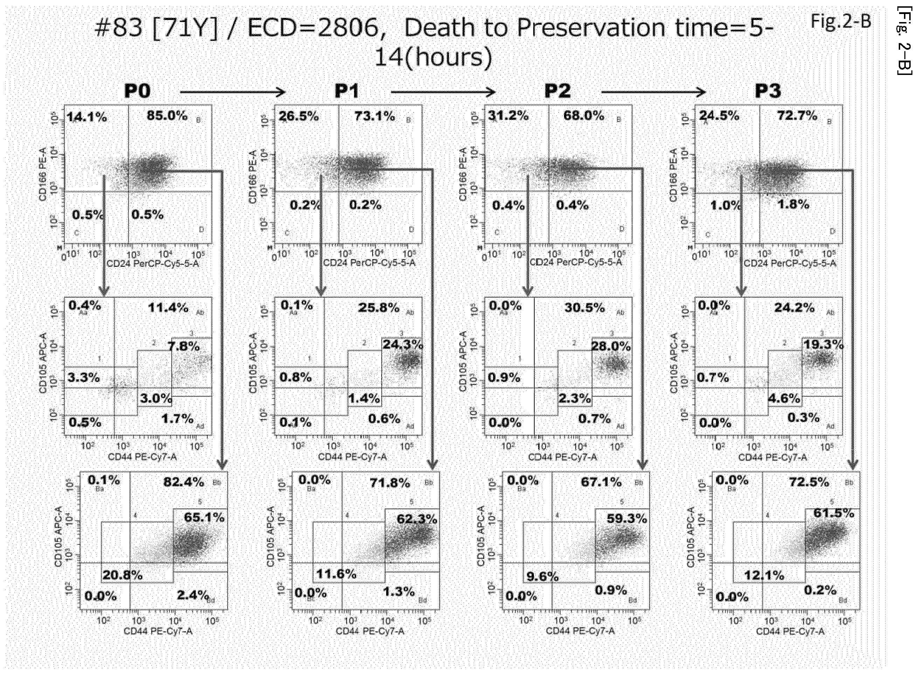

   Fig. 2-B shows typical FACS analysis showing the change in subpopulation compositions depending on the number of passages. The results of FACS analysis for CD44, CD166, CD24, and CD105 expression with respect to primary culture and first-third passage of HCECs of #83 are shown.
[Fig. 3]

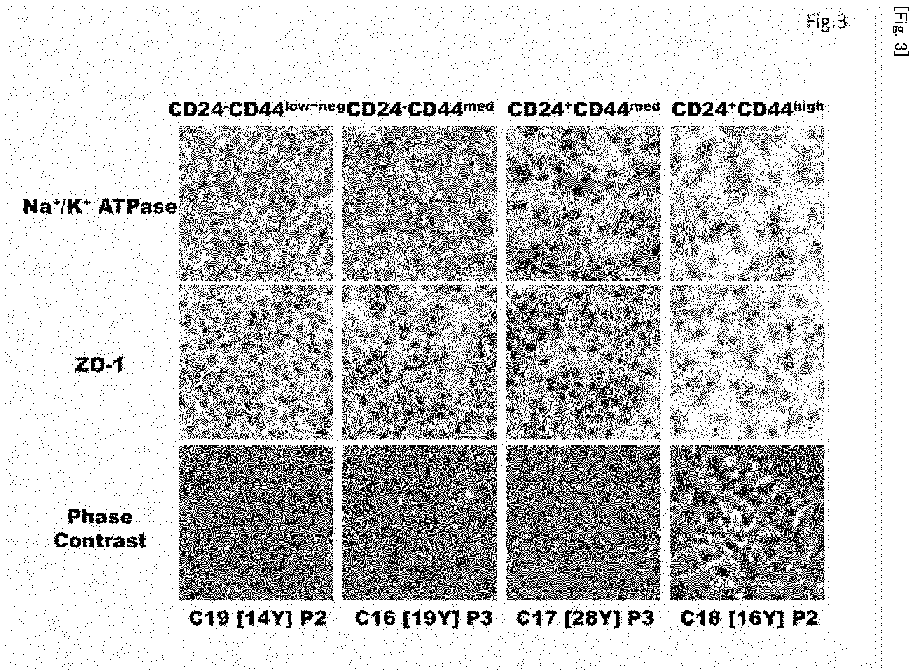

   Fig. 3 shows the marker expression and morphology of subpopulations characterized by the expression of CD44 and CD24. Nuclei were stained with hematoxylin. Fig. 3 shows, from the top, bright field observation images for Na⁺/K⁺ATPase (color emission by 3,3'-diaminobenzidine [DAB], dark brown), bright field observation images for ZO-1 (color emission by 3,3'-diaminobenzidine [DAB], dark brown), and phase contrast microscope images, and from the left, C19 second passage which is CD24-CD44- to +, C16 third passage which is CD24-CD44++, C17 third passage which is CD24+CD44++, and C18 second passage which is CD24+CD44+++.
[Fig. 4]

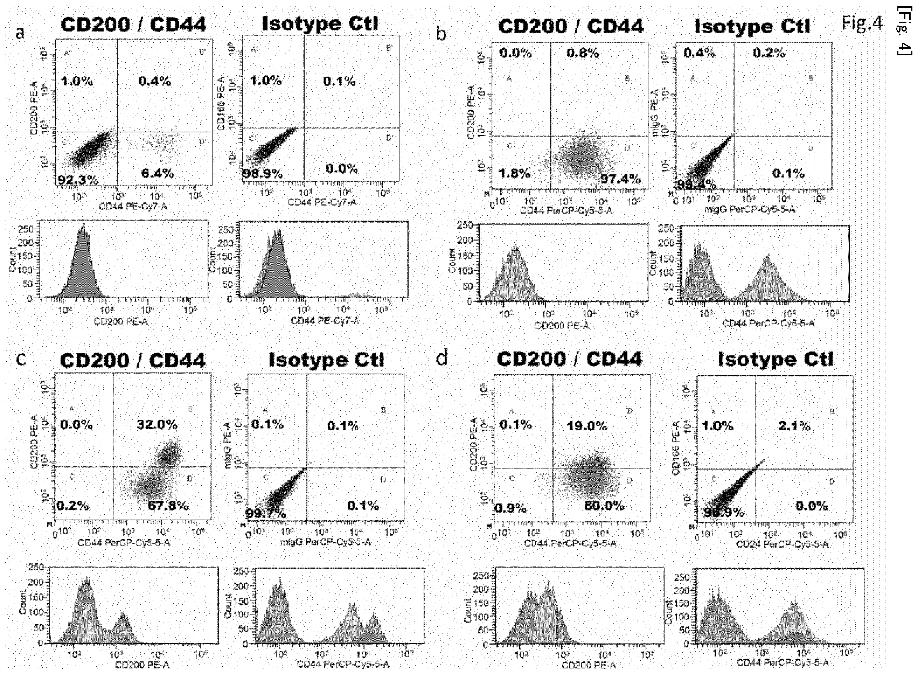

   Fig. 4 shows FACS analysis for CD200 and CD44 expression of each culture. In the dot plots for each culture, the horizontal axis indicates the logarithmic value of expression intensity of human CD44, and the vertical axis indicates the logarithmic value of expression intensity of CD200. For both the vertical and horizontal axes, a dot plot representing the logarithmic value of expression intensity of mouse IgG is shown as a control. In the histograms for each culture, the horizontal axis indicates the logarithmic value of expression intensity of CD200 or CD44 with the expression intensity of mouse IgG (control, gray), while the vertical axis represents the corresponding cell count.
[Fig. 5]

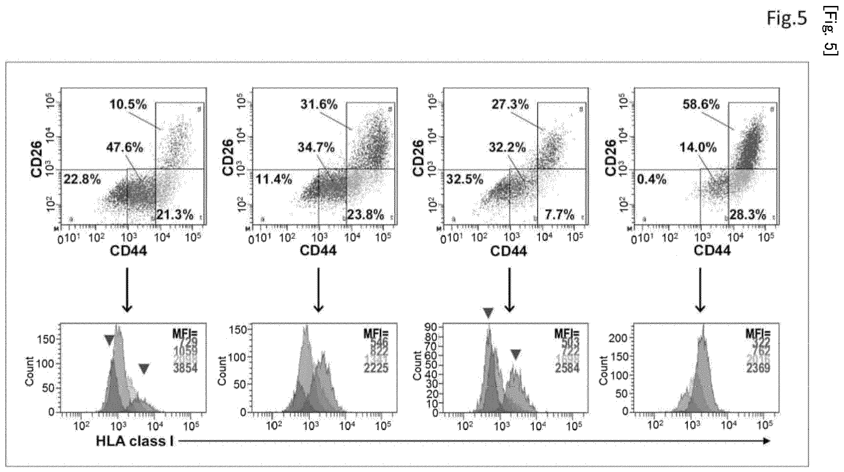

   Fig. 5 shows results of FACS analysis indicating that the surface HLA class I antigen expression decreases with the decrease in CD26 and CD44 expression of each culture. Fig. 5 investigates which subpopulation is desirable for infusion into patients in view of expression of immune rejection related molecules. In the dot plots for each culture, the horizontal axis indicates the logarithmic value of expression intensity of human CD44, and the vertical axis indicates the logarithmic value of expression intensity of CD26. In the histograms for each culture, the horizontal axis indicates the logarithmic value of expression intensity of HLA class I antigens and the vertical axis represents the corresponding cell count. The colors correspond to the cells shown in the dot plots for CD26 and CD44. The numerical values within the histograms represent the Mean of Fluorescence Intensity (MFI) of each histogram. The arrows indicate that the CD44^{neg-low} has a lower expression than CD44^{high}.
[Fig. 6-A]

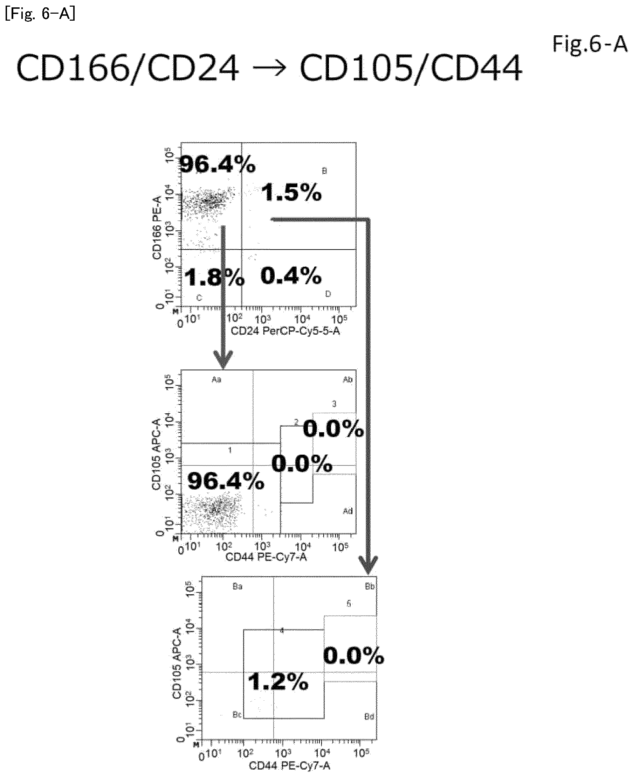

   Fig. 6-A shows results of FACS analysis for expression of CD44, CD166, CD24, and CD105 in a corneal tissue (from a 71 year old donor) immediately after excision of the tissues and set up of a single cell suspension.
[Fig. 6-B]

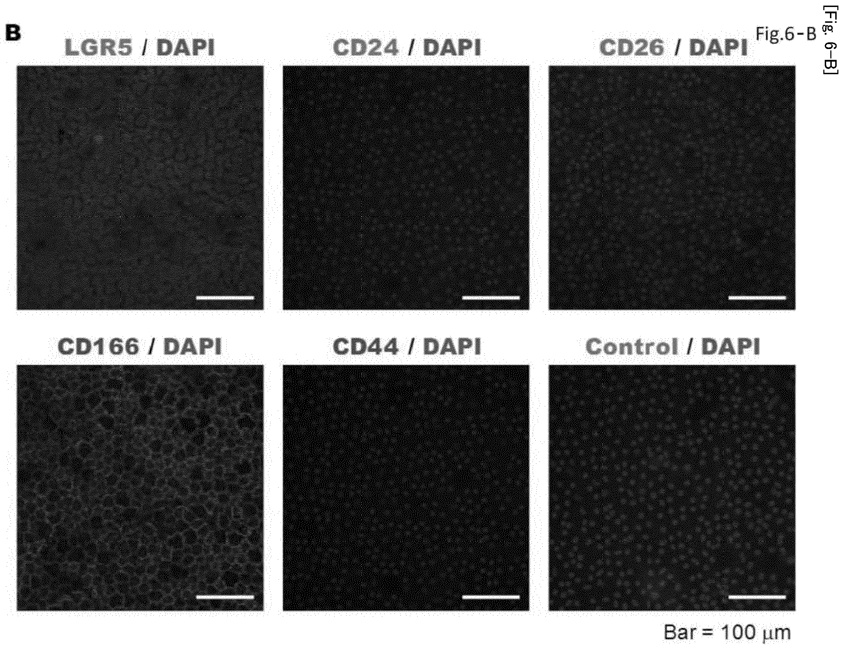

   Fig. 6-B shows results of immunohistochemical staining indicating expression of the markers in a corneal tissue (from a 65 year old donor) handled with as in Fig. 6-B. Fig. 6-B shows staining, in the top row from the left, for LGR5, CD24, and CD26, and, in the bottom row from the left, for CD166, CD44, and control (isotype control), overlaid with DAPI staining. The scale bar is 100 .micro.m.
[Fig. 7]

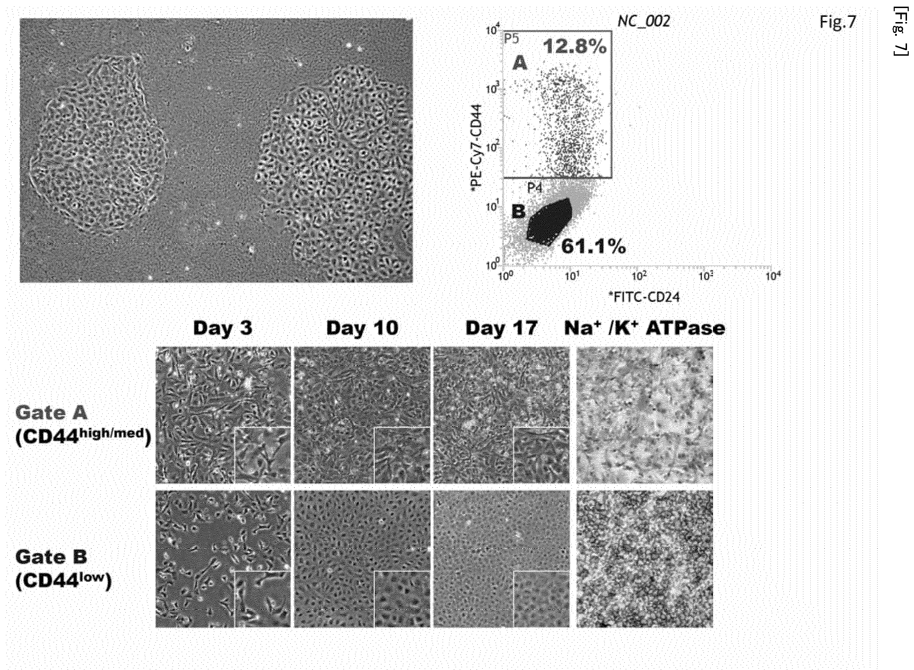

   Fig. 7 shows that cHCECs from subpopulations with distinct intensity of CD44 expression exhibit different morphology. The top left portion shows a phase contrast microscope image of cultured cells before gating by FACS. Top right portion shows FACS analysis on CD44 and CD24 of the cultured cells. Gate A contained 12.8% of cells and Gate B contained 61.1% of cells. The bottom part shows phase contrast microscope images and fluorescence microscope images of cultured cells from each gate. The top row shows cultured cells with medium to high degree of CD44 expression of obtained from Gate A, and the bottom row shows those with low degree of CD44 expression obtained from Gate B. The bottom part shows, from the left, a phase contrast microscope image on day 3, day 10, and day 17, and bright field observation image of staining with DAPI and anti-Na⁺/K⁺ATPase.
[Fig. 8]

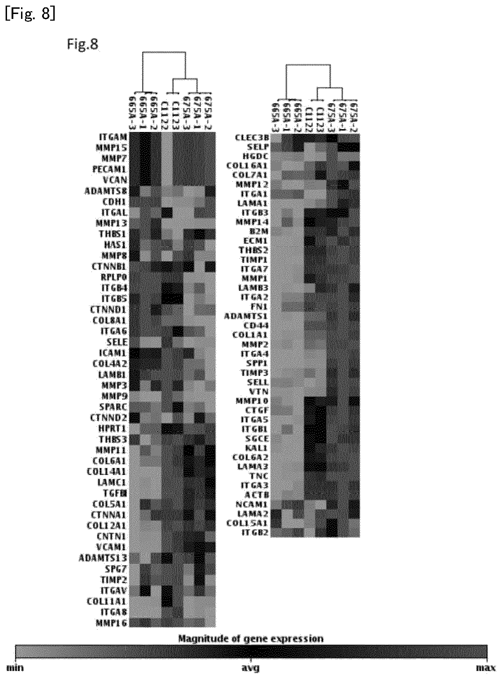

   Fig. 8 shows results of comparison of expression of gene expression in cultured endothelial cells of two separate subpopulations that have undergone either CST or differentiation into effector cells. Hierarchical clustering was used to compare gene signatures. This is shown as a heat map. Red indicates a relatively high expression and green indicates a relatively low expression.
[Fig. 9]

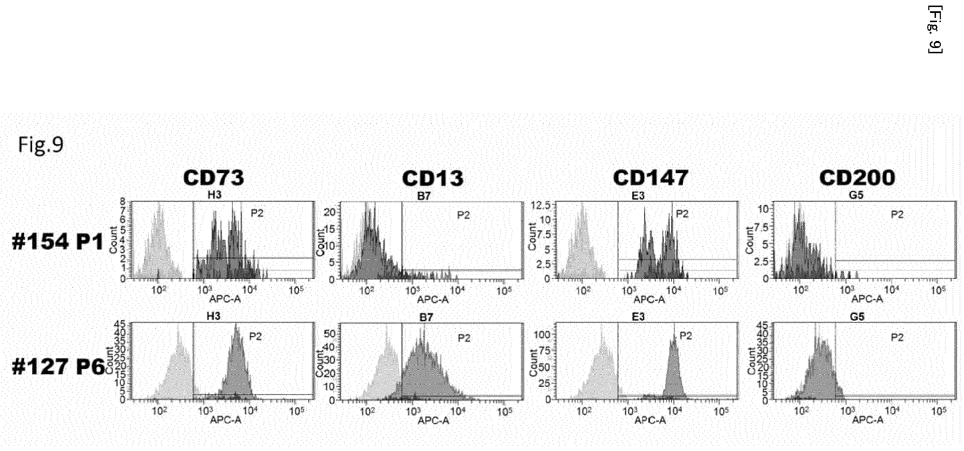

   Fig. 9 illustrates partial results of the expression of cell surface markers of cell subpopulations which are different among cHCECs by FACS analysis. Fig. 9 shows histograms representing the expression intensity of each marker for two FACS gated subpopulations (CD166+CD105-CD24-CD44- to + (blue) and CD166+CD105-CD24+CD44+++ (red)) of #154 (first passage, top) and #127D (sixth passage, bottom). The horizontal axis indicates the logarithmic value of expression intensity of CD73, CD13, CD147 or CD200 with the expression intensity of the negative control (label by isotype control antibody, gray). The vertical axis indicates the corresponding cell count.
[Fig. 10-A]

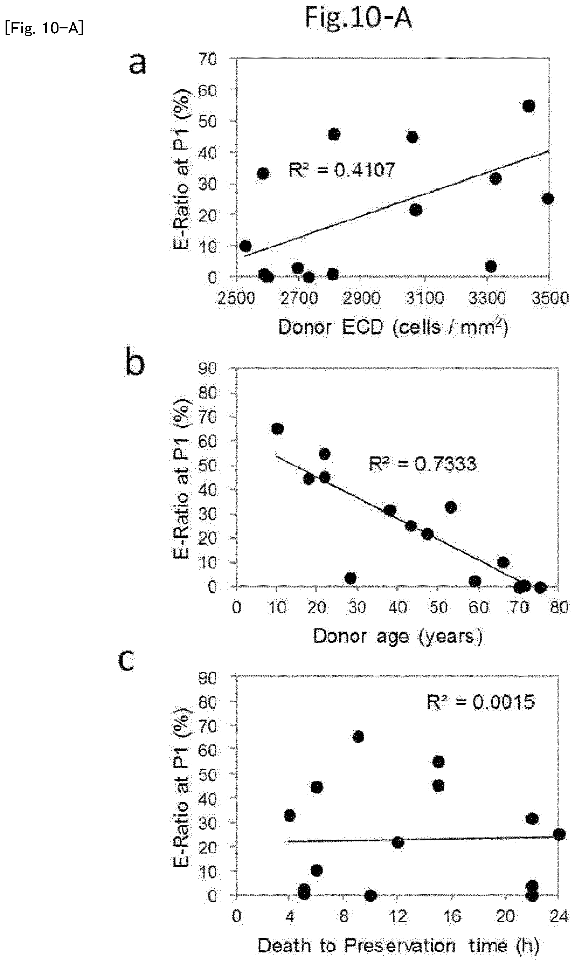

   Fig. 10-A shows the correlation between the ratios of effector cells (E-ratio) and other donor parameters. In each graph, each plot represents a distinct tissue donor, and the vertical axis represents the E-ratio in the first passage culture. In the top row, the horizontal axis indicates the endothelial cell density of a donor, where the correlation coefficient for the E-ratio and the endothelial cell density of a donor is 0.4107. In the middle row, the horizontal axis indicates the age of a donor, where the correlation coefficient for the E-ratio and age of a donor is 0.7333. In the bottom row, the horizontal axis indicates cell death during the preservation period, where the correlation coefficient for the E-ratio to cell death during preservation period is 0.0015.
[Fig. 10-B]

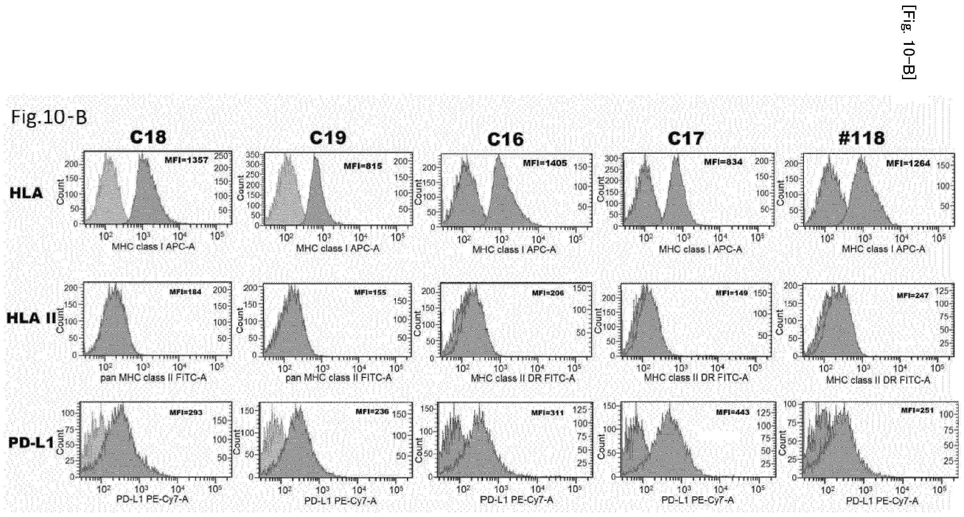

   Fig. 10-B shows results of FACS analysis for three types of immune rejection associated molecules of five different lot of cultured HCE cells. Fig. 10-B investigates which subpopulation is suitable for infusion into patients in view of expression of an immune associated molecules. The horizontal axis represents expression intensity of each immune rejection associated molecule and the vertical axis represents the corresponding cell count. Fig. 10-B shows, from the left, cultured human corneal endothelial cells of C18, C19, C16, C17, and #118, and shows, from the top, expression of HLAI, HLAII, and PDL1 with a red histogram. MFI represents mean fluorescence intensity for these molecules. The control is shown with a gray histogram. HLAI and PDL1 are positive for each cultured human corneal endothelial cells, but HLAII was mostly negative.
[Fig. 10-C]

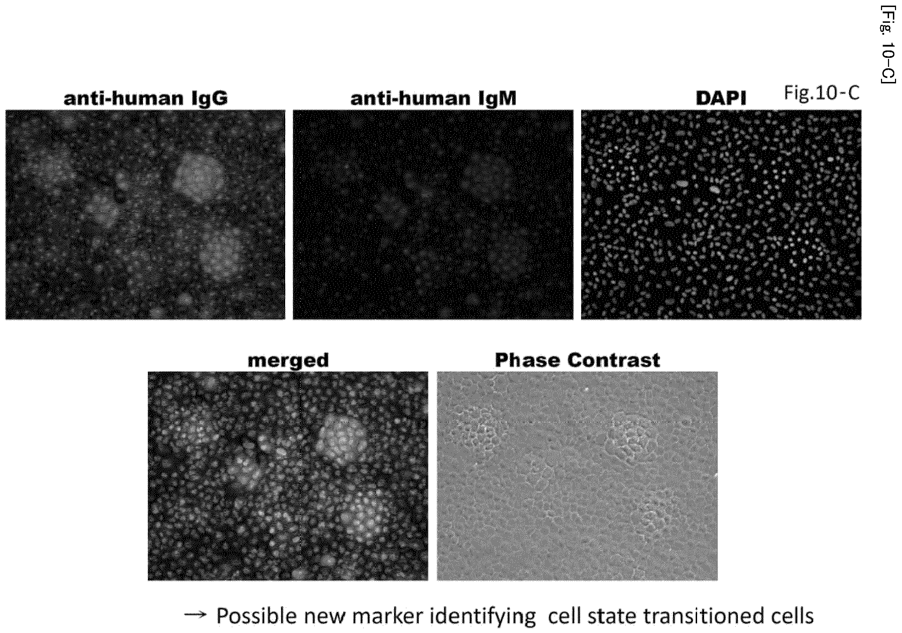

   Fig. 10-C shows fluorescence microscope images and phase contrast microscope images of cultured cells from human corneal endothelial tissues comprising cells that have undergone cell state transition. The left side of the top row shows a fluorescence microscope image of human IgG antibody-labeled cells that have been reacted with serum of a healthy individual, the center of the top row shows a fluorescence microscope image of anti-human IgM antibody-labeled cells that have been reacted with serum of a healthy individual, and the right side of the top row shows a fluorescence microscope image of DAPI-labeled cells that have been reacted with serum of a healthy individual, the left side of the bottom row merges the three fluorescence microscope images in the top row, and the right side of the bottom row shows a phase contrast microscope image. Since IgG and IgM are bound partly to cultured cells from human corneal endothelial tissue that have undergone phase transition, this demonstrates the presence of a natural antibody in the human serum against a cell that has undergone cell state transition, not suitable for infusion therapy.
[Fig. 11-A]

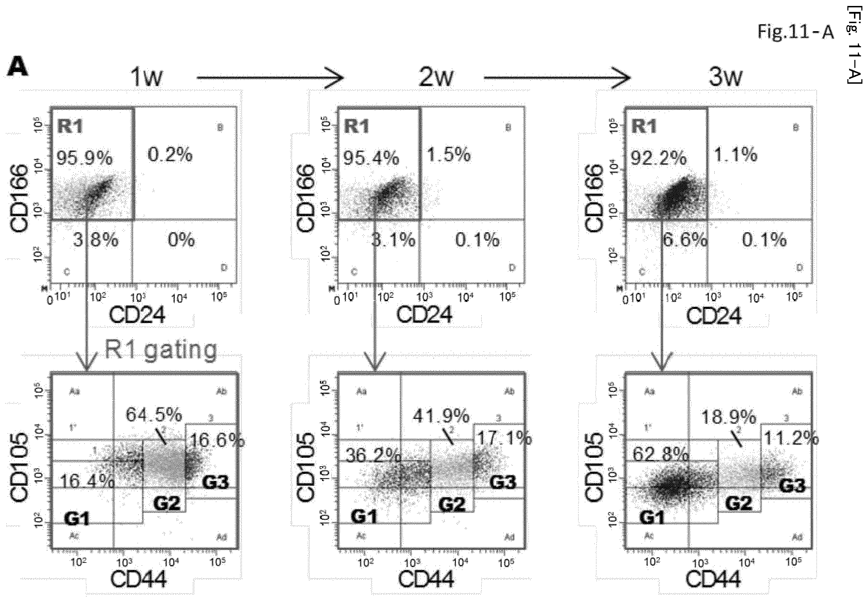

   Fig. 11-A shows that CD44 expression gradually decreases when primary culture is extended. Fig. 11-A shows FACS analysis for CD166, CD24, CD105, and CD44 of cultures collected at, from the left, week 1, week 2, and week 3 of culture.
[Fig. 11-B]

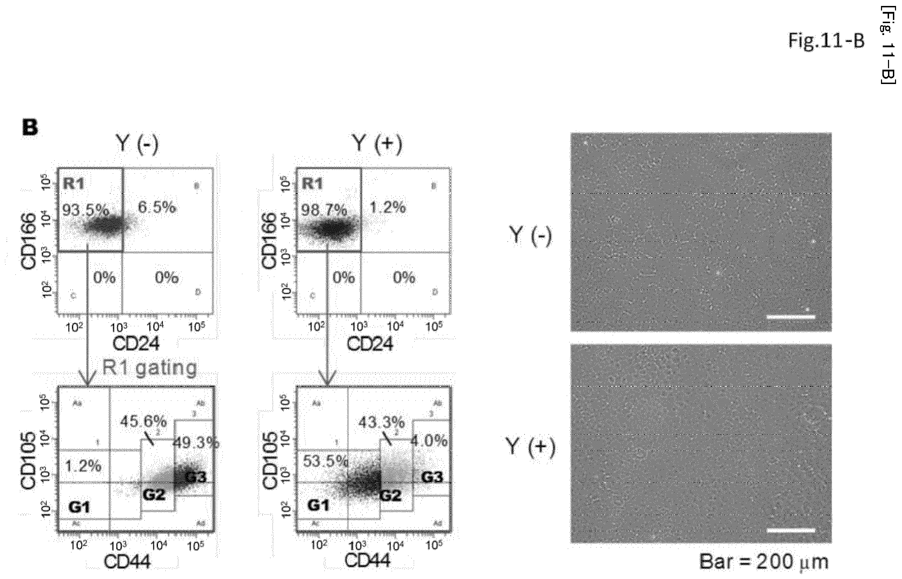

   Fig. 11-B shows the effect due to the presence or absence of addition of [(R)-(+)-trans-(4-pyridyl)-4-(1-aminoethyl)-cyclohexanecarboxamide dihydrochloride monohydrate](Y-27632) by phase contrast microscope images and FACS analysis for CD166, CD24, CD105, and CD44. Y(+) indicates addition of Y-27632 and Y(-) indicates no addition of Y-27632. The scale bar indicates 200 .micro.m.
[Fig. 12-A]

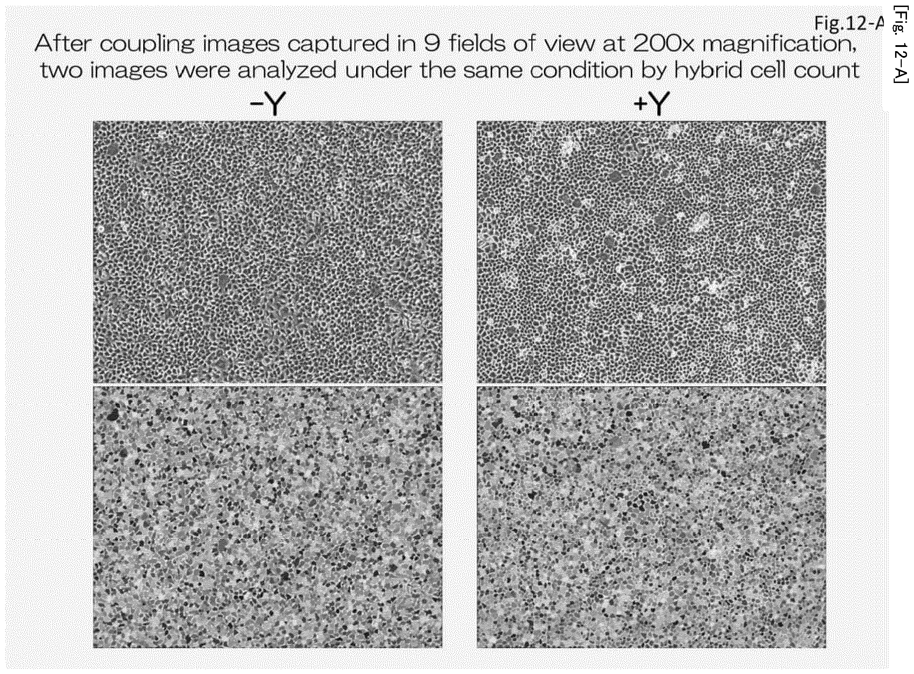

   Fig. 12-A shows that the cell subpopulations with smaller cell area are enriched by adding Y-27632 during the culture. In the phase contrast microscope images, the left side show a case where Y-27632 was not added, and the right side shows a case where Y-27632 was added. The top row shows phase contrast images of cultures on day 47 of culture after washing with PBS. The bottom row shows identification of cell regions in the images on the top row with BZ-H3C Hybrid cell counting software.
[Fig. 12-B]

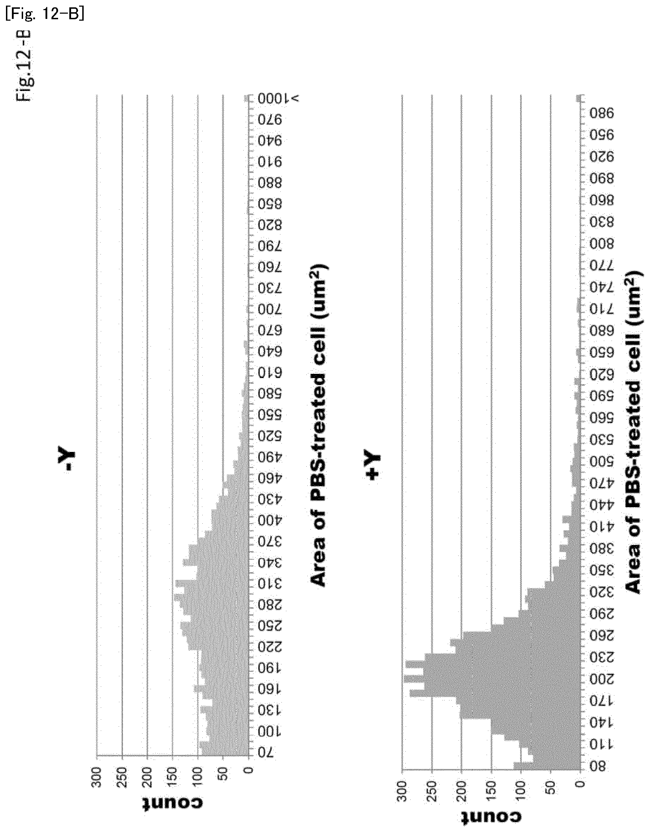

   Fig. 12-B shows histograms demonstrating that cell subpopulations with smaller cell area are enriched by adding Y-27632 during culture. -Y represents a case where Y-27632 was not added and +Y represents a case where Y-27632 was added. The vertical axis indicates the cell count and the horizontal axis indicates the cell area.
[Fig. 13]

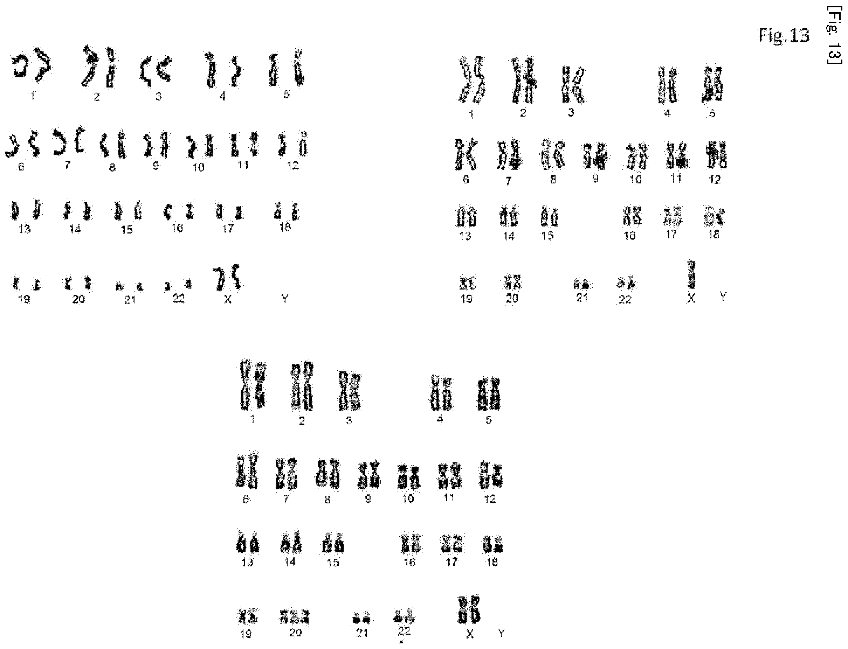

   Fig. 13 shows an example of karyotype aneuploidy. The top left portion shows a normal karyotype. The top right portion shows loss of Y-chromosome. The bottom part shows trisomy on chromosome 20. When the preparation in the top left image is examined, the number of chromosomes for 30 of the counted 30 cells was 46. Detailed karyotyping resulted in 20 cells with the number of chromosomes of 46 and XX. When the preparation in the top right image was examined, the number of chromosomes for 50 of the counted 50 cells was 45. Detailed karyotyping resulted in 20 cells with the number of chromosomes of 45, X and -Y. When the preparation in the bottom image was examined, the number of chromosomes for 33 out of counted 50 cells was 46 and the number of chromosomes for 17 cells was 45. Detailed karyotyping resulted in 8 out of 20 cells with the number of chromosomes of 47 and XX, and 12 cells with the number of chromosomes of 46 and XX.
[Fig. 14]

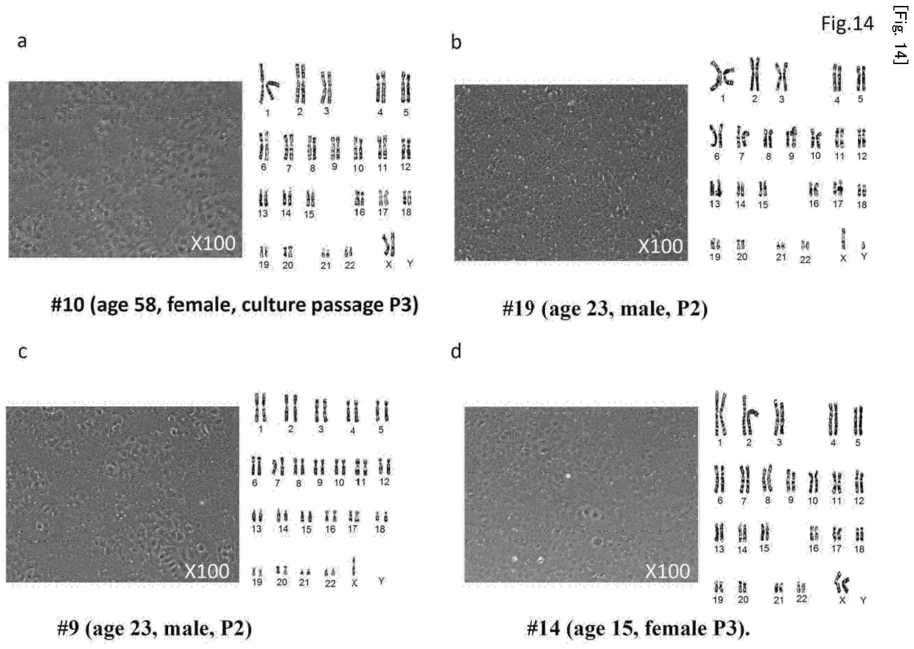

   Fig. 14 shows a typical example of karyotyping. Detailed karyotype and phase contrast microscope images of each cultured cell are shown. a is third passage cHCECs from a 58 year old female, b is second passage cHCECs from a 23 year old male, c is second passage cHCECs from a 23 year old male, and d is third passage cHCECs from a 15 year old female.
[Fig. 15]

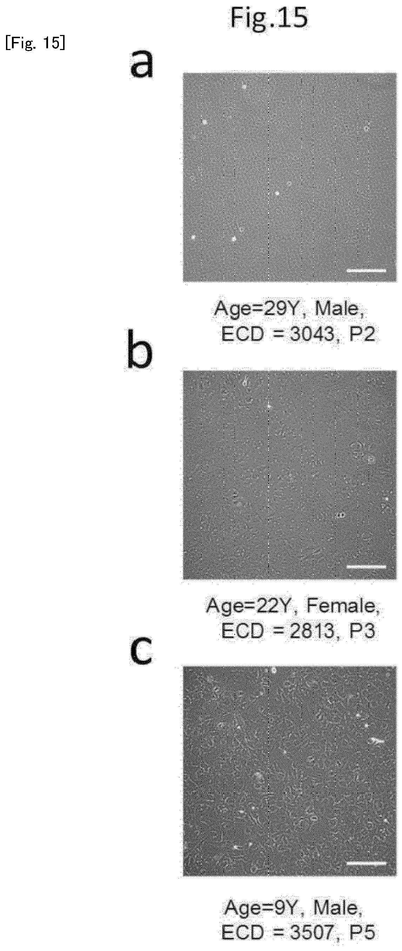

   Fig. 15 shows phase contrast microscope images for different cHCECs. The top row is second passage from a 29 year old male donor. The cells exhibit a hexagonal morphology with no sign of CST. The middle row is a third passage from a 22 year old female with abnormal CST-like morphology. The bottom row is a fifth passage from a 9 year old male with abnormal CST-like morphology. ECD means the cell density in cultures (cells/.micro.m²)
[Fig. 16]

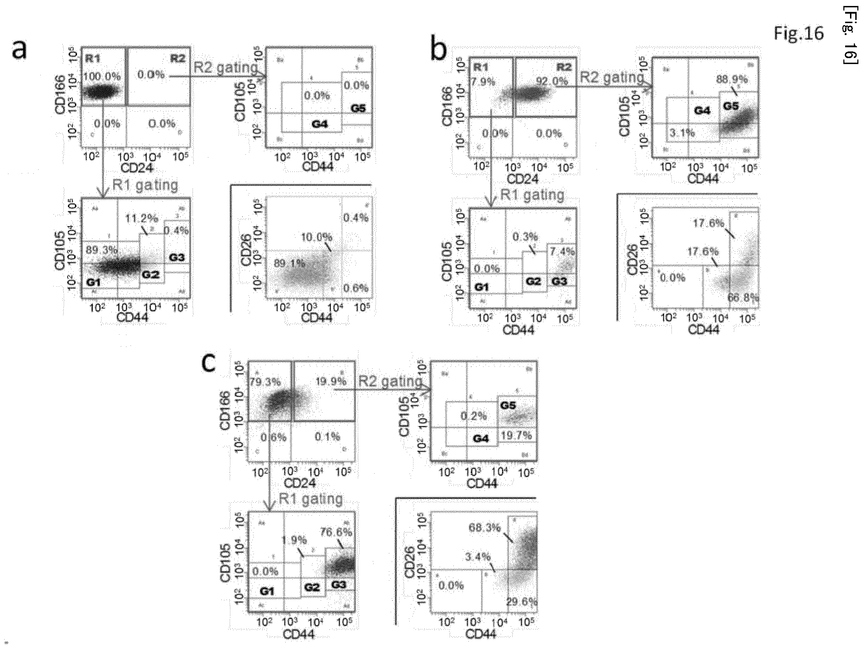

   Fig. 16 shows FACS analysis for different cHCECs. The results of FACS analysis for a, b, and c correspond to the cultured cells shown in the phase contrast microscope images of a, b, and c in Fig. 15, respectively. The top row includes most of subpopulation with CD44-. The middle row demonstrates subpopulation with mostly CD44+++, a culture containing cells with intense CD24 expression. The bottom row is a culture involving cells with intense CD26 expression.
[Fig. 17]

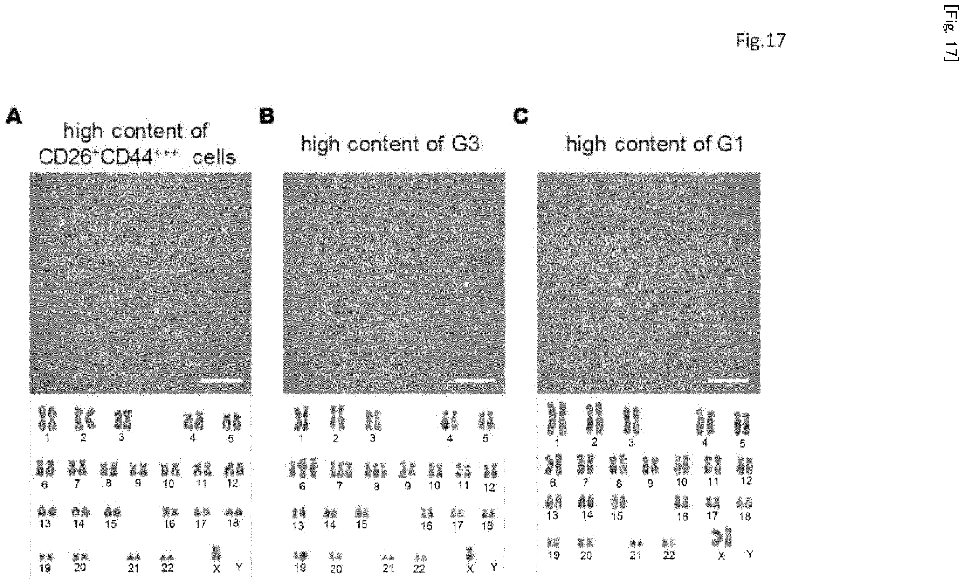

   Fig. 17 shows phase contrast microscope images and chromosome observation images showing karyotype aneuploidy observed when culturing a specific subpopulation. A shows that the subpopulations of CD44⁺⁺⁺, CD166⁺, CD24⁻, and CD26⁺ have lost sex chromosomes. B shows that the subpopulations of CD44⁺⁺⁺, CD166⁺, CD24⁺, and CD26⁻ (G3) exhibit trisomy at a high frequency on chromosomes 6, 7, and 8. C shows that the subpopulations CD44-, CD166⁺, CD105⁻, CD24⁻, and CD26⁻ (G1) do not exhibit aneuploidy.
[Fig. 18]

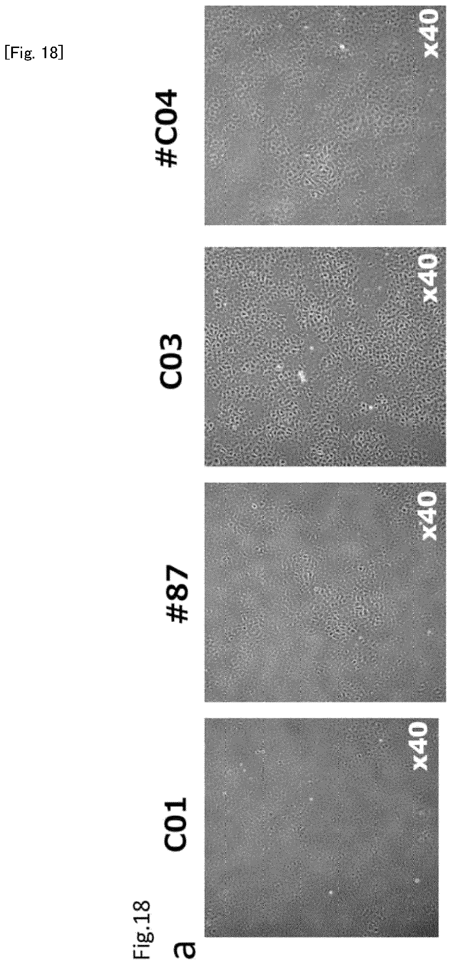

   Fig. 18 shows phase contrast microscope images for 4 types of cHCECs (C01, #87, C03 and #C04) characterizing cHCECs from different donors.
[Fig. 19]

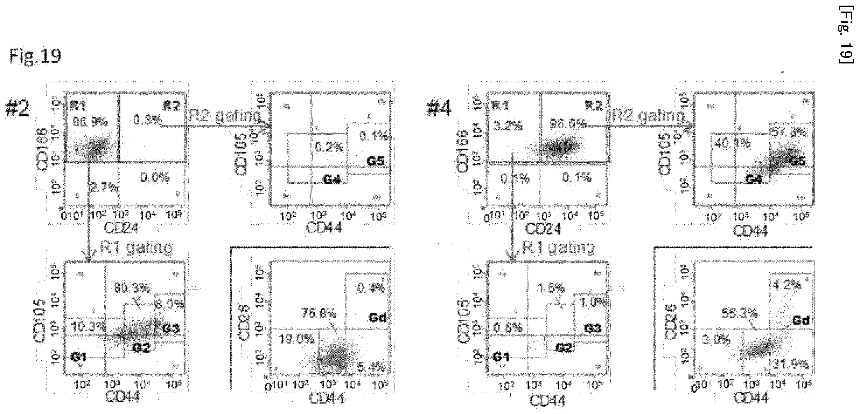

   Fig. 19 shows results of FACS analysis for CD44, CD166, CD24, CD26, and CD105 of 2 types of cHCECs (#2 is from a 57 year old donor, #4 is from a 58 year old donor) characterizing cHCECs from different donors.
[Fig. 20]

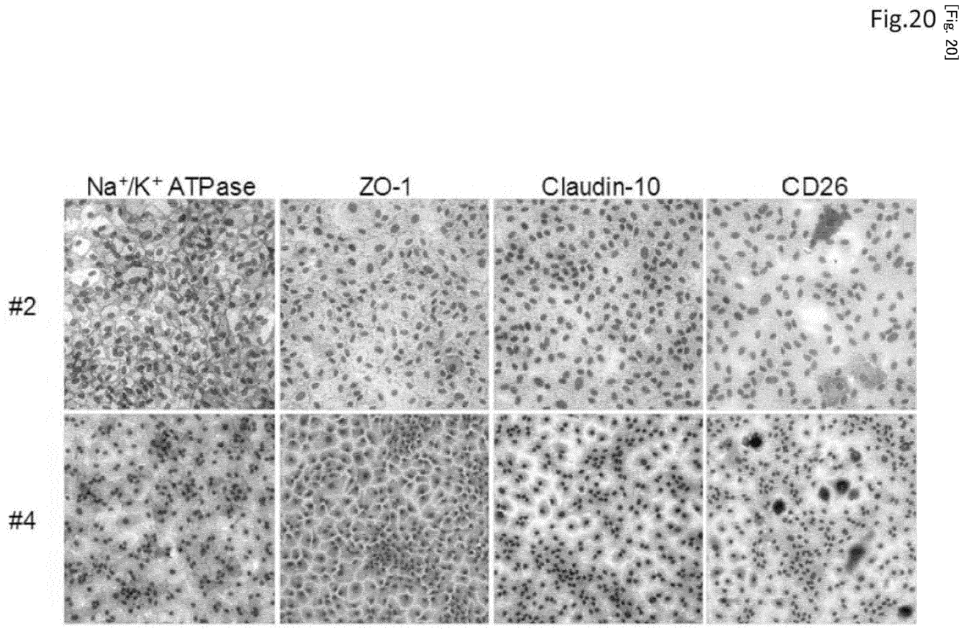

   Fig. 20 shows bright field microscope images (coloring by DAB) after immunostaining for 2 type of cHCECs (#2 is from a 57 year old donor, #4 is from a 58 year old donor) characterizing cHCECs from different donors, showing antibody staining for Na⁺/K⁺ATPase, ZO1, Claudin10, and CD26. Nuclei were stained with hematoxylin.
[Fig. 21]

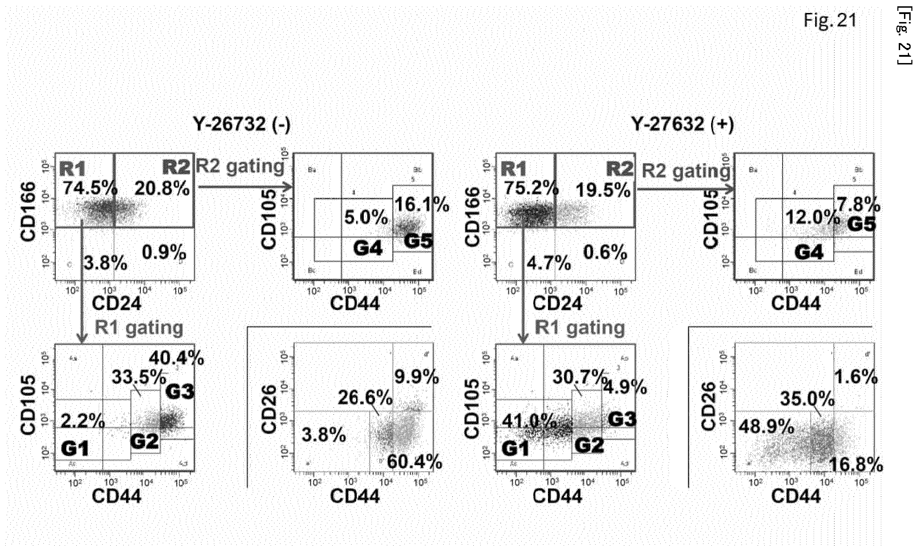

   Fig. 21 shows the subpopulation distribution in culture with or without Y-27632.
[Fig. 22-A]

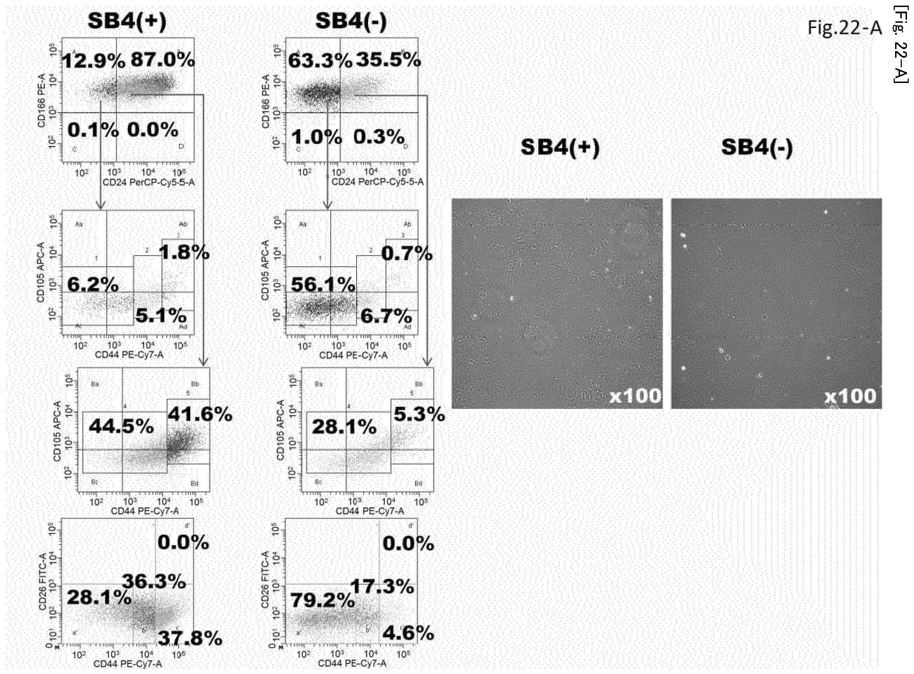

   Fig. 22-A shows that cultures without a TGF-beta inhibitor, SB431542, do not induce morphological changes (CST) in cultured cells. Fig. 22A shows phase contrast microscope images for cases with or without addition of SB431542, and FACS analysis results for CD44, CD166, CD24, CD26, and CD105. SB4(+) represents addition of SB431542 and SB4(-) represents no addition of SB431542.
[Fig. 22-B]

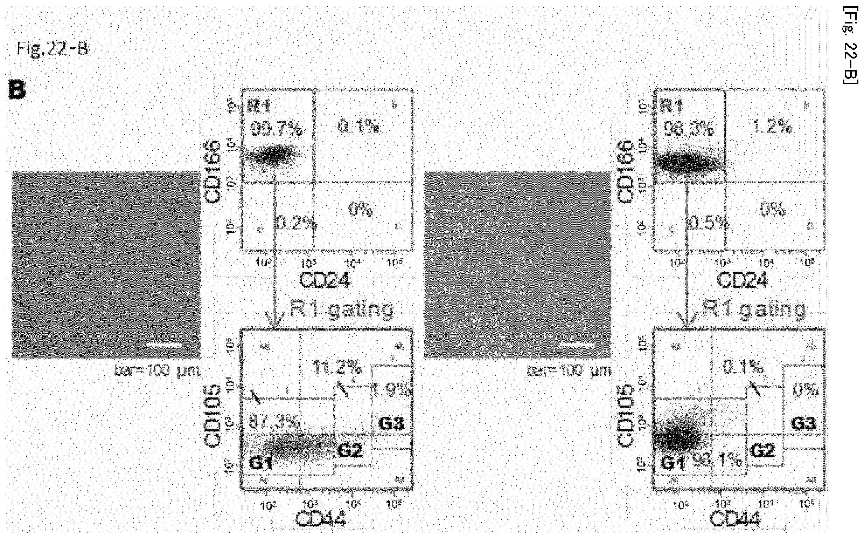

   Fig. 22-B shows phase contrast microscope images and results of FACS gating of cHCECs from two different donors (both 22 years old). For each cHCEC, a phase contrast microscope image is shown on the left and results of FACS gating on the right. Culture was performed without Y-27632. The scale bar indicates 100 .micro.m.
[Fig. 22-C]

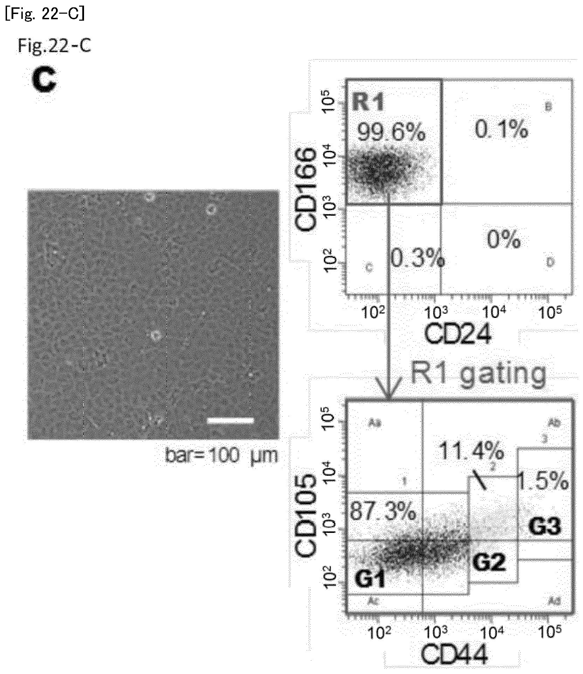

   Fig. 22-C shows results of culturing cells collected from a 71 year old subject by continuously adding Y-27632 throughout the culture period as in Fig. 22-B. A picture of cells is shown on the left and results of FACS gating for CD44, CD166, CD24, CD26, and CD105 are shown on the right. The scale bar indicates 100 .micro.m.
[Fig. 22-D]

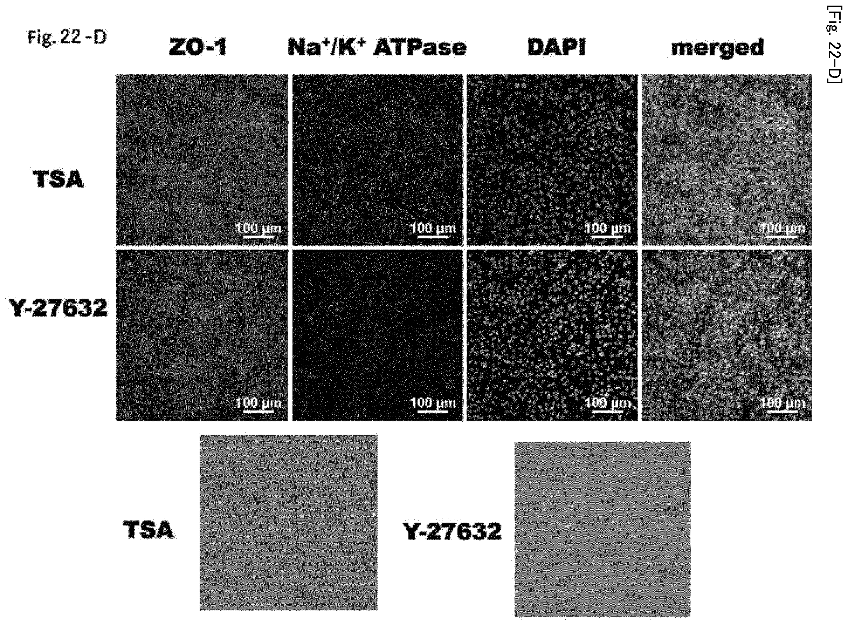

   Fig. 22-D shows representative microscope images of cHCEC treated with two types of agents. In the top diagram, fluorescence microscope images of cHCECs treated with Trichostatin A (TSA) are shown on the top row and those of cHCECs treated with Y-27632 are shown on the bottom row. Fig. 22-D shows, from the left, images of fluorescence of ZO-1, fluorescence of Na+/K+ ATPase, fluorescence of DAPI, and merged images thereof. The bar indicates 100 .micro.m. The bottom diagram shows phase contrast microscope images of cHEHCs treated with Trichostatin A (TSA) or Y-27632, respectively.
[Fig. 23]

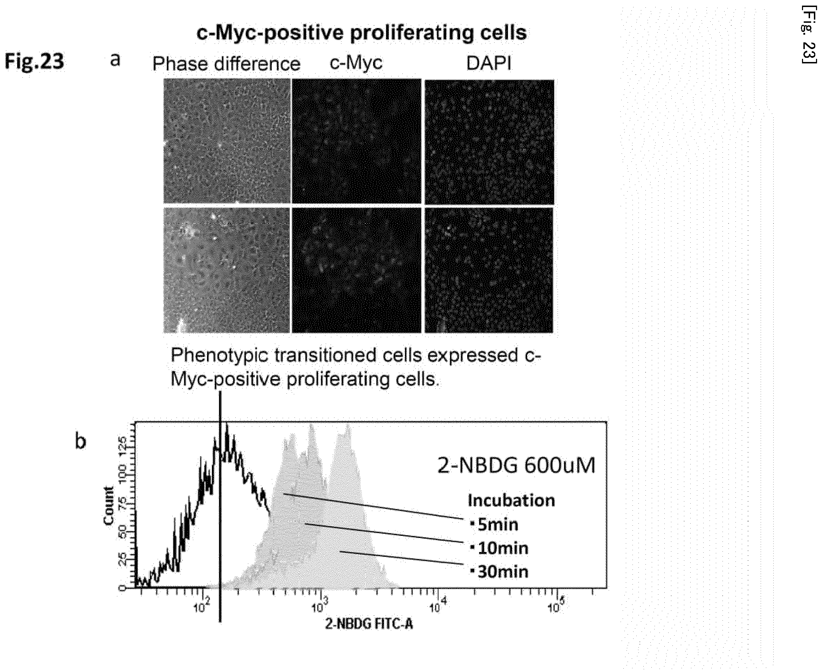

   Fig. 23a shows c-Myc positive phenotypically transitioned cells. The left side shows a phase contrast image, the center part shows fluorescence corresponding to c-Myc, and the right side shows fluorescence of DAPI. The images on the top row and bottom row show microscope images of different portions. Immunocytochemical assessment for c-Myc expression of bulk cultured cHCECs confirmed fluorescence indicating c-Myc expression at the sites of morphologically transformed cell like shapes in a phase contrast microscope. Fig. 23b is a diagram showing glucose uptake of bulk cultured cHCECs by flow cytometry analysis. Detached cHCECs were incubated with 600 .micro.M 2NBDG for 5, 10, 30 minutes at 37.degrees.C. After culture medium was replaced with fresh glucose deleted medium for 15 minutes, the cells were then washed twice with cold FACS buffer (PBS containing 1% BSA), resuspended in ice-cold FACS buffer and subjected to flow cytometry. Samples were analyzed using BD FACS Canto II (BD Biosciences) at FITC range (excitation 490 nm, emission 525 nm band pass filter). The mean fluorescence intensities of different groups were analyzed by BD FACS Diva software and corrected for autofluorescence from unlabeled cells. A single peak of 2-NBDG uptake was exhibited in all incubation times. Fig. 23b shows, from the left, group without incubation, group incubated for 5 minutes, group incubated for 10 minutes, and group incubated for 30 minutes.
[Fig. 24-A]

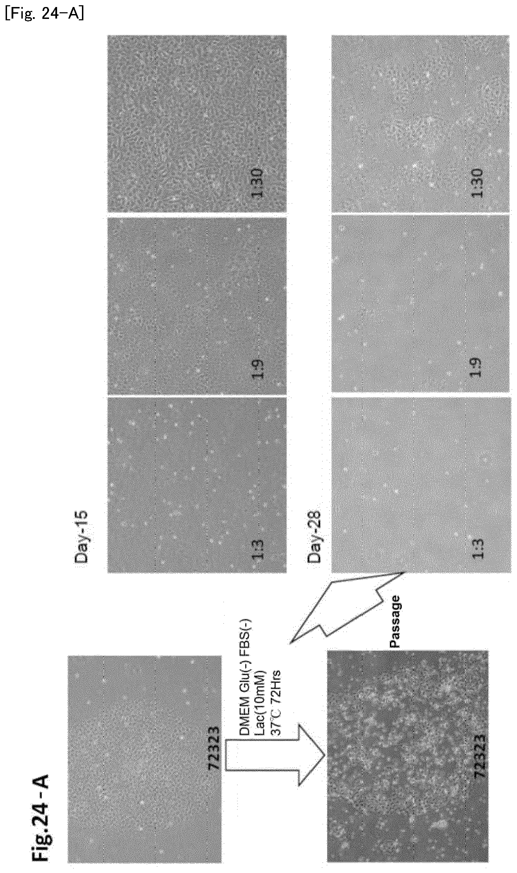

   Fig. 24-A shows morphological changes detected by a phase contrast microscope in cHCEC cultures in glucose starved DMEM without FBS in the presence of lactate. After passage to P3 under normal culture conditions, cultured cells were incubated for 72 hours with DMEM containing 10 mM of lactate and free of glucose. The resultant cHCECs were further cultured at the three distinct dilution passages (1:3, 1:9, 1:30) under normal conditions for 4 weeks.
[Fig. 24-B]

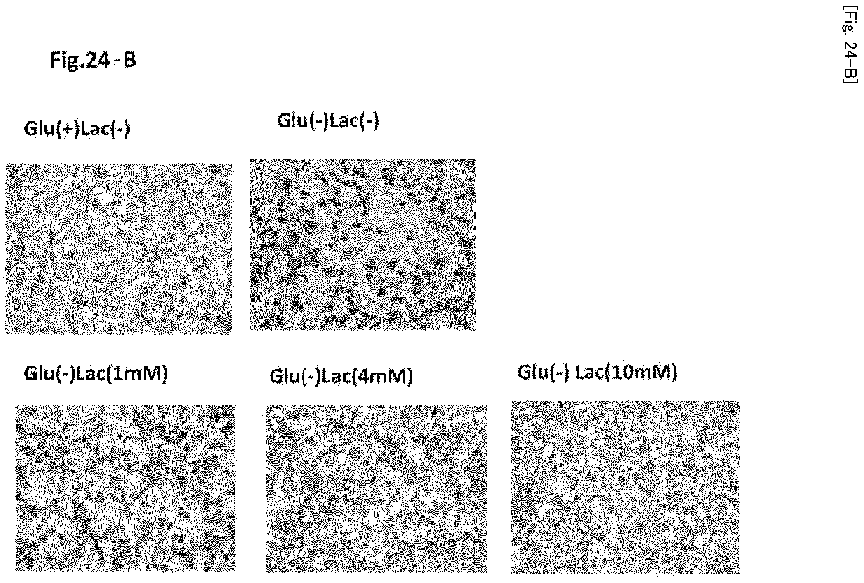

   Fig. 24-B shows immunohistochemical straining of Na⁺/K⁺-ATPase before and after glucose starvation for assessing partial elimination of a cHCEC subpopulation in bulk culture. Na⁺/K⁺-ATPase was used as a function related marker of HCECs. The efficiency of recovery from the effect of the deletion was clearly dependent on the concentration of the added lactate.
[Fig. 25-A]

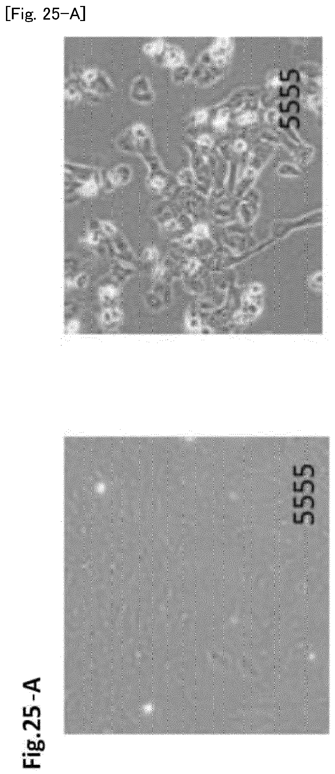

   Fig. 25-A is a picture showing a morphological change of cHCECs (#55) before and after Lac treatment. Left: before Lac treatment. Right: after Lac treatment.
[Fig. 25-B]

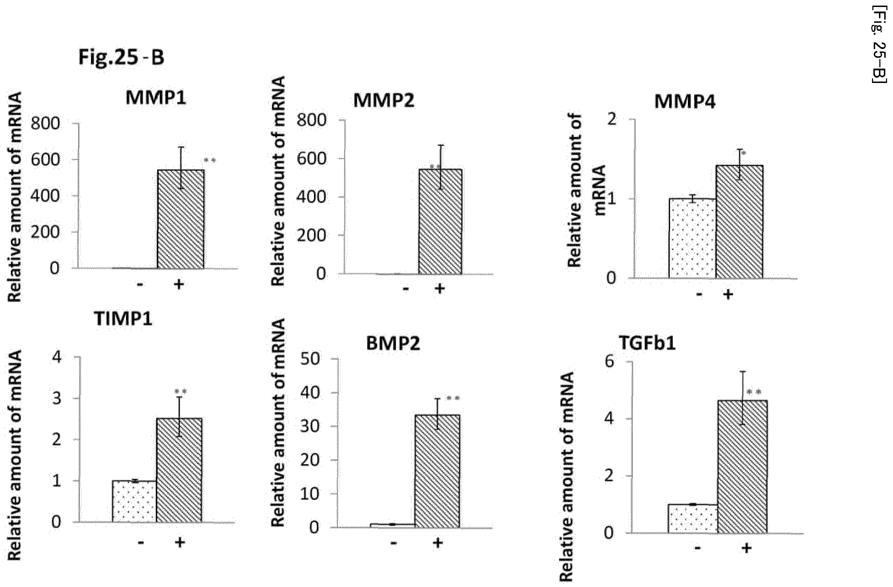

   Fig. 25-B shows results of analyzing the change in expression of a gene associated with CST such as EMT, cell senescence and fibrosis in cHCECs (#55) before and after Lac treatment by quantitative real time PCR. The expression intensity is shown as a relative expression intensity while assuming the expression intensity of each gene before treatment as 1.
[Fig. 25-C]

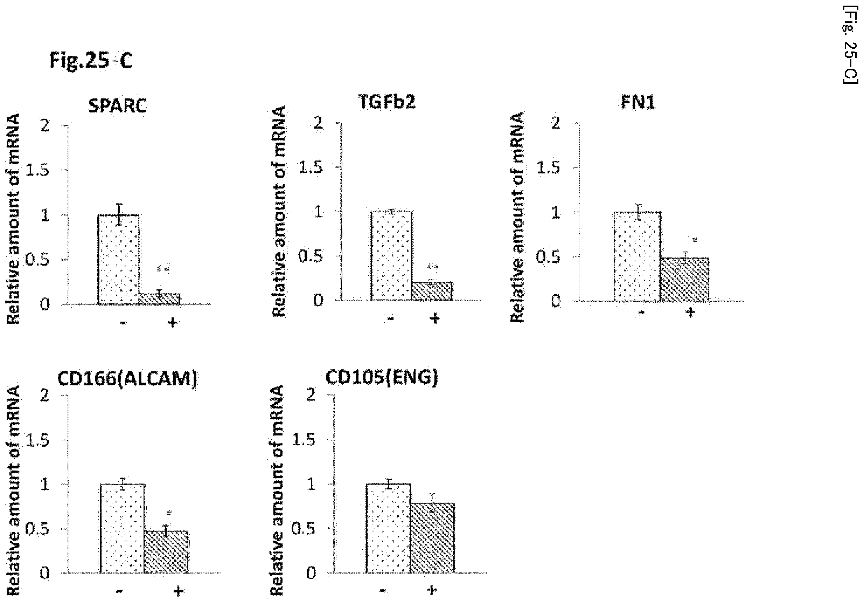

   Fig. 25-C shows results of analyzing the change in expression of a gene associated with CST such as EMT, cell senescence and fibrosis in cHCECs (#55) before and after Lac treatment by quantitative real time PCR. The expression intensity is shown as a relative expression intensity while assuming the expression intensity of each gene before treatment as 1.
[Fig. 25-D]

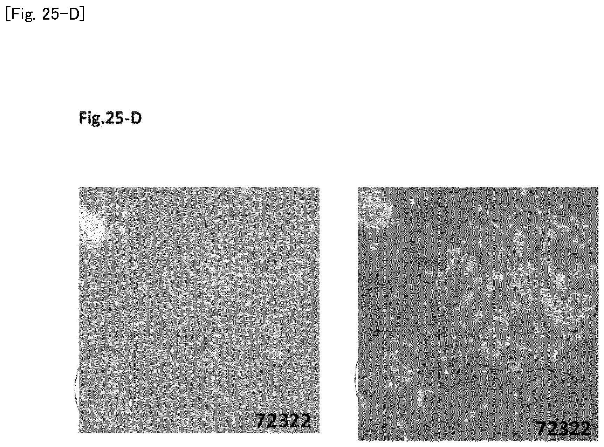

   Fig. 25-D is a diagram showing a morphological change of cHCECs (#72) before and after Lac treatment. Left: before Lac treatment. Right: after Lac treatment.
[Fig. 25-E]

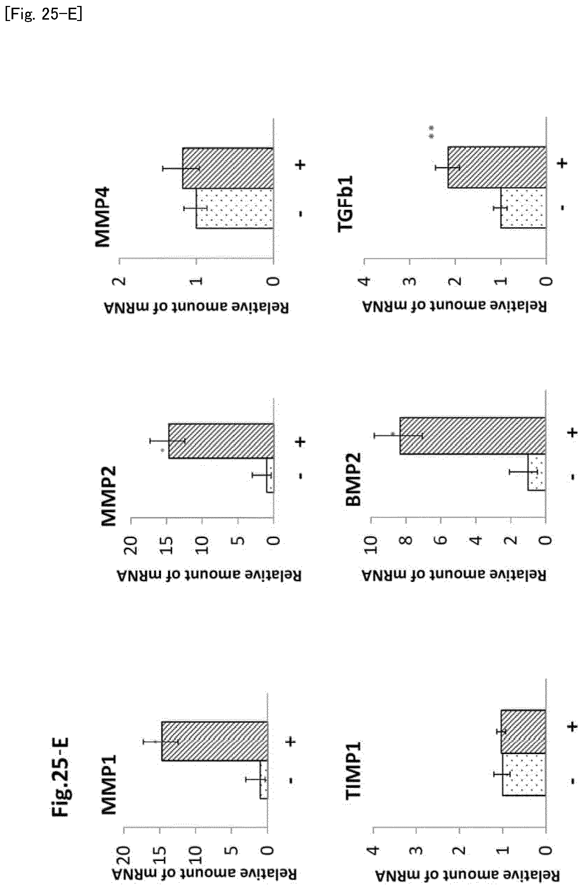

   Fig. 25-E shows results of analyzing the change in expression of a gene associated with CST such as EMT, cell senescence and fibrosis in cHCECs (#72) before and after Lac treatment by quantitative real time PCR. The expression intensity is shown as a relative expression intensity while assuming the expression intensity of each gene before treatment as 1.
[Fig. 25-F]

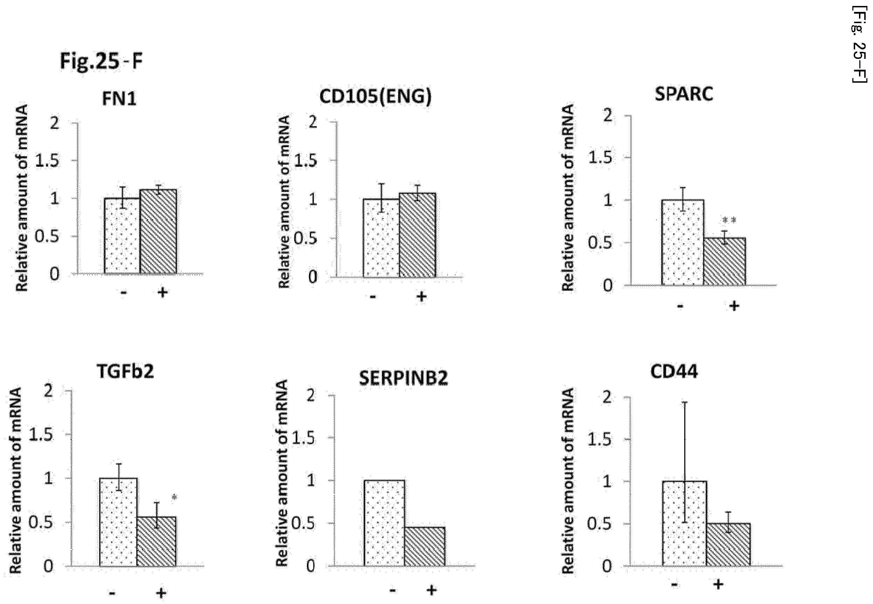

   Fig. 25-F shows results of analyzing the change in expression of a gene associated with CST such as EMT, cell senescence and fibrosis in cHCECs (#72) before and after Lac treatment by quantitative real time PCR. The expression intensity is shown as a relative expression intensity while assuming the expression intensity of each gene before treatment as 1.
[Fig. 25-G]

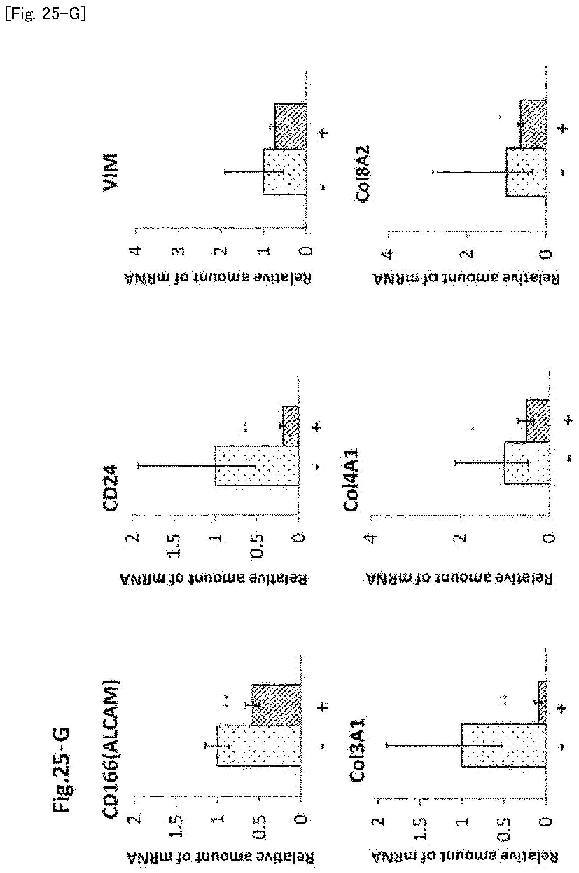

   Fig. 25-G shows results of analyzing the change in expression of a gene associated with CST such as EMT, cell senescence and fibrosis in cHCECs (#72) before and after Lac treatment by quantitative real time PCR. The expression intensity is shown as a relative expression intensity while assuming the expression intensity of each gene before treatment as 1.
[Fig. 25-H]

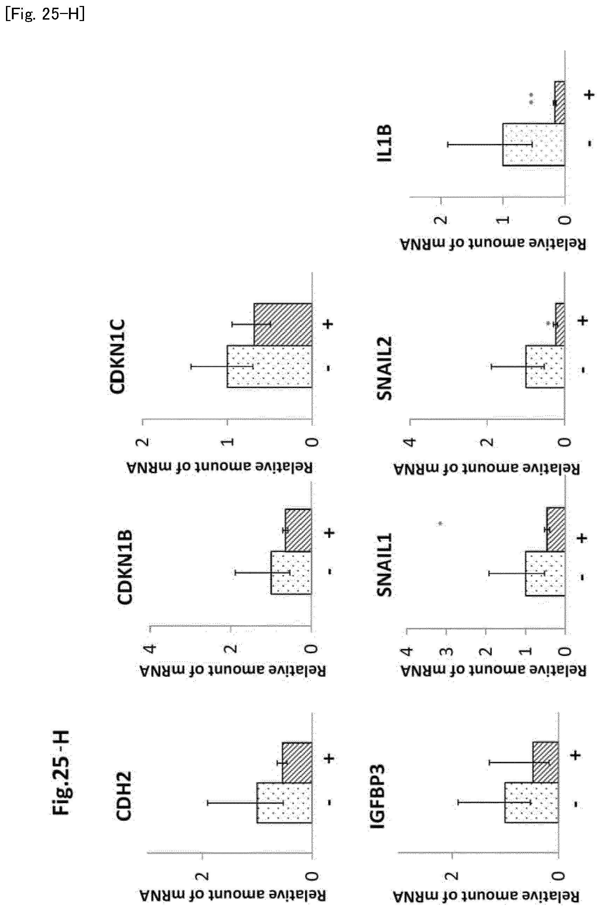

   Fig. 25-H shows results of analyzing the change in expression of a gene associated with CST such as EMT, cell senescence and fibrosis in cHCECs (#72) before and after Lac treatment by quantitative real time PCR. The expression intensity is shown as a relative expression intensity while assuming the expression intensity of each gene before treatment as 1.
[Fig. 26-A]

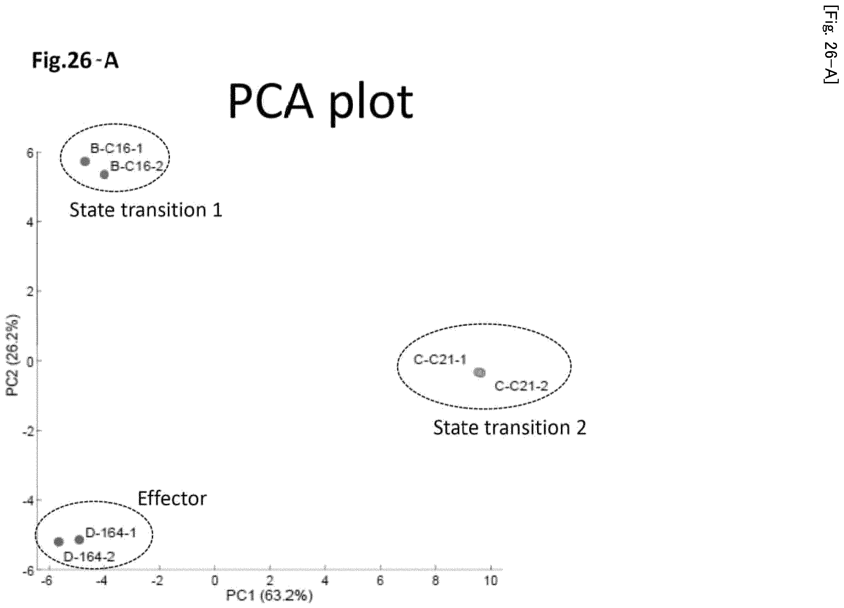

   Fig. 26-A shows relative intracellular metabolite signaling positions in three lots of cHCECs (164P1, C16P6, and C21P3; effector cell, cell state transitioned cell 1, and cell state transitioned cell 2, respectively) as PCA analysis.
[Fig. 26-B]

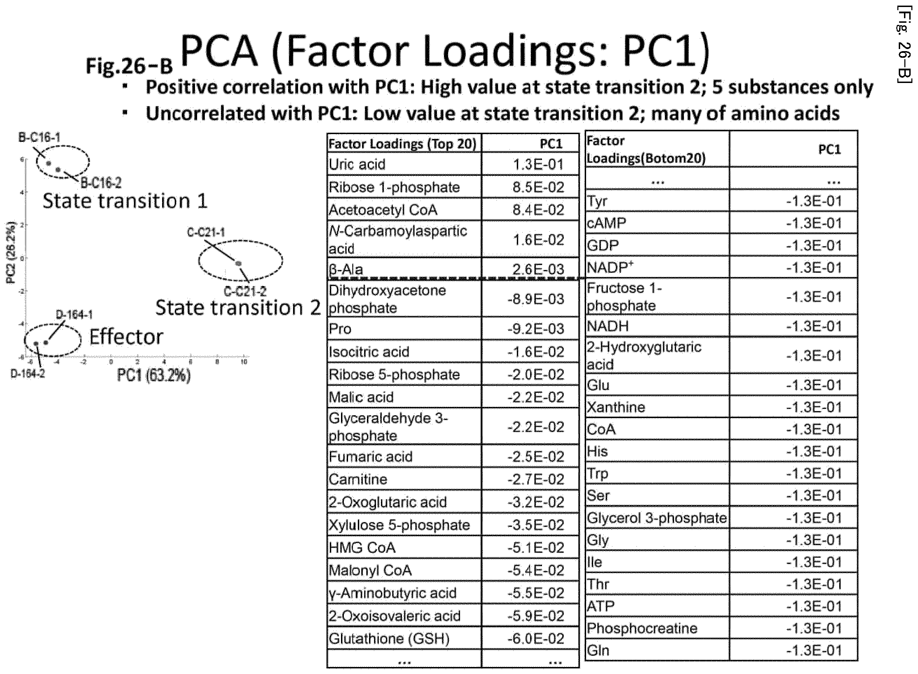

   Fig. 26-B shows typical metabolites in a PC1 component of an intracellular metabolite signal in three lots of cHCECs (164P1, C16P6, and C21P3; effector cell, cell state transitioned cell 1, and cell state transitioned cell 2, respectively).
[Fig. 26-C]

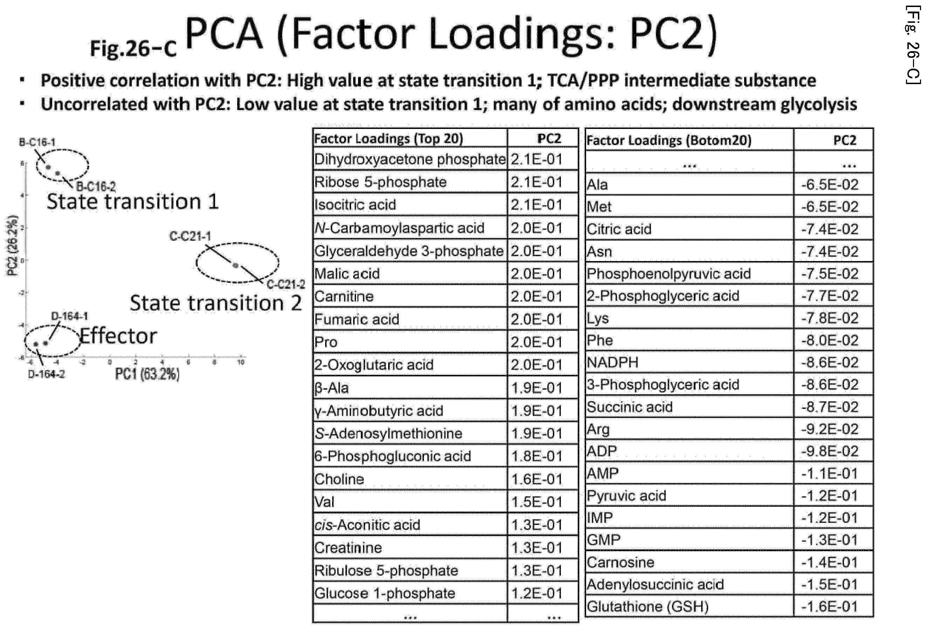

   Fig. 26-C shows typical metabolites in a PC2 component of an intracellular metabolite signal in three lots of cHCECs (164P1, C16P6, and C21P3; effector cell, cell state transitioned cell 1, and cell state transitioned cell 2, respectively).
[Fig. 26-D]

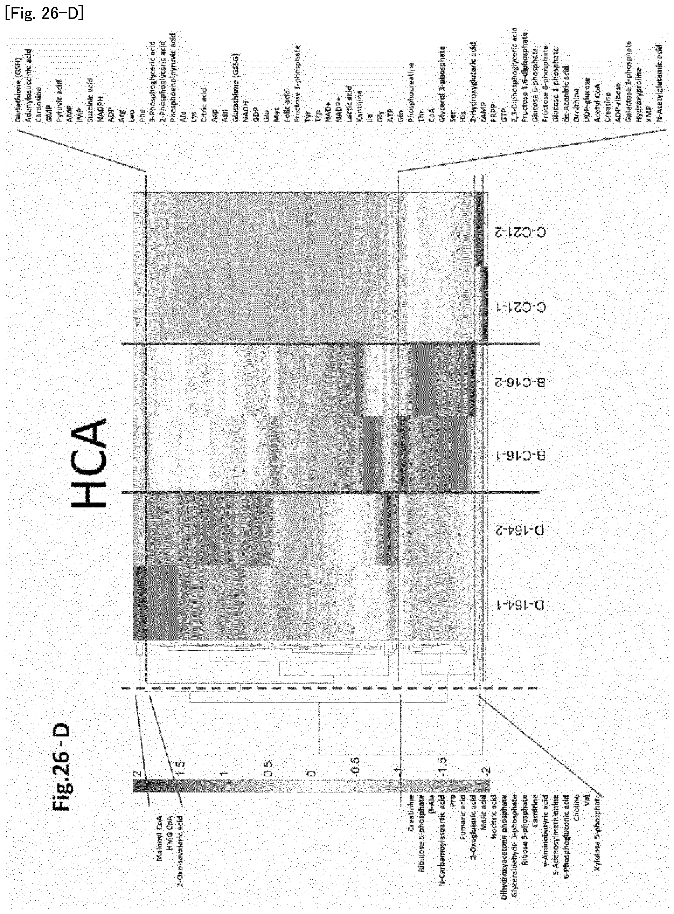

   Fig. 26-D is a diagram showing a hierarchical clustering (HCA) using standardized intensity of each metabolite in two samples each from each lot. High standardized intensity of each metabolite is shown in red and low standardized intensity of each metabolite is shown in green.
[Fig. 26-E]

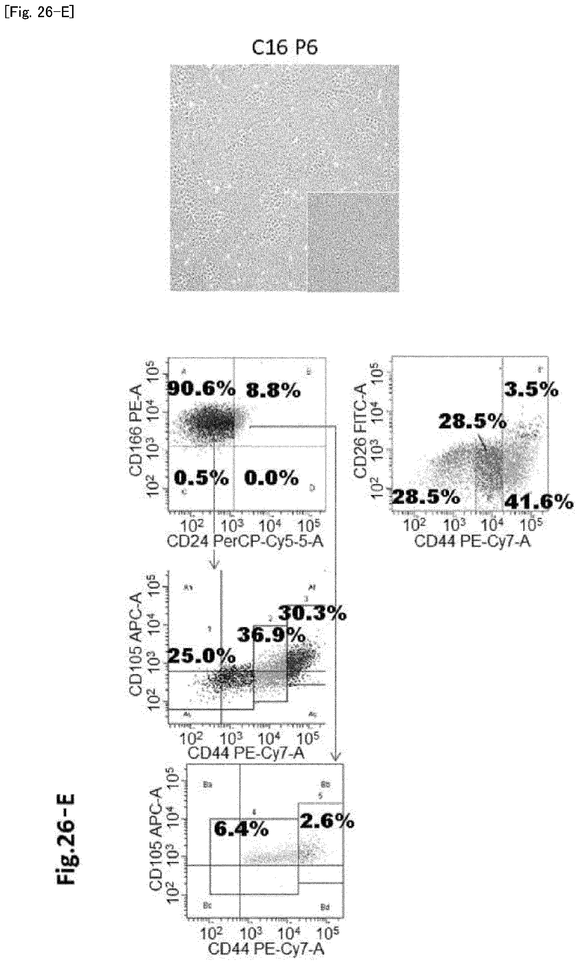

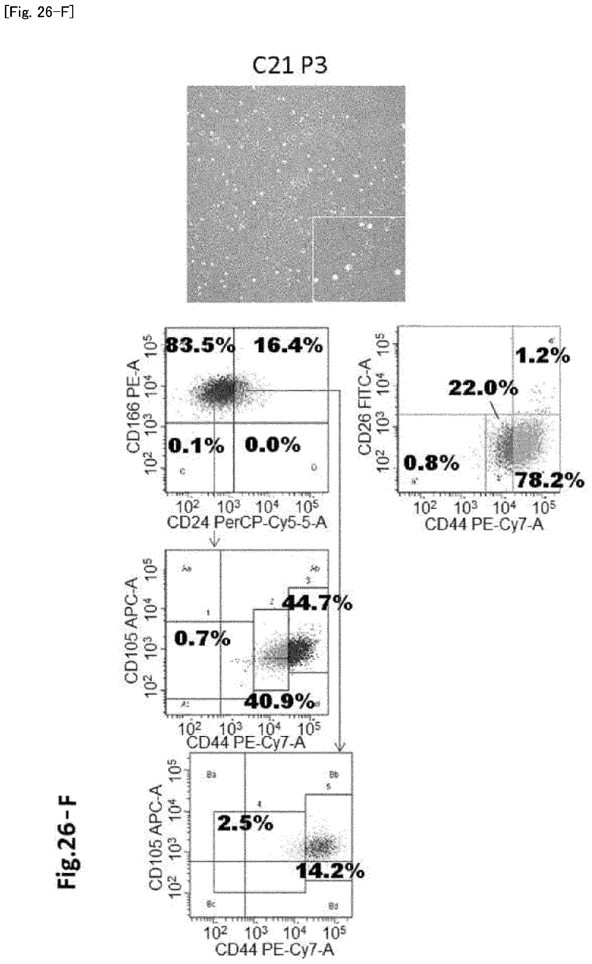

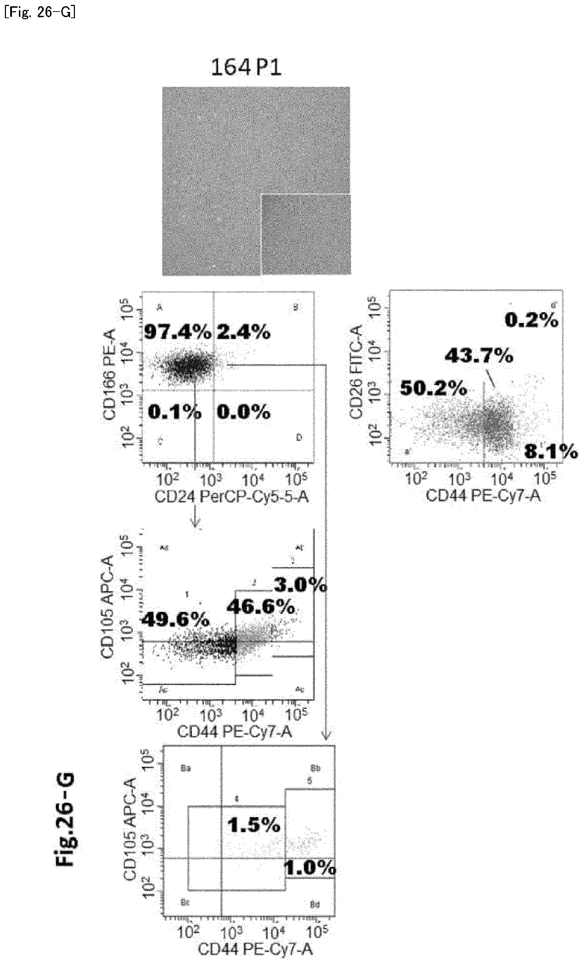

   Figs. 26-E, 26-F, and 26-G are diagrams showing morphological difFerences in three lots of cHCECs with flow cytometry analysis. FACS analysis of each cHCEC is shown in the bottom row. In addition, the subpopulation composition of each lot with cell surface antigen expression classified by the same standard as Fig. 1 is shown.
[Fig. 26-F]
   Figs. 26-E, 26-F, and 26-G are diagrams showing morphological difFerences in three lots of cHCECs with flow cytometry analysis. FACS analysis of each cHCEC is shown in the bottom row. In addition, the subpopulation composition of each lot with cell surface antigen expression classified by the same standard as Fig. 1 is shown.
[Fig. 26-G]
   Figs. 26-E, 26-F, and 26-G are diagrams showing morphological difFerences in three lots of cHCECs with flow cytometry analysis. FACS analysis of each cHCEC is shown in the bottom row. In addition, the subpopulation composition of each lot with cell surface antigen expression classified by the same standard as Fig. 1 is shown.
[Fig. 26-H]

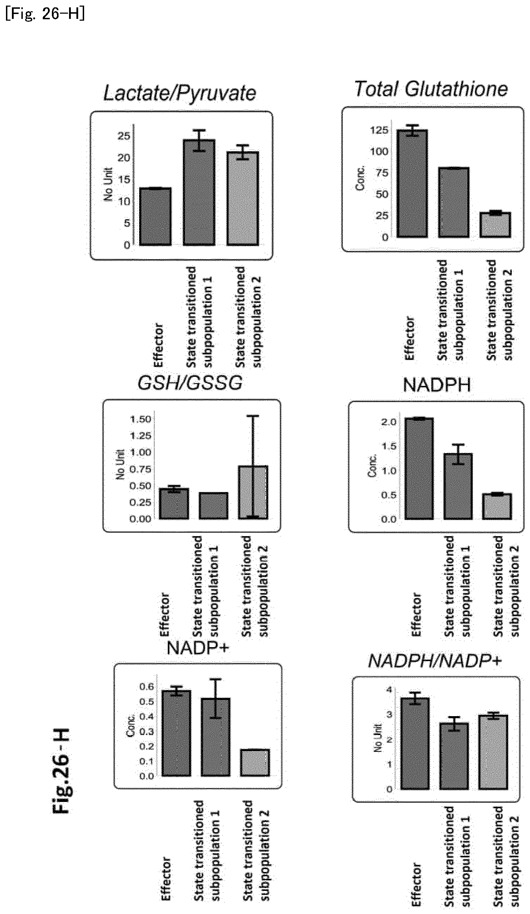

   Fig. 26-H is a graph showing the amounts of metabolites regulating intracellularly relevant physiological functions and lactate/pyruvate ratio in each cHCEC lot. Examples of markers include GSH/GSSG, total glutathione, NADP+, NADPH, and NADPH/NADP.
[Fig. 27-A]

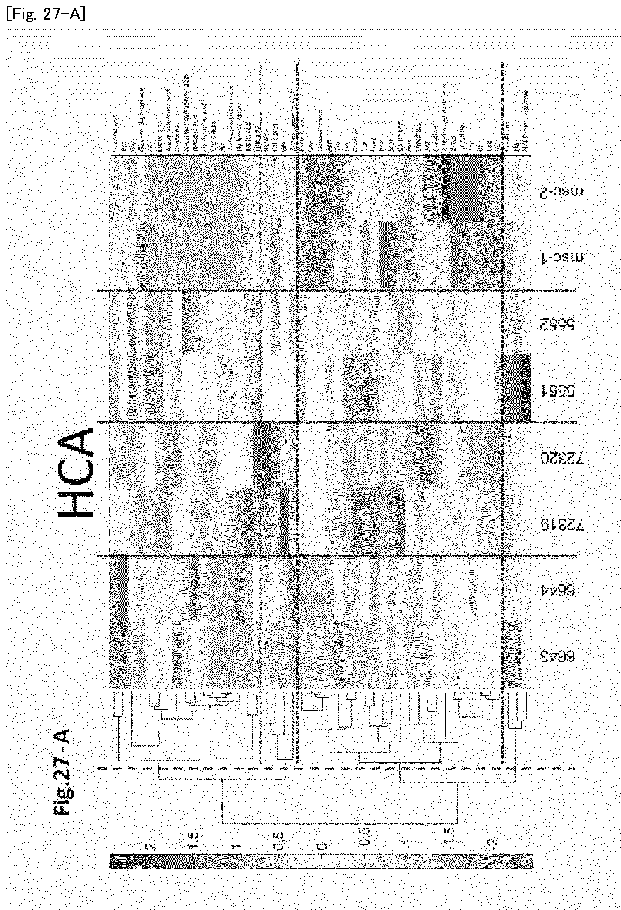

   Fig. 27-A shows a characteristic assessment of a metabolite change in different lot of cHCECs cultured in conditioned medium. Fig. 27-A shows hierarchical clustering of metabolomic profile. A cluster of metabolites correlating with the presence of CST was identified. High intensity of each metabolite is shown in red and low intensity is shown in green. The cluster was divided into at least 4 metabolite subclusters: from the top, metabolite increased in all cHCECs (#66, #72, and #55), metabolite increased in mainly #72 and #55 but not in #66, metabolite that most notably decreased in #66, and metabolite that is generally present in the three groups.
[Fig. 27-B]

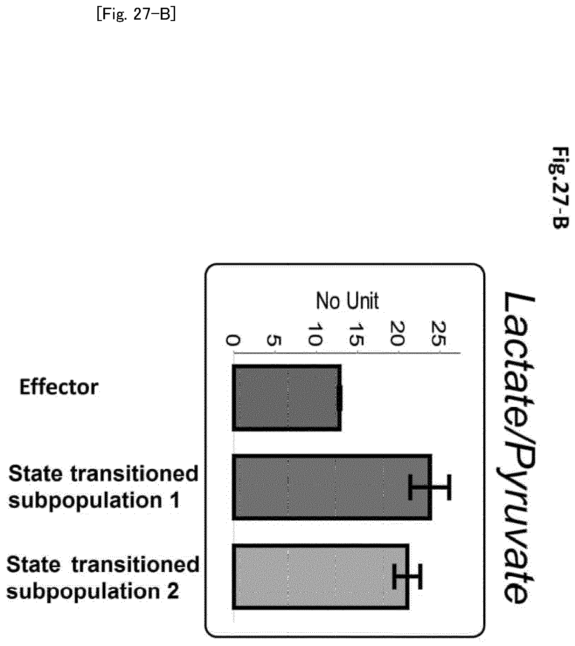

   Fig. 27-B is a graph showing that the lactate/pyruvate ratio is higher in cell state transitioned 1 and 2 cells than in effector cells.
[Fig. 27-C]

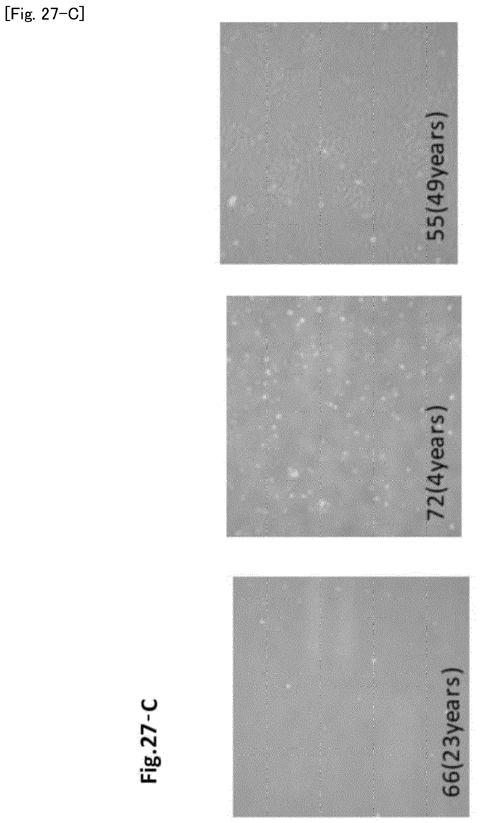

   Fig. 27-C provides microscope images showing the morphology of #66, #72, and #55.
[Fig. 28-A]

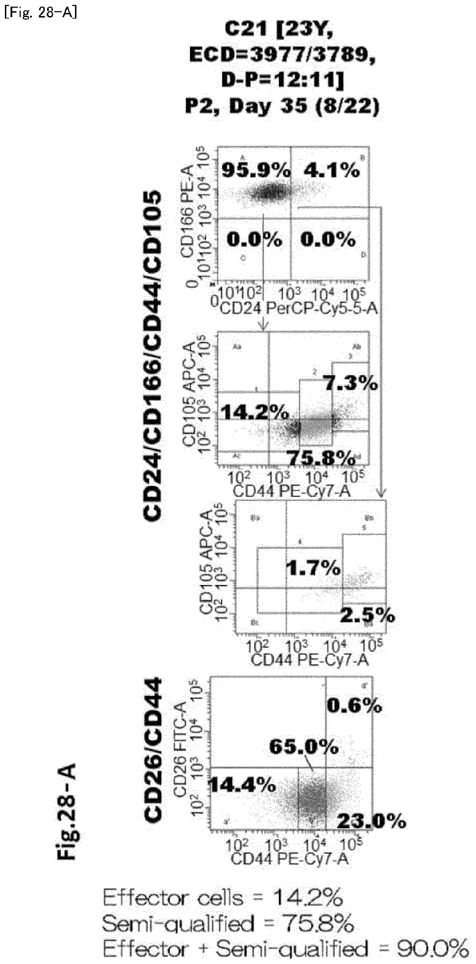

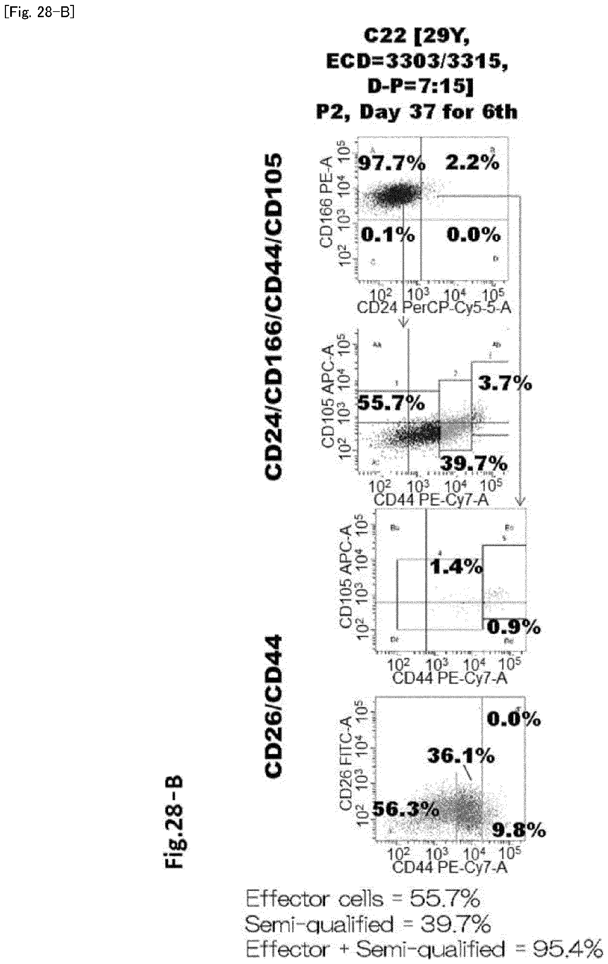

   Figs. 28-A and 28-B show results of FACS analysis on cHCECs without CD44+++ cell, CD24+ cell, or CD26+ cell as four distinct cHCECs with different subpopulation compositions produced under the GMP condition. The baseline values of the expression intensity of CD166, CD24, CD26, CD44, and CD105 were as described above. Figs. 28-A show each of the composition of effector cells, intermediately differentiated cells, non-intended cells in each lot based on their surface CD expression.
[Fig. 28-B]
   Figs. 28-A and 28-B show results of FACS analysis on cHCECs without CD44+++ cells, CD24+ cells, or CD26+ cells as four distinct cHCECs with different subpopulation compositions produced under the GMP condition. The baseline values of the expression intensity of CD166, CD24, CD26, CD44, and CD105 were as described above. Figs. 28-B show each of the composition of effector cells, intermediately differentiated cells, non-intended cells in each lot based on their surface CD expression.
[Fig. 28-C]

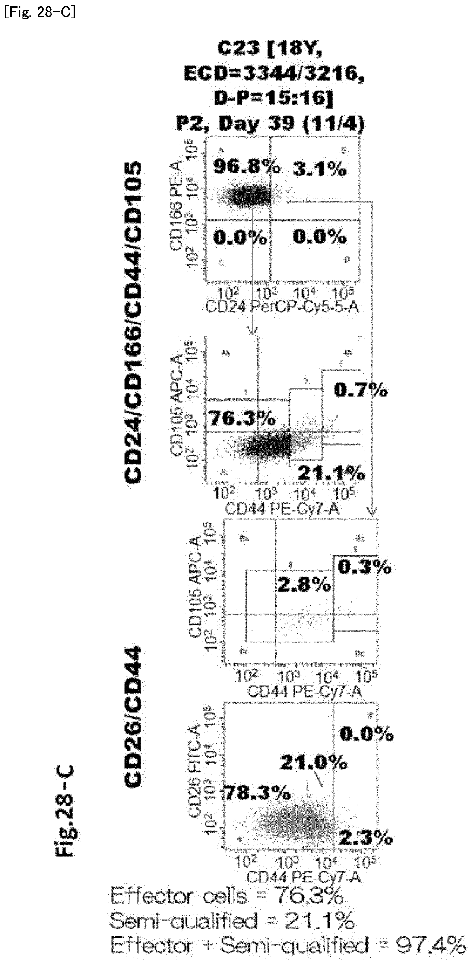

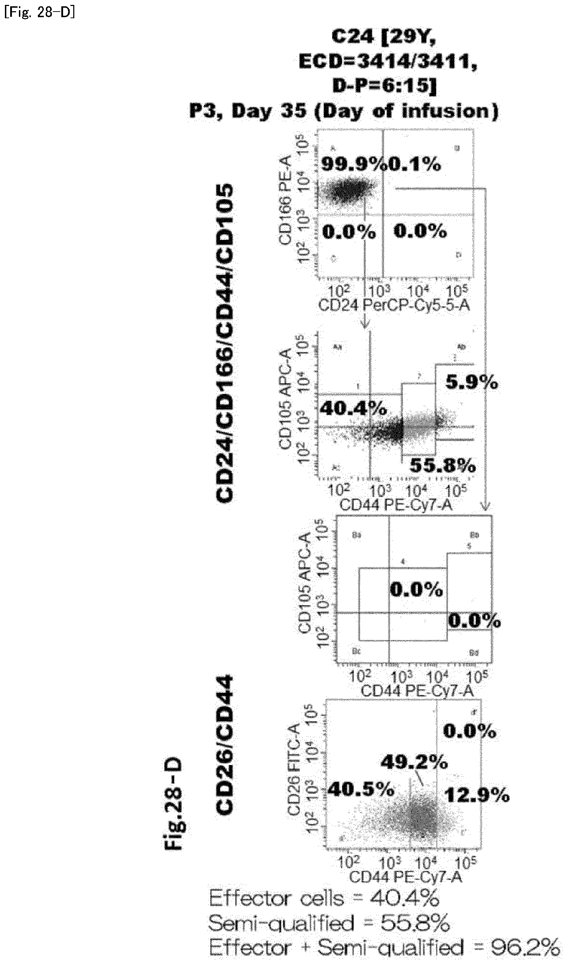

   Figs. 28-C and 28-D show results of FACS analysis on cHCECs without CD44+++ cells, CD24+ cells, or CD26+ cells as four distinct cHCECs with different subpopulation compositions produced under the GMP condition. The baseline values of the expression intensity of CD166, CD24, CD26, CD44, and CD105 were as described above. Figs.28C show each of the composition of effector cells, intermediately differentiated cells, non-intended cells in each lot based on their surface CD expression.
[Fig. 28-D]
   Figs. 28-C and 28-D show results of FACS analysis on cHCECs without CD44+++ cells, CD24+ cells, or CD26+ cells as four distinct cHCECs with different subpopulation compositions produced under the GMP condition. The baseline values of the expression intensity of CD166, CD24, CD26, CD44, and CD105 were as described above. Figs.28-D show each of the composition of effector cells, intermediately differentiated cells, non-intended cells in each lot based on their surface CD expression.
[Fig. 28-E]

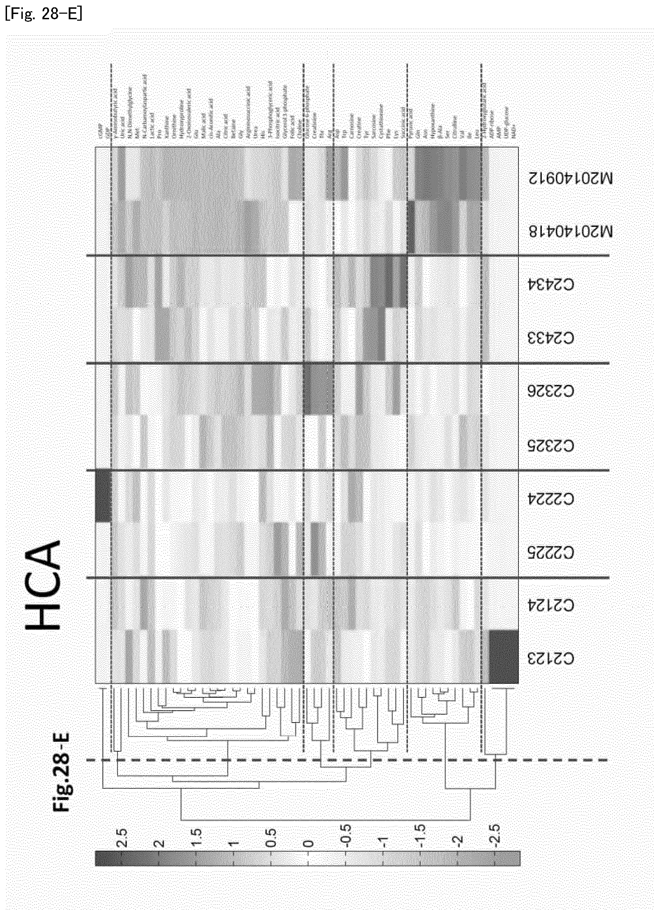

   Fig. 28-E shows hierarchical clustering for four cHCECs and conditioned media with different subpopulation compositions. High intensity of each metabolite is shown in red and low standardized intensity of each metabolite is shown in green. The cluster was divided into four metabolite subclusters.
[Fig. 28-F]

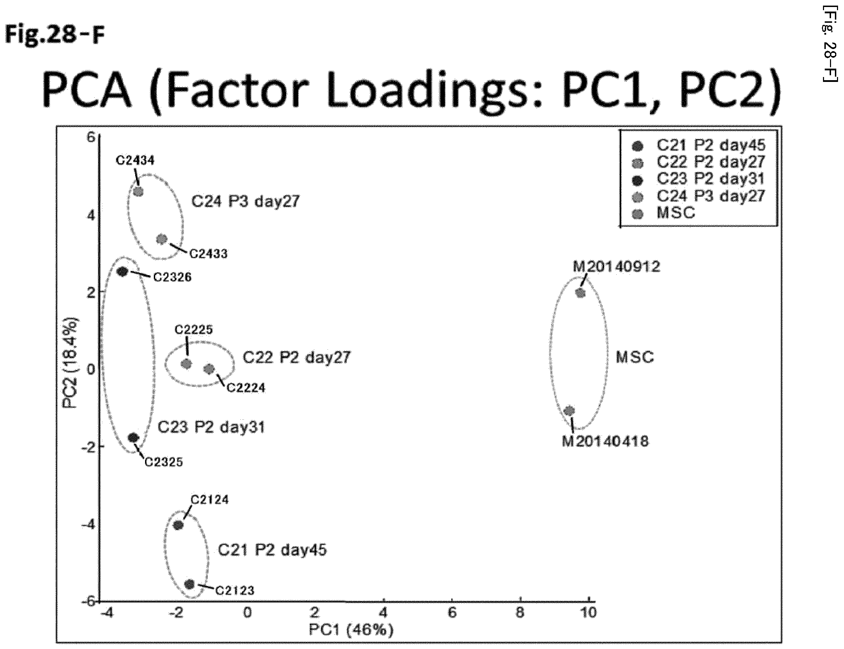

   Fig. 28-F shows PCA analysis for four cHCECs and conditioned media with different subpopulation composition.
[Fig. 28-G]

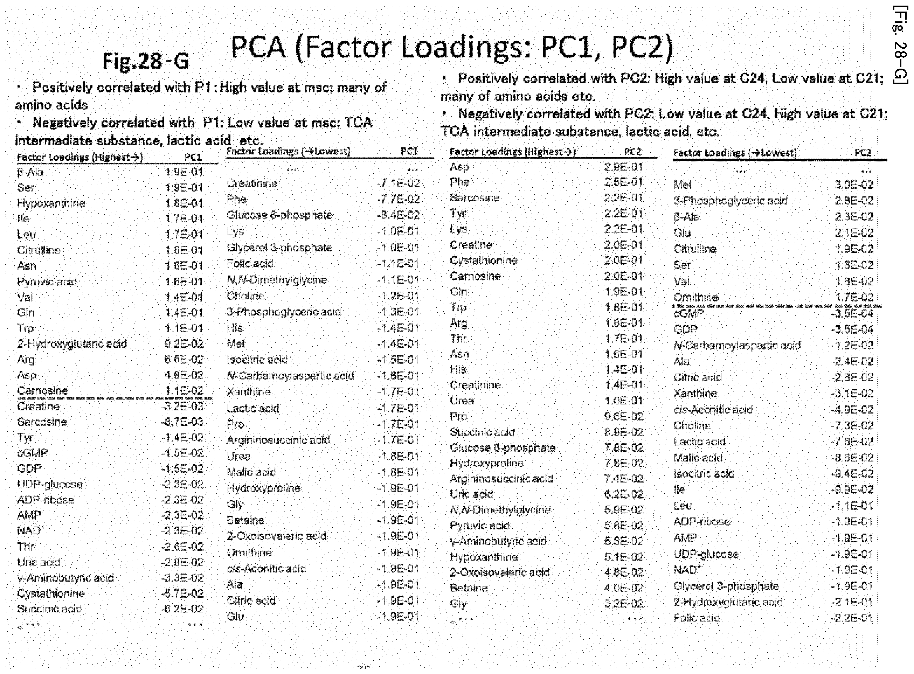

   Fig. 28-G shows main metabolites correlating with PC1 and PC2 in PCA analysis for four cHCECs with different subpopulation compositions.
[Fig. 28-H]

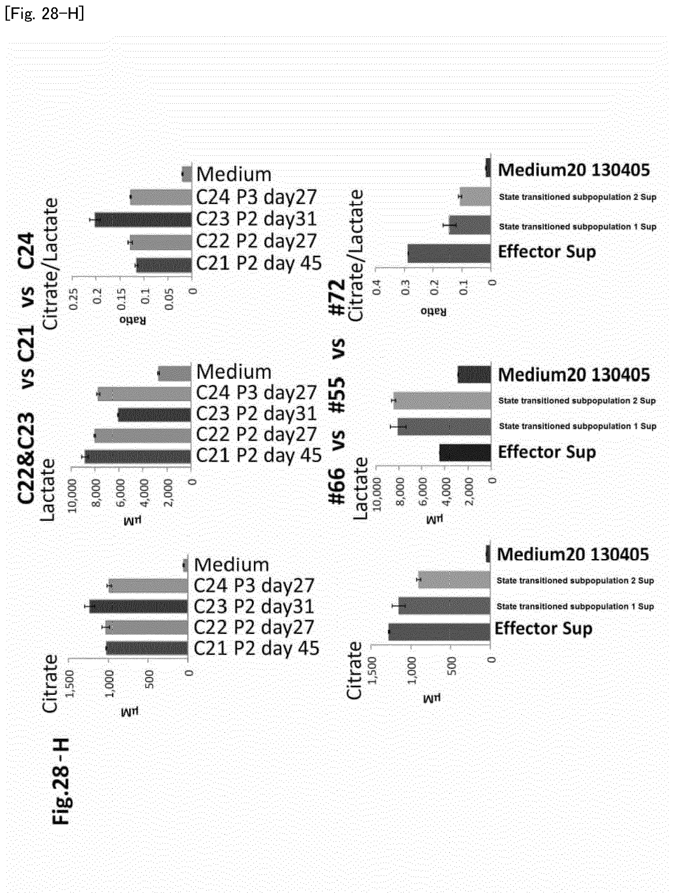

   Fig. 28-H shows, in the top row, the difference in the citrate/lactate ratio among C21, C22, C23, and C24, which are different only in the proportion of CD44- to CD44+ vs. CD44++, and shows, in the bottom row, the difference in the citrate/lactate ratio among #66, #55, and #72, which are different in the CD44+++ subpopulation content.
[Fig. 29-A]

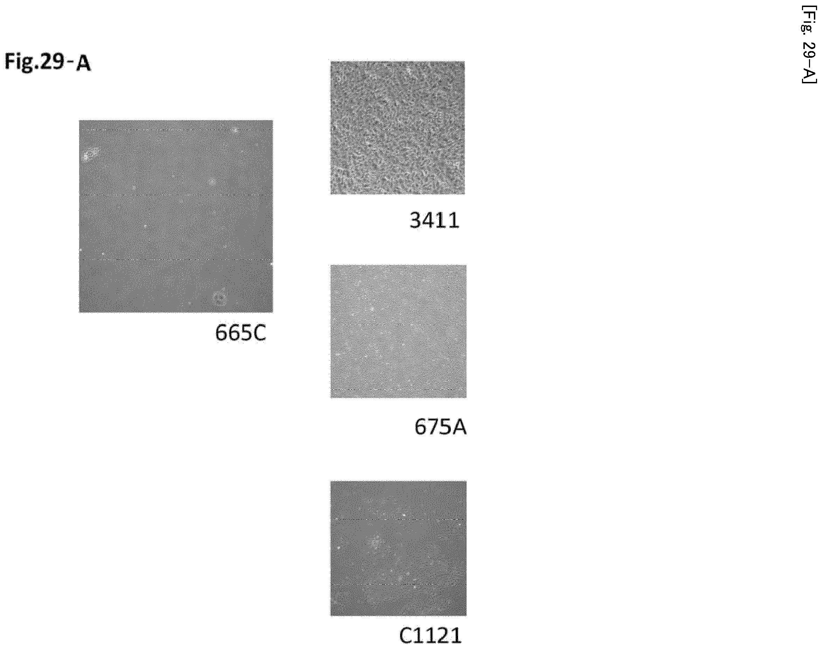

   Fig. 29-A shows microscope images of 665C (effector cell), 3411 (culture lot with unknown constituent culture cell subpopulation), 675A (culture lot comprised of a culture cell subpopulation with morphologically recognized cell state transition), and C1121 (cell with state transition comprising an island cluster (assumed to be senescent cell)).
[Fig. 29-B]

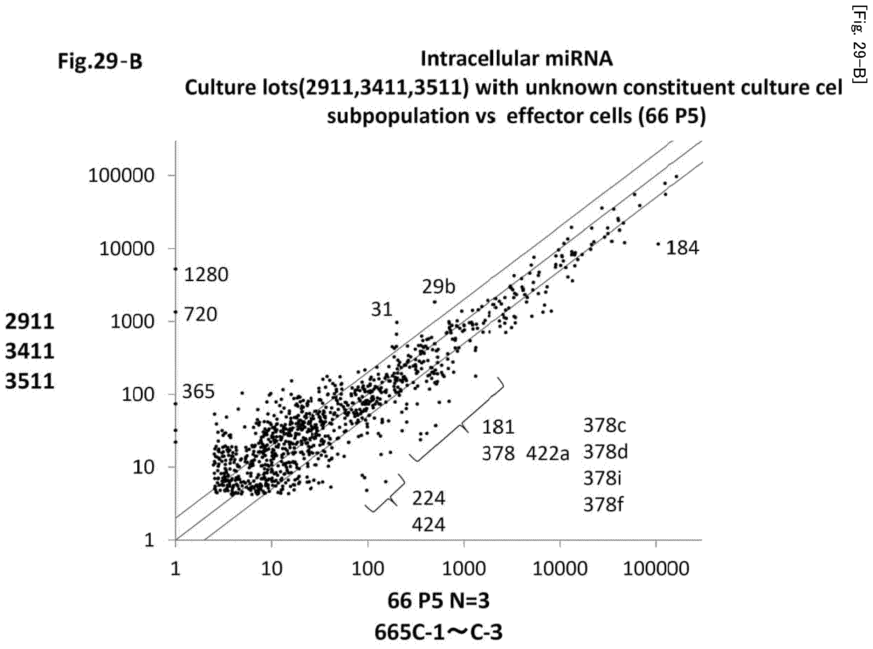

   Fig. 29-B is a scatter plot of relative amounts of various intracellular miR for comparing intracellular miR profiles detected using 3D gene (Toray) between effector cells (#66 P5) and cHCECs with unknown constituent cell subpopulation (2911, 3411, and 3511). The horizontal axis is the relative amount of miR in effector cells. The vertical axis is the relative amount of miR in the culture lot with unknown constituent subpopulation.
[Fig. 29-C]

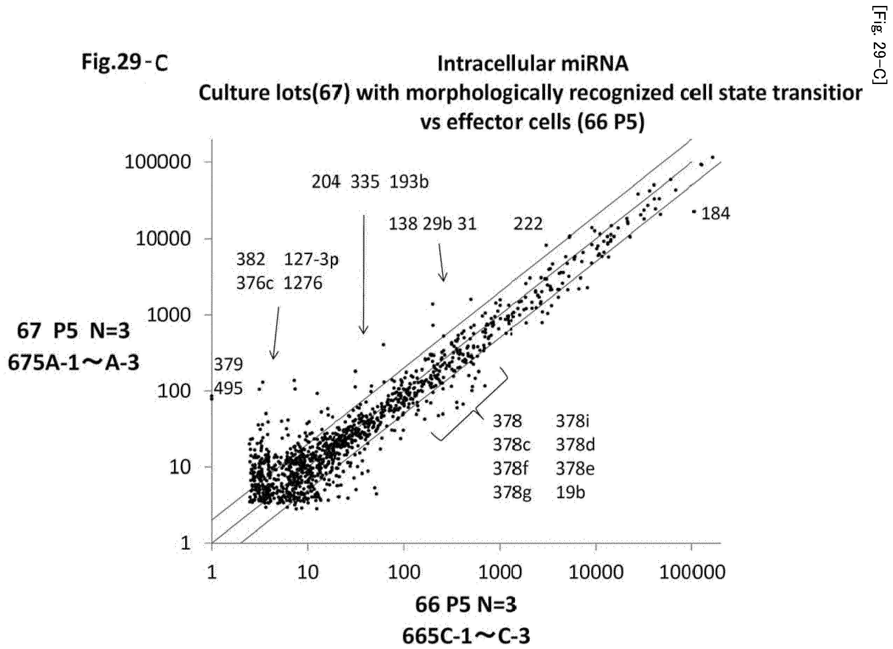

   Fig. 29-C is a scatter plot of relative amounts of various intracellular miR profiles detected using 3D gene (Toray) between effector cells (#66 P5) and cHCECs comprised of a culture cell subpopulation with morphologically recognized cell state transition (675A1-A3). The horizontal axis is the relative amount of miR in effector cells. The vertical axis is the relative amount of miR in the culture lot comprised of a cHCEC subpopulation with morphologically recognized cell state transition.
[Fig. 29-D]

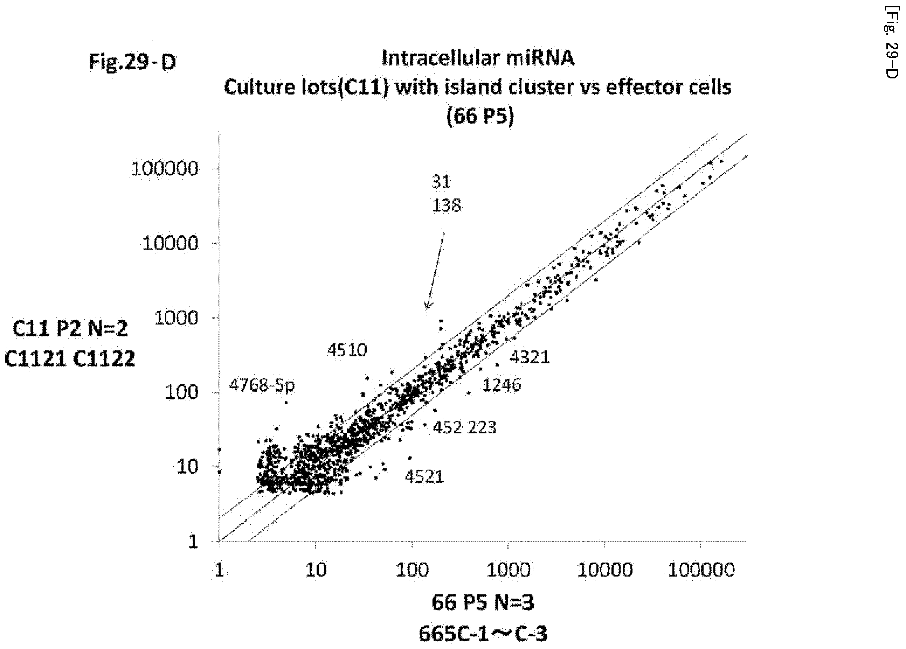

   Fig. 29-D is a scatter plot of relative amounts of various intracellular miR profiles detected using 3D gene (Toray) between effector cells (#66 P5) and cells with cell state transition comprising an island cluster (C1121 and C1122). The horizontal axis is the relative amount of miR in effector cells. The vertical axis is the relative amount of miR in the cells with cell state transition comprising an island cluster.
[Fig. 30-A]

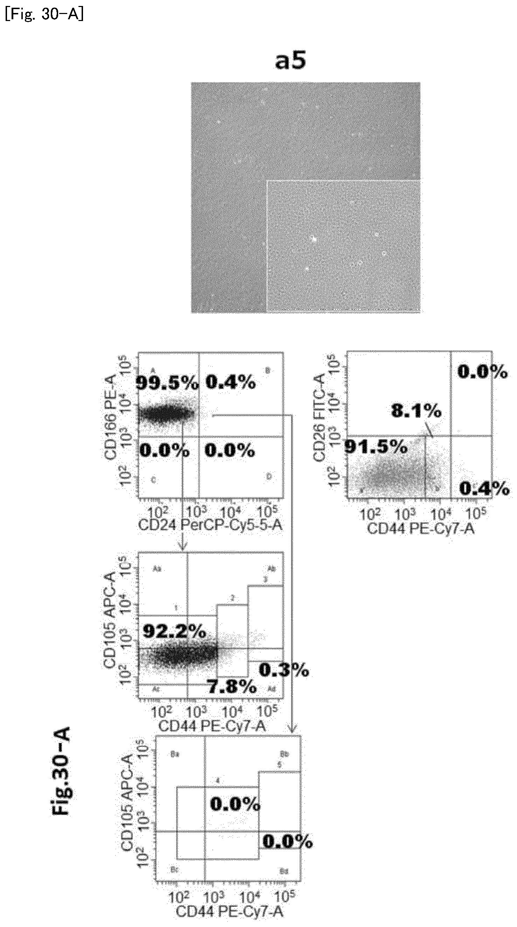

   Fig. 30-A shows a subpopulation composition based on CD expression measured by FACS of a5 subjected to 3D gene analysis. The image on the top row shows the morphology of a5, and the images on the bottom row show CD expression measured by FACS. The baseline values of the expression intensity of CD166, CD24, CD26, CD44, and CD105 were as described above. a5 contains a subpopulation comprised of mainly CD44-CD24-CD26-.
[Fig. 30-B]

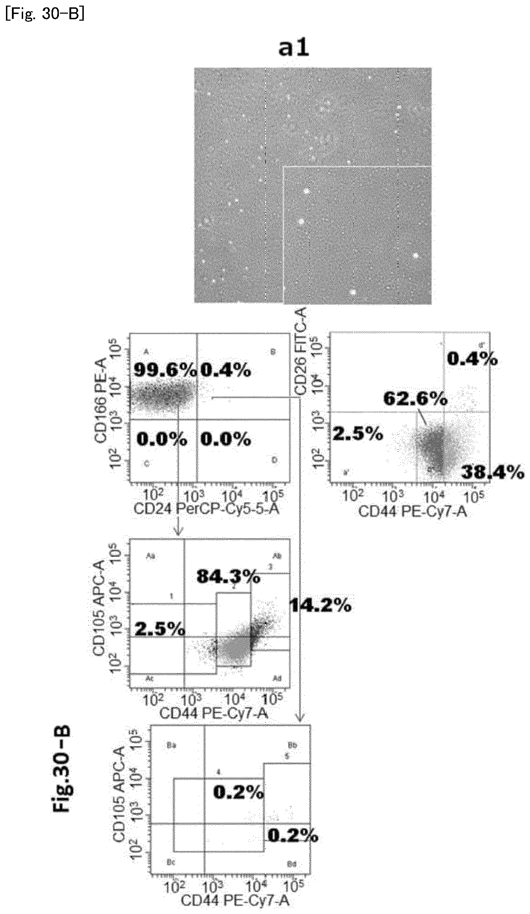

   Fig. 30-B shows a subpopulation composition based on CD expression measured by FACS of a1 subjected to 3D gene analysis. The image on the top row shows the morphology of a1, and the images on the bottom row show CD expression measured by FACS. The baseline values of the expression intensity of CD166, CD24, CD26, CD44, and CD105 were as described above. a1 contains a subpopulation comprised of mainly CD44++CD24-CD26-.
[Fig. 30-C]

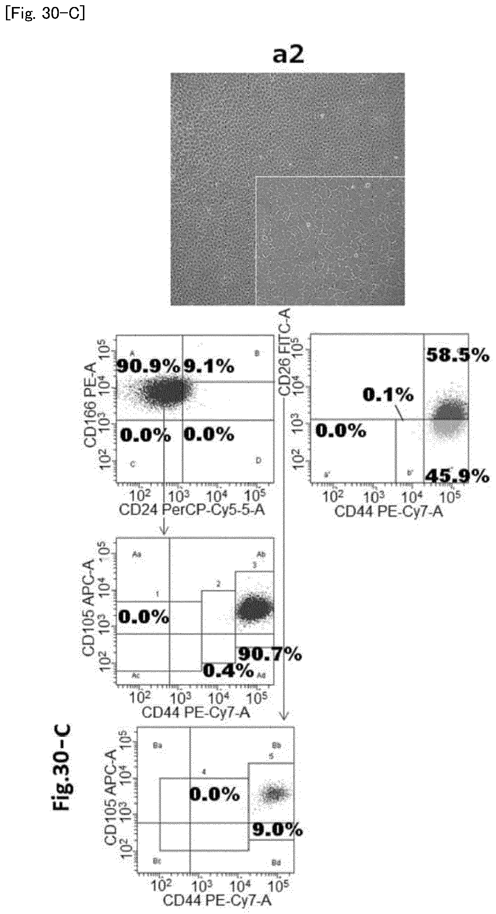

   Fig. 30-C shows a subpopulation composition based on CD expression measured by FACS of a2 subjected to 3D gene analysis. The image on the top row shows the morphology of a2, and the images on the bottom row show CD expression measured by FACS. The baseline values of the expression intensity of CD166, CD24, CD26, CD44, and CD105 were as described above. a2 contains a subpopulation comprised of mainly CD44+++CD24-CD26++.
[Fig. 31-A]

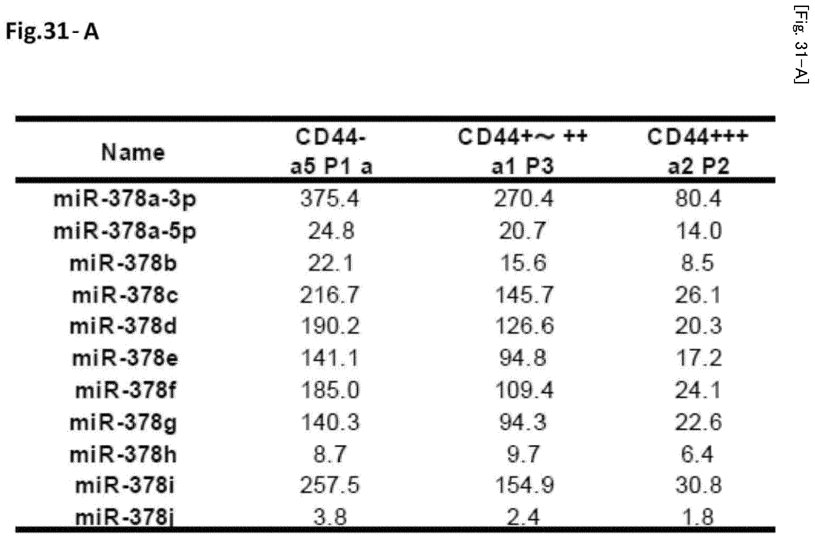

   Fig. 31-A is a table summarizing the relative expression intensity of various intracellular miR of the miR378 family in a5, a1 and a2 cells.
[Fig. 31-B]

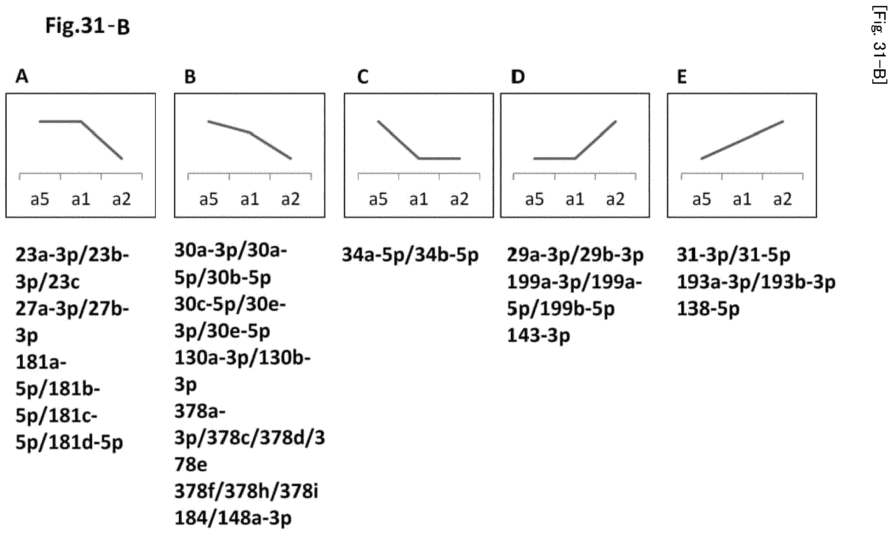

   Fig. 31-B shows the classification of intracellular miRNA into five classes by the change in expression intensity among each cell. The expression of each intracellular miR is classified as displayed under each graph.
[Fig. 32-A]

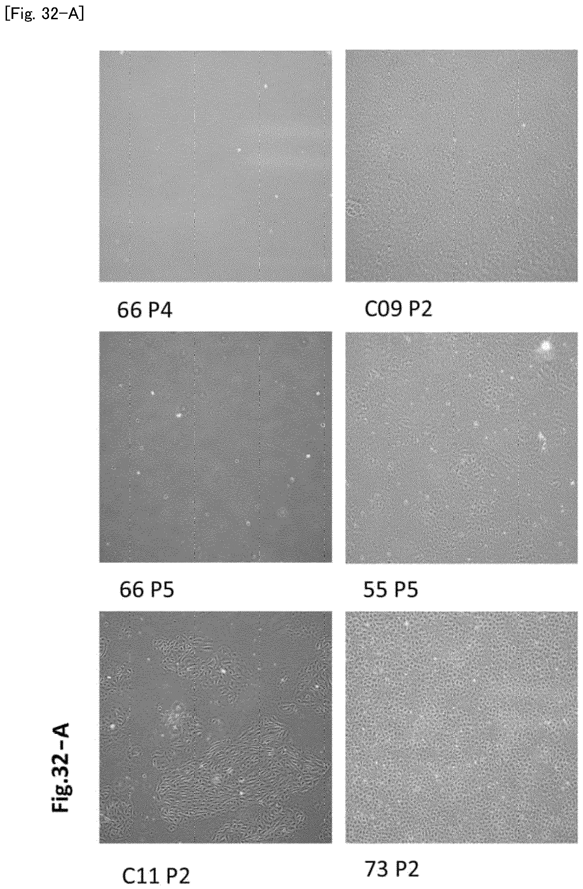

   Fig. 32-A is a picture showing the morphology of #66 P4 (effector cell, fourth passage), #66 P5 (effector cell, fifth passage), C11 P2 (second passage), C09 P2 (second passage), #55 P5 (fifth passage) and #73 P2 (second passage).
[Fig. 32-B]

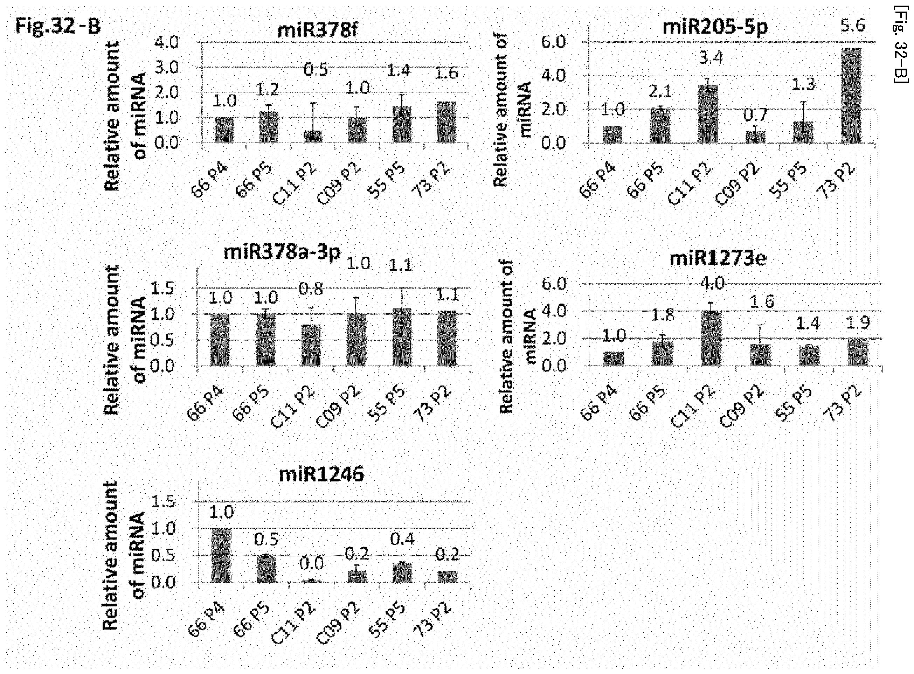

   Fig. 32-B is a diagram showing the relative expression intensity of miR in culture supernatant of each cell of #66 P4 (effector cell, fourth passage), #66 P5 (effector cell, fifth passage), C11 P2 (second passage), C09 P2 (second passage), #55 P5 (fifth passage) and #73 P2 (second passage). The secreted miR shows a pattern of change in expression that is characteristic for each cell. The relative expression intensity is represented with the expression intensity in #66 P4 (effector cell, fourth passage) as 1.
[Fig. 33-A]

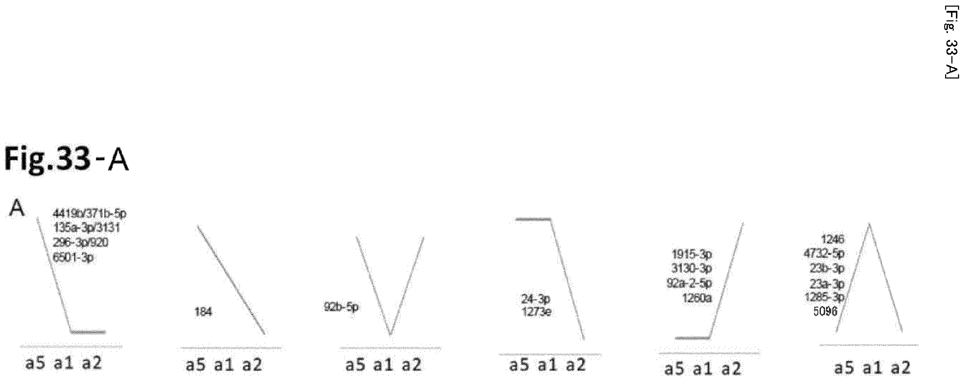

   Figs. 33-A to 33-B show a pattern of several secreted miR having a clear tendency of expression for each cell. Fig. 33-A shows a summary of a change in a secreted miR expression pattern.
[Fig. 33-B]

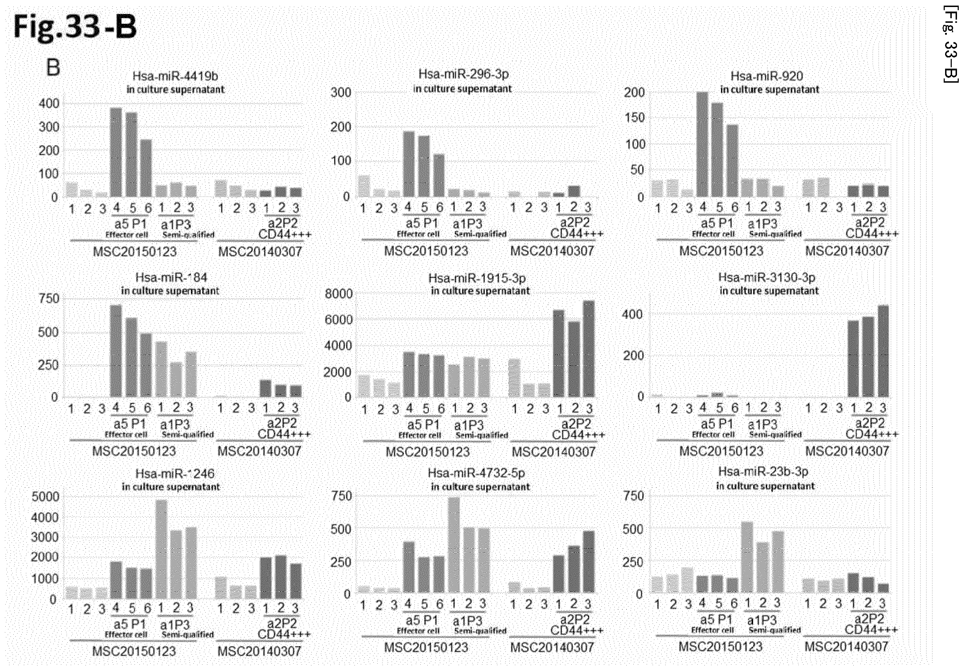

   Figs. 33-A to 33-B show a pattern of several secreted miR having a clear tendency of expression for each cell. Fig. 33-B shows a graph indicating the specific expression intensity for each cell of secreted miR which belongs to each pattern.
[Fig. 34-A]

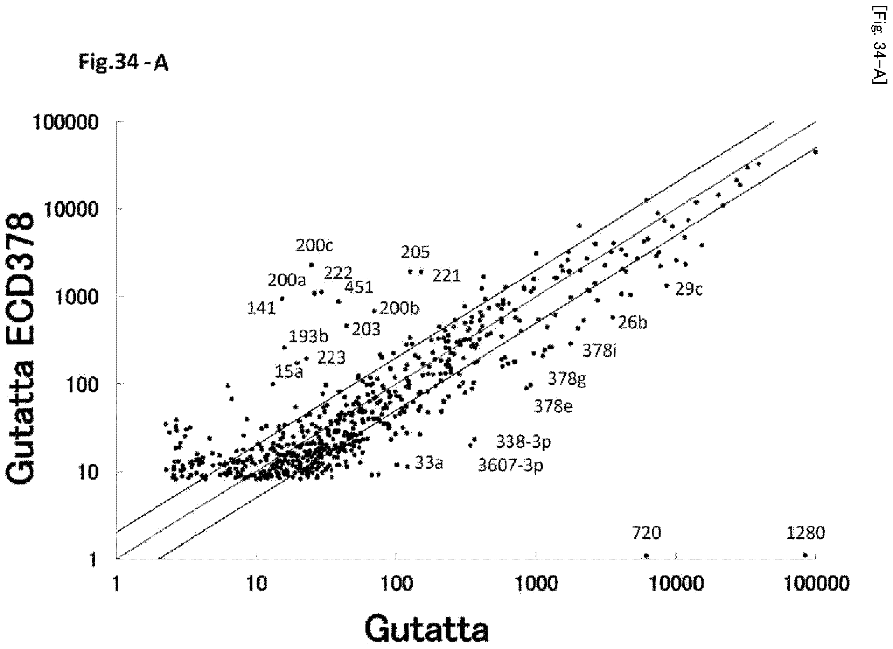

   Fig. 34-A shows a comparison of miR profiles in fresh corneal endothelial tissues. Fig. 34-A is a scatter plot showing a comparison of miR profiles of tissues with intermediate ECD levels with gutatta and tissues with low ECD levels (ECD 378) with gutatta.
[Fig. 34-B]

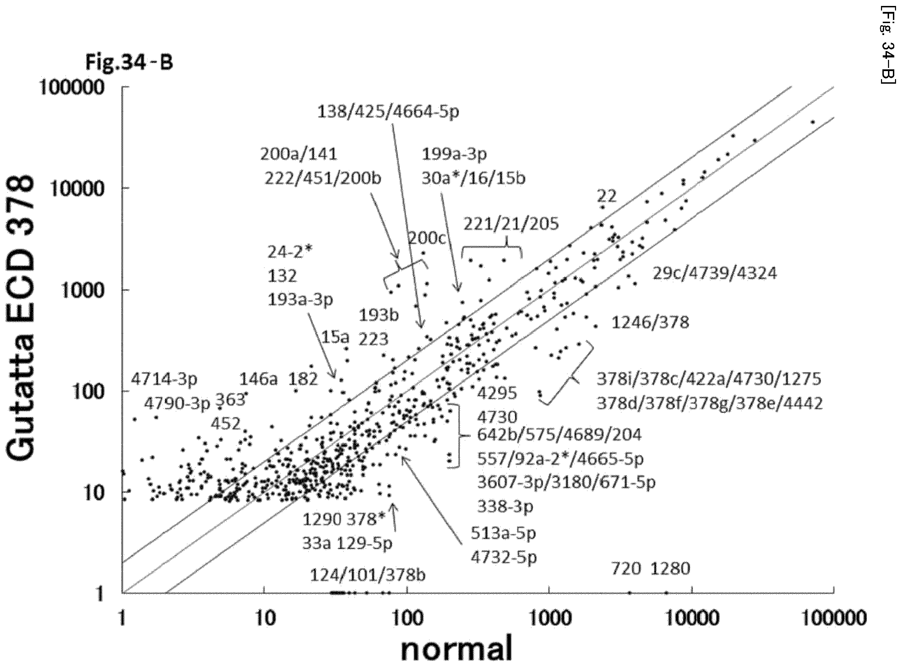

   Fig. 34-B shows a comparison of miR profiles in fresh corneal endothelial tissues. Fig. 34-B is a scatter plot showing a comparison of miR profiles of tissues with low ECD level (ECD 378) with gutatta and normal tissues.
[Fig. 34-C]

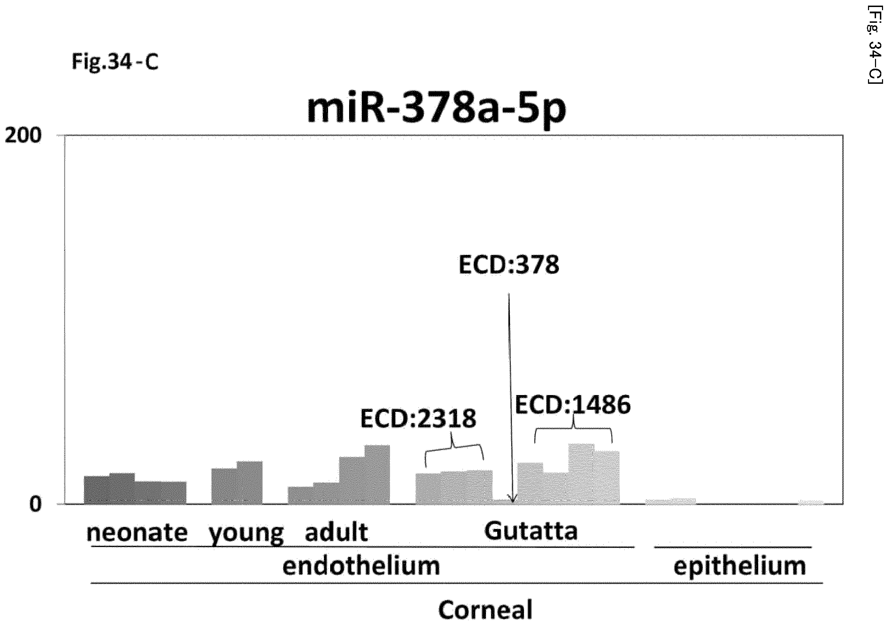

   Fig. 34-C is a graph showing the expression of miR-378a-5p for corneal epithelium tissues, corneal endothelium tissues from a neonate, young individual, adult, and adult corneal endothelium tissues with a different ECD level with gutatta. The miR of the 378 family upregulated in the corneal endothelium tissues than epithelium tissues was drastically reduced in endothelium tissue with lower ECD with gutatta.
[Fig. 34-D]

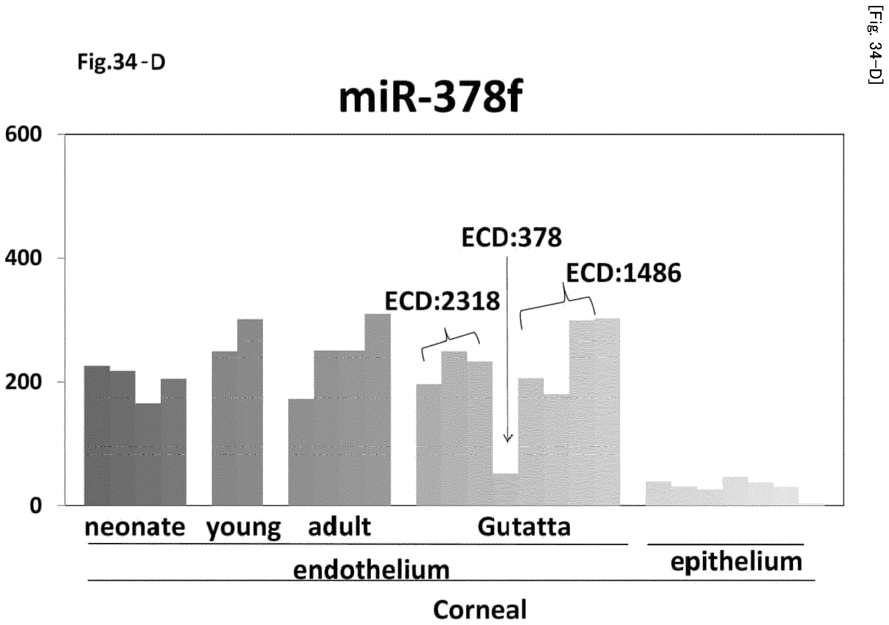

   Fig. 34-D is a graph showing the expression of miR-378f for corneal epithelium tissues, corneal endothelium tissues from a neonate, young individual, adult, and adult corneal endothelia with a different ECD level with gutatta. The miR of the 378 family upregulated in the corneal endothelium tissues than epithelium tissues was drastically reduced in endothelium tissue with lower ECD tissue than with gutatta.
[Fig. 34-E]

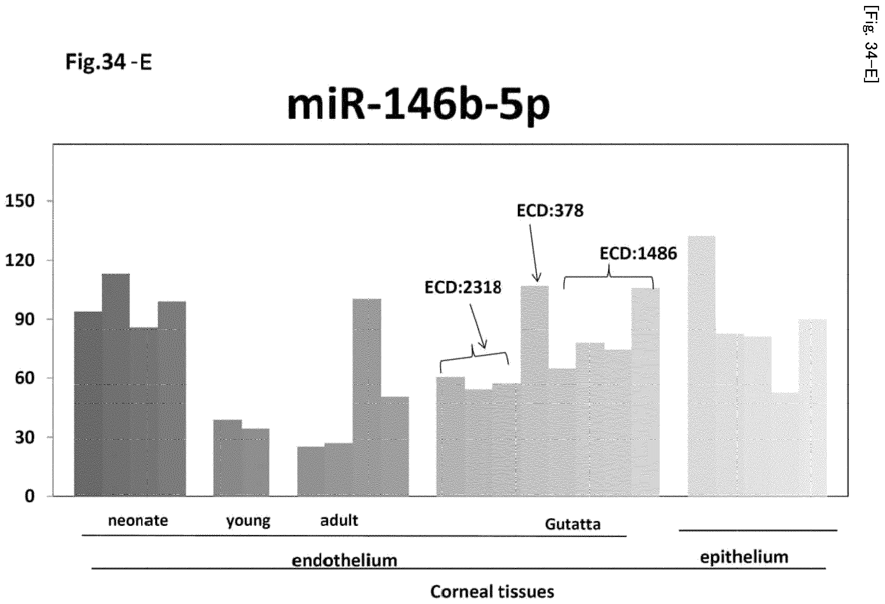

   Fig. 34-E is a graph showing the expression of miR-146b-5p for corneal epithelium tissues, corneal endothelium tissues from a neonate, young individual, adult, and adult corneal endothelium tissues with a different ECD level with gutatta.
[Fig. 34-F]

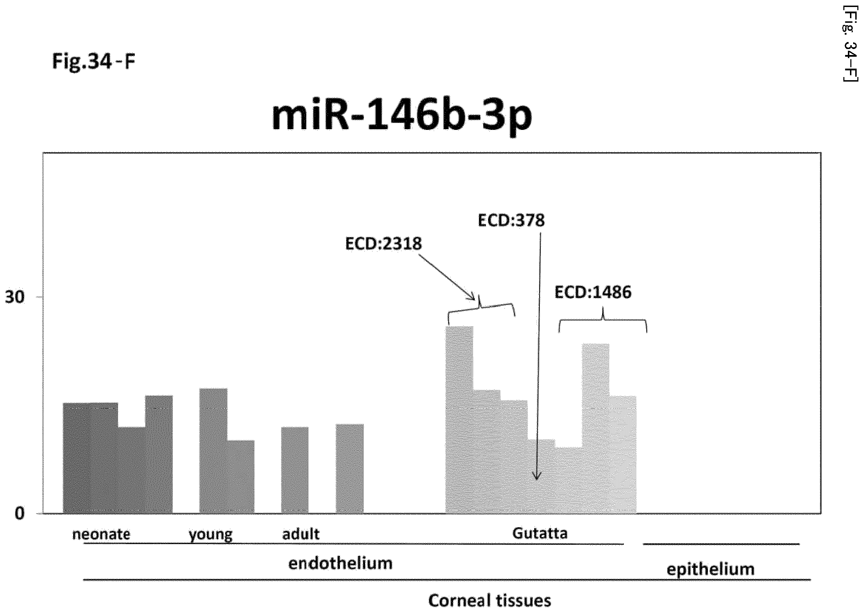

   Fig. 34-F is a graph showing the expression of miR-146b-3p for corneal epithelium tissues, corneal endothelium tissues from a neonate, young individual, adult, and adult corneal endothelium tissues with a different ECD level with gutatta.
[Fig. 34-G]

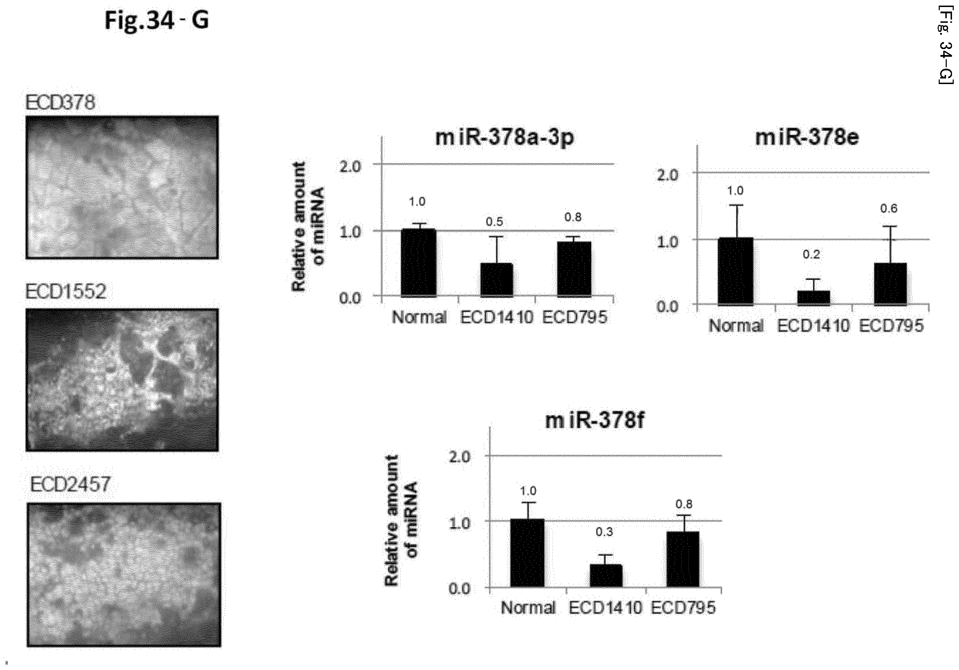

   Fig. 34-G provides images showing the morphology of cells with ECD378, ECD1552, and ECD2457 in the left column. The right column of Fig. 34-G shows results of Q-RT-PCR for the miR378 family (a-3p, e, and f) in normal tissue, tissue with ECD795, and tissue with ECD1410. The expression intensity is shown by relative amounts while assuming the expression intensity in normal tissue as 1.
[Fig. 35-A]

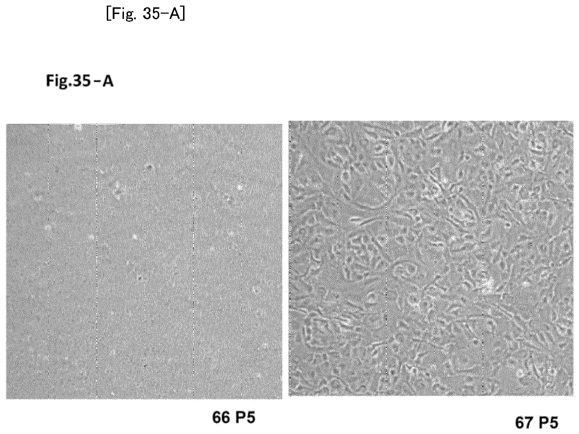

   Fig. 35-A is a picture showing the morphology of 66P5 (effector cell, fifth passage) and 67P5 (cell with CST clearly recognized, fifth passage).
[Fig. 35-B]

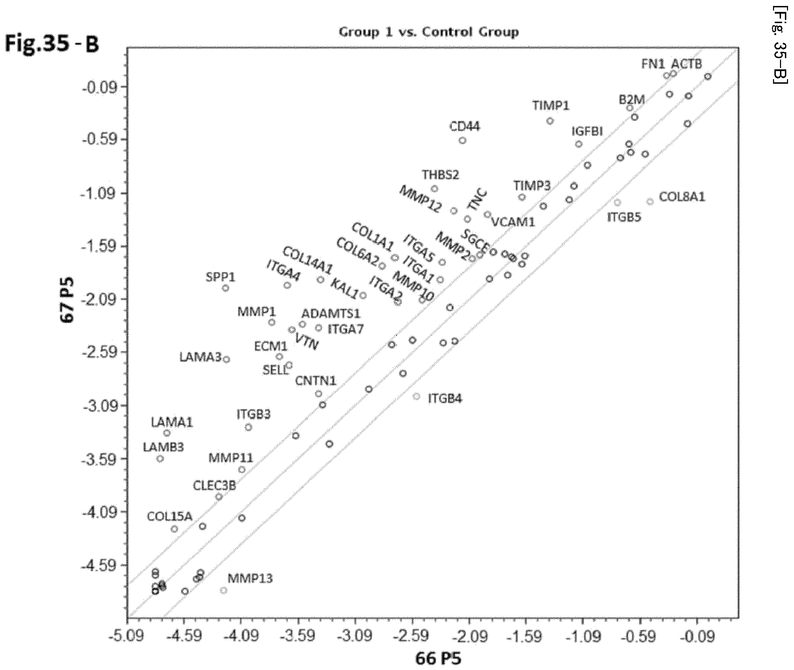

   Fig. 35-B is a scatter plot showing the difference in the expression intensity of various genes between 66P5 (effector cells, fifth passage) and 67P5 (cell with CST clearly recognized, fifth passage).
[Fig. 35-C]

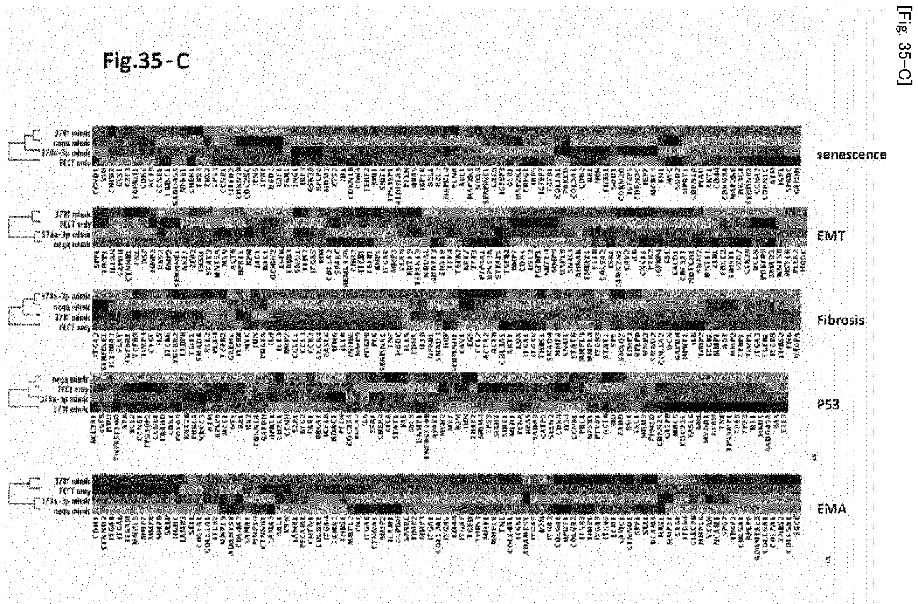

   Fig. 35-C is a diagram showing results of preliminary transfection of miR378a-3p or 5f mimetics into the CD44+++ cHCEC subpopulation which were not detected for the expression of miR378a-3p or 5f. Fig. 35-C shows heat maps of gene signatures after transfection of two miR mimetics, which were assayed with a PCR array of senescence, EMT, fibrosis, p53, and EMA. The transfected cells showing upregulation of numerous gene signatures such as collagen, ITG, and MMP families and CD44. For each cell and gene, red indicates relatively high expression and green indicates relatively low expression.
[Fig. 35-D]

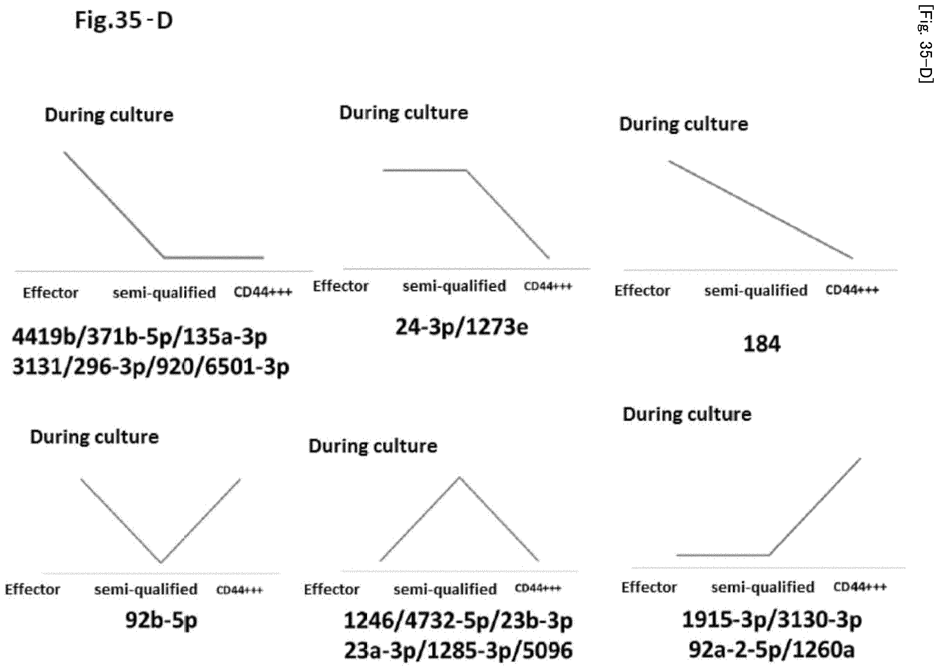

   Fig. 35-D shows classified expression patterns of secreted miR different in each subpopulation (effector, intermediately differentiated, CD44+++). miR in culture supernatant for each subpopulation is classified into six patterns as shown in Fig. 35-D.
[Fig. 35-E]
   The top row of Fig. 35-E shows the morphological images and the content of subpopulations with distinct level of the expression of CD markers in different culture lots of cHCECs, a1, a2, and a5. The bottom row of Fig. 35-E is a volcano plot comparing the miR expression profiles among culture supernatant of cultures, a1, a2, and a3 shown in A.
[Fig. 36]

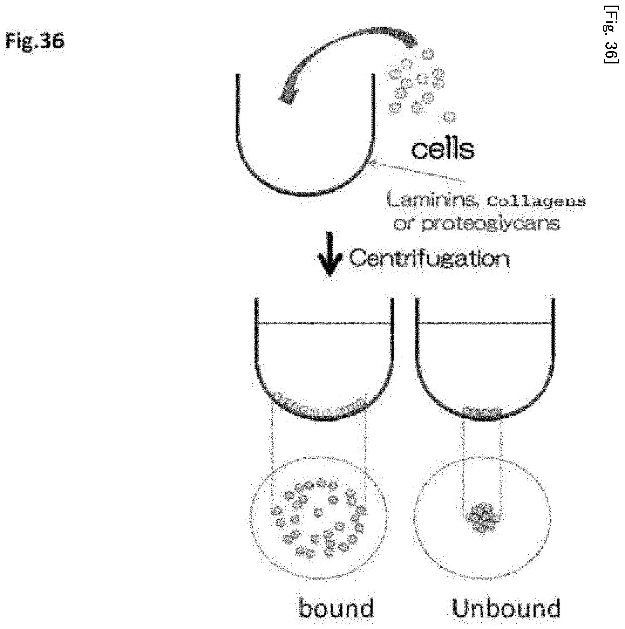

   Fig. 36 shows a schematic diagram of centrifugal cell adhesion assay for testing the binding capability of HCECs. Cultured HCECs were added to U-bottomed 96-well plates pre-coated with collagens, laminins, or proteoglycans. The plates were then centrifuged. The adherent cells were assessed under a phase contrast microscope.
[Fig. 37]

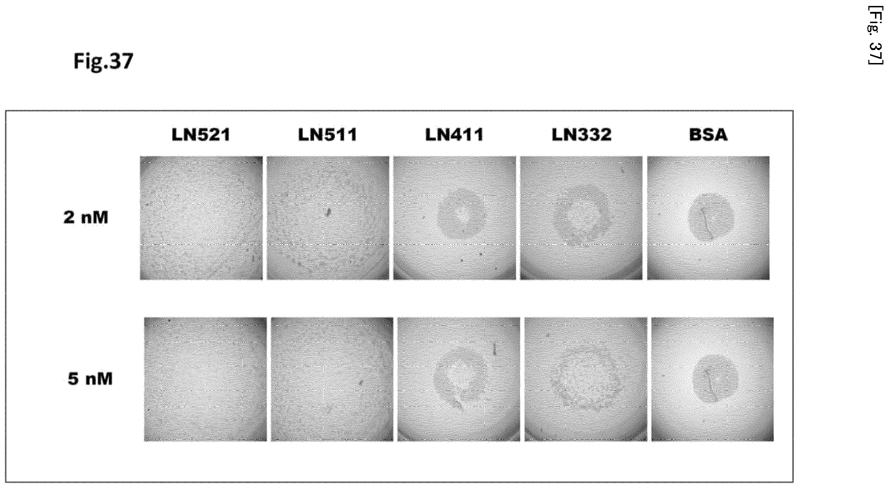

   Fig. 37 shows cultured HCECs bound to laminin in a centrifugation assay. The top row shows results of binding to a plate coated at a concentration of 2 nM, and the bottom row shows results of binding to a plate coated at a concentration of 5 nM. Fig. 37 shows, from the left, laminin-521, laminin-511, laminin-411, laminin-332, and BSA.
[Fig. 38]

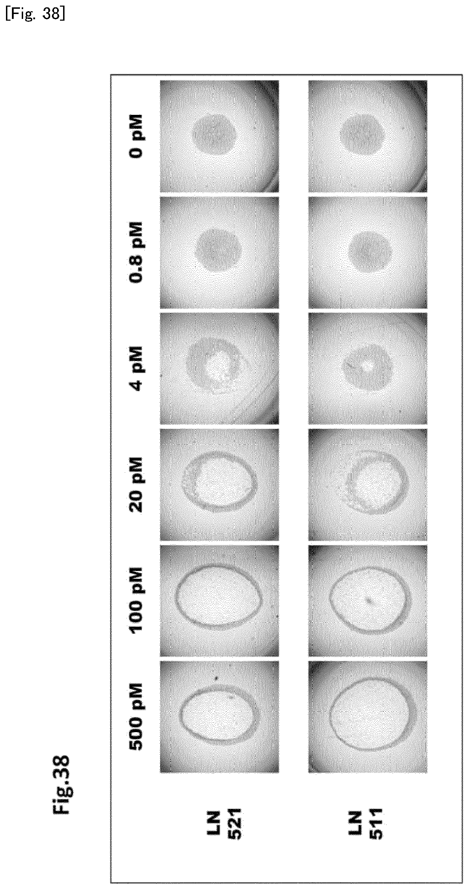

   Fig. 38 shows cultured HCECs binding to laminin 521 and laminin 511 in a concentration dependent manner in a centrifugation assay. The top row shows cultured HCECs binding to laminin-521, and the bottom row shows cultured HCECs binding to laminin-511. Fig. 38 shows, from the left, coating concentration of laminin, 500 pM, 100 pM, 20 pM, 4 pM, 0.8 pM, and 0 pM.
[Fig. 39]

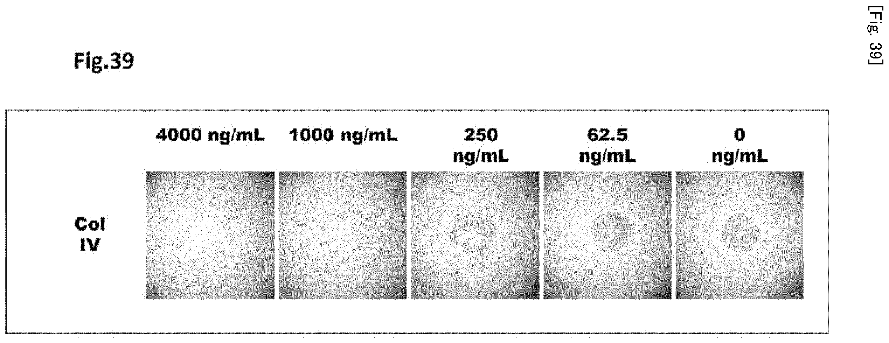

   Fig. 39 shows cultured HCECs binding to type IV collagen in a concentration dependent manner in a centrifugation assay. Fig. 39 shows, from the left, coating concentration of type IV collagen, 4000 ng/mL, 1000 ng/mL, 250 ng/mL, 62.5 ng/mL, and 0 ng/mL.
[Fig. 40]

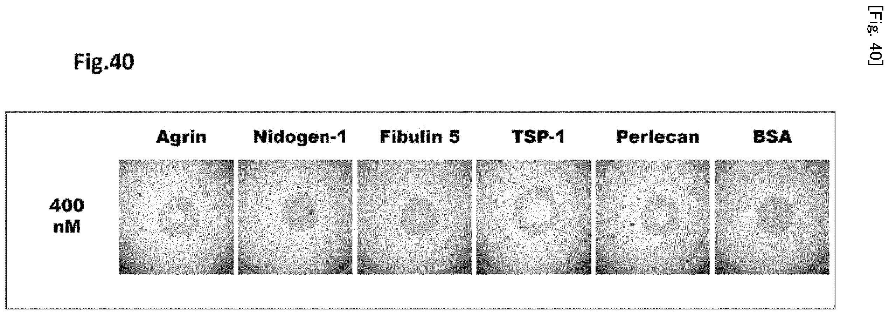

   Fig. 40 shows binding of cultured HCECs to various proteoglycan and glycoprotein in a centrifugation assay. Fig. 40 shows, from the left, coating with agrin, nidogen-1, fibulin 5, TSP-1, perlecan, and BSA (all with coating concentration of 400 nM).
[Fig. 41]

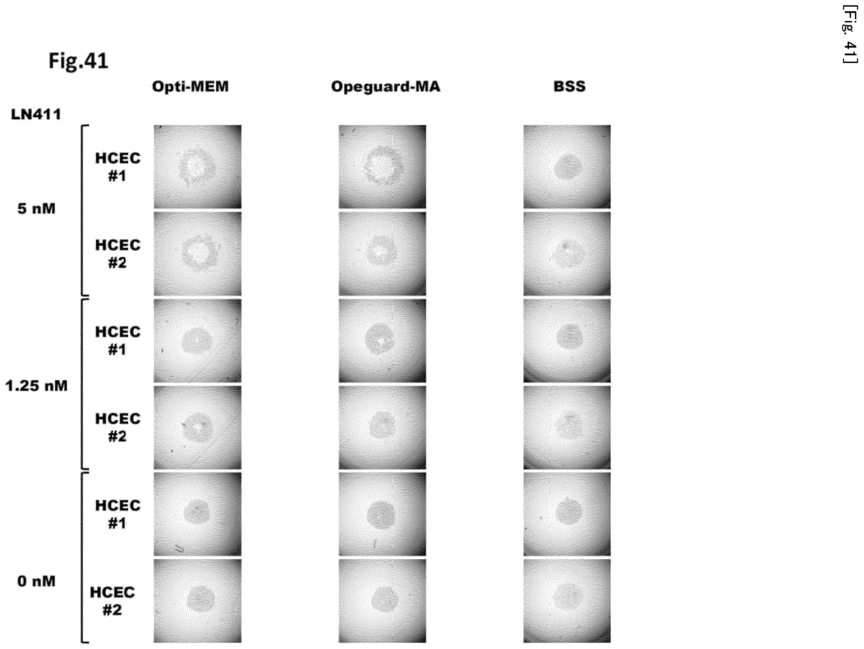

   Fig. 41 shows binding of cultured HCECs in different media to laminin-411. Fig. 41 shows, from the left, Opti-MEM, Opeguard-MA, and BSS. Laminin 411 concentrations of 5 nM, 1.25 nM and 0 nM were used.
[Fig. 42]

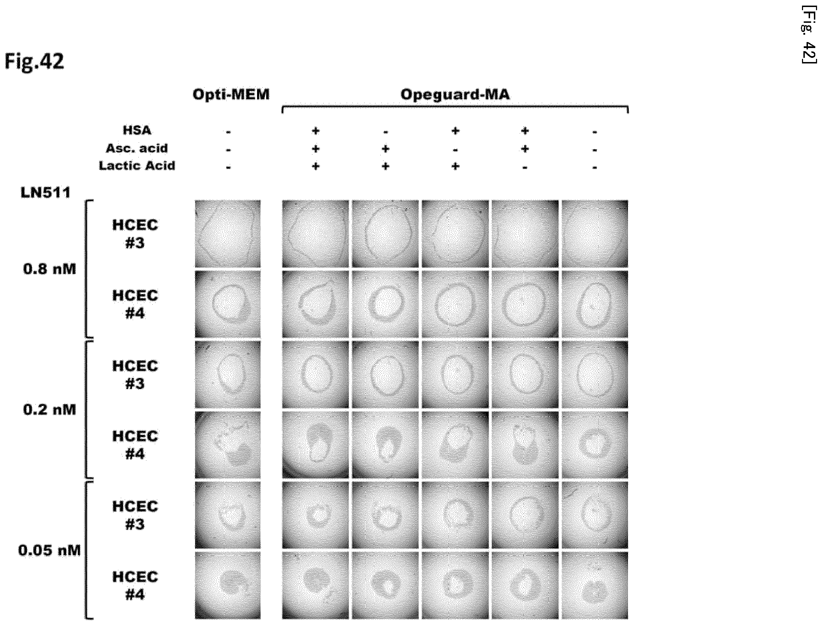

   Fig. 42 shows a change in binding affinity of cultured HCECs to laminin-511 in the presence or absence of addition of human serum albumin (HSA), ascorbic acid, lactate (an aqueous humour constituent). Laminin-511 concentrations of 0.8 nM, 0.2 nM, and 0.05 nM were used.
[Fig. 43]

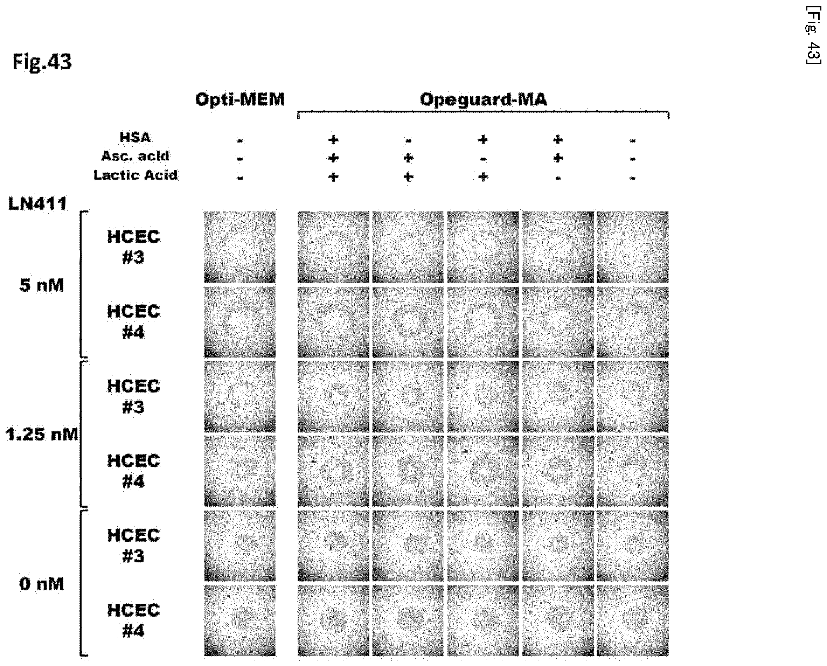

   Fig. 43 shows a change in binding affinity of cultured HCECs to laminin-411 in the presence or absence of addition of human serum albumin (HSA), ascorbic acid, lactate (an aqueous humour constituent). Laminin-411 concentrations of 5 nM, 1.25 nM, and 0 nM were used.
[Fig. 44]

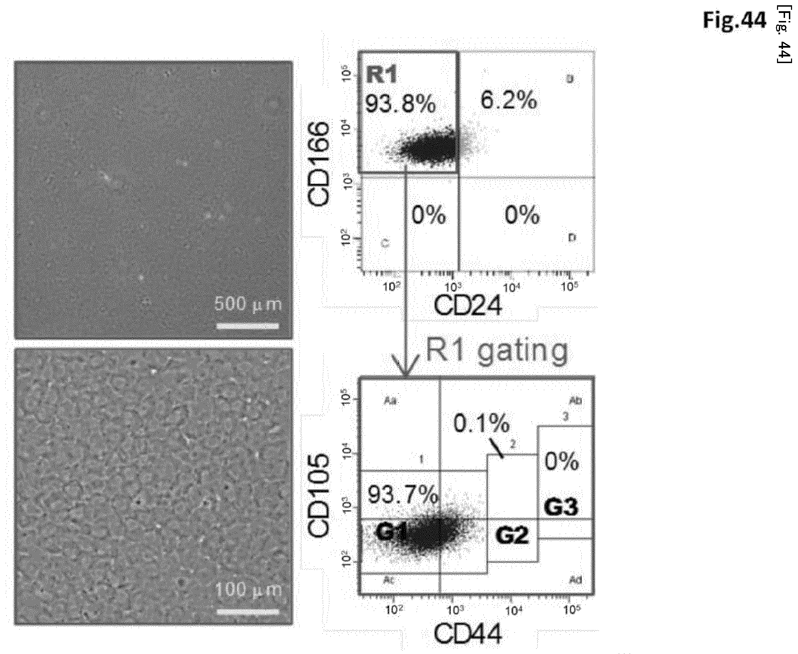

   Fig. 44 shows a cHCEC subpopulation with a hexagonal shape without signs of CST which was prepared by magnetic bead cell sorting (MACS). The left panel shows phase contrast microscope image of a cHCEC subpopulations without signs of CST. The scale car on the top row indicates 500 .micro.m and the scale bar on the bottom row indicates 100 .micro.m. The right panel shows results of flow cytometry for measuring the purity of cHCEC subpopulation provided for the analysis.
[Fig. 45]

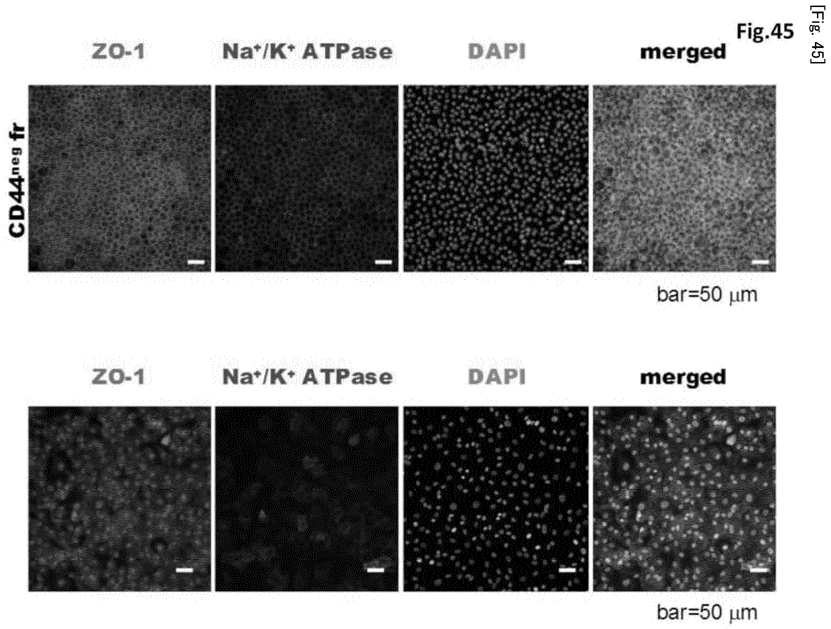

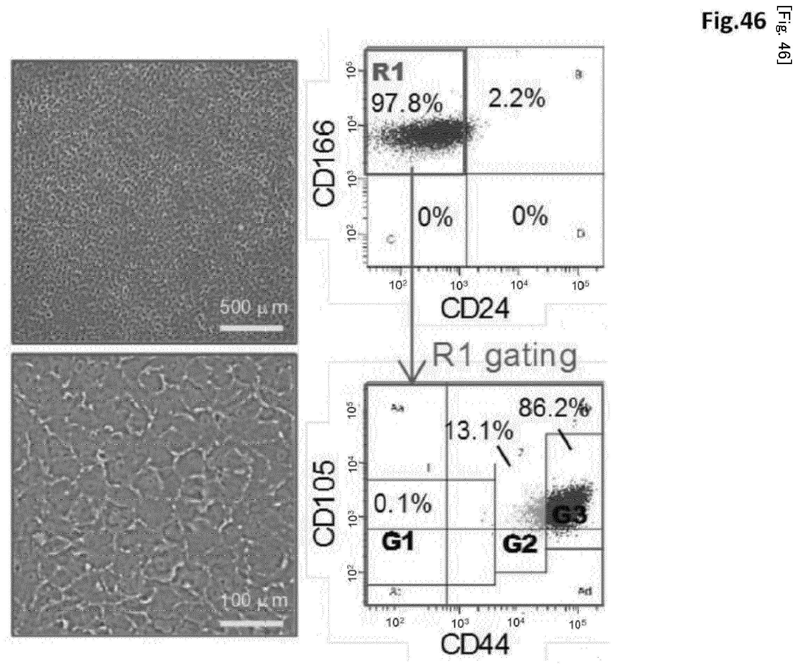

   Fig. 45 shows representative fluorescence microscope images of two subpopulations. The top row shows the subpopulation in Fig. 44, and the bottom row shows the subpopulation in Fig. 46. Fig. 45 shows, from the left, fluorescence of ZO-1, fluorescence of Na⁺/K⁺ATPase, fluorescence of DAPI, and merged image thereof. The bar indicates 50 .micro.m.
[Fig. 46]
   Fig. 46 shows a subpopulation with an EMT phenotype prepared by magnetic bead cell sorting (MACS). The left side shows a phase contrast microscope image of a subpopulation with an EMT phenotype. The scale bar on the top row indicates 500 .micro.m and the scale bar on the bottom row indicates 100 .micro.m. The right panel shows results of flow cytometry for measuring the purity of a subpopulation provided for the analysis.
[Fig. 47]

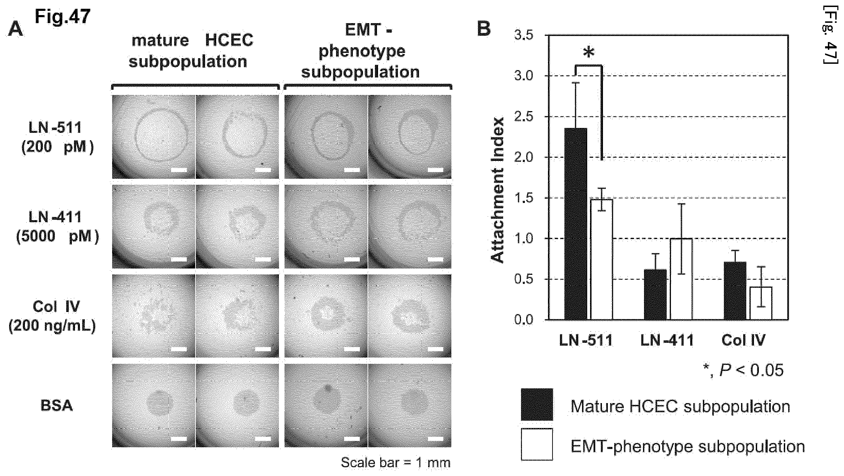

   Fig. 47 shows a comparison of the binding ability of HCEC subpopulations to a constituent of the Descemet's membrane. Top panel: each subpopulation used was examined through preparation by controlling culture conditions or by magnetic cell sorting and staining cells with a cell surface marker. Subsequently, a centrifugal cell adhesion assay was performed. A mature HCEC subpopulation and EMT phenotype subpopulation were used. The bottom panel: The binding ability of HCEC subpopulations to laminin and type IV collagen was compared by centrifugation cell adhesion assay. The attachment index was calculated as follows. Attachment index = (.delta.base (laminin or collagen)-.delta.BSA)/.delta.BSA..delta.(Greek letter) indicates area.
[Fig. 48]

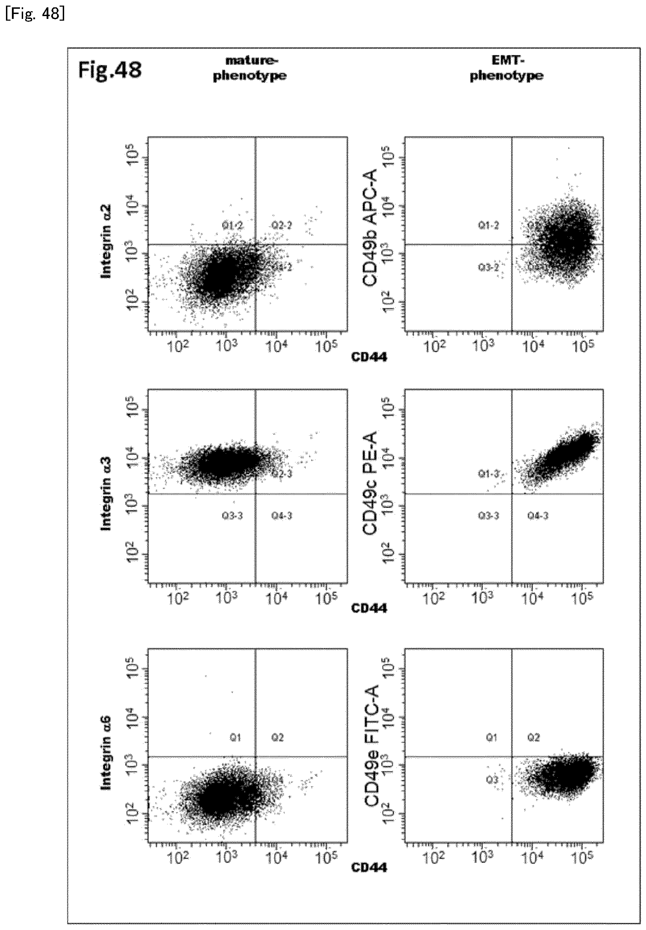

   Fig. 48 shows expression of integrin alpha subunits in an HCEC subpopulation. The top row shows integrin alpha2 expression, middle row shows integrin alpha3 expression, and the bottom row shows integrin alpha6 expression. The left column indicates mature phenotype and the right column indicates EMT phenotype.
[Fig. 49-A]

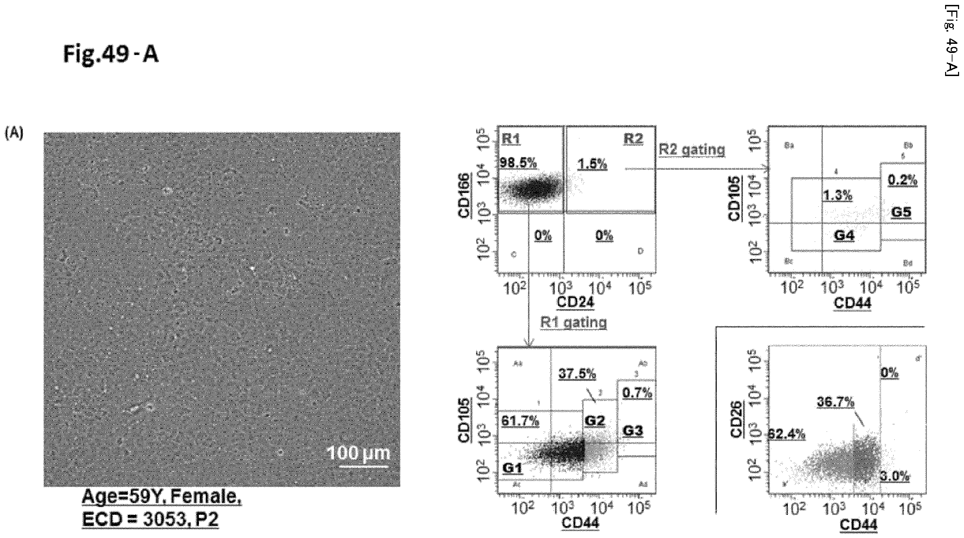

   Fig. 49-A shows expression of cell surface markers and phase contrast microscope images of cHCECs used in Example 7. FACS analysis was performed as follows. Cells were detached from the culture dish to analyze the expression of CD166, CD24, CD44, CD105, and CD26 by FACS.
[Fig. 49-B]

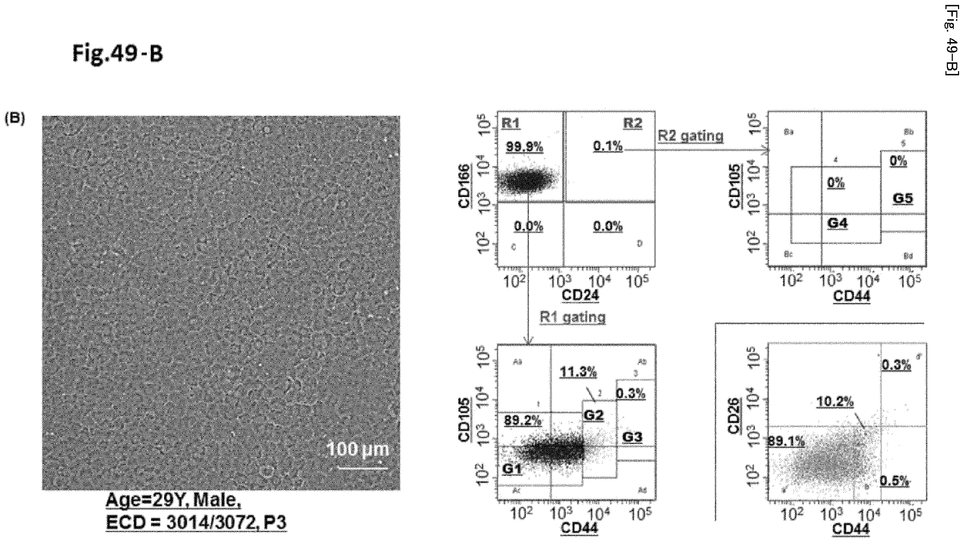

   Fig. 49-B shows expression of cell surface markers and phase contrast microscope images of cHCECs used in Example 7. FACS analysis was performed as follows. Cells were detached from the culture dish to analyze the expression of CD166, CD24, CD44, CD105, and CD26 by FACS.
[Fig. 50]

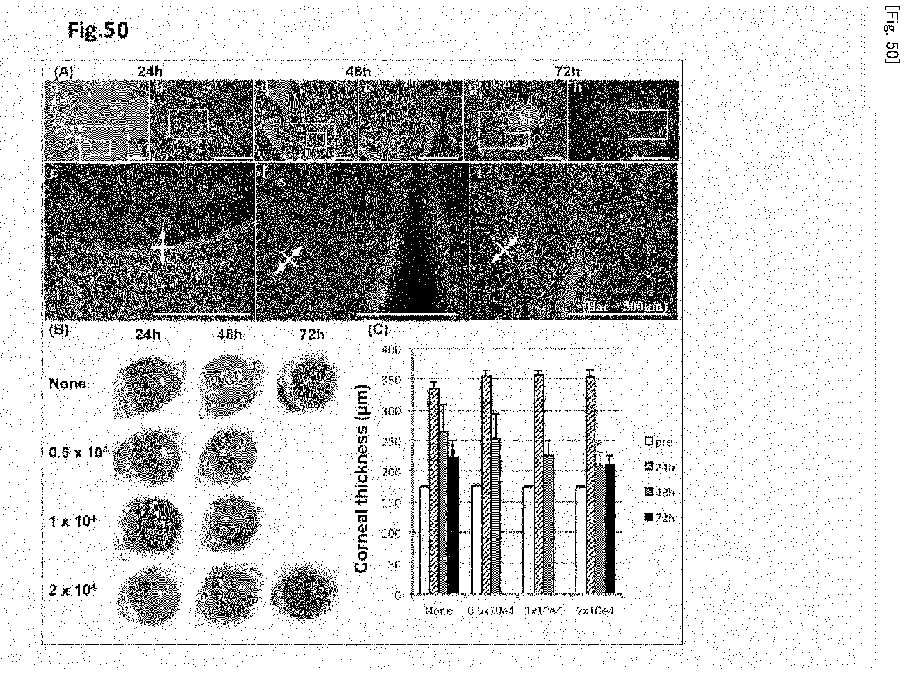

   Fig. 50 shows the cell attached to endothelium nuclei, corneal clarity, and central corneal thickness on the endothelial surface after cryo-treated freeze damage to the endothelium. The corneas mounted horizontally 24-72 hours after freeze damage were stained with DAPI to observe the loss and recovery of mouse endothelial cells.
   (A) The white dotted lines indicate region where endothelium is lost (a, d, and g). The dotted line and solid line squares (a, d, and g) of tissue mounted horizontally indicate the regions in Fig. 50 (b), (e), and (h), and Fig. 50 (c), (f), and (i), respectively. The arrows indicate the peripheral edge between normal endothelia and lost endothelia.
   (B) shows the clinical appearance after injection of 0-2.0 x 10⁴ HCECs into an eye with freeze damage (24 hours, 48 hours, and 72 hours). (C) shows the corneal thickness before and after injection (24 hours, 48 hours, and 72 hours) (*p < 0.05).
[Fig. 51]

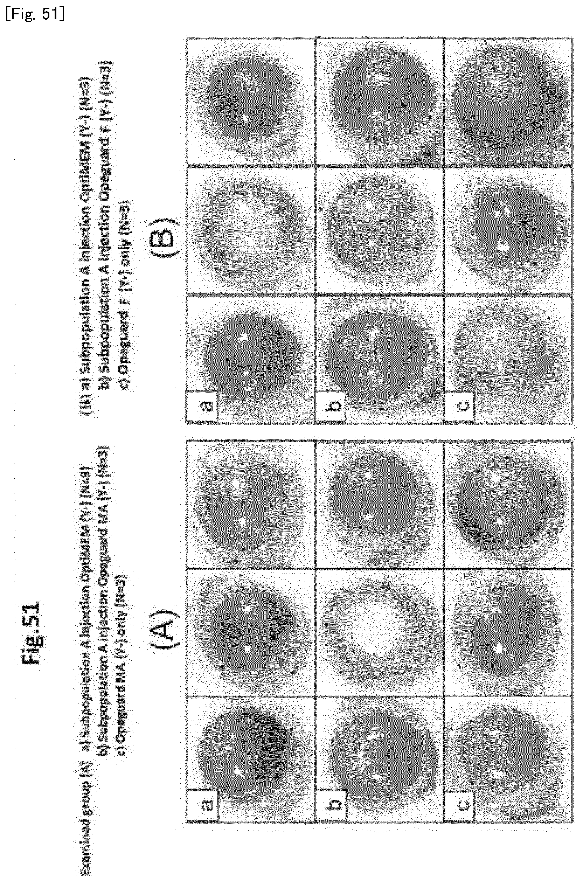

   Fig. 51(A) shows corneal clarity 48 hours after infusion of cHCECs in different cell suspension vehicles. Eyes of BALB/c were freeze damaged, 2.0 x 10⁴ HCECs were suspended in Opti-MEM (a) and Opeguard MA (b) and injected into the anterior chamber. As a control, cell-free Opeguard MA only (c) was injected into the anterior chamber (for each case, N=3). Fig. 51(B) performed an experiment similar to (A) by suspending cells in Opti-MEM (a) and Opeguard F (b). As a control, cell-free Opeguard F only (c) was injected into the anterior chamber (for each case, N=3).
[Fig. 52]

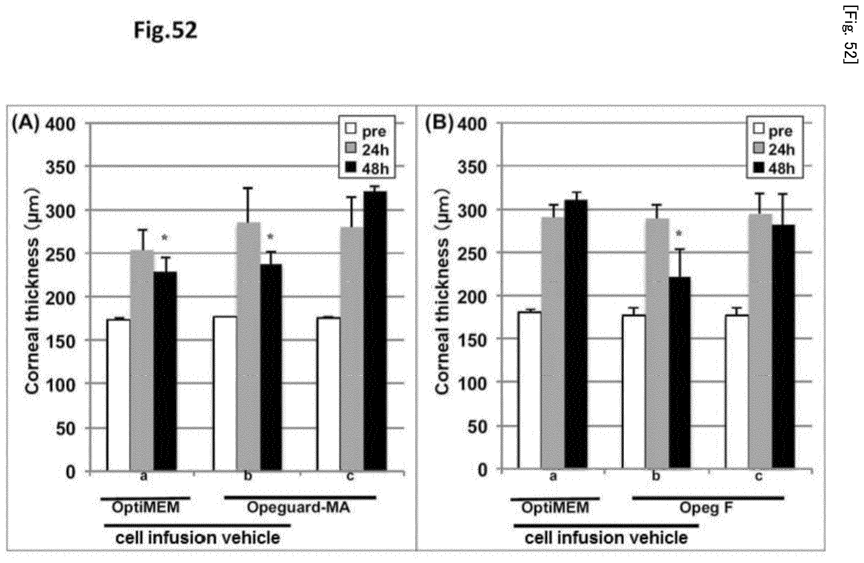

   Fig. 52(A) shows the corneal thickness after infusion of cHCECs in different cell suspension vehicles. Eyes of BALB/c were freeze damaged, 2.0 x 10⁴ HCECs were suspended in Opti-MEM (a) and Opeguard MA (b) and injected into the anterior chamber. As a control, cell-free Opeguard MA only (c) was injected into the anterior chamber (for each case, N=3). Fig. 52(B) performed an experiment similar to Fig. 52(A) by suspending cells in Opti-MEM (a) and Opeguard F (b). As a control, cell-free Opeguard F only (c) was injected into the anterior chamber (for each case, N=3). The corneal thickness before injection, after 24 hours, and after 48 hours was assessed. * indicates a statistical significant difference (p < 0.05).
[Fig. 53]

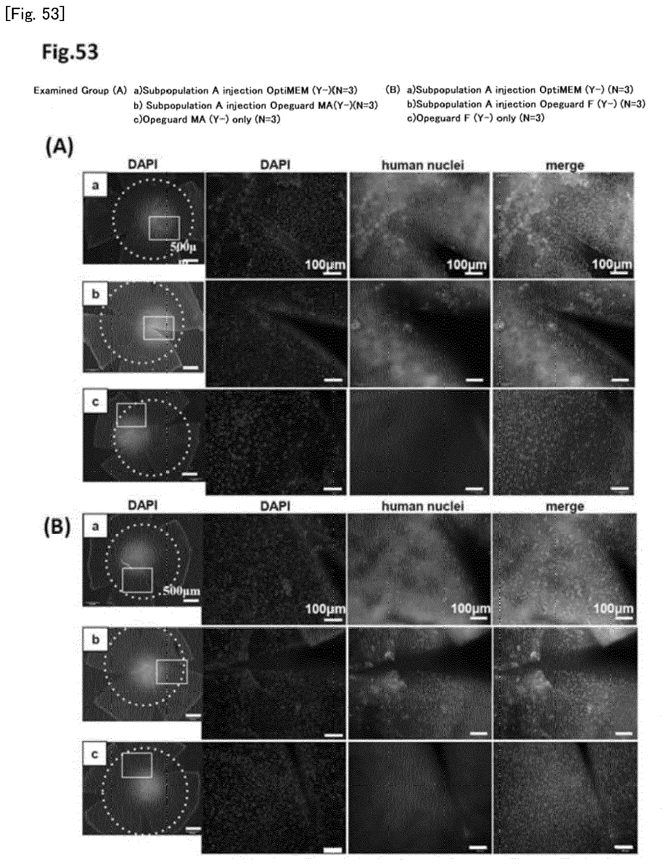

   Fig. 53(A) provides fluorescence microscope images showing adhesion of HCECs infused into anterior chamber in different cell suspension vehicles. Eyes of BALB/c were freeze damaged, 2.0 x 10⁴ HCECs were suspended in Opti-MEM (a) and Opeguard MA (b) and injected into the anterior chamber. As a control, cell-free Opeguard MA only (c) was injected into the anterior chamber (for each case, N=3). Fig. 53(B) performed an experiment similar to 53(A) by suspending cells in Opti-MEM (a) and Opeguard F (b). As a control, cell-free Opeguard F only (c) was injected into the anterior chamber (for each case, N=3). Immediately after assessing corneal clarity and corneal thickness after 48 hours, these corneas were stained with anti-human nuclear antibody (identification of injected HCECs and distinction of host derived CEC) and DAPI. The dotted line circles indicate regions with freeze damage. DAPI (red), anti-human nuclear antibody (green), and merged image show high magnification expanded diagrams of white square regions in the image of DAPI (blue).
[Fig. 54-A(a)]

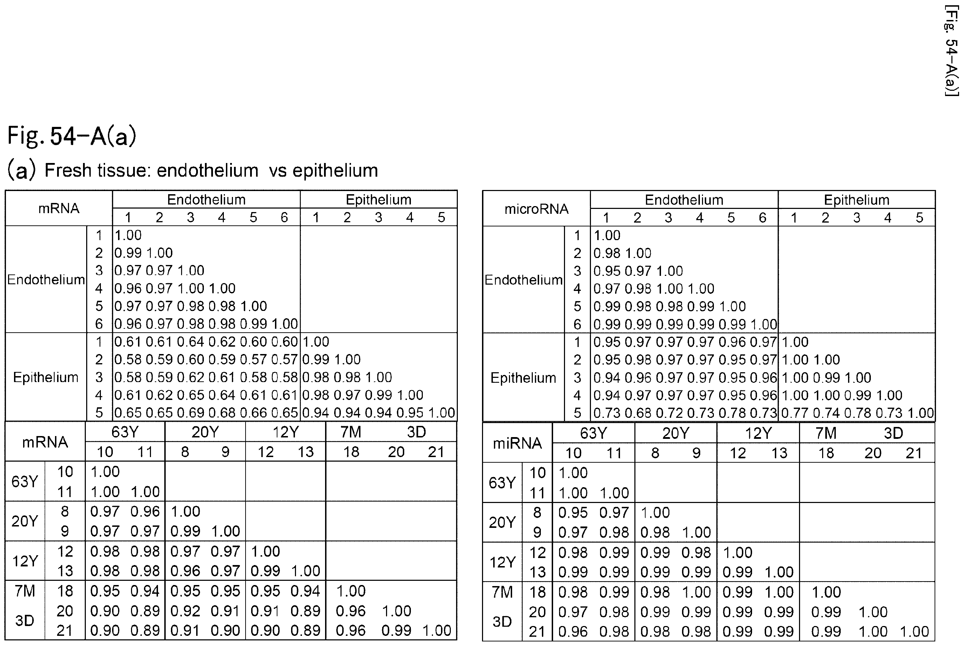

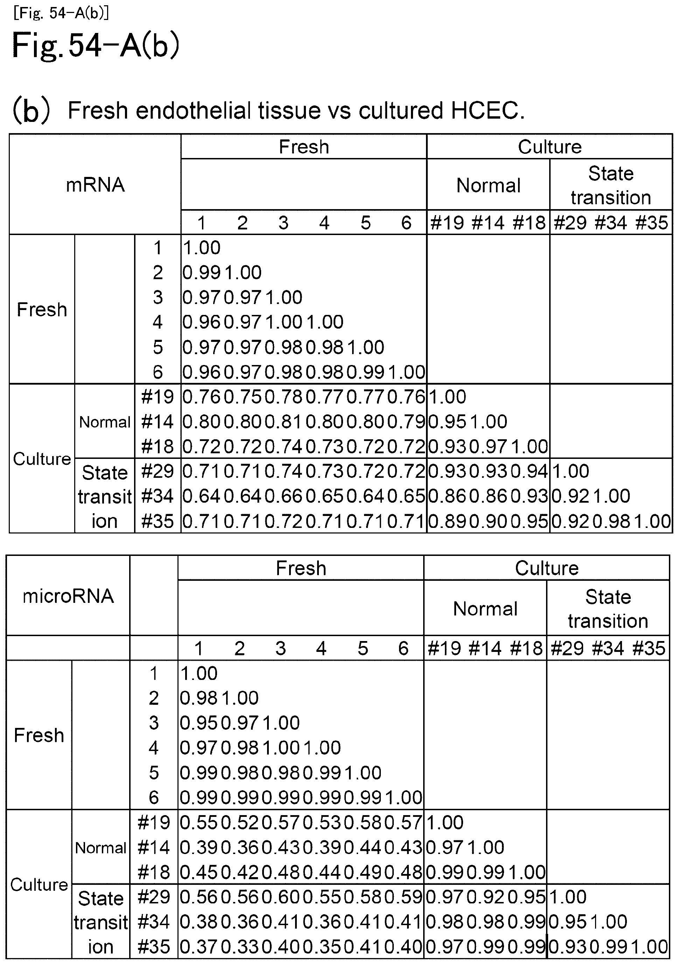

   Fig. 54-A(a) to 54-A(b) shows results of analyzing the mRNA and miRNA signatures of human corneal endothelium (Endo)/epithelium (EP) tissue and cHCECs by 3D-Gene. Analysis was performed with Human_25K_Ver 2.1 and Human_miRNA_Ver 17. Fig. 54-A(a) shows the correlation coefficients of mRNA (top left) and miRNA (top right) between each of the six human corneal endothelial tissues and five human corneal epithelial tissues, and the correlation coefficients of mRNA (bottom left) and miRNA (bottom right) signatures between each of the fresh tissues of seven donors.
[Fig. 54-A(b)]
   Fig. 54-A(a) to 54-A(b) shows results of analyzing the mRNA and miRNA signatures of human corneal endothelium (Endo)/epithelium (EP) tissue and cHCECs by 3D-Gene. Analysis was performed with Human_25K_Ver 2.1 and Human_miRNA_Ver 17. Fig. 54-A(b) shows the correlation coefficients of mRNA (left) and miRNA (right) signatures between each of the six fresh tissues, three normal cHCECs and three cHCECs that have undergone cell state transition.
[Fig. 54-B]

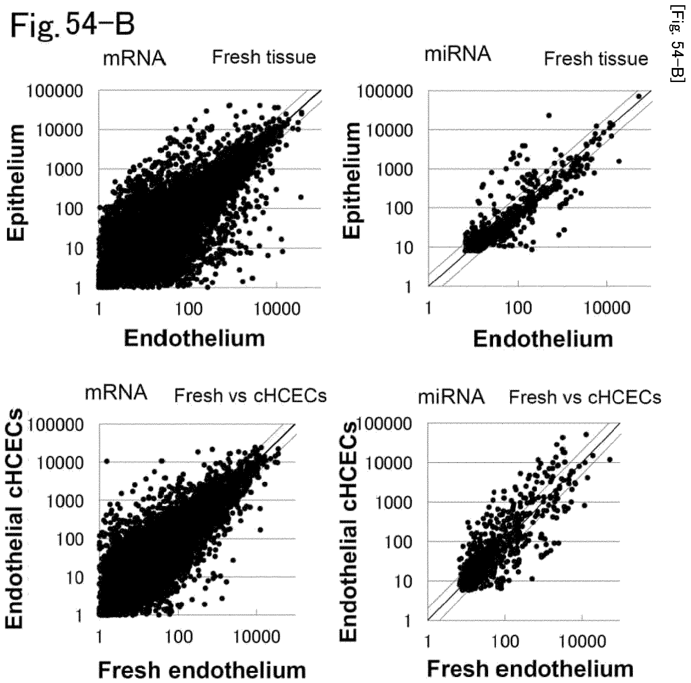

   Fig. 54-B shows results of analyzing the mRNA and miRNA signatures of human corneal endothelium (Endo)/epithelium (EP) tissue and cHCECs by 3D-Gene. Analysis was performed with Human_25K_Ver 2.1 and Human_miRNA_Ver 17. Fig. 54-B shows scatter plots of Endo, EP, and cHCEC genes and miR expression profiles. The values are average values after global normalization. Straight lines representing 2-fold change and 1/2 fold change are shown in the scatter diagrams.
[Fig. 55-A]

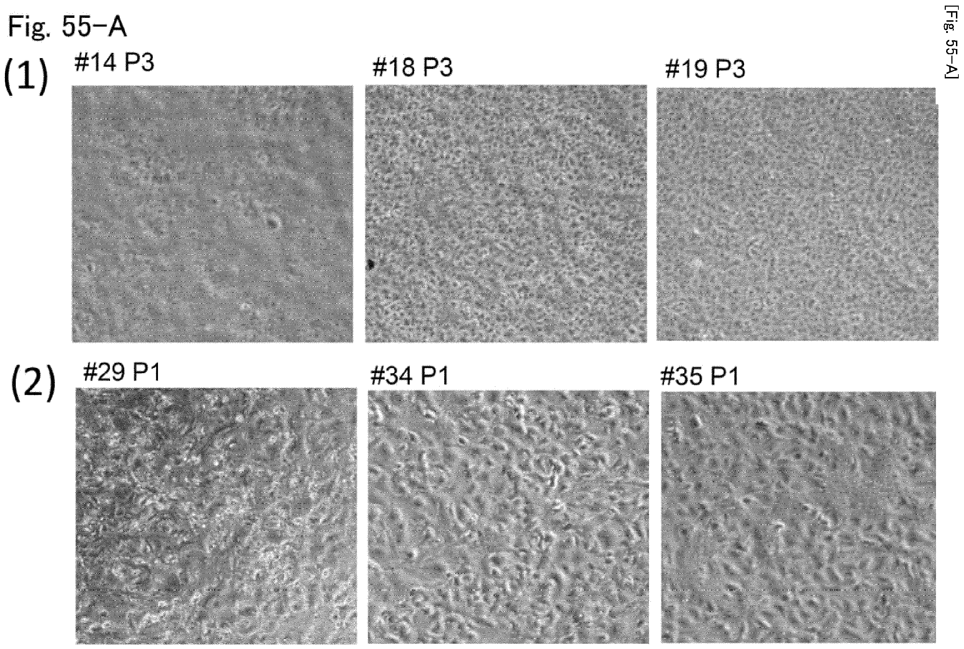

   Fig. 55-A to 55-B shows comparison of gene signatures among cHCECs without CST (#14, #18, and #19), and with CST (#29, #34, and #35) and fresh tissues (endo tissue of 8 and 9 of 20Y and 12 of 12Y) using RT2 profiler PCR-Array for senescence, EMT, and fibrosis. In Fig. 55-A, (1) shows phase contrast microscope images of, from the left, #14 third passage, #18 third passage, and #19 third passage. (2) shows phase contrast microscope images of, from the left, #29 first passage, #34 first passage, and #35 first passage.
[Fig. 55-B]

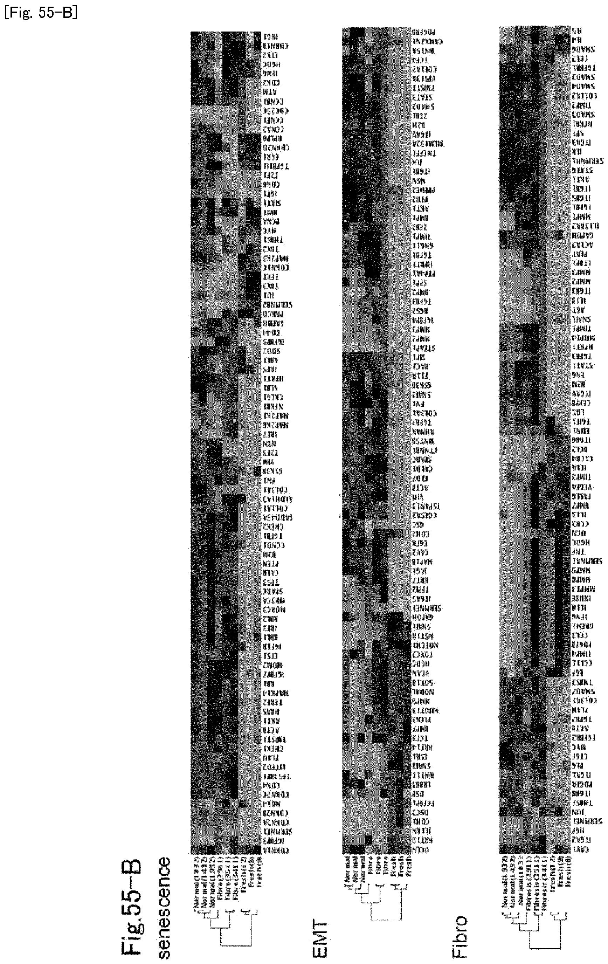

   Fig. 55-A to 55-B shows comparison of gene signatures among cHCECs without CST (#14, #18, and #19), and with CST (#29, #34, and #35) and fresh tissues (endo tissue of 8 and 9 of 20Y and 12 of 12Y) using RT2 profiler PCR-Array for senescence, EMT, and fibrosis. In Fig. 55-B, mRNA extracted from cHCECs without CST and with CST, and fresh Endo tissues was used to analyze senescence, EMT and fibrosis by a microarray. Hierarchical clustering was used to compare gene signatures, which are shown as heat maps. Red indicates relatively high expression intensity and green indicates relatively low expression intensity.
[Fig. 56-A]

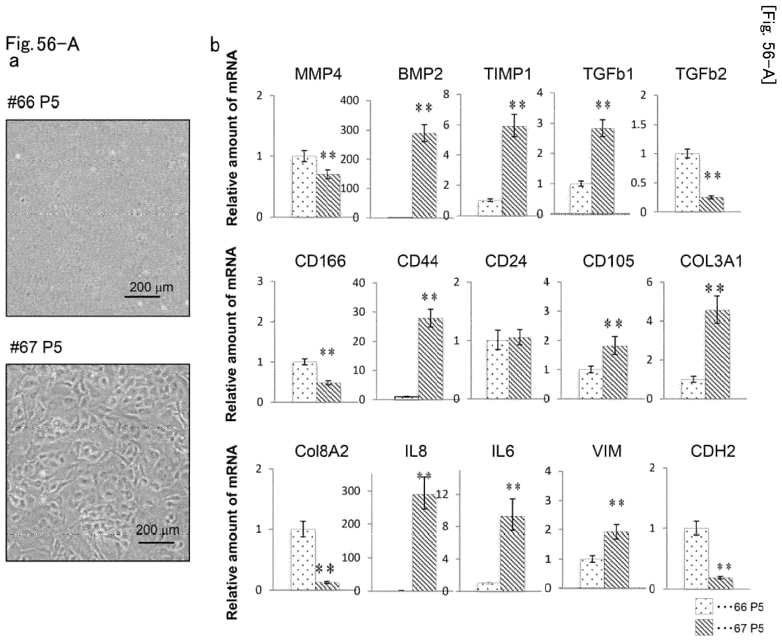

   Fig. 56-A shows results of comparing expression of each mRNA in the two cHCECs (#66 fifth passage (effector cell) and #67 fifth passage with CST) by qRT-PCR. a shows phase contrast microscope images of #66 fifth passage and #67 fifth passage. These two cultures were morphologically different. b is a result of measuring and comparing the expression intensity of mRNA for some of the 50 candidate genes by qRT-PCR. In each bar graph, the left column represents #66 fifth passage and the right bar represents #67 fifth passage. The vertical axis represents the relative expression intensity of mRNA while assuming the expression intensity of #66 fifth passage as 1.
[Fig. 56-B]

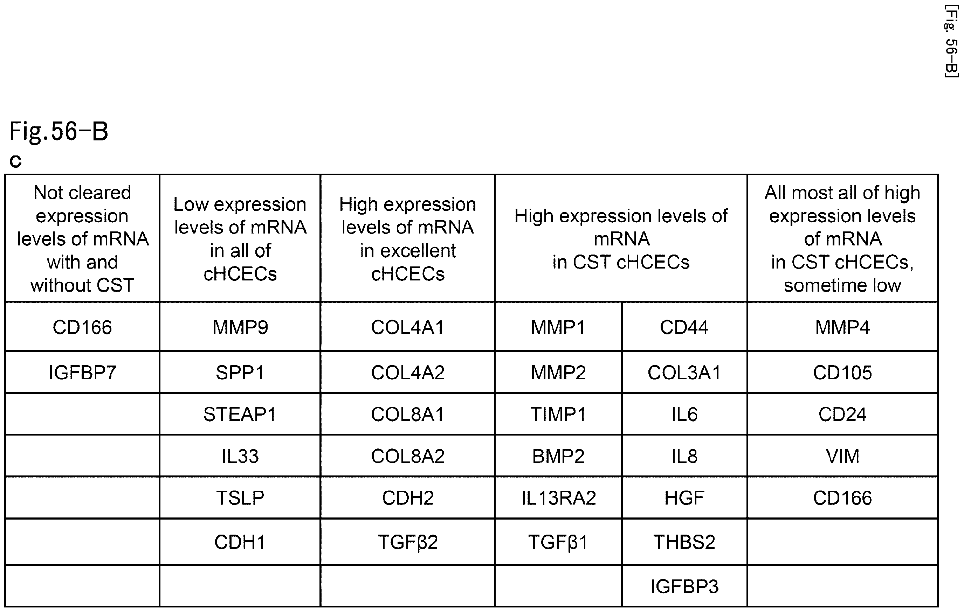

   Fig. 56-B is a table summarizing genes with different mRNA expression intensity between #66 and #67.
[Fig. 57-A]

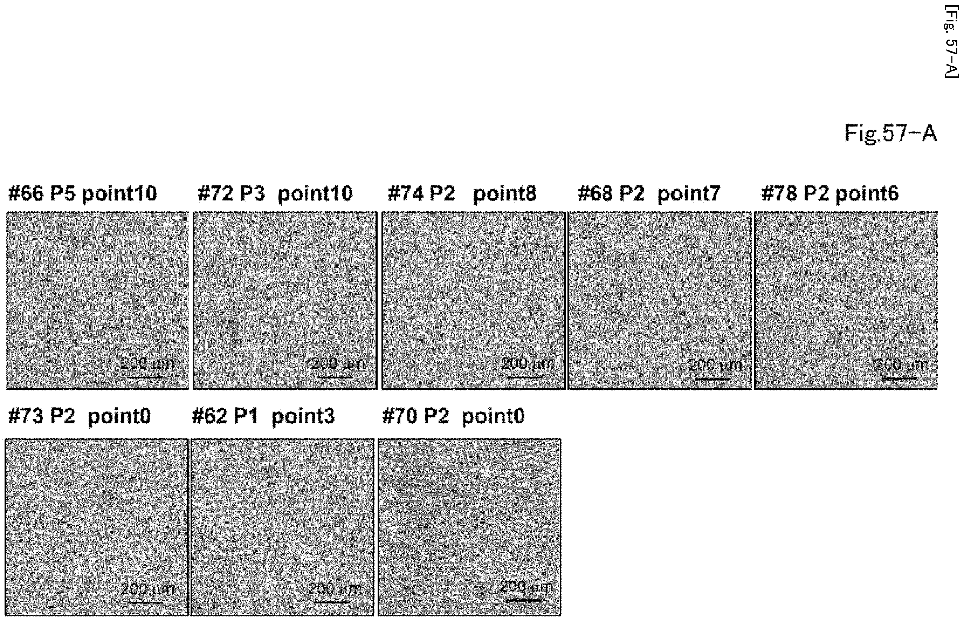

   Fig. 57-A shows pictures of cultures provided for the analysis of selected genes by qRT-PCR. In Fig. 57-A, phase contrast microscope images of each culture are shown with donor number, number of passages, and points assigned for morphological classification of cHCECs. Higher point means the higher quality of cHCECS.
[Fig. 57-B]

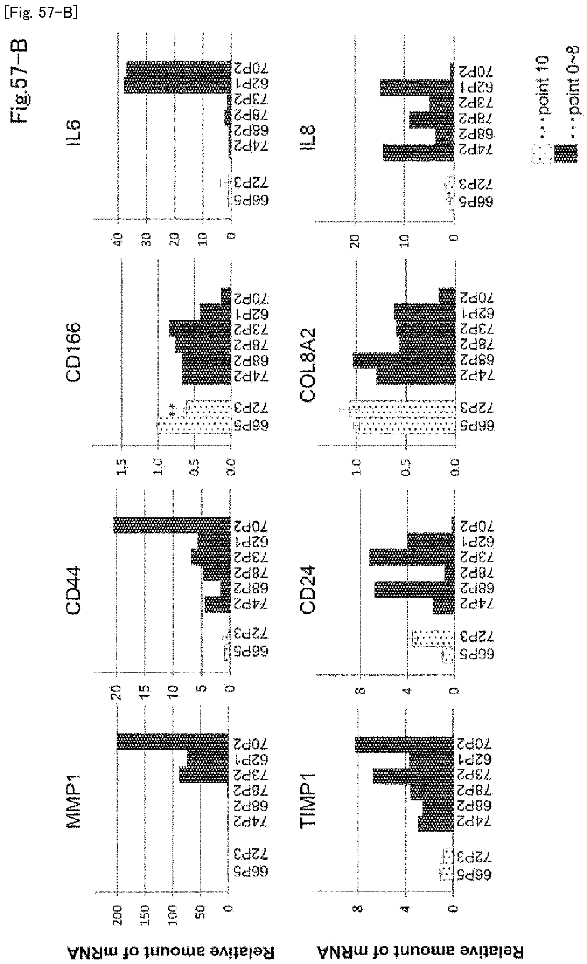

   Fig. 57-B shows graphs of the expression intensity of selected genes by qRT-PCR among morphologically classified cHCECs. In Fig. 57-B, the vertical axis shows the relative intensity of gene expression while assuming the expression intensity of #66 fifth passage as 1 for each gene, and the horizontal axis shows the type of culture from which mRNA was extracted. Cultures with 10 points are shown with a light column and cultures with 0-8 points are shown with a dark column.
[Fig. 58-A]

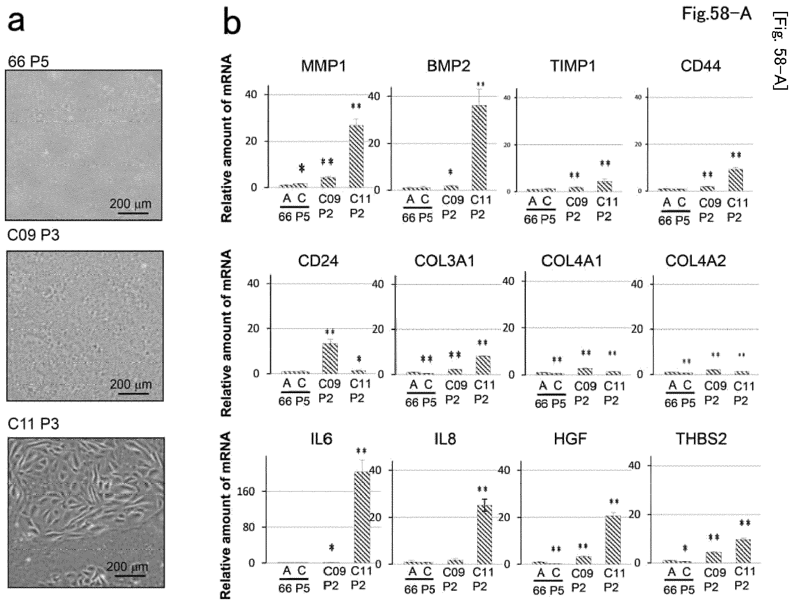

   Fig. 58-A shows pictures of cultures provided for the analysis of selected cells by qRT-PCR among cHCECs manufactured at a cell processing center under GMP. a shows phase contrast microscope images of #66 fifth passage, C09 (from 16 year old donor) third passage, and C11 (from 26 year old donor) third passage. The miR expression levels were assessed by qRT-PCR. For each column for each gene, the columns represent, from the left, culture well A for #66 fifth passage, culture well C for #66 fifth passage, C09 (from 16 year old donor) third passage, and C11 (from 26 year old donor) third passage. The vertical axis represents the relative intensity of gene expression while assuming the expression intensity of culture well A of #66 fifth passage as 1.
[Fig. 58-B]

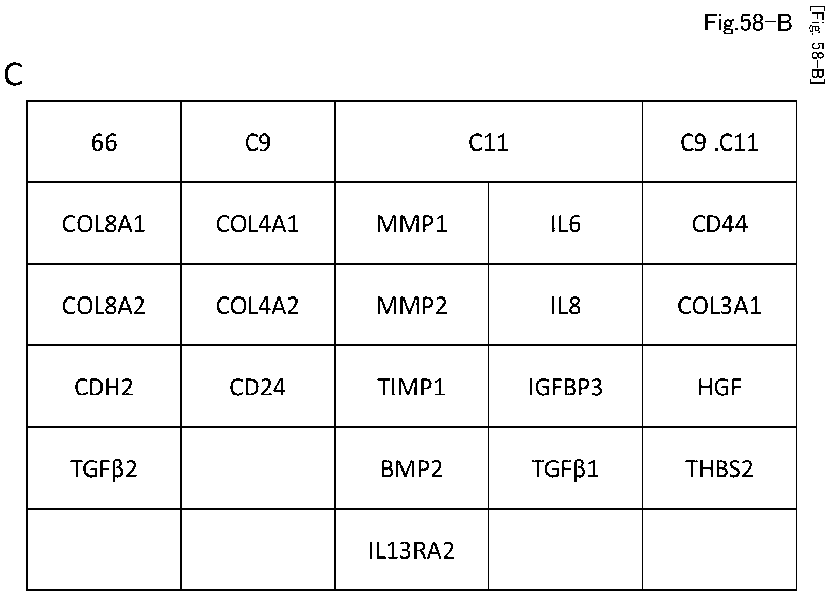

   Fig. 58-B is a table summarizing genes with high expression in each culture as a result of analyzing cHCECs by qRT-PCR among cHCECs manufactured at a cell processing center under GMP.
[Fig. 59-A]

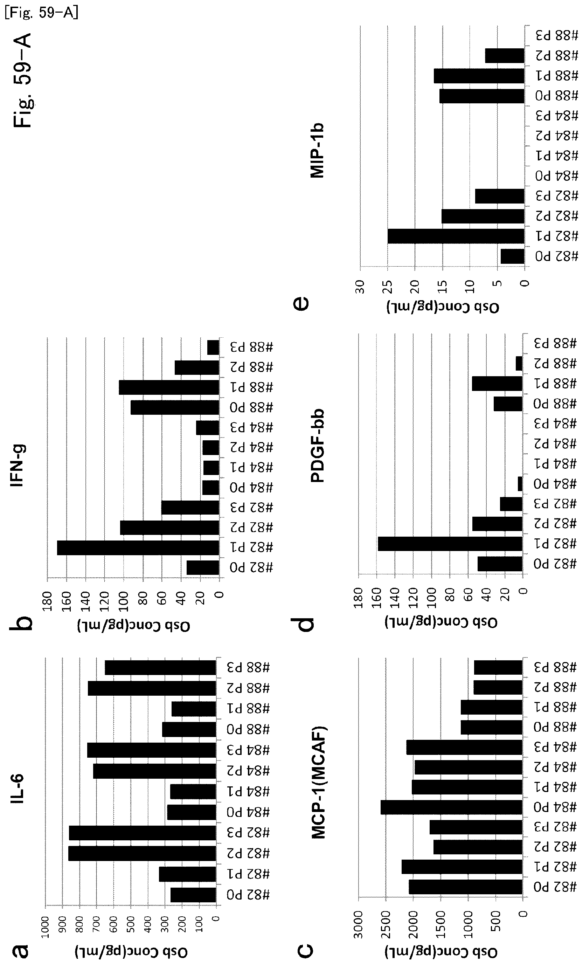

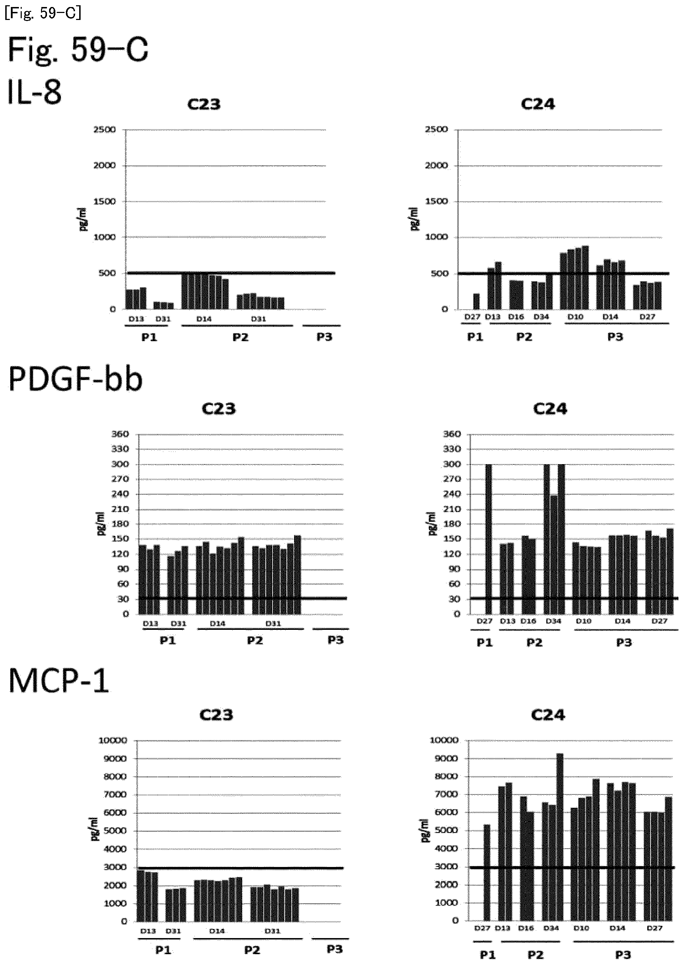

   Fig. 59-A shows results of analyzing the amount of cytokines in supernatant of cultured HCECs #82 (P0-P3, 72 year old donor, ECD = 3192/3409), #84 (P0-3, 75 year old donor, ECD = 2598), and #88 (P0-3, 10 year old donor, ECD = 3879) by Bio-Plex. A, B, C, D, and E show quantitative results for IL-6, IFN-gamma, MCP-1, PDGF-bb, and MIP-1b, respectively.
[Fig. 59-B]

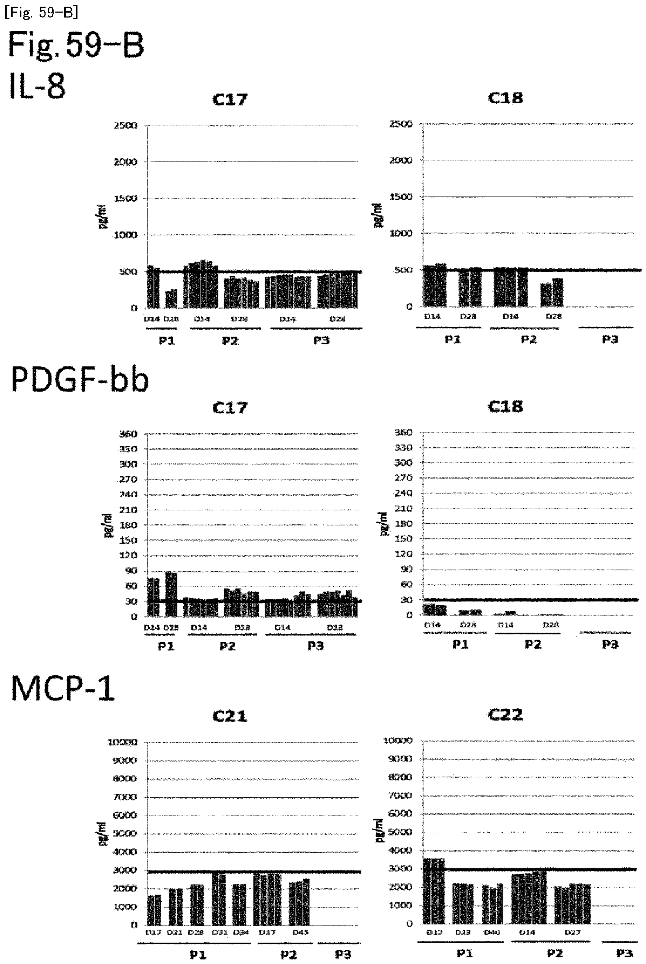

   Figs. 59-B to 59-C show an ELISA assay in culture supernatant to evaluate the quality of cHCECs. Quantification was repeated three times by ELISA. The graphs show the amount of [L8, PDGFbb, or MCP1 secreted in the culture of C17, C18, C23, or C24. P1 indicates first passage, P2 indicates second passage, and P3 indicates third passage. Quantification was performed on cultures with various days of culture.
[Fig. 59-C]
   Figs. 59-B to 59-C show an ELISA assay in culture supernatant to evaluate the quality of cHCECs. Quantification was repeated three times by ELISA. The graphs show the amount of [L8, PDGFbb, or MCP1 secreted in the culture of C17, C18, C23, or C24. P1 indicates first passage, P2 indicates second passage, and P3 indicates third passage. Quantification was performed on cultures with various days of culture.
[Fig. 60-A]

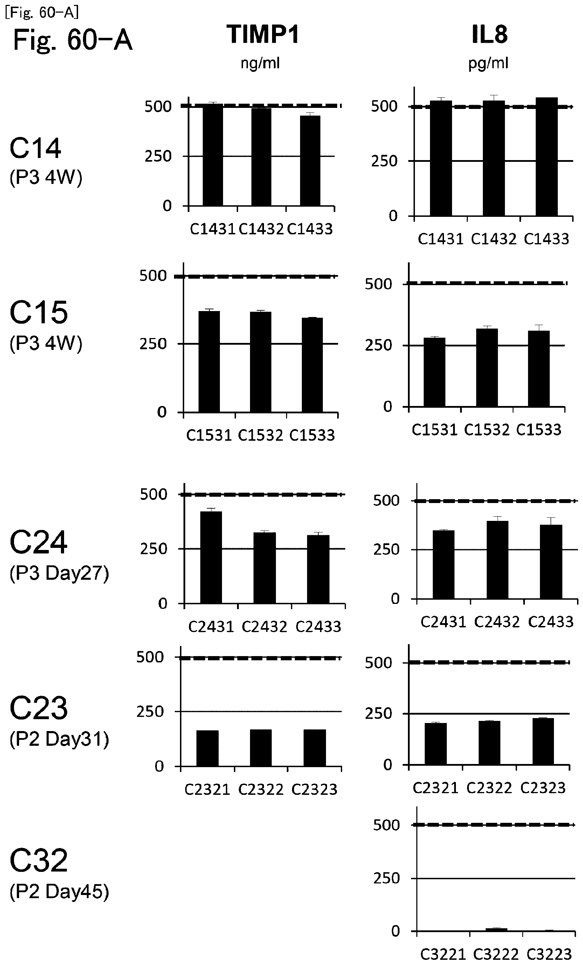

   Figs.60-A to 60-B show an ELISA assay of culture supernatant to evaluate the quality of cHCECs. Quantification was repeated three times. The graphs show the amount of TIMP1, [L8, PDGFbb, or MCP1 secreted in each culture. Figs.60-A to 60-B shows, from the top, week 4 of C14 third passage, week 4 of C15 third passage, day 27 of C24 third passage, day 31 of C23 second passage, and day 45 of C32 second passage.
[Fig. 60-B]
   Figs.60-A to 60-B show an ELISA assay of culture supernatant to evaluate the quality of cHCECs. Quantification was repeated three times. The graphs show the amount of TIMP1, [L8, PDGFbb, or MCP1 secreted in each culture. Figs.60-A to 60-B shows, from the top, week 4 of C14 third passage, week 4 of C15 third passage, day 27 of C24 third passage, day 31 of C23 second passage, and day 45 of C32 second passage.
[Fig. 61-A]

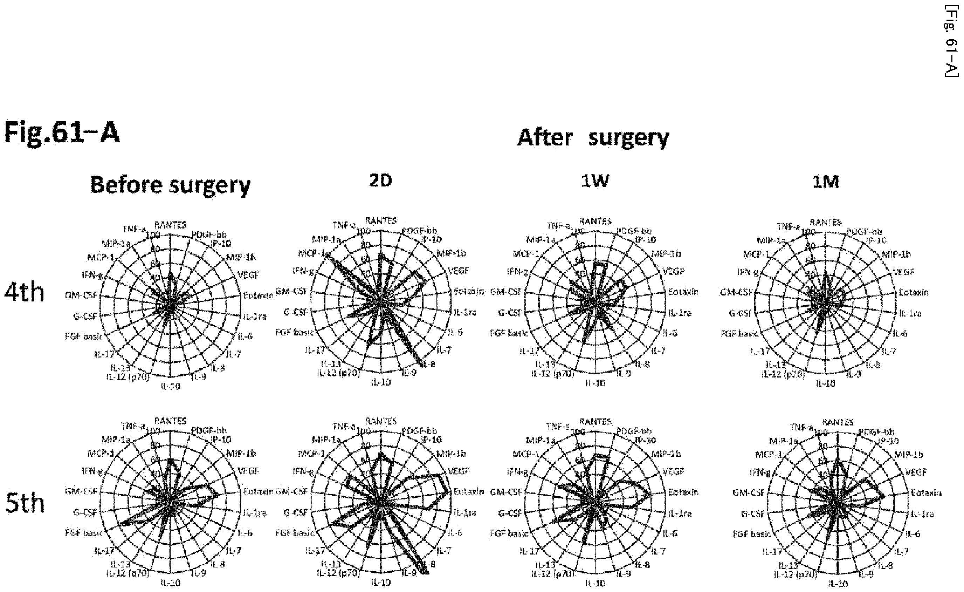

   Fig. 61-A shows diagrams of cytokine profiles. Fig. 61-A shows profiles of cytokine levels of serum of patients infused with a low quality cHCEC. Fig. 61-A shows a comparison of profiles of pre cHCEC infusion, 2 days after surgery, 1 week after surgery, and 1 month after surgery. The amount of cytokines in the serum of patients at each time is represented by a relative value. Fig. 61A shows that a low quality cells elicit an unintended biological response.
[Fig. 61-B]

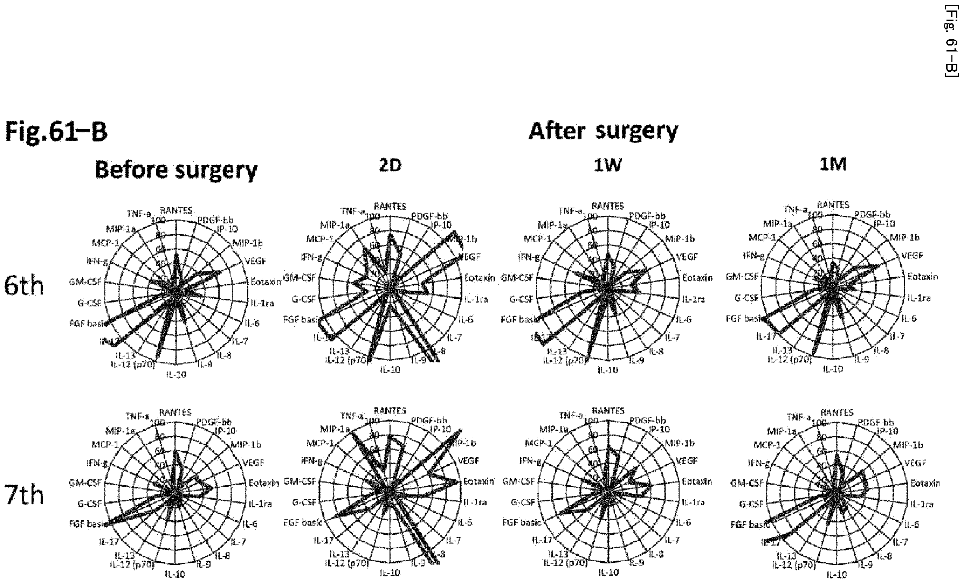

   Fig. 61-B shows diagrams of cytokine profiles. Fig. 61-B shows profiles of cytokine levels of serum of patients infused with a low quality cHCEC. Fig. 61-B shows an example of surgery that is different from that in Fig. 61-A. Fig. 61-B shows a comparison of serum cytokine profiles of pre cHCEC infusion surgery, 2 days after surgery, 1 week after surgery, and 1 month after surgery. The amount of cytokines in the serum of patients at each time is represented by a relative value. Fig. 61-B shows that a low quality cell elicits an unintended biological response.
[Fig. 62]

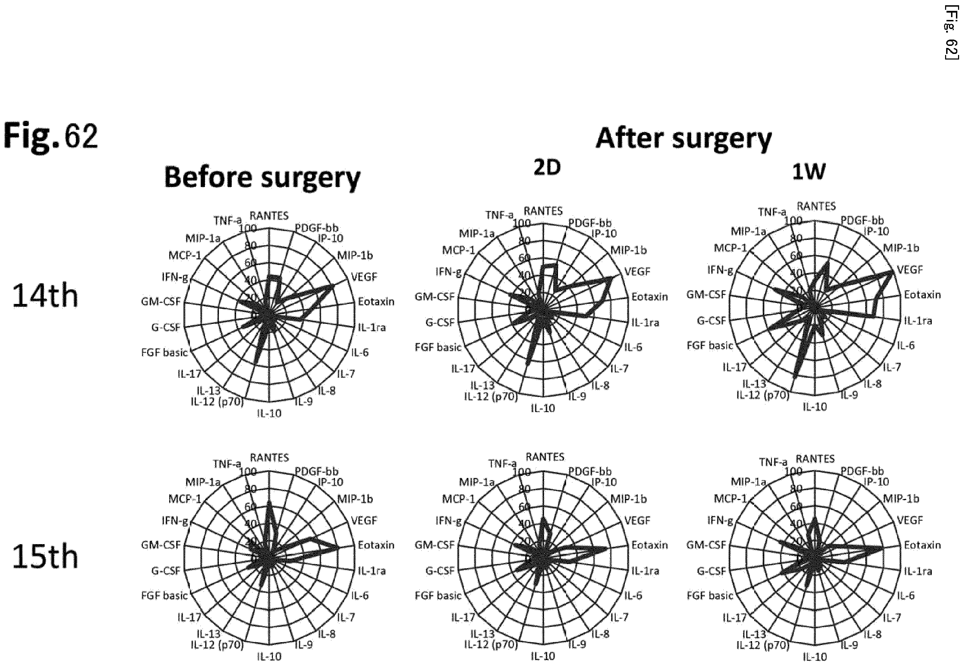

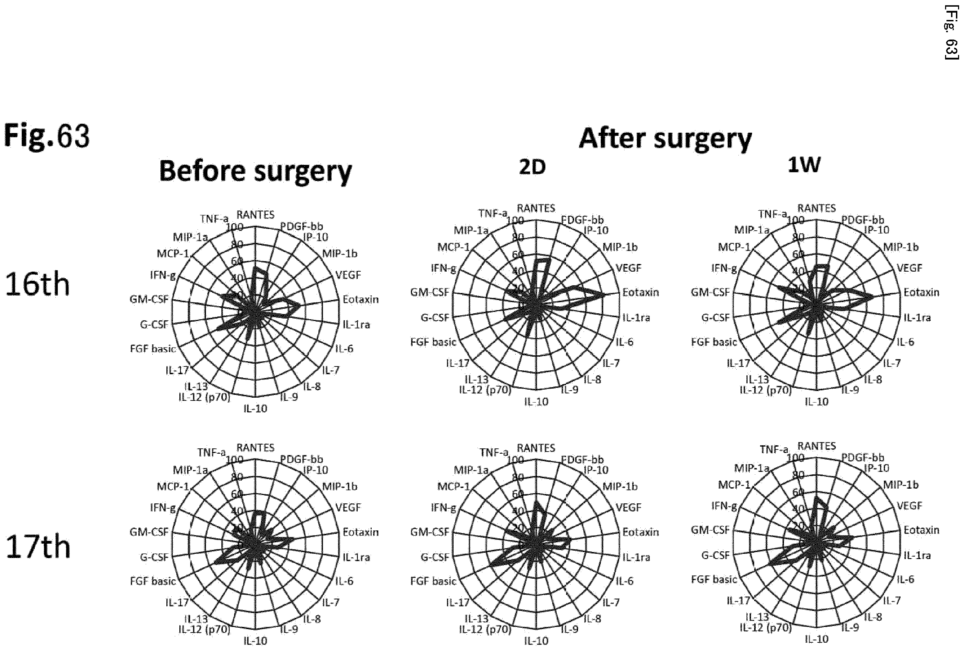

   Figs. 62 and 63 show diagrams of cytokine profiles. Figs. 62 to 63 show profiles of cytokine levels of serum of patients infused with high quality cHCECs. Figs.62 to 63 show a comparison of profiles of pre cHCEC infusion surgery, 2 days after surgery, and 1 week after surgery. The amount of cytokines in the serum of patients at each time is represented by a relative value. Figs.62 to 63 show that a high quality cell tends not to elicit an unintended biological response.
[Fig. 63]
   Fig. 63 shows diagrams of cytokine profiles. Fig. 63 shows profiles of cytokine levels of serum of patients infused with high quality cHCECs. Fig. 63 shows an example of surgery that is different from that in Fig. 62. Fig. 63 shows a comparison of profiles of pre cHCEC infusion surgery, 2 days after surgery, and 1 week after surgery. The amount of cytokines in the serum of patients at each time is represented by a relative value. Fig. 63 shows that a high quality cell tends not to elicit an unintended biological response.
[Fig. 64-A]

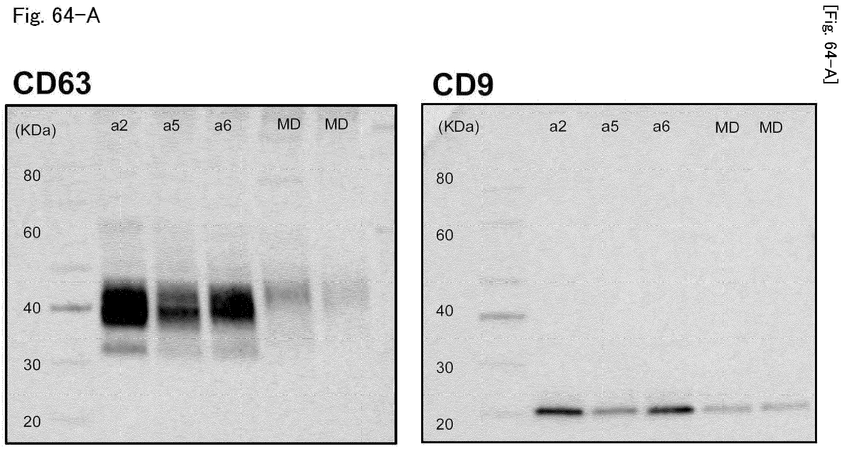

   Fig. 64-A is a diagram showing results of Western blot using anti-CD63 and anti-CD9 antibodies for detecting the secreted exosomes in culture supernatants from cHCECs either with or without CST.
[Fig. 64-B]

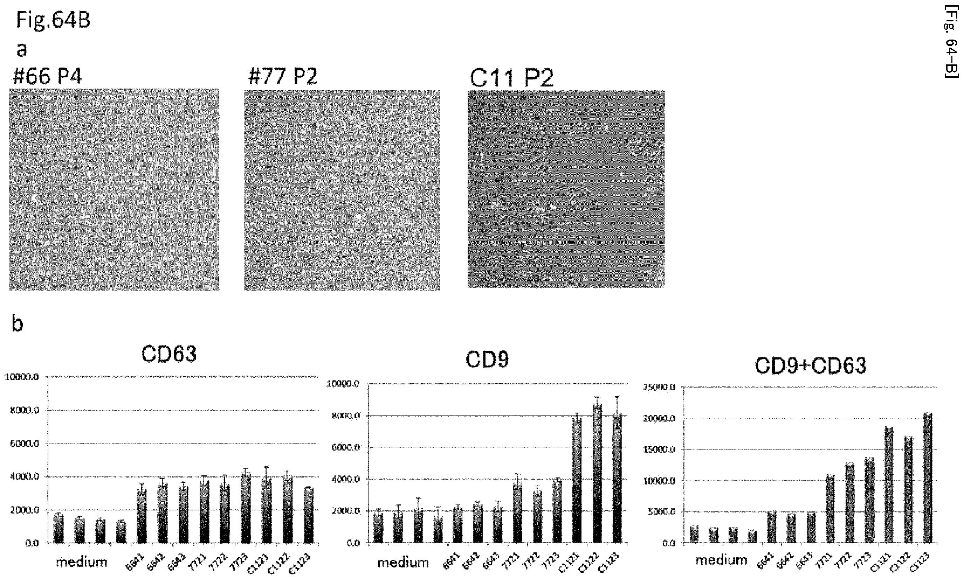

   Fig. 64-B describes phase contrast microscope images showing the morphology of cells of #66 (fourth passage), #77 (second passage) and C11 (second passage) in the top row. Fig. 64-B is a graph showing the amount of exosome detected by ExoScreen by using CD9 and/or CD63 in each cell culture media in the bottom row.
[Fig. 65]

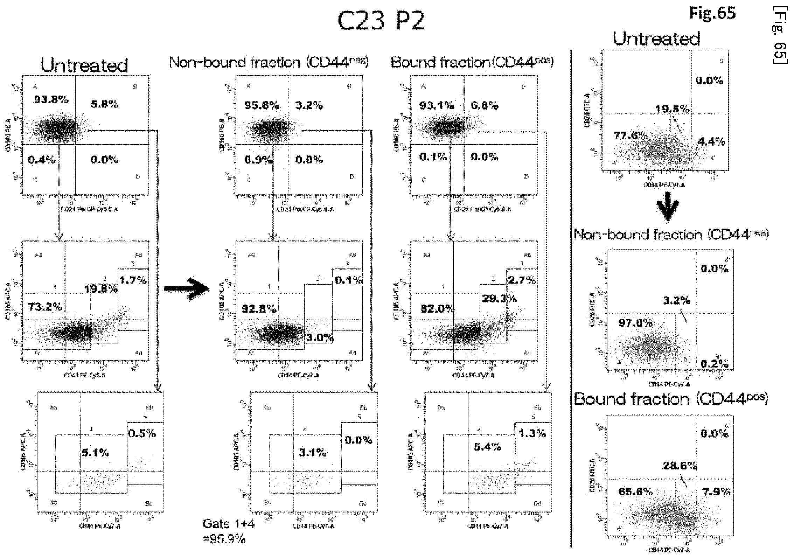

   Fig. 65 provides results of FACS analysis showing a change in the cell population composition by using magnetic bead cell sorting (MACS) with CD44 magnetic beads. C23 (second passage) cHCECs were subjected to the analysis. Dot plots with gating show, from the left, untreated with MACS, non-bound fraction from MACS, and bound fraction from MACS. Fig. 65 shows analysis on expression of CD105 and CD44 after gating for expression of CD166 and CD24. The dot plots for expression of CD26 and CD44 on the right side show, from the top, untreated with MACS, non-bound fraction from MACS, and bound fraction from MACS.
[Fig. 66]

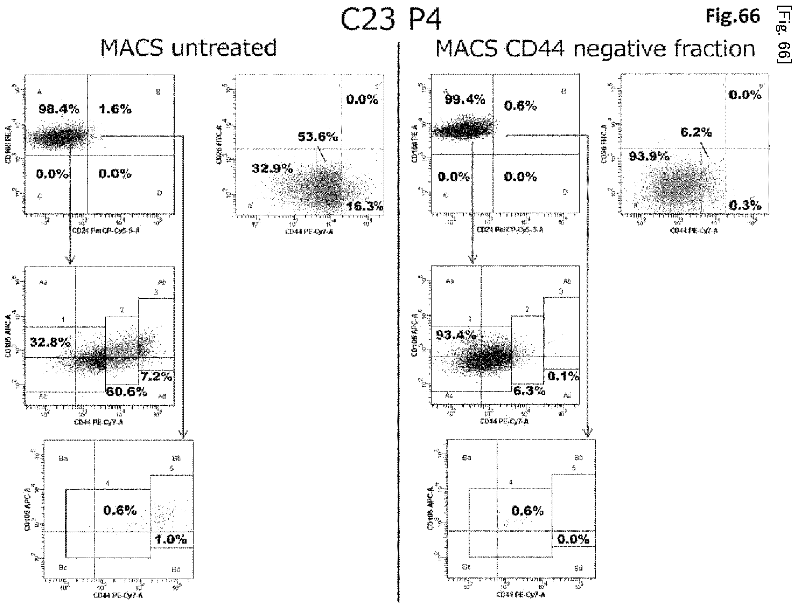

   Fig. 66 provides results of FACS analysis showing a change in the cell population composition by using magnetic bead cell sorting (MACS) with CD44 magnetic beads. C23 fourth passage cHCECs were subjected to the analysis. The left side shows a case that was untreated with MACS and the right side shows non-bound fraction from MACS. Fig. 66 shows analysis on expression of CD105 and CD44 and analysis on expression of CD26 and CD44 after gating for expression of CD166 and CD24.
[Fig. 67]

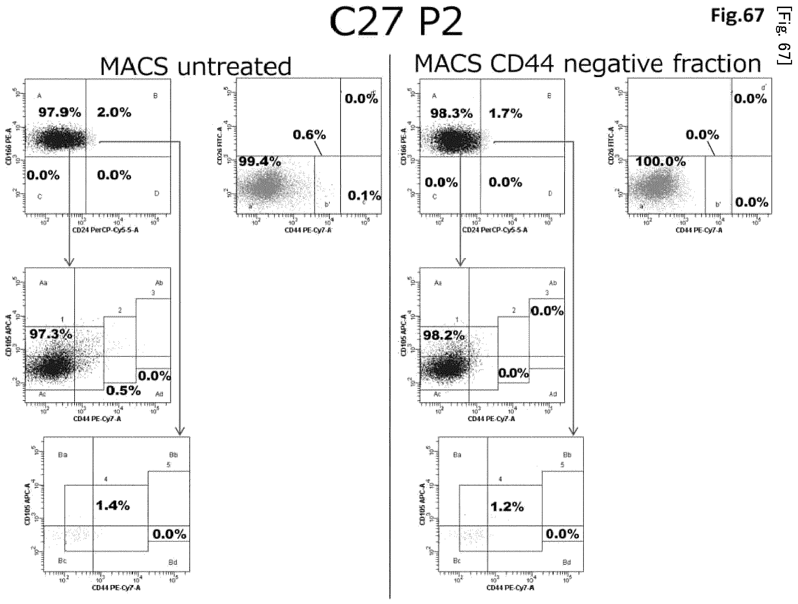

   Fig. 67 provides results of FACS analysis showing a change in the cell population composition by using magnetic bead cell sorting (MACS) with CD44 magnetic beads. C27 second passage cHCECs were subjected to the analysis. The left side shows a case that was untreated with MACS and the right side shows non-bound fraction from MACS. Fig. 67 shows analysis on expression of CD105 and CD44 and analysis on expression of CD26 and CD44 after gating for expression of CD166 and CD24.
[Fig. 68]

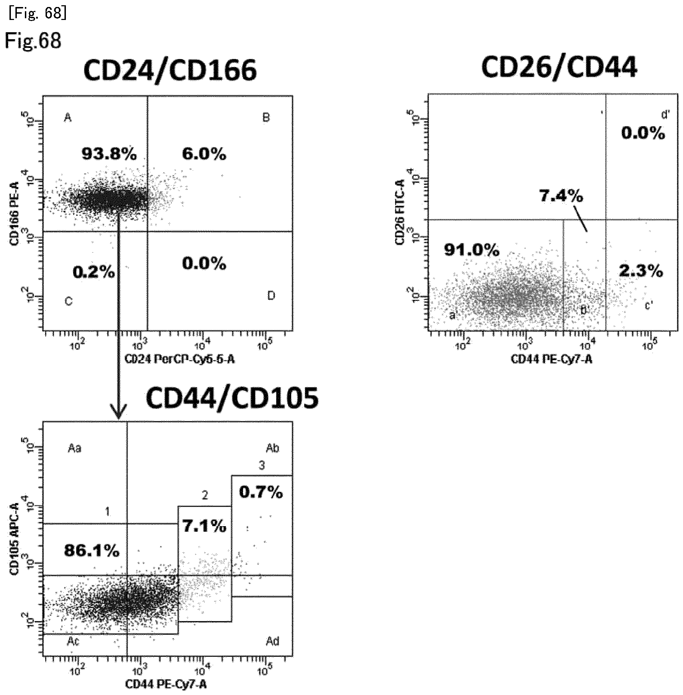

   Fig. 68 is a diagram showing the method to estimate the content of effector (E-ratio) in a cultured cell population by FACS analysis. The gate is set as followings in measurements using PE-Cy7-labeled anti-human CD44 antibodies (BD Biosciences) and setting the Area Scaling Factor of Blue laser of FACS Canto II to 0.75 and voltage of PE-Cy7 to 495. First, fractions A, B, C, and D are set in the dot plot (top row left) where the X axis is CD24 and the Y axis is CD166. At this time, fraction A is CD24 negative and CD166 positive, fraction B is CD24 positive and CD166 positive, fraction C is CD24 negative and CD166 negative, and fraction D is CD24 positive and CD166 negative. The proportion of fraction B when target cells for analysis are 100% is considered the content of non-intended cell C [CD24 positive cell]. Fractions 1, 2, and 3 are further set as in the diagram in the bottom row on the left in a dot plot where the X axis is CD44 and the Y axis is CD105 for fraction B. The proportion of fraction 1 at this time is the E-ratio, the total value of the proportion of fractions 1 and 2 is the "effector cell + progenitor cell" content, and the proportion of fraction 3 is the non-intended cell A [CD44 strongly positive cell] content. Further, a separate dot plot where the X axis is CD44 and the Y axis is CD26 is created and fractions a', b', c', and d' are set as in the diagram on the top row right side. The proportion of fraction B when the target cells of analysis is 100% is the non-intended cell B [CD26 positive cell] content.
[Fig. 69]

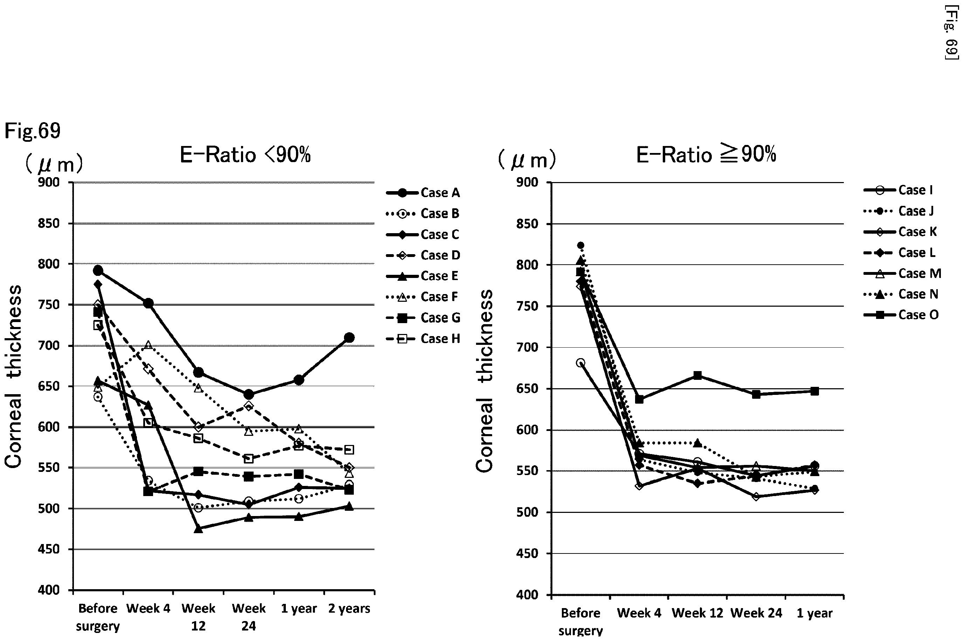

   Fig. 69 shows post-surgery corneal thickness depend on the cell quality (E-ratio) infused and provides graphs showing results of measuring the corneal thickness before cell infusion, after 1 month, after 3 months, and after 6 months (after 4 weeks, after 12 weeks, and after 24 weeks, respectively), and after 1 year and after 2 years for patients A-N who were injected with a cell population having an E-ratio of less than 90% and patients I-O who were infused with a cell population having an E-ratio of 90% or greater. The horizontal axis is time (before surgery, after 1 month, after 3 months, and after 6 months (after 4 weeks, after 12 weeks, and after 24 weeks, respectively), and after 1 year and after 2 years), and the vertical axis is the corneal thickness. It can be understood that thinning is extremely better accomplished at an early stage by the functional cells having an E-ratio of 90% or greater.
[Fig. 70]

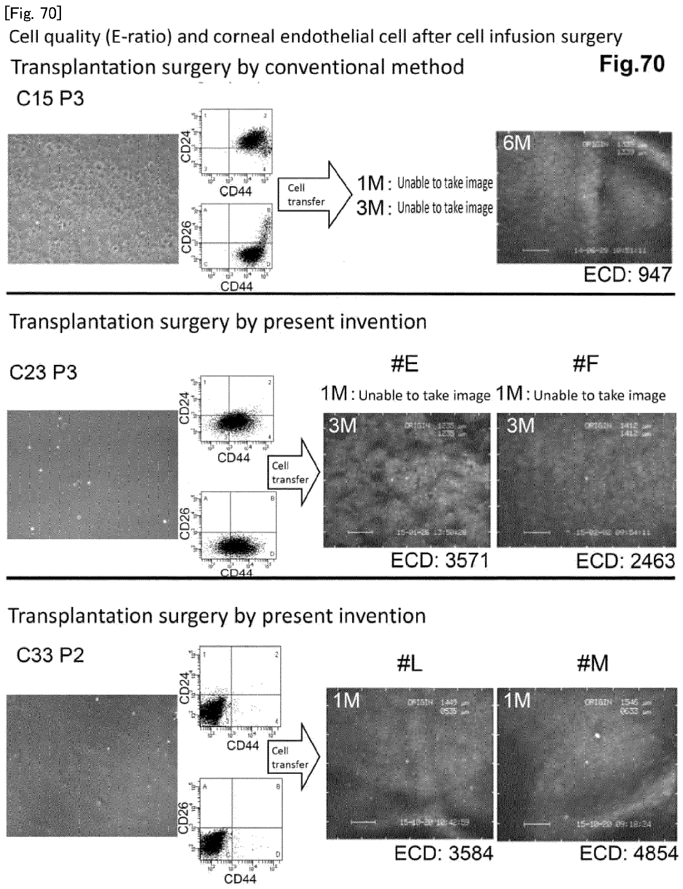

   Fig. 70 shows a comparison of the clinical outcome with cHCECs distinct in their E-ratio. The top row shows results of cHCEC infusion surgery by C15 (passage 3) with very low E-ratio. The middle row shows results of cHCEC infusion surgery by C23 (passage 3) (E-ratio < 90%) according to the present invention. The bottom row shows results of cHCEC infusion surgery by C32 (passage 2) (E-ratio >= 90%) according to the present invention. In each row, Fig. 70 shows, from the left, results of a phase contrast microscope picture, FACS analysis based on CD24, CD26, and CD44 of infused cells, and specular microscopy picture after the infusion surgery on the right side as well as the numerical value of ECD in the endothelium tissue (cells/mm²). When a cell population with low E-ratio (<10%) was infused, cells attached to endothelium tissue could not be detected after 1 month or 3 month due to opacity. Only after6 months cells attached to endothelium tissue could be detected. When the infusion surgery (with subpopulation selection, E-ratio < 90%) according to the present invention was used, cells could not be detected after one month due to opacity, but this state improved after 3 months such that cells could be detected. As shown in the bottom row, when transplantation surgery (with subpopulation selection, E-ratio >= 90%) according to the present invention was used, opacity already improved after 1 month, such that cells could be detected. It was revealed that the cells prepared by the technology firstly developed in the present invention exert an effect notably earlier compared to infusion of cHCECs with low E-ratio.
[Fig. 71]

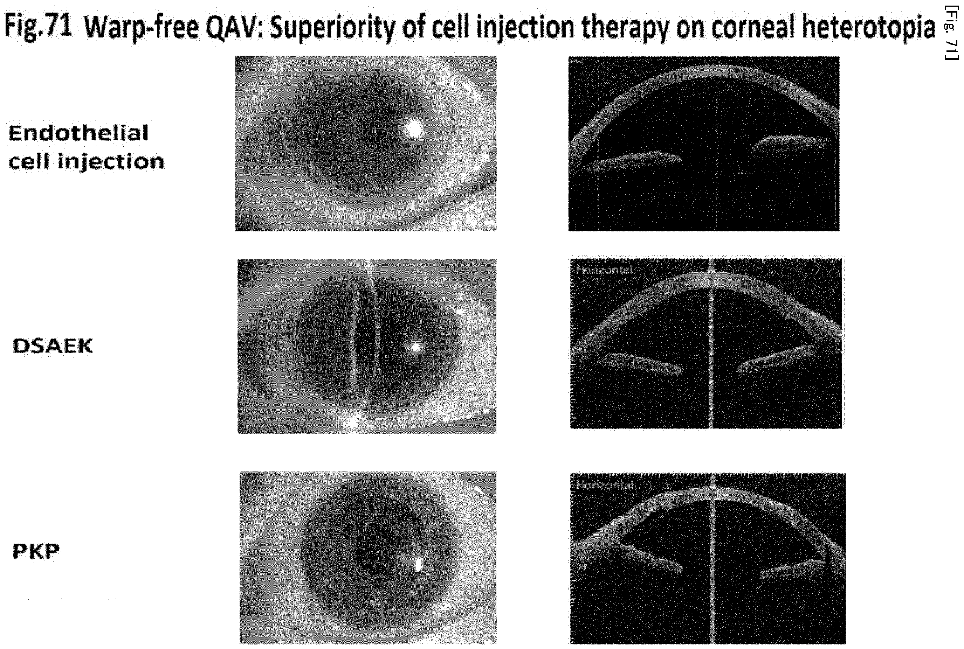

   Fig. 71 shows post-surgery results in cultured endothelial cell infusion of the present invention (top row), DSAEK (conventional method; middle row), and PKO (corneal transplantation, conventional method; bottom row). The left side shows a picture of an eye ball after each surgery and the center of the top row shows the distribution of corneal thickness. The right side shows horizontal cross-sectional pictures. While endothelial injection of the present invention resulted in a corneal reconstitution without distortion, resulting in recovering a good QAV, DSAEK resulted in a surface with distortion and a surface due to the surgical method. PKP results in a notable distortion.

### [Description of Embodiments]

The embodiment of the present invention is disclosed hereinafter. To avoid complicating the disclosure with repeating the same content, explanation is appropriately omitted. Throughout the entire specification, a singular expression should be understood as encompassing the concept thereof in the plural form, unless specifically noted otherwise. Thus, singular articles (e.g., "a", "an", "the" and the like in case of English) should also be understood as encompassing the concept thereof in the plural form unless specifically noted otherwise. Further, the terms used herein should be understood to be used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

First, the terms and common techniques used in the present invention are explained.

As used herein, "corneal endothelium" and "human corneal endothelium" are used in the meaning that is commonly used in the art. The cornea is one of the lamellar tissues constituting an eye. A cornea is transparent and positioned at a part closest to the external environment. In humans, it is understood that the cornea is comprised of five layers, in order from the outside (body surface), of corneal epithelium, Bowman's membrane (external boundary), Lamina propria, Descemet's membrane (internal boundary), and corneal endothelium. Unless specifically noted otherwise, parts other than epithelium and endothelium may be collectively called "corneal stroma", which is also called as such herein.

As used herein, a cell desired from corneal endothelial tissue is referred to as "corneal endothelial tissue derived cell". Further, a cell that becomes a corneal endothelial cell by differentiation is referred to as a "corneal endothelial progenitor cell".

As used herein, "human functional corneal endothelial cell capable of eliciting a corneal endothelial functional property when infused into an anterior chamber of a human eye" refers to "cell with functionality of a corneal endothelium, having the ability to express a corneal endothelial functional property (referred to as "human corneal endothelial functional property" when referring to humans, or simply referred to hereinafter although not especially limiting, as "corneal endothelial functional property") when infused into the anterior chamber of a human eye. This is also referred to as the "corneal endothelial property possessing functional cell" especially for abbreviation. When used for a human cell, this is referred to as "human functional corneal endothelial cell capable of eliciting a human corneal endothelial functional property when infused into an anterior chamber of a human eye". Since the present invention is mainly concerned with human corneal cells, it is understood that a human cell is referred unless specifically noted otherwise. As used herein, the corneal endothelial property possessing functional cells of the invention encompass "functional mature differentiated corneal endothelial cell" having a corneal endothelial functional property as such without further processes and "intermediately differentiated corneal endothelial cell", which lacks some of the functions, but is used similarly or exert the same function as a functional mature differentiated corneal endothelial cell after infusion.

As used herein, "corneal endothelial functional property" refers to a functional property that a mature differentiated cornea has in a normal state.

As used herein, "functional mature differentiated corneal endothelial cell" refers to a mature differentiated corneal endothelium and any cell having its function (e.g., the above-described corneal endothelial functional property). This is referred to as a functional mature differentiated human corneal endothelial cell for human cells. In particular, a corneal endothelial functional property can be confirmed from forming a small hexagonal cobble-stone shape and using an energy metabolism system by mitochondrial function. It is possible to determine whether the property can have a therapeutic effect when infused (e.g., into the anterior chamber of a human eye). However, this is not limited thereto. A corneal endothelial functional property can be judged or determined by using a surrogate marker as an indicator. Judgment can be made by any one of the 8 types of such surrogate markers or a combination thereof, including (1) retention of endothelial pumping/barrier functions (including Claudin expression), (2) adhesion/attachment to a specific laminin, (3) secreted cytokine profile, (4) produced micro RNA (miRNA) profile, (5) produced metabolite profile, (6) saturated cell density upon in vitro culture (7) spatial size and distribution of cells obtained in culturing, and (8) cell retention in case of cell infusion after freeze damage cryo treatment by liquid nitrogen on a mouse cornea.
(1) Retention of endothelial pumping/barrier functions can be judged by using a pumping function measuring method or a barrier function measuring method commonly used for corneal endothelia. Examples of such judgment include techniques of applying the methods described in Wigham C, Hodson S.: Current Eye Research, 1, 37-41, 1981, Hodson S, Wigham C.: J Physiol., 342:409-419, 1983, Hatou S., Yamada M., Akune Y., Mochizuki H., Shiraishi A., Joko T., Nishida T., Tsubota K.: Investigative Ophthalmology & Visual Science, 51, 3935-3942, 2010 by using a Ussing chamber utilized in case of a sheet form. Claudin expression can be confirmed by using a known approach in the art such as immunological approach. Any immunological approach known in the art can be used to confirm Claudin expression. However, the cells of the present invention are expected to be infused in a suspension. In such a case, it is thus preferable to assess a corneal endothelial function by applying Claudin expression, or any one of (2)-(8) or a combination thereof.
(2) Adhesion/attachment to a specific laminin can be judged using adhesion to laminin 511 (composite of alpha5 chain, beta1 chain, and gamma1 chain), laminin 521 (composite of alpha5 chain, beta2 chain, and gamma1 chain), or a functional fragment thereof (e.g., laminin 511-E8 fragment) and/or increase in integrin (e.g., alpha3beta1, alpha6beta1 or the like) expression with respect thereto as an indicator. Such an approach can be implemented by a cell adhesion assay illustrated in Example 6.

In this regard, laminin alpha chains are discussed. "alpha5 chain" (LAMA5) is a subunit of a protein-laminin of a cell adhesion molecule in an extracellular matrix, and is called LAMA5, KIAA1907, or the like. For human LAMA5, the sequences of the gene and protein are registered as NCBI registration numbers NM_005560 and NP_005551, respectively. OMIM is identified by the accession number 601033. Laminin beta chains are discussed. "beta1 chain" (LAMB1) is a subunit of a protein (laminin) of a cell adhesion molecule in an extracellular matrix, and is called LAMB1, CLM, LIS5, or the like. For human LAMB1, the sequences of the gene and protein are registered as NCBI registration numbers NM_002291 and NP_002282, respectively. OMIM is identified by the accession number 150240. "beta2 chain" (LAMB2) (laminin S) is a subunit of a protein (laminin) of a cell adhesion molecule in an extracellular matrix, and is called LAMB2, LAMS, NPHS5, or the like. For human LAMB2, the sequences of the gene and protein are registered as NCBI registration numbers NM_002292 and NP_002283, respectively. OMIM is identified by the accession number 150325. Laminin gamma chains are discussed. "gamma1 chain" (LAMC1) is a subunit of a protein (laminin) of a cell adhesion molecule in an extracellular matrix, and is called LAMC1, LAMB2, or the like. For human LAMC1, the sequences of the gene and protein are registered as NCBI registration numbers NM_002293 and NP_002284, respectively. OMIM is identified by the accession number 150290.
(3) Secreted cytokine profiles can be judged by measuring the production level of cytokines profiles in "serum" or "anterior aqueous humour" explained elsewhere herein. Such cytokines include, but are not limited to, RANTES, PDGF-BB, IP-10, MIP-1b, VEGF, EOTAXIN, IL-1ra, IL-6, IL-7, IL-8, IL-0, IL-10, IL-12 (p70), IL-13, IL-17, FGFbasic, G-CSF, GM-CSI, IFN-gamma, MCP-1, MIP-1a, TNF-alpha, and the like. Specifically, analysis can be performed using a cytokine measuring kit and analysis system such as Bio-Plex for integrated analysis of cytokines. The approach thereof is exemplified in Example 9.
(4) The produced microRNA (miRNA) profile can be judged by measurement using the analytical approach for "miRNA profile" explained elsewhere herein. For instance, judgment can be materialized by using a method of analyzing a microRNA expression profile described in Example 5 or 9. For example, Toray's "3D-Gene" human miRNA oligochip (miRBase version 17) can be used for the implementation thereof. Total RNAs obtained from samples of both tissue and cells, which is labeled with total miRNA obtained from supernatant and those labeled with a label such as Hy5 by using a kit such as miRCURY LNA(R) microRNA Power Labeling Kits (Exiqon, Vedbaek, Denmark) are prepared. Labeled microRNA is separately hybridized to the surface of a microRNA chip and incubated under a suitable condition (e.g., 32.degrees.C for 16 hours). After this microRNA chip is washed and dried in an ozone-free environment, a scanner such as 3D-Gene scanner 3000 (Toray Industries Inc., Tokyo, JAPAN) can be used for scanning, and 3D-Gene Extraction software (Toray) can be used for analysis.
(5) The produced metabolite profile can be judged, for example, by the method described in Example 4. The metabolic extract of an intracellular metabolite is prepared from a cHCEC culture container having methanol containing an internal standard reagent such as Internal Standard Solution (Human Metabolome Technologies; HMT, Inc., Tsuruoka, Japan). The medium is replaced and cell extract is treated (treatment condition is exemplified in Example 4). CE-MS analysis is preformed to analyze the metabolite. Metabolome analysis can be measured according to the method developed by Soga, et al. (Soga, D. et al., T. Soga, et al., Anal. Chem. 2002; 74: 2233-2239 Anal. Chem. 2000; 72: 1236-1241; T. Soga, et al., J. Proteome Res. 2003; 2: 488-494) and automatic integration software (MasterHands, Keio University, Tsuruoka, Japan (M. Sugimoto, et al., Metabolomics, 2009; 6: 78-95) and MassHunter Quantitative Analysis B.04.00, Agilent Technologies, Santa Clara, CA, USA) is appropriately used for analysis. From the HMT metabolite database, the peak is annotated by a hypothetical metabolite, standardized, and calculated based on the m/z value measured by MT and TOFMS in CE. Hierarchical cluster analysis (HCA) and principal component analysis (PCA) can be performed for metabolome measurement.
(6) The saturated cell density during in vitro culture can be judged by measuring the cell density by using appropriate culture conditions described herein. This may be measured in parallel with the cell size. Photo-taking phase contrast microscope images are taken using an equipment comprising an image capturing system such as a BZ X-700 Microscope system (Keyence, Osaka, Japan) by an inverted microscope system (CKX41, Olympus, Tokyo, Japan). The density can be quantified by using a cell counting software (e.g., BZ-H3C Hybrid cell count software (Keyence)). Preferred saturated cell density in the present invention is described elsewhere herein.
(7) The spatial size and distribution of cells obtained in culture can be judged by taking pictures of cells and taking measurements with any software or the like or by measuring the special size and distribution of cells by using appropriate culture conditions described herein. This can be materialized by using raw image processing software such as BZ-H3C Hybrid cell count software (Keyence). The preferred saturated cell density in the present invention is described elsewhere herein.
(8) Cell retention in case of cell infusion after freeze damage with cryo treatment by liquid nitrogen on mouse cornea can be judged by making a mouse model exemplified in Example 7. Specifically, the center region (e.g., 2mm) of a cornea of a suitable mouse (e.g., BALB/c) is pretreated by low temperature damage to remove an endothelial cell to make a model. The cell to be judged is injected into the ocular anterior chamber of the model. The characteristics of the corneal clarity are clinically observed. The corneal thickness is assessed by a pachymeter. The adhesion of HCECs is histopathologically tested by human nuclear staining and the cells are examined whether they have a function. These approaches are exemplified in Example 8.

A cell that is not derived from corneal endothelium (e.g., cell produced by having induced pluripotent stem cell (iPS cell), embryonic stem cell (ES cell) or the like differentiate into corneal endothelia), although it is unclear whether it is completely identical to a corneal endothelial cell in a living body, is within the scope of the functional corneal endothelial cell or functional mature differentiated corneal endothelial cell capable of eliciting a corneal endothelial functional property when infused into the anterior chamber of a human eye of the present invention, as long as it has a corneal endothelial functional property. In the explanation or experiment set forth herein, the human functional mature differentiated corneal endothelial cell of the present invention is also called "human functional mature differentiated corneal endothelial cells", "functional mature differentiated human corneal endothelial cell" or the like. While such a cell may also be called "a5" cell, "cells of interest", "qualified cell", simply as "effector cell" or the like, they are all used synonymously. Further, "functional mature differentiated corneal endothelial cell" with enhanced functionality may be referred to as "high quality" functional mature differentiated corneal endothelial cell. Such a high quality cell can be provided by selectively propagating those that are CD44 negative. Although not wishing to be bound by any theory, it is understood that a collection of only mature differentiated functional corneal endothelial cells that are negative has higher quality and higher identity with mature differentiated cells in a living tissue.

As used herein, "intermediately differentiated corneal endothelial cell" refers to a cell that can exert a corneal endothelial functional property (human corneal endothelial functional property for human cells) of a functional mature differentiated corneal endothelial cell but does not have a complete corneal endothelial functional property as in functional mature differentiated corneal endothelial cells and exerts at least a part of the functions thereof. Such a cell, when used for human cells, is referred to as "intermediately differentiated corneal endothelial cells" or "human intermediately differentiated corneal endothelial cell". Meanwhile, it should be noted that the present invention primarily targets humans. Since "intermediately differentiated corneal endothelial cell" has an ability that could function as a functional mature differentiated corneal endothelial cell after infusion into the ocular anterior chamber, they can be used in the present invention. "Intermediately differentiated corneal endothelial cell", up to a certain level, exerts an effect when mixed in and used in infusion therapy or at least would not inhibit the therapeutic effect. Such an intermediately differentiated corneal endothelial cell can mature into, differentiate into, and function as a functional mature differentiated corneal endothelial cell in a living body after infusion (e.g., after infusion into the anterior chamber of a human eye). In the explanation or experiment set forth herein, such a cell is also referred to as an "a1" cell, simply "corneal endothelial semi-functional cell", "semi-functional cell", "intermediately differentiated effector cell", "cell of secondary interest" and the like, which are synonymous.

As used herein, "corneal endothelial nonfunctional cell" is a cell other than the corneal endothelial property possessing functional cell of the invention (i.e., "functional mature differentiated corneal endothelial cell" and "intermediately differentiated corneal endothelial cells"). Such a cell may be called "non-intended cell", "non-qualified cell", "unintended cell", nonfunctional cell", or the like. Such a cell includes the "a2" fraction.

As used herein, "cell indicator" refers to any indicator indicating that a certain cell is the corneal endothelial property possessing functional cell of the invention (e.g., functional mature differentiated corneal endothelial cell or intermediately differentiated corneal endothelial cell). Since a cell indicator is a property of mature differentiated human corneal endothelia and any cell with the function thereof, it is also referred to as "functional cell indicator". The specific property is also referred to as the "cell functional property". For instance, a case where an applicable cell has a cell functional property that is homologous to that of an a5 cell refers to the applicable cell having a value that corresponds to the range of each value of the cell indicator exhibited by a5.

As used herein, "transform" refers to a trait of a cell changing to a non-normal state, including a normal cell undergoing unrestrained cell division, i.e., oncogenesis, and especially dynamic metaplasia (dedifferentiation to be a stem cell or changes beyond the realm of basic form of tissue). Examples of transformation include cell state transition (CST) such as EMT, fibrosis, epithelial mesenchymal transition, senescence, dedifferentiation and the like. A corneal endothelial cell often undergoes transformation such as epithelial mesenchymal transition such that it is no longer a functional mature differentiated corneal endothelial cell in many cases. The manufacturing method of the present invention encompasses manufacturing methods that can convert such a cell which has undergone epithelial mesenchymal transition into a functional mature differentiated corneal endothelial cell by allowing such cell to mature and differentiate after dedifferentiation.

As used herein "epithelial mesenchymal transition" (EMT; also referred to as epithelial mesenchymal transformation) refers to a process of an epithelial cell losing cell polarity thereof and the ability to adhere to a surrounding cell and acquiring the ability to migrate and infiltrate to change into a mesenchymal-like cell.

In this regard, starting cells in various samples and manufacturing methods that can be used herein may be functional mature differentiated corneal endothelial cells, cells of interest, or sample considered as comprising a substance derived therefrom that enables gene expression. For example, a cell directly isolated from a corneal endothelium (also referred to as corneal endothelial tissue derived cells) or cell that has acquired a corneal endothelium-like function from differentiation can be used. A corneal endothelial tissue derived cell can be obtained by a known method (Koizumi N, Okumura N, Kinoshita S., Experimental Eye Research. 2012; 95: 60-7). Preferably, a cell and the like obtained from a corneal endothelium donor can be used as a cell sample. Further, cultured cells comprising the corneal endothelial property possessing functional cells of the invention or functional mature differentiated corneal endothelial cells, which were differentiated and induced in vitro, can be used as the sample. The cells can be differentiated and induced in vitro into the corneal endothelial property possessing functional cells of the invention or functional mature differentiated corneal endothelial cells by processing with a known method such as the AMED method or the like <Ueno M, Matsumura M, Watanabe K, Nakamura T, Osakada F, Takahashi M, Kawasaki H, Kinoshita S, Sasai Y:, Proc Natl Acad Sci USA. 103(25): 9554-9559, 2006.> while using a known cell such as ES cell, iPS cell, or bone marrow stromal cell as the starting material.

In the present invention, "miRNA" is an abbreviation of microRNA and refers to RNA which is encoded on the genome and generated though a multistep generation process. Various types of miRNAs have been discovered. Generally, RNA has a length of 20-25 bases, but the length is not limited thereto. Known miRNAs are registered at microRNA database, miRBase (hGp://www.mirbase.org/) or the like. miRNA is generally denoted as "mir" for progenitors and "miR" for mature forms. While a registration number is appended after miR (mir), a lower case alphabet is appended in case of similarity. When defined by appending the origin of the generation process, 5p is appended for a chain on the 5' terminus side and 3p is appended to a chain on the 3' terminus side. To distinguish species, hsa is appended for human. They are to be linked with a hyphen to denote, for example, as "hsa-miR-15a-5p" or the like. Since humans are primarily targeted herein, it is understood that humans are intended even without specifically appending hsa. When used with specific distinction in the present invention, it is possible to differentiate "intracellular" miRNA and "secreted" miRNA. One of the features of the present invention is that it was discovered, for the first time worldwide, that miRNA can be used to identify a subpopulation of cells, including the discovery that cell type or subpopulation can be identified with "secreted" miRNA secreted in cell supernatant, which can be used in quality control in cell infusion therapy.

As used herein, "intracellular" miRNA refers to any miRNA that is present in a cell.

As used herein, "secreted" miRNA refers to any miRNA that is secreted and can be detected in culture supernatant. While such miRNA may also be called "cell secreted" miRNA, miRNA "in culture supernatant", "cell supernatant" miRNA, or "cell supernatant/cultured" miRNA in the art, they refer to the same miRNA. Secreted miRNA can be detected without destroying a cell.

As used herein, "high expression", "intermediate expression", and "low expression" of miRNA or the like is used to describe the relative expression intensity of miRNA and refer to relative intensity compared to a standard. For "high expression", "intermediate expression", and "low expression", the expression intensity is high expression > intermediate expression > low expression. It is possible herein to use a value corrected to have identical median expression intensity of all genes detected by assuming that the total number of copies of gene among samples is not notably different after determining genes whose fluorescence intensity has been measured as amount of miR expression (expression intensity). "High expression" and "low intensity" have a statistically significant difference (relative ratio of 2 or greater, p-value of 0.05 or less) in terms of expression intensity. Intermediate expression can be included as needed. When a third expression intensity that is different from the strongest and weakest is found in three or more groups of cells, assessment may include intermediate expression. Further, "high expression" and "intermediate expression" as well as "low expression" and "intermediate expression" may also have statistically significant difference.

For use herein, when specifying the expression property of an a5 cell as CD44 negative to weakly positive CD24 negative CD26 negative, the expression property of a1 as CD44 intermediately positive CD24 negative CD26 negative, and the expression property of a2 as CD44 strongly positive CD24 negative CD26 positive as a representative example, "high expression", "intermediate expression", and "low expression" of miRNA are used to relatively express the expression intensity of a5 cell: a1 cell: a2 cell. It should be noted that each cell can be identified by "high expression" and "low expression" in cases without "intermediate expression".

As used herein, "cell size" is one of the cell indicators of the corneal endothelial property possessing functional cell of the invention, which is measured by techniques that are commonly used in the art. The cell size is expressed, for example, by cell area. As used herein, "cell area" is one of the cell indicators of the corneal endothelial property possessing functional cell of the invention. Cell area can be measured with any software or the like by taking a picture of a cell. Examples of such a measuring approach include a method utilizing image processing software such as BZ-H3C Hybrid cell count software (Keyence). The mean value thereof is referred to as "mean cell area". The arithmetic mean is generally used.

As used herein, "cell density" or "(mean) cell density" is an indicator of a cell expressed by the number of cells present in a certain area. Cell density is measured by any technique that is commonly used in the art. The mean density of a cell population is one of the cell indicators of the corneal endothelial property possessing functional cell of the invention or functional mature differentiated corneal endothelial cell. Arithmetic mean is generally used as the mean. Cell density may be measured in parallel with the cell size and quantified by taking photo-taking phase contrast microscope images using an equipment comprising an image capturing system such as a BZ X-700 Microscope system (Keyence, Osaka, Japan) by an inverted microscope system (CKX41, Olympus, Tokyo, Japan) and using a cell counting software (e.g., BZ-H3C Hybrid cell count software (Keyence)) or the like. The cell density as of saturated cell culture (also referred to as (culture) confluence; saturated cell culture and (culture) confluence as used herein have the same meaning) is used as an indicator. In addition, density as of seeding is also used as a benchmark in the manufacturing method of the present invention. Further, cell density may be used as an indicator of a therapeutic result after infusion.

As used herein, "karyotype abnormality" refers to any karyotype with an abnormality. For humans, karyotype abnormalities can be measured in accordance with the Standard International System for Human Cytogenetic Nomenclature (ISCN) (1995) and definitions thereof.

As used herein, "immunological property", for a certain cell, refers to an immunological response exhibited between the cell and the host derived cell is one of the cell indicators of the corneal endothelial property possessing functional cell of the invention or functional mature differentiated corneal endothelial cell. Examples thereof include no immunological rejection while being allo (allogeneic) infusion.

As used herein, "gene property", for a certain cell, refers to a property such as expression of a gene related to the cell. A gene property is one of the cell indicators of the corneal endothelial property possessing functional cell of the invention or mature differentiated functional corneal endothelial cell.

As used herein, "cytokine profile in serum" is one of the cell indicators of the corneal endothelial property possessing functional cell of the invention or functional mature differentiated corneal endothelial cell and refers to a profile showing at least one of amount, level and the like of cytokine in the serum. Typically, the profile can be displayed by a circle centroid method exemplified herein.

As used herein, "cell surface marker" refers to any biological material expressed on a cell surface. This is also called a cell surface antigen, surface antigen or surface marker. A cell surface marker can be identified as an antigen binding to a monoclonal antibody. Cell surface markers that are called a CD marker or CD antigen in the art are also encompassed. A trait represented by a cell surface marker is also called a cell surface trait, which may be used herein as having the same meaning.

As used herein, "proteinaceous product" refers to any proteinaceous product produced by a cell. "Related biological material of a proteinaceous product (the product)" refers to any biological material related to cell proteinaceous product (e.g., gene encoding the proteinaceous product (DNA), mRNA, protein precursor, and the like), which is one of the cell indicators of the corneal endothelial property possessing functional cell of the invention or functional mature differentiated corneal endothelial cell. Typically, a proteinaceous product is a gene or a product thereof. In the present invention, examples thereof include those with (A) elevated expression in the corneal endothelial property possessing functional cell (including functional mature differentiated corneal endothelial cell) of the present invention (such as COL4A1, COL4A2, COL8A1, COL8A2, CDH2, and TGF-beta2) and (B) decreased expression in the corneal endothelial property possessing functional cell (including functional mature differentiated corneal endothelial cell) of the present invention (such as MMP1, MMP2, TIMP1, BMP2, IL13RA2, TGF-beta1, CD44, COL3A1, IL6, IL8, HGF, THBS2, and IGFBP3).

As used herein, "SASP related protein", "SASP factor" and "SASP mediator" are interchangeably used and refer to any protein related to SASP (abbreviation for Senescence Associated Secretory Phenotype), which is one of the cell indicators of the corneal endothelial property possessing functional cell, functional mature differentiated corneal endothelial cell, or non-intended cell of the present invention. This is also called cell senescence related secretion. SASP is a cell senescence related phenomenon in which various secretory proteins exhibiting action for inducing inflammatory reaction or oncogenesis are highly expressed. Examples of such SASP related proteins include inflammatory cytokines (IL-6), inflammatory chemokines (IL-8, MCP-1, and the like), protease (MMPs and the like), PAI-1, GRO-alpha, and VEGF.

As used herein, "exosome" is also called an exosome complex. An exosome is one of the cell indicators of the corneal endothelial property possessing functional cell of the invention or functional mature differentiated corneal endothelial cell. Examples thereof include CD63, CD9, CD81, HSP70, and the like.

As used herein, "cellular metabolite" refers to any metabolite produced by a cell. "Related biological material of cellular metabolite (the metabolite)" refers to any biological material related to a cellular metabolite (e.g., enzyme that synthesizes the metabolite, metabolizing enzyme, protein associated with a signaling pathway, or the like), which is one of the cell indicators of the corneal endothelial property possessing functional cell of the invention or functional mature differentiated corneal endothelial cell. Examples of metabolites include any product related to products of energy metabolism system in a mitochondrial system, glutathione metabolic system product, methionine metabolic cycle product, lipid metabolite, pentose phosphate pathway product, tricarboxylic acid (TCA) cycle metabolite, glycolytic system metabolite and the like. Tricarboxylic acid (TCA) cycle metabolite and glycolytic system metabolite are especially important. Examples of cellular metabolites and related biological material of the metabolite include succinic acid (succinate), Pro, Gly, glycerol 3-phosphate, Glu, lactic acid (lactate), arginosuccinic acid (arginosuccinate), xanthine, N-carbamoyl aspartic acid (N-carbamoyl aspartate), isocitric acid (isocitrate), cis-aconitic acid (cis-aconitate), citric acid (citrate), Ala, 3-phosphoglyceric acid (3-phosphoglycerate), hydroxyproline, malic acid (malate), uric acid (urate), betaine, folic acid (folate), Gln, 2-oxoisovaleric acid (2-oxoisovalerate), pyruvic acid (pyruvate), Ser, hypoxanthine, Asn, Trp, Lys, choline, Tyr, urea, Phe, Met, carnosine, Asp, ornithine, Arg, creatine, 2-hydroxy glutaminic acid (2-hydroxy glutamate), beta-Ala, citrulline, Thr, Ile, Leu, Val, creatinine, His, and N,N-dimethyl glycine.

As used herein, "autoantibody reactive cell" refers to any cell that reacts to an autoantibody. This is one of the cell indicators for selecting the corneal endothelial property possessing functional cell of the invention or functional mature differentiated corneal endothelial cell. An autoantibody reactive cell can be detected by any technique known in the art. A non-intended cell subpopulation is often reactive to an autoantibody. Thus, reactivity to an autoantibody can be assessed in order to define a cell of interest.

Detection, identification, quality control and the like of the cell of the present invention can be materialized by using an interactive molecule or substance that binds to a substance used as a marker. In the context of the present invention, "interactive molecule" or "substance binding to" a substance used as a marker is a molecule or substance that at least temporarily binds to a molecule such as a substance to be used as a marker (e.g., CD44) and preferably is capable of indicating that the molecule or substance is bound (e.g., labeled or capable of being labeled). A substance that binds a molecule such as CD44 may be a ligand of a molecule such as CD44. Examples thereof include antibodies, antisense oligonucleotides, siRNA, low molecular weight molecules (LMW), binding peptides, aptamers, ribozymes, peptidomimetics and the like, including binding proteins or binding peptide directed to molecules such as CD44 and nucleic acids directed to a gene of a molecule such as CD44. As used herein, "binding protein" or "binding peptide" for a molecule such as CD44 refers to types of proteins or peptides that bind to a molecule such as CD44, including, but not limited to, polyclonal antibodies or monoclonal antibodies directed to a molecule such as CD44, antibody fragments and protein backbones.

As used herein, "protein", "polypeptide", "oligopeptide" and "peptide" are used herein to have the same meaning and refer to an amino acid polymer of any length. The polymer may be straight, branched or cyclic. An amino acid may be a naturally-occurring, non-naturally occurring or altered amino acid, but an amino acid is naturally occurring when targeting those contained in a cell.

As used herein, "polynucleotide", "oligonucleotide" and "nucleic acid" are used herein to have the same meaning, and refer to a polymer of nucleotides of any length. The terms also encompass "oligonucleotide derivative" and "polynucleotide derivative". "Oligonucleotide derivative" and "polynucleotide derivative" refer to an oligonucleotide or polynucleotide that comprises a nucleotide derivative or has a bond between nucleotides which is different from normal. The terms are used interchangeably. As used herein, "nucleic acid" is interchangeably used with gene, cDNA, mRNA, oligonucleotide, and polynucleotide. As used herein, "nucleotide" may be naturally-occurring or non-naturally occurring, but those in cells are naturally-occurring. Nucleic acids or nucleotides used as detection means may be considered artificial.

As used herein, "gene" refers to an agent defining a genetic trait. Genes are generally arranged in a certain order on a chromosome. A gene defining the primary structure of a protein is referred to as a structural gene, and a gene affecting the expression thereof is referred to as a regulator gene. As used herein, "gene" may refer to "polynucleotide", "oligonucleotide" and "nucleic acid". "Gene product" is a substance produced based on a gene and refers to proteins, mRNAs or the like.

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity and homology of an amino acid sequence and a base sequence is calculated herein by using a default parameter using a sequence analysis tool, BLAST. For example, identity can be searched by using BLAST 2.2.28 (published on April 2, 2013) of the NCBI. Herein, values for identity generally refer to a value when achieving alignment under the default condition using the above-described BLAST. However, when a higher value is obtained by changing a parameter, the highest value is considered the value of identity. When identity is achieved in a plurality of regions, the highest value thereamong is considered the value of identity. Similarity is a value calculated by taking into consideration a similar amino acid in addition to identity.

An "isolated" substance or biological agent (e.g., nucleic acid, protein, or the like) as used herein refers to a substance or biological agent with no agent that naturally accompanies the substance or biological agent. Meanwhile, as used herein, a "purified" substance or biological agent (e.g., nucleic acid, protein or the like) refers to a substance or a biological agent from which an agent naturally accompanying the substance or biological agent has been at least partially removed. Thus, the purity of a biological agent in a purified biological agent is generally higher than the purity in the normal state of the biological agent (i.e., concentrated). The terms "isolated" and "purified" as used herein refer to the presence of preferably at least about 75% by weight, more preferably at least about 85% by weight, still more preferably at least about 95% by weight, and most preferably at least about 98% by weight of a biological agent of the same type. The substance used in the present invention is preferably an "isolated" or "purified" substance.

As used herein, "marker (substance, protein or gene (nucleic acid))" refers to a substance that can be an indicator for tracking whether a target is in or at risk of being in a certain condition (e.g., functionality, transformation state, diseased state, disorder state, growth capability, level or presence of differentiated state or the like). Examples of such a marker include genes (nucleic acid = DNA level), gene products mRNA, protein and the like), metabolites, enzymes and the like. In the present invention, detection, diagnosis, preliminary detection, prediction, or prediagnosis of a certain state (e.g., disease such as differentiation disorder) can be materialized by using an agent or means specific to a marker associated with such a state, or a composition, kit or system comprising the same or the like. As used herein, "gene product" refers to a protein or mRNA encoded by a gene. It is found in the present specification that a gene product (i.e., molecule such as CD44 or the like), which does not exhibit association with an eye cell, especially corneal endothelial cell, can be used as an indicator for whether the cell has the functionality of a corneal endothelial cell (transformed or not).

"Detection" or "quantification" of polynucleotide or polypeptide expression can be accomplished herein by using a suitable method including, for example, an immunological measuring method and measurement of mRNAs, including a bond or interaction to a detecting agent, inspection agent or diagnostic agent. Examples of a molecular biological measuring method include northern blot, dot blot, PCR and the like. Examples of an immunological measurement method include ELISA using a microtiter plate, RIA, fluorescent antibody method, luminescence immunoassay (LIA), immunoprecipitation (IP), radial immunodiffusion (SRID), turbidimetric immunoassay (TIA), western blot, immunohistochemical staining and the like. Further, examples of a quantification method include ELISA, RIA and the like. Quantification may also be performed by a gene analysis method using an array (e.g., DNA array, protein array). DNA arrays are outlined extensively in (Ed. by Shujunsha, Saibo Kogaku Bessatsu "DNA Maikuroarei to Saishin PCR ho" [Cellular engineering, Extra issue, "DNA Microarrays and Latest PCR Methods"]. Protein arrays are discussed in detail in Nat Genet. 2002 Dec; 32 Suppl: 526-532. Examples of a method of analyzing gene expression include, but are not limited to, RT-PCR, RACE, SSCP, immunoprecipitation, two-hybrid system, in vitro translation, FACS (Fluorescence-activated cell sorting) and the like, in addition to the methods discussed above. Such additional analysis methods are described in, for example, Genomu Kaiseki Jikkenho Nakamura Yusuke Labo Manyuaru [Genome analysis experimental method Yusuke Nakamura Lab Manual], Ed. by Yusuke Nakamura, Yodosha (2002) and the like. The entirety of the descriptions therein is incorporated herein by reference. Flow cytometry used in FACS is an approach for dispersing fine particles in a fluid and allowing small amounts of the fluid to flow to optically analyze individual particles. An approach applying this is FACS (Fluorescence-activated cell sorting). FACS is a technique that can quantitatively measure the amount of antigen expression on a cell surface by flowing a cell stained with a fluorescent antibody on a liquid flow and allowing the cell to pass a focal point of a laser beam to measure fluorescence emitted by individual cells.

As used herein, "expression intensity" refers to the amount of polypeptide, mRNA or the like expressed in a cell, tissue or the like of interest. Examples of such an expression intensity include expression intensity of the polypeptide of the present invention at a protein level assessed by any suitable method including an immunological measurement method such as ELISA, RIA, fluorescent antibody method, western blot, and immunohistochemical staining by using the antibody of the present invention, and the expression intensity of the polypeptide used in the present invention at an mRNA level assessed by any suitable method including a molecular biological measuring method such as northern blot, dot blot, and PCR. "Change in expression intensity" refers to an increase or decrease in the expression intensity of the polypeptide used in the present invention at a protein level or mRNA level assessed by any suitable method including the above-described immunological measuring method or molecular biological measuring method. A variety of detection or diagnosis based on a marker can be performed by measuring the expression intensity of a certain marker.

As used herein, "decrease" or "suppression" of activity or expression product (e.g., protein, transcript (RNA or the like)) or synonyms thereof refers to: a decrease in the amount, quality or effect of a specific activity, transcript or protein; or activity that decreases the same. Among decreases, "elimination" refers to activity, expression product or the like being less than the detection limit and especially referred to as "elimination". As used herein, "elimination" is encompassed by "decrease" or "suppression".

As used herein, "increase" or "activation" of activity or expression product (e.g., protein, transcript (RNA or the like)) or synonyms thereof refers to: an increase in the amount, quality or effect of a specific activity, transcript or protein; or activity that increases the same.

As used herein, an "antibody" includes, in a broad sense, polyclonal antibodies, monoclonal antibodies, multi-specific antibodies, chimeric antibodies, anti-idiotype antibodies, and fragments thereof such as Fv fragments Fab' fragments, F(ab')₂ and Fab fragments, as well as other conjugates or functional equivalents produced by recombination (e.g., chimeric antibodies, humanized antibodies, multifunctional antibodies, bispecific or oligospecific antibodies, single chain antibodies, scFV, diabodies, sc(Fv)₂ (single chain (Fv)₂), and scFv-Fc). Furthermore, such an antibody may be fused, by a covalently bond or recombination, with an enzyme such as alkaline phosphatase, horseradish peroxidase, or alpha galactosidase. The antibodies to CD44 or the like used in the present invention are sufficient if they bind to their proteins such as CD44, regardless of the origin, type, shape or the like thereof. Specifically, known antibodies such as a non-human animal antibody (e.g., a mouse antibody, a rat antibody, or a camel antibody), a human antibody, a chimeric antibody, or a humanized antibody can be used. In the present invention, a monoclonal or polyclonal antibody can be utilized as an antibody, but a monoclonal antibody is preferable. It is preferable that antibodies bind specifically to their proteins such as CD44.

As used herein, "means" refers to anything that can be a tool for accomplishing an objective (e.g., detection, diagnosis, therapy). As used herein, "selective recognizing means" in particular refers to means capable of recognizing (detecting) a certain subject differently from others.

The detecting agent or diagnostic agent of the present invention or other medicaments can be in a form of a probe or a primer. The probes and primers of the present invention can specifically hybridize to a molecule such as CD44. As described herein, the expression of a molecule such as CD44 is an indicator for whether it is a normal or transformed cell in a corneal endothelial cell. Further, such expression is useful as an indicator of the level of transformation. Thus, the probes and primers according to the present invention can be used to identify a corneal endothelial cell as a normal or transformed cell and/or the degree of transformation. In one embodiment, the probes and primers of the present invention only need to be able to detect the expression of a molecule such as CD44 and refer to a polymer consisting of bases or base pairs such as multiple deoxyribonucleic acids (DNA) or ribonucleic acids (RNA). It is known that double stranded cDNA can be used in tissue in situ hybridization. The probes and primers of the present invention also include such double stranded cDNA. Examples of especially preferred probes and primes in detecting RNA in a tissue include RNA probes (riboprobes).

As used herein, "(nucleic acid) primer" refers to a substance required for initiating a reaction of a polymeric compound to be synthesized in a polymer synthesizing enzyme reaction. A synthetic reaction of a nucleic acid molecule can use a nucleic acid molecule (e.g., DNA, RNA or the like) complementary to a portion of a sequence of a polymeric compound to be synthesized. A primer can be used herein as marker detecting means.

Examples of nucleic acid molecules generally used as a primer include those with a nucleic acid sequence with a length of at least 8 contiguous nucleotides, which is complementary to a nucleic acid sequence of a gene of interest (e.g., markers of the present invention). Such a nucleic acid sequence may be a nucleic acid sequence preferably with a length of at least 9 contiguous nucleotides, more preferably with a length of at least 10 contiguous nucleotides, and still more preferably with a length of at least about 11 contiguous nucleotides, a length of at least about 12 contiguous nucleotides, a length of at least about 13 contiguous nucleotides, a length at least about of 14 contiguous nucleotides, a length of at least about 15 contiguous nucleotides, a length of at least about 16 contiguous nucleotides, a length of at least about 17 contiguous nucleotides, a length of at least about 18 contiguous nucleotides, a length of at least about 19 contiguous nucleotides, a length of at least about 20 contiguous nucleotides, a length of at least about 25 contiguous nucleotides, a length of at least about 30 contiguous nucleotides, a length of at least about 40 contiguous nucleotides, or a length of at least about 50 contiguous nucleotides. Such nucleic acid sequences used as a primer include nucleic acid sequences that are at least 70% homologous, more preferably at least 80% homologous, still more preferably at least 90% homologous or at least 95% homologous to the aforementioned sequences. While a sequence that is suitable as a primer may vary depending on the nature of the sequence intended to be synthesized (amplified), those skilled in the art can appropriately design a primer in accordance with an intended sequence. Designs of such primers are well known in the art. Primers may be designed manually or by using a computer program (e.g., LASERGENE, PrimerSelect, DNAStar).

The primers according to the present invention can also be used as a primer set consisting of two or more types such primers.

The primers and primer sets according to the present invention can be used a as a primer and a primer set according to a conventional method in a known method for detecting a gene of interest by using a nucleic acid amplification method such as PCR, RT-PCR, real-time PCR, in situ PCR, or LAMP.

The primer sets according to the present invention can be selected such that the nucleotide sequence of a protein of interest of a molecule such as CD44 or the like can be amplified by a nucleic acid amplification method such as PCR. Nucleic acid amplification methods are well known, and selection of a primer pair in a nucleic acid amplification method is evident to those skilled in the art. For instance, primers can be selected in PCR such that one of the two primers (primer pair) conjugates a plus strand of a double stranded DNA of a protein of interest of a molecule such as CD44 and the other primer conjugates the minus strand of the double stranded DNA, and one of the primers conjugate to an extended chain extended by the other primer. For the LAMP method (WO 00/28082), three regions F3c, F2c, and F1c are defined from the 3' terminus and three regions B1, B2, and B3 are defined from the 5' terminus on the target gene, and these 6 regions can be used to design four types of primers. The primers of the present invention can be chemical synthesized based on the nucleotide sequences disclosed herein. Primer preparation is well known and can be prepared according to, for example, "Molecular Cloning, A Laboratory Manual 2nd ed." (Cold Spring Harbor Press (1989)), "Current Protocols in Molecular Biology" (John Wiley & Sons (1987-1997)).

As used herein, "probe" refers to a substance that can be means for search, which is used in a biological experiment such as in vitro and/or in vivo screening. Examples thereof include, but are not limited to, a nucleic acid molecule comprising a specific base sequence, a peptide comprising a specific amino acid sequence, a specific antibody, a fragment thereof and the like. A probe is used herein as means for marker detection.

Examples of nucleic acid molecules generally used as a probe include those with a nucleic acid sequence with a length of at least about 8 contiguous nucleotides, which is complementary to a nucleic acid sequence of a gene of interest. Such a nucleic acid sequence may be a nucleic acid sequence preferably with a length of at least about 9 contiguous nucleotides, more preferably with a length of at least 10 contiguous nucleotides, and still more preferably with a length of at least about 11 contiguous nucleotides, a length of at least about 12 contiguous nucleotides, a length of at least about 13 contiguous nucleotides, a length at least about of 14 contiguous nucleotides, a length of at least about 15 contiguous nucleotides, a length of at least about 20 contiguous nucleotides, a length of at least about 25 contiguous nucleotides, a length of at least about 30 contiguous nucleotides, a length of at least about 40 contiguous nucleotides, or a length of at least about 50 contiguous nucleotides. Such nucleic acid sequences used as a probe include nucleic acid sequences that are at least about 70% homologous, more preferably at least about 80% homologous, still more preferably at least about 90% homologous or at least about 95% homologous to the aforementioned sequences.

In one embodiment, the detecting agent of the present invention can be labeled. Alternatively, the detecting agent of the present invention may be coupled to a tag.

As used herein, "label" refers to an entity (e.g., substance, energy, electromagnetic wave or the like) for distinguishing a molecule or substance of interest from others. Such a method of labeling includes RI (radioisotope) method, fluorescence method, biotin method, chemiluminescent method and the like. When a plurality of markers of the present invention or agents or means for capturing the same are labeled by a fluorescence method, labeling is performed with fluorescent substances having different fluorescent emission maximum wavelengths. It is preferable that the difference in fluorescent emission maximum wavelengths is 10 nm or greater. When labeling a ligand, any label that does not affect the function can be used. Examples of labels actually used in FACS include FITC, PE, PerCP-Cy5.5, PE-Cy7, APC, Alexa^{™}Fluor^{™}488, and Alexa^{™}Fluor647. In a typical example of immunostaining, Alexa^{™}Fluor488, Alexa^{™}Fluor555 or Alexa^{™}Fluor594, and Alexa^{™}Fluor647 can be combined for use. Alexa^{™}Fluor is a water-soluble fluorescent dye obtained by modifying coumarin, rhodamine, fluorescein, cyanine or the like. This is a series compatible with a wide range of fluorescence wavelengths. Relative to other fluorescent dyes for the corresponding wavelength, Alexa^{™}Fluor is very stable, bright and has a low level of pH sensitivity. Combinations of fluorescent dyes with a fluorescence maximum wavelength of 10 nm or greater include a combination of Alexa^{™}555 and Alexa^{™}633, combination of Alexa^{™}488 and Alexa^{™}555 and the like. When a nucleic acid is labeled, any label can be used that can bind to a base portion thereof, but e a cyanine dye (e.g., Cy3, Cy5 or the like of the CyDye^{™} series), rhodamine 6G reagent, N-acetyoxy-N2-acetylaminofluorene (AAF), AAIF (iodine derivative of AAF) or the like can be used. Examples of labels in actual use include DAPI and Hoechst 33342. Examples of a fluorescent substance with a difference in fluorescent emission maximum wavelengths of 10 nm or greater include a combination of Cy5 and a rhodamine 6G reagent, a combination of Cy3 and fluorescein, a combination of a rhodamine 6G reagent and fluorescein and the like. The present invention can utilize such a label to alter a subject of interest to be detectable by the detecting means to be used. Such alteration is known in the art. Those skilled in the art can appropriately carry out such a method in accordance with the label and subject of interest.

According to one embodiment of detection in the present invention, a molecule such as CD44 in a cell sample or expression of a gene of the molecule can be detected by hybridizing the probe according to the present invention with a nucleic acid sample (mRNA or a transcription product thereof) and directly or indirectly detecting a hybridization complex, i.e., nucleotide double strand. For detailed procedure of hybridization methods, the following can be referred: "Molecular Cloning, A Laboratory Manual 2nd ed." (Cold Spring Harbor Press (1989), especially Section 9.47-9.58), "Current Protocols in Molecular Biology" (John Wiley & Sons (1987-1997), especially Section 6.3-6.4), "DNA Cloning 1: Core Techniques, A Practical Approach 2nd ed." (Oxford University (1995), for conditions, especially Section 2.10).

Detection of expression of a molecule such as CD44 or genes of these molecules utilizing a hybridization method can be implemented by, for example, (a) contacting a polynucleotide derived from a test sample with the probe according to the present invention; and (b) detecting a hybridization complex. In step (a), mRNA prepared from a test sample of interest or complementary DNA (cDNA) transcribed from the mRNA can be contacted with the probe as the polynucleotide derived from a test cell sample. In a method of detection using a probe, the probe can be labeled for use. Examples of the label include labels utilizing radioactivity (e.g., ³²P, ¹⁴C, and ³⁵S), fluorescence (e.g., FITC and europium), and an enzyme reaction such as chemiluminescence (e.g., peroxidase and alkaline phosphatase) or the like. A hybridization product can be detected using a well-known method such as Northern hybridization, Southern hybridization, colony hybridization, or the like. Since a cell from which a hybridization complex is detected is a cell expressing a molecule such as CD44, the cell can be determined as having a high proliferation ability (an undifferentiated cell, a progenitor cell, a stem cell or the like) and/or a high differentiation ability.

According to another embodiment of detection according to the present invention, expression of molecules such as CD44 or genes of these molecules in a sample can be detected by amplifying a nucleic acid sample (mRNA or a transcription product thereof) by a nucleic acid amplification method using the primer or the primer set according to the present invention and detecting the amplification product.

Detection of expression of molecules such as CD44 or genes of these molecules utilizing a nucleic acid amplification method can be implemented, for example, by (i) performing a nucleic acid amplification method using the primer or the primer set according to the present invention while using a polynucleotide derived from a test sample as a template; and (ii) detecting the formed amplification product.

In step (i), mRNA prepared from a test sample of interest or complementary DNA (cDNA) transcribed from the mRNA can be used as a template. An amplification product can be detected using a nucleic acid amplification method such as PCR, RT-PCR, real time PCR, or a LAMP method. A cell from which an amplification product is detected is highly likely a normal corneal endothelial cell for a normal corneal endothelial cell marker and highly likely a transformed corneal endothelial cell for a transformed corneal endothelial cell marker. Thus, the cell can be determined to be a normal or transformed cell.

As the immunological method, a known method such as an immunohistological staining method, an enzyme immunometric assay, a Western blotting method, an agglutination method, a competition method, or a sandwich method can be applied to a sample obtained by subjecting a cell sample to an appropriate treatment as needed such as separation of a cell or an extraction operation. The immunohistological staining method can be performed, for example, by a direct method using a labeled antibody, an indirect method using a labeled antibody to the above antibody, or the like. As a labeling agent, a known labeling substance such as a fluorescent substance, a radioactive substance, an enzyme, a metal, or a dye can be used.

In the present invention, "Rho kinase" refers to serine/threonine kinase which is activated with activation of Rho. Examples thereof include ROKalpha (ROCK-II: Leung, T. et al., J. Biol. Chem., 270, 29051-29054, 1995), p160ROCK (ROKbeta, ROCK-I: Ishizaki, T. et al., The EMBO J., 15(8), 1885-1893, 1996) and other proteins having serine/threonine kinase activity.

Examples of Rho kinase inhibitors include compounds disclosed in the following documents: US Patent No. 4678783, Japanese Patent No. 3421217, International Publication No. WO 95/28387, International Publication No. WO 99/20620, International Publication No. WO 99/61403, International Publication No. WO 02/076976, International Publication No. WO 02/076977, International Publication No. WO 2002/083175, International Publication No. WO 02/100833, International Publication No. WO 03/059913, International Publication No. WO 03/062227, International Publication No. WO 2004/009555, International Publication No. WO 2004/022541, International Publication No. WO 2004/108724, International Publication No. WO 2005/003101, International Publication No. WO 2005/039564, International Publication No. WO 2005/034866, International Publication No. WO 2005/037197, International Publication No. WO 2005/037198, International Publication No. WO 2005/035501, International Publication No. WO 2005/035503, International Publication No. WO 2005/035506, International Publication No. WO 2005/080394, International Publication No. WO 2005/103050, International Publication No. WO 2006/057270, International Publication No. WO 2007/026664 and the like. Such compounds can be manufactured by the methods described in the respective documents where the compounds are disclosed. The specific examples thereof include 1-(5-isoquinolinesulfonyl) homopiperazine or a salt thereof (e.g., fasudil or fasudil hydrochloride), (+)-trans-4-(1- aminoethyl)-1-(4-pyridylcarbamoyl)cyclohexane((R)-(+)-trans-(4-pyridyl)-4-(1-aminoethyl)-cyclohexanecarboxamide) or a salt thereof (e.g., Y-27632 ((R)-(+)-trans-(4-pyridyl)-4-(1-aminoethyl)-cyclohexanecarboxamide dehydrochloride monohydrate) and the like) and the like. For these compounds, a commercially available product (Wako Pure Chemical Industries, Ltd, Asahi Kasei Pharma Corporation and the like) can also be preferably used.

As used herein, "diagnosis" refers to identifying various parameters associated with a disease, disorder, condition (e.g., bullous keratopathy, Fuchs endothelial dysfunction) or the like in a subject to determine the current or future state of such a disease, disorder, or condition. The condition in the body can be investigated by using the method, apparatus, or system of the present invention. Such information can be used to select and determine various parameters of a formulation or method for the treatment or prevention to be administered, disease, disorder, or condition in a subject or the like. As used herein, "diagnosis" when narrowly defined refers to diagnosis of the current state, but when broadly defined includes "early diagnosis", "predictive diagnosis", "prediagnosis" and the like. Since the diagnostic method of the present invention in principle can utilize what comes out from a body and can be conducted away from a medical practitioner such as a physician, the present invention is industrially useful. In order to clarify that the method can be conducted away from a medical practitioner such as a physician, the term as used herein may be particularly called "assisting" "predictive diagnosis, prediagnosis or diagnosis".

As used herein, "therapy" refers to the prevention of exacerbation, preferably maintaining of the current condition, more preferably alleviation, and still more preferably disappearance of a disease or disorder (e.g., bullous keratopathy, Fuchs endothelial dysfunction) in case of such a condition, including being capable of exerting a prophylactic effect or an effect of improving a disease of a patient or one or more symptoms accompanying the disease. Preliminary diagnosis with suitable therapy may be referred to as "companion therapy" and a diagnostic agent therefor may be referred to as "companion diagnostic agent".

As used herein, "combined use" of a certain pharmaceutical ingredient (e.g., cellular medicament of the present invention or the like) with another pharmaceutical ingredient (e.g., combined agent such as ROCK inhibitor or the like) is intended to encompass concomitant (co) administration and continuous administration. Continuous administration is intended to encompass administration of a medicament (one or more types) with a medicament of the present invention (one or more types) or the like to a subject in various orders. An agent for "combined use" and administration of a certain pharmaceutical ingredient (e.g., cellular medicament of the present invention or the like) with another pharmaceutical ingredient (e.g., ROCK inhibitor or the like) may also be called a "combined agent" or "combined drug".

The term "prognosis" as used herein refers to prediction of the possibility of progression or death due to a disease such as bullous keratopathy or Fuchs endothelial dysfunction. A prognostic agent is a variable related to natural course of a disease, which affects the rate of recurrence of outcome of a patient who has experienced the disease. Examples of clinical indicators associated with exacerbation in prognosis include any cell indicator used in the present invention. A prognostic agent is often used to classify patients into subgroups with different pathological conditions.

As used herein, "detecting drug (agent)" or "inspection drug (agent)" broadly refers to all agents capable of detecting or inspecting a target of interest.

As used herein, "diagnostic drug (agent)" broadly refers to all agents capable of diagnosing a condition of interest (e.g., disease such as corneal endothelial disease).

As used herein, "therapeutic drug (agent)" broadly refers to all agents capable of treating a condition of interest (e.g., diseases such as corneal endothelial disease). In one embodiment of the present invention, "therapeutic drug" may be a pharmaceutical composition comprising an effective ingredient and one or more pharmacologically acceptable carriers. A pharmaceutical composition can be manufactured, for example, by mixing an effective ingredient and the above-described carriers by any method known in the technical field of pharmaceuticals. Further, mode of usage of a therapeutic drug is not limited, as long as it is used for therapy. A therapeutic drug may be an effective ingredient alone or a mixture of an effective ingredient and any ingredient. Further, the shape of the above-described carriers is not particularly limited. For example, the carrier may be a solid or liquid (e.g., buffer solution). It should be noted that a medicament includes drugs (prophylactic drug) for prevention and medicaments (therapeutic drugs) for improving the condition of a corneal endothelial disease.

As used herein, "prevention" refers to the action of taking a measure against a disease or disorder (e.g., corneal endothelial disease) from being in such a condition prior to being in such a condition. For example, it is possible to use the agent of the present invention to perform diagnosis, and optionally use the agent of the present invention to prevent or take measures to prevent the disease or the like.

As used herein, "prophylactic drug (agent)" broadly refers to all agents capable of preventing a condition of interest (e.g., corneal endothelial disease or the like).

The composition, medicament, agent (therapeutic agent, prophylactic agent) and the like of the present invention generally comprise a therapeutically effective amount of medicament or effective ingredient and a pharmaceutically acceptable carrier or excipient. As used herein, "pharmaceutically acceptable" means that government regulatory agency-approved or pharmacopoeia or other commonly recognized pharmacopoeia-listed substance for use in animals and more specifically in humans. As used herein "carrier" refers to a culture, infusion vehicle, irrigating solution, diluent, adjuvant, excipient or vehicle administered in conjunction with a medicament. The cellular medicament of the present invention comprises a cell as the main component. The carrier is thus preferably capable of maintaining a cell such as culture, infusion vehicle, or irrigating solution. In addition to the cellular medicament, the present invention may use other medicaments in combination such as a steroid agent, antimicrobial, or ROCK inhibitor. Such a medicament can have the same dosage form as common medicaments. Such a carrier can be an aseptic liquid such as water or oil, including but not limited to liquids derived from petroleum, animal, plant or synthesis, as well as peanut oil, soybean oil, mineral oil, sesame oil and the like. When a medicament (composition) is intravenously administered, saline and aqueous dextrose are preferred carriers. Preferably, aqueous saline solution and aqueous dextrose and glycerol solution are used as a liquid carrier of an injectable solution. For oral administration of a medicament, water is the preferred carrier. Suitable excipients include light anhydrous silicic acid, crystalline cellulose, mannitol, starch, glucose, lactose, sucrose, gelatin, malt, rice, wheat flour, chalk, silica gel, sodium stearate, glyceryl monostearate, talc, sodium chloride, powdered skim milk, glycerol, propylene, glycol, water, ethanol, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, saccharose, carboxymethylcellulose, corn starch, inorganic salt and the like. When desired, the composition can contain a small amount of wetting agent or emulsifier or pH buffer. These compositions can be in a form of solution, suspension, emulsion, tablet, pill, capsule, powder, sustained release mixture or the like. It is also possible to use traditional binding agents and carriers, such as tryglyceride, to prepare a composition as a suppository. Oral preparation can also comprise a standard carrier such as medicine grade mannitol, lactose, starch, magnesium stearate, sodium saccharin, cellulose, or magnesium carbonate. Examples of a suitable carrier are described in E. W. Martin, Remington's Pharmaceutical Sciences (Mark Publishing Company, Easton, U.S.A). Such a composition contains a therapeutically effective amount of therapy agent and preferably in a purified form, together with a suitable amount of carrier, such that the composition is provided in a form suitable for administration to a patient. A preparation must be suitable for the administration format. In addition, the composition may comprise, for example, a surfactant, excipient, coloring agent, flavoring agent, preservative, stabilizer, buffer, suspension, isotonizing agent, binding agent, disintegrant, lubricant, fluidity improving agent, corrigent or the like.

When the present invention is administered as a medicament, various delivery systems are known, and such systems can be used to administer the medicament of the present invention to a suitable site (e.g., ocular anterior chamber). A typical dosage form of the cellular medicament of the present invention is injection into the anterior chamber. In such a case, a cell can be suspended in an infusion vehicle and injected with a needle (e.g., 26G needle) into the anterior chamber. Other combined use drugs can use the same dosage form as common medicaments. Examples of such a system include liposomes, microparticles, encapsulation in a microcapsule and the like. The methods of administration include, but not limited to, intradermal, intramuscular, intraperitoneal, intravenous, subcutaneous, intranasal, epidural and oral pathways. A medicament can be administered by any suitable pathway, such as by injection, bolus injection, or absorption through epithelial or mucocutaneous lining (e.g., oral cavity, rectum, intestinal mucosa or the like). In addition, an inhaler or mistifier using an aerosolizing agent can be used as needed. Moreover, cells can also be administered together. Administration can be systemic or local or topical.

In a preferred embodiment, a composition can be prepared as a pharmaceutical composition adapted to administration to humans in accordance with a known method. Such a composition can be administered by injection or infusion. When a composition is to be administered by injection, the composition can be distributed by using an injection bottle containing cell infusion solution, aseptic agent-grade water or saline.

The low molecule or polymer medicament such as combined drug (e.g., antibiotic or ROCK inhibitor) of the present invention can be prepared as a neutral or salt form or other prodrugs (e.g., ester or the like). Pharmaceutically acceptable salts include salts formed with a free carboxyl group, derived from hydrochloric acid, phosphoric acid, acetic acid, oxalic acid, tartaric acid or the like, salts formed with a free amine group, derived from isopropylamine, triethylamine, 2-ethylaminoethanol, histidine, procaine or the like, and salts derived from sodium, potassium, ammonium, calcium, ferric hydroxide or the like.

The amount (cell count, number of administration or the like) of medicament of the present invention that is effective in therapy of a specific disorder or condition may vary depending on the properties of the disorder or condition. However, such an amount can be determined by those skilled in the art by a standard clinical technique based on the descriptions herein. Furthermore, an in vitro assay can be used in some cases to assist the identification of the optimal dosing range. The precise dose to be used in a preparation may also vary depending on the administration pathway or the severity of the disease or disorder. Thus, the dose should be determined in accordance with the judgment of the attending physician or the condition of each patient. The dosage is not particularly limited, but any cell density and amount described herein or within a range between any two values thereof can be used, such as 1.5 × 10⁶ cells. The dosing interval is not particularly limited, but may be, for example, a single administration or 1 or 2 administration every 1, 7, 14, 21, or 28 days or 1 or 2 administrations in the range of period between any two values described above. The dosage, dosing interval, and dosing method may be appropriately selected depending on the age or weight of the patient, symptom, target disease or the like. Further, it is preferable that a therapeutic drug contains a therapeutically effective amount, or an amount effective for exerting a desired effect, of effective ingredients. When a marker indicating a pathological condition significantly decreases after administration, the presence of a therapeutic effect may be acknowledged. The effective dose can be estimated from a dose-reaction curve obtained from an in vitro or animal model testing system.

"Patient" in one embodiment of the present invention primarily assumes humans, but may be mammals other than humans when applicable.

### (Preferred embodiments)

Preferred embodiments of the present invention are described hereinafter. The embodiments are provided hereinafter for better understanding of the present invention. It is understood that the scope of the present invention should not be limited to the following descriptions. Thus, it is apparent that those skilled in the art can appropriately make modifications within the scope of the present invention by referring to the descriptions herein. It is understood that the following embodiments of the present invention can be used alone or in combination.

### (Human functional corneal endothelial cells capable of eliciting a human corneal endothelial functional property when infused into an anterior chamber of a human eye)

In one aspect, the present invention provides a human functional corneal endothelial cell capable of eliciting a human corneal endothelial functional property when infused into an anterior chamber of a human eye (also referred to as the corneal endothelial property possessing functional cell of the invention). Since the corneal endothelial property possessing functional cell of the present invention has a corneal endothelial functional property of a mature differentiated corneal endothelium and exerts an effect in cell infusion therapy (e.g., capable of eliciting a corneal endothelial functional property when infused into an anterior chamber of a human eye), such a cell can typically be referred to as a human functional corneal endothelial cell capable of eliciting a corneal endothelial functional property when infused into an anterior chamber of a human eye. The corneal endothelial property possessing functional cell of the invention may include functional mature differentiated corneal endothelial cells as well as intermediately differentiated corneal endothelial cells. The functional mature differentiated corneal endothelial cell of the present invention is a mature differentiated cell that exerts a corneal endothelial function. An effector cell, which is the optimal subpopulation for infusion, forms a small hexagonal cobble-stone shape, and utilizes an energy metabolism system by a mitochondrial function.

Cells called "cultured corneal endothelial cells" or "cultured human corneal endothelial cells" have been reported. However, it was not known that such cells are comprised of multiple subpopulations, or there is a subpopulation thereamong that is particularly optimal for cell infusion therapy. Thus, the significance of the present invention that has revealed this is considerable. In particular, prior to the disclosure of the present invention, the problem related to heterogeneity of cells associated with regenerative medicine was not clearly recognized for human corneal endothelial cells. The significance of discovering and solving such a problem is considerable. This is because although human corneal endothelial cells (HCEC) cannot undergo cell division in vivo such that the cell cycle stops at the G1 phase, the ability to proliferate is still retained and it was understood that it is very difficult to culture an HCEC for a long period of time in view of recent studies.

An example of application of the present invention is noteworthy especially in terms of using an "allo" functional mature differentiated human corneal endothelial cell, which is high quality, free of karyotype abnormality, and does not elicit immunological rejection, as a suspension to enable regeneration of a corneal endothelial function by infusion into the anterior chamber. The medical technique using the cell of the present invention enables therapy where a corneal endothelial cell from especially young donors is cultured, expanded, and amplified ex vivo, and then a cell suspension is infused into the anterior chamber of a bullous keratopathy patient. The safety and clinical POC (proof of concept) have been demonstrated/established by the present invention in human applications in clinical studies based on the guidelines for clinical studies using a human stem cell.

One of the reasons the cells of the present invention were able to be provided is the discovery that cells used in infusion therapy are mixtures of heterogeneous cell subpopulations and only some of them are "human functional corneal endothelial cells capable of eliciting a human corneal functional property when infused into an anterior chamber of a human eye" that can be used in therapy.

The present invention revealed that karyotype abnormalities occur in a subpopulation selective manner and there are autoantibodies that react subpopulation selectively in corneal endothelial cells. It was revealed that the corneal endothelial property possessing functional cell of the invention, especially functional mature differentiated corneal endothelial cell, does not have such an abnormality, and relative to other subpopulations, the expression of HLA class I antigen associated with immunological rejection is relatively low, and expression of CD200 antigen, which had been so far speculated to be a cell marker, was negative. It was also revealed that cytokine (SASP related protein) production associated with cell senescence is high in a non-intended cell.

Prior to the disclosure of the present invention, reproducible culturing means were limited. Attempts to grow a cultured human corneal endothelial cell without cell state transition (CST) such as fibrosis, cell senescence, or epithelial mesenchymal transition (EMT) or karyotype abnormality in vitro was notably difficult due to complete lack of knowledge related to the cell properties thereof and knowledge/report related to whether a cell population is comprised of multiple subpopulations or a cell population produced according to culture conditions exhibits stable composition, such that analysis from such viewpoints were not even conducted.

Cultured HCECs tend to undergo CST to have senescent phenotype, EMT, and fibroblastic morphology. The inventors identified a clear cell surface marker identifying such HCECs to enable HCEC populations that can be applied to reconstruction of dysfunctional human corneal endothelial tissue to be defined.

Several CD markers were selected to define a SP while considering the report on karyotype aneuploidy in cHCECs and plasticity of a metabolic profile of cHCECs. The selected markers were CD166, CD44, CD49e, CD73, CD105, CD90, CD133, CD26, and CD24, which were all involved in some way to mesenchymal stem cell (MSC), cancer stem cell (CSC) or transformation during CST (Davies S, Beckenkamp A, Buffon A. Biomed Pharmacother. 2015; 71:135-8; Roberta Pang, et al., Stem Cell, 6, 2010, 603-615; Krawczyk N, et al., Biomed Res Int. 2014; 2014: 415721. Epub 2014 May 8; Irollo E, Pirozzi G. Am J Transl Res. 2013 Sep 25; 5:563-81; Williams K, et al., Exp Biol Med (Maywood). 2013; 38:324-38; Zhe Shi, et al., Mol Cell Biochem (2015) 401: 155-164). While this selection appeared appropriate, this was because a normal stem cell is a cell that survives the longest in tissue and it is highly likely that a mutation accumulates due to passage of time, such that a CSC may arise from a transit-amplifying cell (B.J. Huntly Cancer Cell, 6 (2004), 587-596; C.H. Jamieson et al., N. Engl. J. Med., 351 (2004), pp. 657-667).

Prior to the disclosure of the present invention, a specific cell surface marker (group) of a human functional mature differentiated corneal endothelial cell in its true sense was not known. Glypican-4 and CD200 were proposed as HCEC markers for distinguishing HCECs from corneal stromal fibroblasts (Cheong YK et al., Invest Ophthalmol Vis Sci. 2013; 54: 4538-4547). However, it was discovered that practical issues with HCEC culture are in the coexistence of a vulnerable cultured human corneal endothelial cell that has undergone transformation. This was overcome by the manufacturing method provided by the present invention.

Flow cytometry analysis shows the presence of several types of subpopulations in cultured human corneal endothelial cells, and one type of specific cultured human corneal endothelial cell subpopulation with surface expression of CD166 positive, CD105 negative, CD44 negative, CD24 negative, and CD26 negative is a representative subpopulation without CST. This is a new finding that enables application of this subpopulation to clinical use. The combination of CD markers defined herein is suitable for quality control to guarantee the functional feature of a cultured human corneal endothelial cell for clinical applications. The inventors proceeded further with research to discover a cell indicator that more suitably reflects a corneal endothelial functional property of a functional matured differentiated corneal endothelial cell.

In one embodiment, the corneal endothelial property possessing functional cell of the invention has a corneal expression property of the cell indicator defined herein.

Cell indicators that the corneal endothelial property possessing functional cell of the invention may have include cell surface markers (CD markers and the like), cell product property, cell morphology indicator, genetic property of a cell and the like. Specific examples of cell indicators can include cell surface markers (CD markers and the like); property of proteinaceous product and related biological material of the product; expression property of SASP related protein; expression of miRNA (e.g., intracellular miRNA, secreted miRNA or the like); property of exosome; expression property of cell metabolite and related biological material of the metabolite; cell size; cell density and presence of autoantibody reactive cell. The functional mature differentiated corneal endothelial cell of the present invention has such cell indicators that exhibit a cell functional property in a specific range or level or a combination thereof. Thus, it is possible to determine whether a cell is the functional mature differentiated corneal endothelial cell of the present invention by defining a specific range or level of cell functional property or a combination thereof for a specific cell indicator. The specific range or level of cell functional property or a combination thereof unique to the functional mature differentiated corneal endothelial cell of the present invention was first identified in the present invention, whereby various cell subpopulations are identified to allow controlling and testing quality and thus achieving a highly effective therapy. Such cell indicator and the specific range or level of cell functional property or a combination thereof is specifically discussed in more detail below.

In a specific embodiment, the corneal endothelial property possessing functional cell of the invention has a cell functional property comprising CD166 positive and CD133 negative. Additional important cell functional property that is important includes the property of expressing CD44. The expression intensity thereof is not limited to, but is preferably CD44 negative to intermediately positive, more preferably CD44 negative to weakly positive, and still more preferably CD44 negative. The present invention has discovered that a corneal endothelial cell or cell differentiated into corneal endothelium-like form can be confirmed to be functional by confirming the cell to be CD166 positive and CD133 negative. In addition, it is possible to find out whether a cell is functional with high precision by confirming, in addition to the above, the expression of CD44 to be low (CD44 negative to intermediately positive, preferably CD44 negative to weakly positive).

Thus, in a preferred embodiment, the corneal endothelial property possessing functional cell of the invention has a cell functional property comprising CD166 positive, CD133 negative and CD44 negative to weakly positive. Although not wishing to be bound by any theory, a corneal endothelial cell or cell differentiated into corneal endothelium-like form was confirmed to be a functional mature differentiated corneal endothelial cell with high functional quality by having three such cell markers. Such functionality is demonstrated in results of clinical researches as accomplishing a high level of therapeutic effect in a short period of time (e.g., about one month) in terms of values in a corneal endothelial cell clarity test (specular), i.e., level exceeding about 1000 (cells/mm²), level exceeding about 2000 (cells/mm²), preferably a level exceeding about 2300 (cells/mm²), more preferably a level exceeding about 2500 (cells/mm²), or in some cases a level exceeding about 3000 (cells/mm²).

More preferably, the corneal endothelial property possessing functional cell of the invention has a cell functional property comprising CD166 positive, CD133 negative and CD44 negative. Although not wishing to be bound by any theory, a high quality cell with highly guaranteed proliferation ability or the like (also referred to as "high quality" functional mature differentiated corneal endothelial cell herein) can be more suitably provided with further limitation to CD44 negative cells. A "high quality" functional mature differentiated corneal endothelial cell has more stability and improved corneal endothelial functional property.

In another embodiment, the corneal endothelial property possessing functional cell of the invention has a cell functional property comprising CD166 positive, CD133 negative, and CD200 negative. For CD200, CD200 positive has been considered to be a property of a corneal endothelial cell. Meanwhile, the present invention examined each subpopulation in detail to discover that a CD200 positive cell is a large cell with CST that is not suitable for infusion, and CD200 negative is a property of a functional corneal endothelial cell capable of eliciting a human corneal functional property when infused into an anterior chamber of a human eye. Such a property was unexpected from conventional knowledge and is considered a result of careful analysis of subpopulations in the present invention.

In another embodiment, the corneal endothelial property possessing functional cell of the invention has a cell functional property comprising CD166 positive, CD133 negative, CD44 negative to CD44 weakly positive and CD90 negative to week positive. This can further guarantee the homogeneity of cells. Alternatively, the cell surface antigens comprise CD166 positive, CD133 negative, and CD44 negative to intermediate positive and CD90 negative phenotypes. In another embodiment, the cell surface antigens comprise CD166 positive, CD133 negative, and CD44 negative to CD44 weak positive phenotypes, or alternatively the cell expresses a cell surface antigen comprising CD44 negative to CD44 weak positive phenotype.

The corneal endothelial property possessing functional cell of the invention may further have an additional cell functional property. Such a cell functional property may include, but not limited to, one or more expression properties among the following expression properties: CD90 negative (CD90 negatively to weakly positive), CD105 negative to weakly positive, CD24 negative, CD26 negative, LGR5 negative, SSEA3 negative, MHC1 weakly positive (especially weakly positive relative to a cell with state transition), MHC2 negative, PDL1 positive, ZO-1 positive, Na⁺/K⁺ATPase positive, Claudin 10 positive and the following Table 1A:

**[Table 1A]**

| **Cell surface marker** | **Functional cell** |
|---|---|
| CD59 | **Strongly positive** |
| CD147 | **Strongly positive** |
| CD81 | **Strongly positive** |
| CD73 | **Strongly positive** |
| CD49c | **Strongly positive** |
| CD166 | **Strongly positive** |
| CD56 | **Intermediately positive** |
| CD54 | **Intermediately positive** |
| B2-uGlob | **Intermediately positive** |
| CD47 | **Intermediately positive** |
| CD46 | **Intermediately** positive |
| CD141 | **Intermediately positive** |
| CD151 | **Intermediately positive** |
| CD98 | **Weakly positive** |
| CD165 | **Weakly positive** |
| CD340 (Her2) | **Weakly positive** |
| CD58 | **Weakly positive** |
| CD201 | **Weakly positive** |
| CD140b | **Weakly positive** |
| EGF-r | **Weakly positive** |
| CD63 | **Weakly positive** |
| CD9 | **Negative** |
| CD49b | **Negative** |
| CD227 | **Negative** |
| CD90 | **Negative** |
| CD44 | **Negative** |

(wherein the value of median fluorescence intensity of each marker/ negative control (staining by isotype control antibody) is
30 or greater: strongly positive
10 or greater and less than 30: intermediately positive
5 or greater and less than 10: weakly positive

(it should be noted that weakly positive, intermediately positive, and strongly positive are collectively referred to as "positive"), and
less than 5: negative). Alternatively, the group may be the group consisting of CD105 negative to weak positive, CD24 negative, CD26 negative, LGR5 negative, SSEA3 negative, MHC1 weak positive, MHC2 negative, ZO-1 positive, Na⁺/K⁺ATPase positive.

Various genes used in the present invention are identified by the following accession numbers.

**[Table 1B-1]**

| NCBI _{Gene Name} | Amino Acid Sequence Accession | Nucleic Acid Sequence Accession |
|---|---|---|
| MMP2 | NM_001127891.2 | NP_001121363.1 |
| | NM 001302508.1 | NP_001289437.1 |
| | NM_001302509.1 | NP_001289438.1 |
| | NM_001302510.1 | NP_001289439.1 |
| | NM_004530.5 | NP_004521.1 7 |
| ILF3 (Another name for MMP4) | NM_001137673.1 | NP_001131145.1 |
| | NM_004516.3 | NP_004507.2 |
| | NM_012218.3 | NP_036350.2 |
| | NM_017620.2 | NP_060090.2 |
| | NM_153464.2 | NP_703194.1 |
| MMP9 | NM_004994.2 | NP_004985.2 |
| SPP1 | NM_000582.2 | NP_000573.1 |
| | NM_001040058.1 | NP_001035147.1 |
| | NM_001040060.1 | NP_001035149.1 |
| | NM_001251829.1 | NP_001238758.1 |
| | NM_001251830.1 | NP_001238759.1 |
| TIMP1 | NM_003254.2 | NP_003245.1 |
| BMP2 | NM_001200.3 | NP_001191.1 |
| STEAP1 | NM_012449.2 | NP_036581.1 |
| SPARC | NM_001309443.1 | NP_001296372.1 |
| | NM_001309444.1 | NP_001296373.1 |
| | NM_003118.3 | NP_003109.1 |
| IL13RA2 | NM_000640.2 | NP_000631.1 |
| TGF *β* 1 | NM_000660.5 | NP_000651.3 |
| TGF *β* 2 | NM_001135599.2 | NP_001129071,1 |
| | NM_003238.3 | NP_003229.1 |
| EGFR | NM_005228.3 | NP_005219.2 |
| | NM_201282.1 | NP_958439.1 |
| | NM_201283.1 | NP_958440.1 |
| | NM_201284.1 | NP_958441.1 |
| FN1 | NM_001306129.1 | NP_001293058.1 |
| | NM_001306130.1 | NP_001293059.1 |

**[Table 1B-2]**

| | | |
|---|---|---|
| | NM_001306131.1 | NP_001293060.1 |
| | NM_001306132.1 | NP_001293061.1 |
| | NM_002026.2 | NP_002017.1 |
| | NM_054034.2 | NP_473375.2 |
| | NM_212474.1 | NP_997639.1 |
| | NM_212476.1 | NP_997641.1 |
| | NM_212478.1 | NP_997643.1 |
| | NM_212482.1 | NP_997647.1 |
| EGR1 | NM_001964.2 | NP_001955.1 |
| SERPIN82 | NM_001143818.1 | NP_001137290.1 |
| | NM_002575.2 | NP_002566.1 |
| CD44 | NM_000610.3 | NP_000601.3 |
| | NM_001001389.1 | NP_001001389.1 |
| | NM_001001390.1 | NP_001001390.1 |
| | NM_001001391.1 | NP_001001391.1 |
| | NM_001001392.1 | NP_001001392.1 |
| | NM_001202555.1 | NP_001189484.1 |
| | NM_001202556.1 | NP_001189485.1 |
| | NM_001202557.1 | NP_001189486.1 |
| ALCAM (Another name for CD166) | NM_001243280.1 | NP_001230209.1 |
| | NM_001243281.1 | NP_001230210.1 |
| | NM_001243283.1 | NP_001230212.1 |
| | NM_001627.3 | NP_001618.2 |
| ENG (Another name for CD105) | NM_000118.3 | NP_000109.1 |
| | NM_001114753.2 | NP_001108225.1 |
| | NM_001278138.1 | NP_001265067.1 |
| CD24 | NM_001291737.1 | NP_001278666.1 |
| | NM_001291738.1 | NP_001278667.1 |
| | NM_001291739.1 | NP_001278668.1 |
| | NM_013230.3 | NP_037362.1 |
| COL1A2 | NM_000089.3 | NP_000080.2 |
| COL3A1 | NM_000090.3 | NP_000081.1 |
| COL4A1 | NM_001303110.1, | NP_001290039.1. |
| COL4A2 | NM_001846.2. | NP_001837.2. |

**[Table 1B-3]**

| | | |
|---|---|---|
| COL5A1 | NM_000093.4. | NP_000084.3. |
| COL8A1 | NM_001850.4 | NP_001841.2 |
| | NM_020351.3 | NP_065084.2 |
| COL8A2 | NM_005202.3 | NP_005193.1 |
| | NM_001294347.1 | NP_001281278.1 |
| IL6 | XM_011515390.1 | XP_011513692.1 |
| | XM_005249745.3 | XP_005249802.1 |
| | XM_011515391.1 | XP-_011513693.1 |
| CXCL8 (Another name for IL8) | NM_000584.3 | NP_00575.1 |
| 1L10 | NM_000572.2 | NP_000563.1 |
| IL18 | NM_001243211.1 | NP_001230140.1 |
| | NM_001562.3 | NP_001553.1 |
| IL33 | NM_001199640.1 | NP_001186569.1 |
| | NM.001199641.1 | NP_001186570.1 |
| | NM_001314044.1 | NP_001300973.1 |
| | NM_001314045.1 | NP_001300974.1 |
| | NM_001314046.1 | NP_001300975.1 |
| | NM_001314047.1 | NP_001300976.1 |
| | NM_001314048.1 | NP_001300977.1 |
| | NM_033439.3 | NP_254274.1 |
| TSLP | NM_033035.4 | NP_149024.1 |
| | NM_138551.4 | NP_812561.2 |
| CDH1 | NM_001317184.1 | NP_001304113.1 |
| | NM_001317185.1 | NP_001304114.1 |
| | NM_001317186.1 | NP_001304115.1 |
| | NM_004360.4 | NP_004351.1 |
| CDH2 | NM_001308176.1 | NP_001295105.1 |
| | NM_001792.4 | NP_001783.2 |
| VIM | NM_003380.3 | NP_003371.2 |
| CDKN1B | NM_004064.4 | NP_004055.1 |
| CDKN1C | NM_000076.2 | NP_000067.1 |
| | NM_001122630.1 | NP_001116102.1 |

**[Table 1B-4]**

| | | |
|---|---|---|
| | NM_001122631.1 | NP_001116103.1 |
| CDKN2C | NM_001262.2 | NP_001253.1 |
| | NM_078826.2 | NP_523240.1 |
| NOX4 | NM_001143836.2 | NP_001137308.1 |
| | NM_001143837.1 | NP_001137309.1 |
| | NM_001291926.1 | NP_001278855.1 |
| | NM_001291927.1 | NP_001218856.1 |
| | NM_001291929.1 | NP_001278858.1 |
| | NM_001300995.1 | NP_001281924.1 |
| | NM_016931.4 | NP_058627.1 |
| HGF | NM_000601.4 | NP_000592.3 |
| | NM_001010931.2 | NP_001010931.1 |
| | NM_001010932.1 | NP_001010932.1 |
| | NM_001010933.2 | NP _001010933.1 |
| | NM_001010934.2 | NP_001010934.1 |
| THBS2 | NM_003247.3 | NP_003238.2 |
| LGR5 | NM_001277226.1 | NP_001264155.1 |
| | NM_001277227.1 | NP_001264156.1 |
| | NM_003667.3 | NP_003658.1 |
| IGFBP3 | NM_000598.4 | NP_000589.2 |
| | NM_001013398.1 | NP_001013416.1 |
| IGFBP4 | NM_001552.2 | NP_001543.2 |
| IGFBP7 | NM_001253835.1 | NP_001240764.1 |
| | NM_001553.2 | NP_001544.1 |
| ITG81 | NM_002211.3 | NP 002202.2 |
| | NM_033668.2 | NP_391988.1 |
| | NM_133376.2 | NP_596867.1 |
| WNT5A | NM_001256105.1 | NP_001243034. t |
| | NM_003392.4 | NP_003383.2 |
| SNAIL1 | NM_005985.3 | NP_005976.2 |
| SNAIL2 | NM_003068.4 | NP_003059.1 |
| IGFBP5 | NM_000599.3 | NP_000590.1 |

**[Table 1B-5]**

| | | | |
|---|---|---|---|
| MAP1B | | NM_045909.3 | NP_005900.2 |
| Serpine1 | | NM_000602.4 | NP_000593.1 |
| | ZE82 | NM_001171653.1 | NP_001165124.1 |
| | | NM_014795.3 | NP_055610.1 |
| TGFbR2 | | NM_001024847.2 | NP_001020018.1 |
| ITGA3 | | NM_002204.3 | NP_002195.1 |
| ITGA5 | | NM_002205.3 | NP_002196.3 |

The same applies to all other proteins mentioned in the present invention. Thus, it is understood that the existing protein or nucleic acid names not only refer to proteins or nucleic acids shown in the sequence listing, but also include functionally active derivatives.

As used herein, the expression intensity of a cell indicator marker such as a CD marker is indicated as negative (may be indicated as -; when - and +/- are distinguished, both are encompassed) for substantially no expression. As used herein, negative encompassed dull positive. Not negative, i.e., expression significantly observed is indicated as positive (i.e., may be indicated as + when indicated in two categories of + and -). When expression levels are especially distinguished, the intensity thereof is classified into three levels and identified by weakly positive, intermediately positive, and strongly positive. In context of the displayed graph of results from FACS measurement or the like, they may be indicated by the number of +s. Weakly positive, intermediately positive, and strongly positive may be indicated as +, ++, and +++, respectively, which are synonymous. In such a case distinctions can be made as "weakly positive", "intermediately positive", and "strongly positive". This may be referred simply as positive when distinction is not made. Intensity that do not reach weakly positive is generally referred to as negative. Such levels of intensity are used in a manner commonly used in the art. These levels are relative and are defined below. For instance, "-" refers to expression that is substantially not observed. Expression that is observed is classified into three levels, weakly positive, intermediately positive, and strongly positive. Signals can be classified into negative, weakly positive, intermediately positive, and strongly positive for FACS separation.

Specific levels indicated as negative, dull positive, weakly positive, intermediately positive, and strongly positive to indicate signal intensity in FACS can be identified by using mean fluorescence signal intensity (MFI). Cell distribution can be displayed as a histogram and shown as negative, dull positive, weakly positive, intermediately positive, and strongly positive after relative comparison. The baseline of judgment for a more specific measurement value is explained. Specifically, the following levels are examples thereof.

The intensity of expression of a cell indicator marker such as a CD marker is typically different in terms of fluorescence intensity due to the type of fluorescence of a label or equipment setting. Herein, the range of weak fluorescence intensity is about less than 3800, range of intermediate fluorescence intensity is about 3800 or greater and less than 27500, and range of strong fluorescent intensity is about 27500 or greater under the following condition: PE-Cy7-labeled anti-human CD44 antibody (BD Biosciences) is used and Area Scaling Factor of Blue laser of FACS Canto II is set to 0.75 and the voltage of PE-Cy7 is set to 495. In the Examples of the present specification, the mean fluorescence intensity of negative control (isotype control) at this setting was about 50 (range of 55 +/- 25; while there may be a small deviation even under the same setting depending on the cell lot, those skilled in the art can carry out the test while understanding such deviations). Thus, in view of "range of weak fluorescence intensity is about less than 3800, range of intermediate fluorescence intensity is about 3800 or greater and less than 27500, and range of strong fluorescent intensities is about 27500 or greater", mean fluorescence intensity of negative control (isotype control) PE-Cy7: about 50 [approximately 33-80]. Thus, weak: < 76-fold, intermediate: 76 to 550-fold, and strong > 550-fold. A staining intensity pattern that is the same as the negative control (isotype control) is determined to be negative, and positive when the pattern is shifted even slightly.

When used in the present specification, examples of other settings include the following.
*Area Scaling Factor: FSC=0.5, Blue laser= 0.75, Red laser=0.8
*voltage: FSC=270, SSC=400, FITC=290, PE=290, PerCP-Cy5.5=410, PE-Cy7=495, APC=430
*Examples of mean fluorescence intensity of negative control (isotype control) include the following.
   FITC: about 130 [approximately 65-225]
   PE: about 120 [approximately 73-204]
   PerCP-Cy5.5: about 120 [approximately 74-191]
   PE-Cy7: about 50 [approximately 33-80]
   APC: about 110 [approximately 67-196]

In another embodiment, for detection in case of using a Lyoplate experiment (Examples, Table 2), Alexa Fluor 647-labeled secondary antibody (attached to kit) is used for measurement. In such a case, weakly positive, intermediately positive, and strongly positive can be defined as follows. Specifically, the value of median fluorescence intensity of each marker/negative control (staining by isotype control antibody) on the left leads to the category on the right.
30 or greater: strongly positive
10 or greater and less than 30: intermediately positive
5 or greater and less than 10: weakly positive
(it should be noted that weakly positive, intermediately positive, and strongly positive are collectively referred to as "positive"), and
less than 5: negative

Such intensity of a cell marker can be readily assessed by techniques such as fluorescence activating cell sorting, immunohistochemical technique or the like (not limited thereto). For the above-described markers and expression levels thereof, "negative" refers to lack of expression of the markers or notably low levels thereof and "positive" refers to notable expression. Transition of a cell marker from "negative" to "positive" indicates a change from lack of expression or low level of expression to high level or notable level of expression. The term "weakly positive" refers to weak expression, i.e., low level of expression and is also denoted as "low expression". Since "intermediately positive" refers to readily detectable intermediate level of expression, it is also denoted as "intermediate expression". "Strongly positive" refers to notable expression which is very readily detectable strong expression, i.e., high level of expression, and is also denoted as "high expression", In this regard, transition from "weakly positive" to "intermediately positive", "intermediately positive" to "strongly positive" or "strongly positive" to "intermediately positive", or "intermediately positive" to "weakly positive" expression can be readily confirmed. For example, a non-intended cell exhibit CD44 strongly positive, progenitor cell exhibits CD44 intermediately positive, and the mature differentiated functional corneal endothelial cell of the present invention exhibits CD44 negative or CD44 weakly positive. As shown for instance in the Examples, two or more cell surface markers or the like can be used to classify a cell into a subpopulation or the like.

Additional cell indicators used in the present invention include expression intensities of MHC-1 and MHC-2, which are both associated with lack of immunological rejection. Since the present invention is used clinically in cell infusion therapy, no or low immunological rejection is preferred.

Additional cell indicators used in the present invention include ZO-1 and Na⁺/K⁺ ATPase. They are properties that are closely related to expression of functionality of a human corneal endothelial cell. Thus, it is preferred that they are both clearly expressed (+) in a regular manner.

The present invention can also use a proteinaceous product or related biological material of the product as a cell indicator. In the present invention, examples thereof include those with (A) elevated expression in the corneal endothelial property possessing functional cell of the invention (including functional mature differentiated corneal endothelial cell) and (B) decreased expression in the corneal endothelial property possessing functional cell of the invention (including functional mature differentiated corneal endothelial cell) such as CD44. The proteinaceous product or related biological material of the present invention can determine whether a target cell is the corneal endothelial property possessing functional cell of the invention (functional mature differentiated corneal endothelial cell or intermediately differentiated corneal endothelial cell) by one or a combination or more cell indicators. In a certain embodiment, multiple proteinaceous products or related biological materials of the product can be selected and combined from (A), selected and combined from (B), or proteinaceous products or related biological materials of the product from (A) and (B) can be combined and used. Although not wishing to be bound by any theory, this is because these genes have a relatively high expression and cell suitable for therapy and cell that is not can be clearly categorized. For (A), it is understood that an intermediately differentiated corneal endothelial cell also exhibits a similar tendency as a functional mature differentiated corneal endothelial cell.

In addition, one or more of the following can be used in a preferred embodiment (may partially overlap with those described above).

**[Table 1C]**

| | | | | | | |
|---|---|---|---|---|---|---|
| MMP1 | TGFβ1 | COL1A2 | IL6 | CDH1 | NOX4 | IGFBP3 |
| MMP2 | TGFβ2 | COL3A1 | IL8 | CDH2 | HGF | IGFBP4 |
| MMP4 | EGFR | COL4A1 | IL10 | VIM | THBS2 | IGFBP7 |
| MMP9 | FN1 | COL4A2 | IL18 | CDKN1B | LGR5 | ITGB1 |
| SPP1 | EGR1 | COL5A1 | IL33 | CDKN1C | | WNT5A |
| TIMP1 | SERPINB2 | COL8A1 | TSLP | CDKN2C | | SNAIL1 |
| BMP2 | CD44 | COL8A2 | | | | SNAIL2 |
| STEAP1 | CD166 | | | | | |
| SPARK | CD105 | | | | | |
| IL13RA2 | CD24 | | | | | |

Viewed in the morphological basis, it is known to have the following properties, and such information can be used to use an appropriate marker. That is, since MMP9, SPP1, STEAP1, IL33, TSLP, and CDH1 have low expression intensity, it is necessary to be creative for quantitative experiments. COL4A1, COL4A2, COL8A1, COL8A2, CDH2, and TGF-beta2 have elevated expression in the corneal endothelial property possessing functional cell of the invention or functional mature differentiated corneal endothelial cell. MMP1, MMP2, TIMP1, BMP2, IL13RA2, TGF-beta1, CD44, COL3A1, IL6, IL8, HGF, THBS2, and IGFBP3 have elevated expression in a nonfunctional cell. MMP4, CD105, and CD24 have distinct expression intensity between the corneal endothelial property possessing functional cell of the invention or functional mature differentiated corneal endothelial cell and a nonfunctional cell. CD166 and IGFBP7 can also be used.

While the corneal endothelial property possessing functional cell provided by the present invention enables a revolutionary therapeutic method in clinical applications, quality control is necessary. Thus, a highly reliable method therefor is required. The present invention revealed that diversity of genes can be used for identification and quality control of a functional mature differentiated corneal endothelial cell that does not undergo cell state transition (CST) or karyotype abnormality (aneuploidity). The morphological features of corneal endothelial cells greatly differ among cultures even when the same culture protocol is used. One of the largest obstacles to applying a corneal endothelial cell to cell infusion therapy is how to verify a corneal endothelial cell as meeting the cell quality as the corneal endothelial property possessing functional cell of the invention or a functional mature differentiated corneal endothelial cell. Meanwhile, the present invention can solve this obstacle by utilizing genetic diversity.

In one embodiment, the corneal endothelial property possessing functional cell of the invention can have a property specific to the functionality of a specific cytokine or a related substance thereof. Examples of such a property include, but are not limited to, high PDGF-BB production, low IL-8 production, low MCP-1 production, high TNF-alpha production, high IFNgamma production, high IL-IR antagonist production, low VEGF production and the like. Cytokine levels reflecting a state of attaching others such as an inflammatory cell are not preferable as an indicator. Cytokine levels reflecting a normal state is preferred.

As used herein, "high production" and "low production" are relative and determined as being high or low compared to a generally observed level for each cytokine or the like. For instance, when used in the present invention such as culture with the culturing method exemplified in Example 4 (using a basal medium prepared with Opti-MEM-I (Life Technologies Corp., Carlsbad, CA, USA), 8% fetal bovine serum (FBS), 5 ng/mL epidermal growth factor, 20 .micro.g/mL ascorbic acid, 200 mg/L calcium chloride, 0.08% chondroitin sulfate, and 50 .micro.g/mL of gentamicin, and appropriate condition medium (Nakahara, M. et al. PLOS One (2013) 8, e69009)), it is typically preferable that PDGF-BB is about 30 pg/ml or greater and IL-8 is about 500 pg/ml. Further, MCP-1 is preferably about 3000 pg/ml or less. TNF-alpha is preferably about 10 pg/ml or greater, and IFNgamma is preferably about 30 pg/ml or greater. IL-1R antagonists are preferably about 40 pg/ml or greater, and VEGF is preferably about 200-500 pg/ml or less.

The advantages of the present invention are also in providing a cell indicator for evaluating the quality beyond visual inspection of the final product, the corneal endothelial property possessing functional cell of the invention or functional mature differentiated corneal endothelial cell. This because it was revealed in the research process of the present invention that product quality defined by mold specification or proteinaceous secreted product does not correspond to pharmacological effects in clinical settings. Although specifications applied in human stem clinical studies were all confirmed to be satisfied, it was found that the proportion constituted by non-intended cells and the amount of production of the protein product MCP-1 are not in a desired range. Thus, the indicator of MCP-1 is also preferably low production in a preferred embodiment.

The cells of the present invention preferably satisfy the following standards in a quality test prior to use.

The visual inspection at this time includes confirming the presence of a hexagonal cobble-stone shape and lack of fibrosis.

**[Table 1D]**

| | |
|---|---|
| **Entry** | Reference value |
| **Visual inspection** | |
| ZO-1 | **Positive** |
| Na-K ATPase | **Positive** |
| Claudin 10 | **Positive** |
| TIMP-1 | < 500ng/mL |
| IL-8 | < 500pg/mL |
| PDGF-bb | > 30pg/mL |
| MCP-1 | < 3000pg/mL |
| **Effector cells (E-ratio)** | > 50% |
| **Non-intended cells A** | < 15% |
| **Non-intended cells B** | < 5% |
| **Non-intended cells C** | < 10% |
| **Karyotype abnormality** | **Negative** |

The method of calculating E-ratios and the method of calculating non-intended cells A, B, and C are the following.

Method of calculating the E-ratio: Typically, fluorescence intensities differ depending on the type of fluorescence of a label or equipment setting. In a measurement under the following condition: PE-Cy7-labeled anti-human CD44 antibody (BD Biosciences) is used and Area Scaling Factor of Blue laser of FACS Canto II is set to 0.75 and the voltage of PE-Cy 7 is set to 495, gates are set as follows: First, fractions A, B, C, and D are set in a dot plot (Fig. 68, top row left side) with X axis as CD24 and Y axis as CD166. At this time, fraction A is CD24 negative and CD166 positive, fraction B is CD24 positive and CD166 positive, fraction C is CD24 negative and CD166 negative, and fraction D is CD24 positive and CD166 negative. The proportion of fraction B, when target cells for analysis is 100%, is considered the content of non-intended cell C [CD24 positive cell]. Fractions 1, 2, and 3 are further set as in the diagram in the bottom row on the left side of Fig. 68 in a dot plot where the X axis is CD44 and the Y axis is CD105 for fraction B. The proportion of fraction 1 at this time is the E-ratio, the total value of the proportion of fractions 1 and 2 is the "functional mature differentiated corneal endothelial cells + intermediately differentiated corneal endothelial cells" content, and the proportion of fraction 3 is the non-intended cell A [CD44 strongly positive cell] content. Further, a separate dot plot where the X axis is CD44 and the Y axis is CD26 is created and fractions a', b', c', and d' are set as in the diagram on the top row right side of Fig. 68. The proportion of fraction B, when the target cell of analysis is 100%, is the non-intended cell B [CD26 positive cell] content (see Fig. 68).

In one embodiment, the present invention provides a cell with a specific miRNA, especially a corneal endothelial cell, based on the discovery for the first time that the functionality of the cell can be identified with miRNA. The present invention provides for the first time the ability to use the difference in the type of miRNA to identify the functionality of the cell with expression thereof. microRNA (miRNA or miR) is a non-coding small molecule RNA that functions as an endogenous regulator of gene expression. The dysregulation thereof is associated with pathogenic factors of various diseases. There is growing evidence suggesting that miRNA plays an import role in various biological processes including cell growth, development, and differentiation (Bartel DP. Cell. 2004; 116:281-297; Croce CM, Cell. 2005; 122:6-7). Expression of miRNA is essential in the regulation of many cell processes including formation, maintenance, and reconstruction of extracellular matrix (ECM) (Rutnam ZJ, Wight TN, Yang BB. Matrix Biol. 2013; 32:74-85), and has a close connection with CST in cHCECs. There is currently no information on miRNA expression related to corneal endothelium which can identify a functional mature differentiated corneal endothelial cell. The present invention, in this context, has discovered miRNA that can be used in identifying a functional mature differentiated corneal endothelial cell. With such miRNA, various types of miRNA expression patterns were found by comparative studies on functional mature differentiated corneal endothelial cells with different phenotypes by a 3D-Gene miRNA microarray platform (sold, for example, by Toray, Kamakura, Japan) and hierarchical clustering. Unique miRNA expression patterns comprising upregulated and downregulated miRNA clusters were found in cultured cells and corresponding culture supernatant. miRNAs in culture supernatant, i.e., secreted miRNAs, can function as a tool for noninvasively identifying a functional mature differentiated corneal endothelial cell suitable for cell therapy by injection into the anterior chamber.

In a representative embodiment with a functional mature differentiated corneal endothelial cell (a5), intermediately differentiated corneal endothelial cell (a1), and corneal endothelial nonfunctional cell (a2), when the expression property of a5 cell is defined as CD44 negative to weakly positive CD24 negative CD26 negative, the expression property of a1 as CD44 intermediately positive CD24 negative CD26 negative, and the expression property of a2 as CD44 strongly positive CD24 negative CD26 positive, at least one miRNA has the a5 property for the corneal endothelial property possessing functional cell of the invention.

In one embodiment, the following is provided as the miRNA markers used in the present invention. Specifically, the property of said miRNA comprises at least one miRNA selected from the group consisting of those the pattern of which are:
(A) functional mature differentiated corneal endothelial cell (a5): intermediately differentiated corneal endothelial cell (a1): corneal endothelial nonfunctional cell (a2) exhibits high expression: high expression: low expression:
   (intracellular) miR23a-3p, miR23b-3p, miR23c, miR27a-3p, miR27b-3p, miR181a-5p, miR181b-5p, miR181c-5p, miR181d-5p
   (cell-secreted) miR24-3p, miR1273e;
(B) a5:a1:a2 exhibits high expression: intermediate expression: low expression:
   (intracellular) miR30a-3p, miR30a-5p, miR30b-5p, miR30c-5p, miR30e-3p, miR30e-5p, miR130a-3p, miR130b-3p, miR378a-3p, miR378c, miR378d, miR378e, miR378f, miR378h, miR378i, miR184, miR148a-3p
   (cell-secreted) miR184;
(C) a5:a1:a2 exhibits high expression: low expression: low expression:
   (intracellular) miR34a-5p, miR34b-5p
   (cell-secreted) miR4419b, miR371b-5p, miR135a-3p, miR3131, miR296-3p, miR920, miR6501-3p;
(D) a5:a1:a2 exhibits low expression: low expression: intermediate to high expression:
   (intracellular) miR29a-3p, miR29b-3p, miR199a-3p, miR199a-5p, miR199b-5p, miR143-3p
   (cell-secreted) miR1915-3p, miR3130-3p, miR92a-2-5p, miR1260a;
(E) a5:a1:a2 exhibits low expression: intermediate expression: high expression:
   (intracellular) miR31-3p, miR31-5p, miR193a-3p, miR193b-3p, miR138-5p
(F) a5:a1:a2 exhibits high expression: low expression: high expression:
   (cell-secreted) miR92b-5p; and
(G) a5:a1:a2 exhibits low expression: high expression: low expression:
   (cell-secreted) miR1246, miR4732-5p, miR23b-3p, miR23a-3p, miR1285-3p, miR5096;
   wherein a property of a cell surface antigen of the a5 is CD44- to weakly positive, CD24 negative CD26 negative, an expression level is relative intensity among 3 types of cells, expression of a cell surface antigen of the a1 being CD44 intermediately positive CD24 negative CD26 negative, and expression of a cell surface antigen of the a2 being CD44 strongly positive CD24 negative CD26 positive.

Expression intensities are strong expression > intermediate expression > low expression, and strong expression and low expression have a statistically significant difference. The following schematically represents the above.

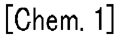

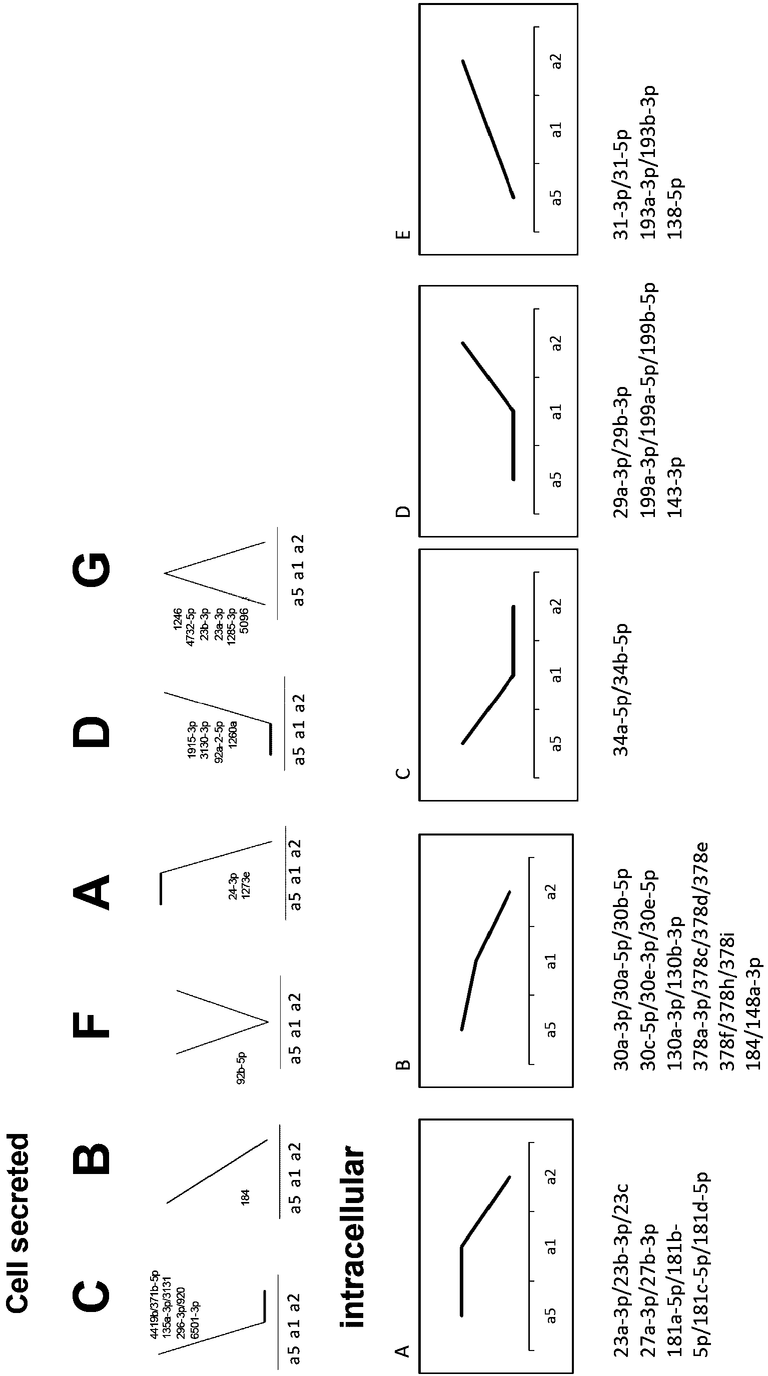

The strong expression, intermediate expression, and weak expression, although the absolute levels vary depending on each miRNA, can be appropriately determined upon actual measurement. Typically, the amount of miR expression (expression intensity) is a comparative value corrected to have identical median expression intensity value of all genes detected by assuming that the total number of copies of gene among samples is not notably different after determining genes whose fluorescence intensity has been measured. High expression and low expression have a significant difference (relative ratio of 2 or greater, p-value of 0.05 or less). Intermediate expression can be included as needed. When a third expression intensity that is different from the strongest and weakest is found in three or more groups of cells, assessment may include intermediate expression.

Various miRNAs used in the present invention are specified by the following numbers. When used in the present invention, relative intensities can be measured based on the information on matured forms (MiRBase), but it is not limited thereto. It is understood that those skilled in the art can similarly measure relative intensities by using information of Stem-Loop and appropriately processing the information.

**[Table 1E-1]**

| **miR** | **Stem loop** | **miRBase accession** | **Notes** |
|---|---|---|---|
| hsa-miR-23a-3p | MI0000079 | MIMAT0000078 | Immature: Homo sapiens |
| | | | miR-23a stem-loop |
| hsa-miR-23b-3p | MI0000439 | MIMAT0000418 | Immature: Homo sapiens |
| | | | miR-23b stem-loop |
| hsa-miR-23c | MI0016010 | MIMAT0018000 | Immature: Homo sapiens |
| | | | miR-23c stem-loop |
| hsa-miR-27a-3p | MI0000085 | MIMAT0000084 | Immature: Homo sapiens |
| | | | miR-27a stem-loop |
| hsa-miR-27b-3p | MI0000440 | MIMAT0000419 | Immature: Homo sapiens |
| | | | miR-27b stem-loop |
| hsa-miR-181a-5p | MI0000289 | MIMAT0000256 | Immature: Homo sapiens |
| | | | miR-181a-1 stem-loop |
| | MI0000269 | | Immature: Homo sapiens |
| | | | miR-181a-2 stem-loop |
| hsa-miR-181b-5p | MI0000270 | MIMAT0000257 | Immature: Homo sapiens |
| | | | miR-181b-1 stem-loop |
| | MI0000683 | | Immature: Homo sapiens |
| | | | miR-181b-2 stem-loop |
| hsa-miR-181c-5p | MI0000271 | MIMAT0000258 | Immature: Homo sapiens |
| | | | miR-181c stem-loop |
| hsa-miR-181d-5p | MI0003139 | MIMAT0002821 | Immature: Homo sapiens |
| | | | miR-181d stem-loop |
| hsa-miR-24-3p | MI0000080 | MIMAT0000080 | Immature: Homo sapiens |
| | | | miR-24-1 stem-loop |
| | MI0000081 | | Immature: Homo sapiens |
| | | | miR-24-2 stem-loop |
| hsa-miR-1273e | MI0016059 | MIMAT0018079 | Immature: Homo sapiens |
| | | | miR-1273e stem-loop |
| hsa-miR-30a-3p | MI0000088 | MIMAT0000088 | Immature: Homo sapiens |
| hsa-miR-30a-5p | | MIMAT0000087 | miR-30a stem-loop |
| hsa-miR-30b-5p | MI0000441 | MIMAT0000420 | Immature: Homo sapiens |
| | | | miR-30b stem-loop |

**[Table 1E-2]**

| | | | |
|---|---|---|---|
| hsa-miR-30c-5p | MI0000736 | MIMAT0000244 | Immature: Homo sapiens |
| | | | miR-30c-1 stem-loop |
| | MI0000254 | | Immature: Homo sapiens |
| | | | miR-30c-2 stem-loop |
| hsa-miR-30e-3p | MI0000749 | MIMAT0000693 | Immature: Homo sapiens |
| hsa-miR-30e-5p | | MIMAT0000692 | miR-30e stem-loop |
| hsa-miR-130a-3p | MI0000448 | MIMAT0000425 | Immature: Homo sapiens |
| | | | miR-130a stem-loop |
| hsa-miR-130b-3p | MI0000748 | MIMAT0000691 | Immature: Homo sapiens |
| | | | miR-130b stem-loop |
| hsa-miR-378a-3p | MI0000786 | MIMAT0000732 | Immature: Homo sapiens |
| | | | miR-378a stem-loop |
| hsa-miR-378c | MI0015825 | MIMAT0016847 | Immature: Homo sapiens |
| | | | miR-378c stem-loop |
| hsa-miR-378d | MI0016749 | MIMAT0018926 | Immature: Homo sapiens |
| | | | miR-378d-1 stem-loop |
| | MI0003840 | | Immature: Homo sapiens |
| | | | miR-378d-2 stem-loop |
| hsa-miR-378e | MI0016750 | MIMAT0018927 | Immature: Homo sapiens |
| | | | miR-378e stem-loop |
| hsa-miR-378f | MI0016756 | MIMAT0018932 | Immature: Homo sapiens |
| | | | miR-378f stem-loop |
| hsa-miR-378h | MI0016808 | MIMAT0018984 | Immature: Homo sapiens |
| | | | miR-378h stem-loop |
| hsa-miR-378i | MI0016902 | MIMAT0019074 | Immature: Homo sapiens |
| | | | miR-378i stem-loop |
| hsa-miR-184 | MI0000481 | MIMAT0000454 | Immature: Homo sapiens |
| | | | miR-184 stem-loop |
| hsa-miR-148a-3p | MI0000253 | MIMAT0000243 | Immature: Homo sapiens |
| | | | miR-148a stem-loop |
| hsa-miR-34a-5p | MI0000268 | MIMAT0000255 | Immature: Homo sapiens |
| | | | miR-34a stem-loop |
| hsa-miR-34b-5p | MI0000742 | MIMAT0000685 | Immature: Homo sapiens |
| | | | miR-34b stem-loop |

**[Table 1E-3]**

| | | | |
|---|---|---|---|
| hsa-miR-4419b | MI0016861 | MIMAT0019034 | Immature: Homo sapiens |
| | | | miR-4419b stem-loop |
| has-miR371b-5p | MI0017393 | MIMAT0019892 | Immature: Homo sapiens |
| | | | miR-371b stem-loop |
| hsa-miR-135a-3p | MI0000452 | MIMAT0004595 | Immature: Homo sapiens |
| | | | miR-135a-1 stem-loop |
| hsa-miR-3131 | MI0014151 | MIMAT0014996 | Immature: Homo sapiens |
| | | | miR-3131 stem-loop |
| hsa-miR-296-3p | MI0000747 | MIMAT0004679 | Immature: Homo sapiens |
| | | | miR-296 stem-loop |
| hsa-miR-920 | MI0005712 | MIMAT0004970 | Immature: Homo sapiens |
| | | | miR-920 stem-loop |
| hsa-miR-6501-3p | MI0022213 | MIMAT0025459 | Immature: Homo sapiens |
| | | | miR-6501 stem-loop |
| hsa-miR-29a-3p | MI0000087 | MIMAT0000086 | Immature: Homo sapiens |
| | | | miR-29a stem-loop |
| hsa-miR-29b-3p | MI0000105 | MIMAT0000100 | Immature: Homo sapiens |
| | | | miR-29b-1 stem-loop |
| | MI0000107 | | Immature: Homo sapiens |
| | | | miR-29b-2 stem-loop |
| hsa-miR-199a-3p | MI0000242 | MIMAT0000232 | Immature: Homo sapiens miR-199a-1 stem-loop |
| hsa-miR-199a-5p | MI0000281 | MIMAT0000231 | or Homo sapiens miR-199a-2 stem-loop |
| hsa-miR-199b-5p | MI0000282 | MIMAT0000263 | Immature: Homo sapiens |
| | | | miR-199b stem-loop |
| hsa-miR-143-3p | MI0000459 | MIMAT0000435 | Immature: Homo sapiens |
| | | | miR-143 stem-loop |
| hsa-miR-1915-3p | MI0008336 | MIMAT0007892 | Immature: Homo sapiens |
| | | | miR-1915 stem-loop |
| hsa-miR-3130-3p | MI0014147 | MIMAT0014994 | Immature: Homo sapiens |
| | | | miR-3130-1 stem-loop |
| | MI0014148 | | Immature: Homo sapiens |
| | | | miR-3130-2 stem-loop |

**[Table 1E-4]**

| | | | |
|---|---|---|---|
| hsa-miR-92a-2-5p | MI0000094 | MIMAT0004508 | Immature: Homo sapiens |
| | | | miR-92a-2 stem-loop |
| hsa-miR-1260a | MI0006394 | MIMAT0005911 | Immature: Homo sapiens |
| | | | miR-1260a stem-loop |
| hsa-miR-31-3p | MI0000089 | MIMAT0004504 | Immature: Homo sapiens |
| hsa-miR-31-5p | | MIMAT0000089 | miR-31 stem-loop |
| hsa-miR-193a-3p | MI0000487 | MIMAT0000459 | Immature: Homo sapiens |
| | | | miR-193a stem-loop |
| hsa-miR-193b-3p | MI0003137 | MIMAT0002819 | Immature: Homo sapiens |
| | | | miR-193b stem-loop |
| hsa-miR-138-5p | MI0000476 | MIMAT0000430 | Immature: Homo sapiens |
| | | | miR-138-1 stem-loop |
| | MI0000455 | | Immature: Homo sapiens |
| | | | miR-138-2 stem-loop |
| hsa-miR-92b-5p | MI0003560 | MIMAT0004792 | Immature: Homo sapiens |
| | | | miR-92b stem-loop |
| hsa-miR-1246 | MI0006381 | MIMAT0005898 | Immature: Homo sapiens |
| | | | miR-1246 stem-loop |
| hsa-miR-4732-5p | MI0017369 | MIMAT0019855 | Immature: Homo sapiens |
| | | | miR-4732 stem-loop |
| hsa-miR-1285-3p | MI0006346 | MIMAT0005876 | Immature: Homo sapiens |
| | | | miR-1285-1 stem-loop |
| | MI0006347 | | Immature: Homo sapiens |
| | | | miR-1285-2 stem-loop |
| hsa-miR-5096 | MI0018004 | MIMAT0020603 | Immature: Homo sapiens |
| | | | miR-5096 stem-loop |

In a preferred embodiment, the miRNA marker used in the present invention comprises at least one selected from (B) or (C) in the above-described categories. This is because when using miRNAs having patterns of (B) and (C), the corneal endothelial property possessing functional cell of the invention (a5 + a1) can be identified by measuring only one marker. When identifying a functional mature differentiated corneal endothelial cell (a5) and intermediately differentiated corneal endothelial cell (a1) from non-intended cell (a2), identification is possible with one marker if, for example a pattern of (A), (B), (D), or (E) is used. Multiple miRNA markers can be used such as a combination of (A) and (C) or use of (B) or (E) when it is desirable to identify three types. miRNA can be identified, for example, by extracting RNA with a known approach and using microarray analysis with an approach described in the Examples to determine the level. For example, a commercially available chip such as Toray's 3D-Gene^{™} human microRNA chip can be used. The resulting data can be processed as an image with a scanner (e.g., 3D-Gene scanner 3000 (Toray Industries Inc., Tokyo, JAPAN)) and processed with a processing software (e.g., 3D-Gene Extraction software (Toray)). The resulting digitized fluorescent signal can be further standardized as raw data. For instance, the median value of fluorescence intensity can be corrected to 25. Alternatively, the standardized level can be corrected to match the 100th value from the highest ranking.

In a preferred embodiment, secreted miRNA is preferably used. This is because secreted miRNAs are capable of the so-called nondestructive identification that does not destroy a cell.

Unlike an approach of identifying a cell such as CD166 positive, CD133 negative, CD105 negative, CD44 negative, CD24 negative, CD26 negative, and CD200 negative with a cell surface marker (CD marker), identification of the corneal endothelial property possessing functional cell of the invention using an miRNA in the present invention is advantageous in that a noninvasive method can be provided.

In one embodiment, the corneal endothelial property possessing functional cell of the invention preferably does not have an exosome exhibiting an abnormal value. Exosomes are membrane vesicles secreted in a cell with a diameter of about 40 nm-150 nm. Related markers can be used to investigate such an abnormal value comprising many proteins with ribonuclease activity. Examples of such a marker include CD63, CD9, CD81, HSP70, and the like. The corneal endothelial property possessing functional cell of the invention preferably has low expression of such markers associated with exosomes. Specific levels can be exemplified by the following experiment. That is, it is typically possible to measure whether a marker is present in an exosome protein in culture supernatant using Exoscreen. Instead of Exoscreen, an exosome protein can be detected by Western blot. The size of the Western detection band can also be visually determined.

The corneal endothelial property possessing functional cell of the invention preferably has a small cell area, i.e., is small cell. In the present invention, a cell area is generally assessed with PBS-treated cells under image captured conditions. That is, a measurement value of hybrid cell count is measured with area while having spaces between cells because an image of PBS-treated cells is taken. That is, the cell area is measured at a lower value than in a mature and differentiated state with tight junction formation at saturated cell culture (confluent) in culture. The present invention has revealed that a functional cell has high quality by having a small area per cell to have the highest cell density in culture. This is recognized as the same or higher level of cell area and cell density as endothelial cells of normal corneal endothelial tissue. Examples of preferred cell area of PBS-treated cells upon saturated cell culture (confluence) include about 250 .micro.m² or less for the mean of the cell population or individual cells, and more preferably about 245 .micro.m² or less, about 240 .micro.m² or less, about 235 .micro.m² or less, about 230 .micro.m² or less, about 225 .micro.m² or less, about 220 .micro.m² or less, about 215 .micro.m² or less, about 210 .micro.m² or less, about 205 .micro.m² or less, about 200 .micro.m² or less and the like. Meanwhile, examples of values achieved as a preferred cell area of the corneal endothelial property possessing functional cell of the invention include, but are not limited to, about 150 .micro.m² or greater, about 155 .micro.m² or greater, about 160 .micro.m² or greater, about 165 .micro.m² or greater, about 170 .micro.m² or greater, about 175 .micro.m² or greater, about 180 .micro.m² or greater, and the like. The cell area can be measured by any approach known in the art. A typical example is a measurement method using phase contrast microscope images. Images can be taken herein using a commercially available system such as an inverted microscope system (CKX41, Olympus, Tokyo, Japan). For measuring area distribution, a target cell can be pretreated to facilitate measurement by washing with PBS(-) three times or the like and a phase contrast microscope image can be obtained by using a commercially available system such as BZ X-700 Microscope system, for example (Keyence, Osaka, Japan). Further, area distribution can be quantified using commercially available software such as BZ-H3C Hybrid cell count software (Keyence).

Thus, the corneal endothelial property possessing functional cell of the invention advantageously has the above-described preferred value in at least one of the cell indicators selected from the group consisting of cell size, cell density, and presence of an autoantibody reactive cell.

The corneal endothelial property possessing functional cell of the invention preferably has a cell functional property homologous to the corneal endothelial property possessing functional cell of the invention (i.e., including functional mature differentiated corneal endothelial cell and intermediately differentiated corneal endothelial cell), preferably a cell functional property homologous to a functional mature differentiate corneal endothelial cell a5, for at least one cell indicator selected from the group consisting of: a cell surface marker; a proteinaceous product and a related biological material of the product; a SASP related protein; intracellular and secreted miRNA; an exosome; a cellular metabolite comprising an amino acid and a related biological material of the metabolite; cell size; cell density and the presence of an autoantibody reactive cell explained herein. For example, any specific numerical value, range, or level described in the explanation regarding each indicator of the present specification is used or a combination thereof may be used as a preferred cell indicator in the present invention. When a certain candidate cell has a value of such cell indicators that the corneal endothelial property possessing functional cell of the invention defined herein, preferably functional mature differentiated corneal endothelial cell should have, the candidate cell is determined to be a functional mature differentiated corneal endothelial cell or intermediately differentiated corneal endothelial cell that expresses a human corneal endothelial functional property when infused into the anterior chamber of a human eye. The following indicators can also be referred in addition to, or in parallel with, the determination with the aforementioned cell indicators. In particular, a function of the corneal endothelial property possessing functional cell of the invention can be confirmed by formation of a small hexagonal cobble-stone shape and use of an energy metabolism system by mitochondrial function, and determined by whether it can be therapeutically effective upon infusion (e.g., into the anterior chamber of the eye). The indicators are not limited thereto, such that surrogate marker-like indicators are also effective. As such an indicator, any one of the following eight types of surrogate markers or a combination thereof can be used: (1) retention of endothelial pumping/barrier functions (including Claudin expression), (2) high adhesion/attachment to laminin 511 or a fragment E8 thereof, (3) secreted cytokine profile; production of PDGFbb, TNFalpha, IFNgamma, or IL-1 receptor antagonist is at or above refrence value, (4) stipulation by produced micro RNA (miRNA) profile, (5) stipulation by produced metabolite profile, (6), saturated cell density during in vitro culture, (7) spatial size or distribution of cells obtained in culture; and (8) adhesion to corneal endothelial surface in case of cell infusion after freeze damage by cryo treatment by liquid nitrogen on a mouse cornea. In particular, although not wishing to be bound by any theory, this is because a proteinaceous product or a related biological material of the product can mostly determine whether cells are CST cells, and miRNA can remove part or all of the unintended cells, and cell metabolite or a related biological material of the metabolite can distinguish an intermediately differentiated corneal endothelial cell from a functional mature differentiated corneal endothelial cell, such that a higher quality functional corneal endothelial cell can be selectively propagated in cultures.

In a preferred embodiment, the corneal endothelial property possessing functional cell of the invention, especially mature differentiated corneal endothelial cell, does not have a karyotype abnormality. The biggest obstacle in applying cHCECs to cell injection regenerative medicine is that cHCECs in many cases exhibit aneuploidy during culture with several passages, as demonstrated by Miyai et al (Miyai T, et al., Mol Vis. 2008; 14:942-50). Aneuploidy observed in cHCECs is induced in culture due to cell division. In this regard, the inventors provide a new finding, i.e., the presence or absence of aneuploidy in cHCECs is closely associated with a specific cell subpopulation that is dominant among cHCECs. The inventors have discovered that a specific cell subpopulation without aneuploidy appears along with a specific pattern of surface phenotype with a functional mature differentiated corneal endothelial cell present in corneal tissue. Refined culture conditions for selectively growing a cell subpopulation consisted mostly of functional mature differentiated corneal endothelial cells without karyotype abnormality was successfully established such that a safe and stable regenerative medicament can be provided by infusing a functional mature differentiated corneal endothelial cell into the anterior chamber in the form of cell suspension for treating a corneal endothelial disorder such as bullous keratopathy. In this manner, the present invention discovered that a karyotype abnormality occurs subpopulation selectively, which was not known up to this point. In addition, use of the technique in the present invention enables selection of a subpopulation that is substantially free of karyotype abnormalities.

The present invention also revealed that a phenomenon where autoantibodies are also substantially absent is subpopulation selectively seen in the corneal endothelial property possessing functional cell of the invention. In the present invention, a specific subpopulation can be selected in order to select a subpopulation that is substantially free of an autoantibody. Autoantibodies can be measured by an approach known in the art. For instance, the following procedure is exemplified. First, HCECs are fixed with methanol and washed twice with PBS. The HCECs are permeabilized with PBS-0.2% Tx-100 (room temperature, 15 minutes) and blocked with 1% BSA/PBS (1 hour or longer at room temperature). Subsequently, 250 .micro.L of serum from a healthy individual diluted 5 or 25 fold with 1% BSA/PBS is added to wells and incubated overnight at 4.degrees.C. The HCECs are then washed 4 times with PBS-0.2% Tx-100, then 1% BSA/PBS comprising Alexa Fluor 488-labeled anti-human IgG (5 .micro.g/mL) and Alexa Fluor 647-labeled anti-human IgM (5 .micro.g/mL) is added (250 .micro.L/well). The HCECs are then incubated for 1 hour at room temperature and washed twice with PBS-0.2% Tx-100 and washed once with PBS. Nuclei are stained with DAPI (5 .micro.g/mL) (at room temperature for 15 minutes), washed with PBS, and examined with an inverted fluorescence microscope (BZ-9000) (see Fig. 10-C).

In another aspect, the present invention provides a cell population comprising corneal endothelial property possessing functional cells of the invention, especially functional mature differentiated corneal endothelial cells.

The cell population of the present invention preferably has a mean cell density at saturated cell culture (confluence) of at least about 1500 cells/mm² or higher, at least about 1600 cells/mm² or higher, at least about 1700 cells/mm² or higher, at least about 1800 cells/mm² or higher, at least about 1900 cells/mm² or higher, or at least about 2000 cells/mm² or higher. Since a cell population comprising the corneal endothelial property possessing functional cells of the invention has a small cell size, it is understood that the cells are provided at a correspondingly high density. The cell density is a characteristic found with high quality corneal endothelial properties, or conversely, measurement of such cell density can be used as one indicator for selecting high quality mature differentiated functional corneal endothelial cells. Since cell density is a numerical value that is directly related to cell area, cell density can be similarly computed by measuring cell area by any approach known in the art. As discussed above, a typical example thereof includes a measurement method using a phase contrast microscope image, wherein the image can be taken with a commercially available system such as an inverted microscope system (CKX41, Olympus, Tokyo, Japan). For measuring area distribution, target cells can be pretreated to facilitate measurement by washing with PBS(-) three times or the like and a phase contrast microscope image can be obtained for example by using a commercially available system such as BZ X-700 Microscope system (Keyence, Osaka, Japan). Further, area distribution can be quantified using commercially available software such as BZ-H3C Hybrid cell count software (Keyence).

In a preferred embodiment, the mean cell density of the cell population of the present invention is at least about 2100 cells/mm² or higher, at least about 2200 cells/mm² or higher, at least about 2300 cells/mm² or higher, at least about 2400 cells/mm² or higher, or at least about 2500 cells/mm² or higher, but the mean cell density is not limited thereto. The upper limit can be any materializable value. Examples of materializable upper limit include about 3000 cells/mm² or higher, about 3100 cells/mm² or higher, about 3200 cells/mm² or higher, about 3300 cells/mm² or higher, about 3400 cells/mm² or higher, about 3500 cells/mm² or higher, about 3600 cells/mm² or higher, about 3700 cells/mm² or higher, about 3800 cells/mm² or higher, about 3900 cells/mm² or higher, about 4000 cells/mm² or higher, and the like. It is understood that any combination of such upper limit and lower limit is used as the preferred range of cell density of the cell population of the present invention.

The characteristic of such a cell density or cell area can be applied to clinical application suitability assessment of a cultured final cell product with the phase contrast quantification technique of cultured cells by hybrid cell counting. The functional mature differentiated corneal endothelial cells of the present invention are revealed as having a small area per cell and the highest cell density in culture. Cultured human corneal endothelial cells made by the manufacturing method of the present invention exhibit the same level of cell area and cell density as endothelial cells of normal corneal endothelial tissue, i.e., cell area of 216 .micro.m² and cell density of 2582 cells/mm², as exemplified in the Examples.

In one embodiment, the cell population of the present invention is characterized by the presence of the corneal endothelial property possessing functional cells of the invention at a ratio that is higher than a naturally-occurring ratio. This is because therapy that is more effective using a naturally available corneal endothelial cell population can be provided by providing a cell population with a ratio of cells capable of eliciting a corneal endothelial functional property which is higher than the naturally-occurring ratio. The ratio of such cells capable of eliciting a corneal endothelial functional property can be increased because a technique that can identify and select out numerous subpopulations of the corneal endothelial property possessing functional cells of the invention (e.g., functional mature differentiated corneal endothelial cells or intermediately differentiated corneal endothelial cells) is provided.

In a preferred embodiment, it is advantageous that at least 5 % or greater, about 10 % or greater, about 15 % or greater, about 20 % or greater, about 25 % or greater, about 30 % or greater, about 35 % or greater, about 40 % or greater, about 45 % or greater, about 50 % or greater, about 55 % or greater, about 60 % or greater, about 65 % or greater, about 70 % or greater, about 75 % or greater, about 80 % or greater, about 85 % or greater, about 90 % or greater, about 95 % or greater, about 98 % or greater, or about 99 % or greater of cells in the subpopulation of the present invention are the corneal endothelial property possessing functional cells of the invention. In this regard, such cells comprised in a cell population can have a cell functional property belonging to either the so-called a5 or a1. For instance, as cells contained in a cell population, cells comprising a cell functional property including CD166 positive and CD 133 negative and, as needed, CD44 negative to intermediately positive are selected out. Although not wishing to be bound by any theory, the reason the cell population of the present invention achieves an effect is because an excellent therapeutic effect or prophylactic effect is exhibited upon infusion of the cell population into a subject by comprising a certain level of the corneal endothelial property possessing functional cells of the invention. In a preferred embodiment, it is advantageous that about 70% or more of cells in the cell population of the present invention are the corneal endothelial property possessing functional cells of the invention. This because at this level, a cell density (e.g., about 2300 cells/mm²) which is considered a benchmark for successful corneal cell infusion therapy can be achieve by the presence of the corneal endothelial property possessing functional cells of the invention. In a more preferred embodiment, it is advantageous that about 90% or more of cells in the cell population of the present invention are the corneal endothelial property possessing functional cells of the invention. This is because the ratio of the presence of the corneal endothelial property possessing functional cells of the invention at this level cannot be accidentally achieved such that it is necessary to establish a technique and information that can precisely and reliably identify and separate a cell population while this was more or less impossible with conventional techniques. The cell density which is considered a benchmark of successful corneal cell infusion therapy can be calculated by measuring the mean cell density of cells integrated into the human corneal endothelial surface after infusion of a cell population. Such a cell density may be at least about 1000 cells/mm² or greater, preferably at least about 1100 cells/mm² or greater, preferably at least about 1200 cells/mm² or greater, preferably at least about 1300 cells/mm² or greater, preferably at least about 1400 cells/mm² or greater, preferably at least about 1500 cells/mm² or greater, preferably at least about 1600 cells/mm² or greater, preferably at least about 1700 cells/mm² or greater, preferably at least about 1800 cells/mm² or greater, preferably at least about 1900 cells/mm² or greater, preferably at least about 2000 cells/mm² or greater, preferably at least about 2200 cells/mm² or greater, preferably at least about 2300 cells/mm² or greater, preferably at least about 2400 cells/mm² or greater, preferably at least about 2500 cells/mm² or greater, preferably at least about 2600 cells/mm² or greater, preferably at least about 2700 cells/mm² or greater, preferably at least about 2800 cells/mm² or greater, preferably at least about 2900 cells/mm² or greater, and preferably at least about 3000 cells/mm² or greater.

In a more preferred embodiment, the cell population of the present invention is characterized by the ratio of functional mature differentiated corneal endothelial cells which is present at a higher ratio than the naturally-occurring ratio. Functional mature differentiated corneal endothelial cells express a human corneal endothelial functional property upon direct infusion into the anterior chamber of the eye. This is because therapy that is more effective that using a naturally available corneal endothelial cell population can be provided by providing a cell population with a ratio of high quality cells that is higher than the naturally-occurring ratio. The ratio of such cells given high quality functionality can be increased, because a technique that can identify and select out numerous subpopulations of functional mature differentiated corneal endothelial cells is provided.

In a preferred embodiment, it is advantageous that at least 5 % or more, about 10 % or more, about 15 % or more, about 20 % or more, about 25 % or more, about 30 % or more, about 35 % or more, about 40 % or more, about 45 % or more, about 50 % or more, about 55 % or more, about 60 % or more, about 65 % or more, about 70 % or more, about 75 % or more, about 80 % or more, about 85 % or more, about 90 % or more, about 95 % or more, about 98 % or more, or about 99 % or more of cells in the cell population of the present invention are functional mature differentiated corneal endothelial cells. The ratio of such functional mature differentiated corneal endothelial cells may be denoted herein as the "E-ratio". The calculation method of E-ratio is described elsewhere herein, In this regard, such cells comprised in a cell population can have a functional property belonging to the so-called a5. For instance, as cells comprised in a cell population, cells with CD166 positive, CD133 negative, and CD44 negative to weakly positive (preferably CD44 negative) are selected out. Alternatively, cells with CD166 positive, CD133 negative, and CD200 negative can be selected out. Although not wishing to be bound by any theory, the reason the cell population of the present invention with enhanced quality achieves an effect is because an excellent therapeutic effect or prophylactic effect is further exhibited upon infusion of the cell population into a subject by comprising a certain level of functional mature differentiated corneal endothelial cells. In a preferred embodiment, it is advantageous that about 40% or more of cells in the cell population of the present invention are mature differentiated functional corneal endothelial cells. This is because a high quality cell density (e.g., about 1000 cells/mm² or higher, preferably about 2000 cells/mm², and generally about 2300 cells/mm² for cells integrated into the corneal endothelial surface) which is considered a benchmark for a successful corneal cell infusion therapy can be more reliably achieved by the presence of the functional cells at this level. In a more preferred embodiment, it is advantageous that at least about 70% or more, more preferably at least 80% or more, still more preferably at least about 90% or more of cells in the cell population of the present invention are functional mature differentiated corneal endothelial cells. This is because the ratio of the presence of functional cells at this level cannot be accidentally achieved such that it is necessary to establish a technique and information that can precisely and reliably identify and separate a cell population; this was more or less impossible with conventional techniques. The ratio of functional mature differentiated corneal endothelial cells can be further enhanced by using the technique of the invention. For instance, it is possible to provide a cell population in which at least about 95% or more, at least about 96% or more, at least bout 97% or more, at least about 98% or more or at least about 99% or more of cells are functional mature differentiated corneal endothelial cells. In addition, it is demonstrated that a therapeutic result which exceeds about 2300 cells/mm² (e.g., about 3000 cells/mm²) can be achieved in not even one month after infusion by providing such a cell population comprising functional mature differentiated corneal endothelial cells. Thus, a fast and high quality therapeutic technique that was conventionally available is provided.

In one embodiment, the corneal endothelial property possessing functional cells (including functional mature differentiated corneal endothelial cells) or cell population of the present invention is characterized by lower expression of cell degeneration associated antigens or HLA class I antigens associated with immunological rejection relative to other subpopulations. The corneal endothelial property possessing functional cell of the invention, especially functional mature differentiated corneal endothelial cell, does not have autoantibodies seen in other subpopulations. Thus, said cell is recognized as an immunologically stable cell.

In one embodiment, the cell metabolites and related biological materials of the metabolite used in the present invention include, but are not limited to, succinic acid (succinate), Pro, Gly, glycerol 3-phosphate, Glu, lactic acid (lactate), argininosuccinic acid (argininosuccinate), xanthine, N-carbamoyl aspartic acid (aspartate), isocitric acid (isocitrate), cis-aconitic acid (cis-aconitate), citric acid (citrate), Ala, 3-phosphoglyceric acid (3-phosphoglycerate), hydroxyproline, malic acid (malate), uric acid (urate), betaine, folic acid (folate), Gln, 2-oxoisovaleric acid (2-oxoisovalerate), pyruvic acid (pyruvate), Ser, hypoxanthine, Asn, Trp, Lys, choline, Tyr, urea, Phe, Met, carnosine, Asp, ornithine, Arg, creatine, 2-hydroxy glutaminic acid (2-hydroxy glutamate), beta-Ala, citrulline, Thr, Ile, Leu, Val, creatinine, His, N,N-dimethyl glycine, or a combination or relative ratio thereof. Alternatively, the cell metabolites of the present invention include the following.

Group of substances that are reduced by culture (group of substances taken in by a cell): Arg, creatine, total amino acids, Tyr, carnosine, Asp, total essential amino acids, total ketogenic amino acids, Trp, Val, total oxaloacetate related amino acids, total glutamate related amino acids, total acetyl CoA related amino acids, total succinyl CoA related amino acids, citrulline, total BCAA, Fischer ratio, hypoxanthine, Leu, Asn, Ile, 2-hydroxyglutaric acid, pyruvic acid, Ser, citrulline/ ornithine ratio, uric acid, amd beta-alanine

Group of substances that are increased by culture (group of substances excreted by a cell): sarcosine, sedoheptulose 7-phosphate, spermidine, spermine, total adenylic acid (adenylate), total glutathione, total guanylic acid (guanylate), UDP-glucose, XMP, xylulose 5-phosphate, cis-aconitic acid (cis-aconitate), citric acid (citrate), betaine, glucose 6-phosphate, lactic acid (lactate) / pyruvic acid (pyruvate), glycerol 3-phosphate, Ala, lactic acid (lactate), 2-oxoisovaleric acid (2-oxoisovalerate), arginosuccinic acid (arginosuccinate), Glu, hydroxyproline, xanthine, ornithine, total pyruvic acid (pyruvate) related amino acid, Pro, Gly, N,N-dimethyl glycine, choline, urea, folic acid (folate), His, creatinine, Met, Lys, Thr, succinic acid (succinate), gamma-aminobutyric acid (gamma-aminobutyrate), Phe, total non-essential amino acids, total fumaric acid (fumarate) related amino acids, total aromatic amino acids, and total glycogenogenic amino acids.

In particular, the present invention can control quality of the corneal endothelial property possessing functional cell or functional mature differentiated corneal endothelial cell of the present invention by using an elevation of serine, alanine, proline, glutamine, or citric acid (citrate)/lactic acid (lactate) ratio, especially citric acid (citrate)/lactic acid (lactate) ratio in culture supernatant.

A cell property can be noninvasively identified by being able to use a metabolite. It was found, by the metabolome analysis which was performed as a part of the present invention, that an energy metabolizing property is dramatically different from each subpopulation of "cultured human corneal endothelial cells" as the so-called "heterogeneous population" that has been conventionally used. It was revealed that glycolytic energy metabolism system progresses in cell culture supernatant in a cell (transformed cell) subpopulation which has undergone state transition and mitochondria system energy metabolism system progresses in effector cell subpopulation (functional mature differentiated corneal endothelial cells) without karyotype abnormality. Mitochondria are responsible for regulating cell energy production in most somatic cells, and all cell types in a specific state may have different metabolic properties. While a proliferative cell, such as a stem cell tend to prefer glycolysis, mature differentiated cells such as the functional mature differentiated corneal endothelial cells of the present invention are considered to be under more oxidative phosphorylation (OXPHOS) regulation. In view of such knowledge, the quality of cells of the present invention can be heightened by referring to information related to a metabolic profile with increased dependency of OXPHOS activity during differentiation or shift to glycolytic metabolism during cell proliferation, such that this contributes to the optimization of culture and manufacturing conditions of the cell of the present invention. "Cultured human corneal endothelial cells" as a "heterogenous population" have a tendency toward senescent phenotype, endothelial mesenchymal transition (EMT), and cell state transition (CST) to transformed fibroblast-like cell morphology. The inventors have discovered a method of using culture supernatant to distinguish subpopulations in cHCECs in terms of the secreted metabolites thereof. CST subpopulations exhibit tendency toward anaerobic glycolysis instead of mitochondria dependent OXPHOS. Products for safe and stable regenerative medicine using a metabolically defined functional mature differentiated corneal endothelial cell can be provided in a form of cell suspension.

In the present invention, for example an extracellular flux analyzer can be purchased and metabolites, oxygen consumption in culture in addition to proteinaceous products and secreted miRNAs can be continuously tracked to provide a process control method during production of products. It is shown that an energy metabolism system in a mitochondria system is most progressed in the functional mature differentiated corneal endothelial cell of the present invention. The present invention can utilize lactic acid (lactate), pyruvic acid (pyruvate), lactic acid (lactate)/pyruvic acid (pyruvate), citric acid (citrate)/lactic acid (lactate), Ser, Pro/Ser, Leu, Ile(branched chain amino acids) and Gln in culture and practice and investigate the usefulness as an assessment method that should be applied in trials and manufacture.

In other embodiments, the functional mature differentiated corneal endothelial cells or cell populations of the present invention have excellent specific genetic form relative to other subpopulations. Examples of "genetic property" include genes corresponding to any cell functional property exhibited by the corneal endothelial property possessing functional cell of the invention (e.g., functional mature differentiated corneal endothelial cell or intermediately differentiated corneal endothelial cell) in any gene product described in the above-described section of proteinaceous products.

In another embodiment, the functional mature differentiated corneal endothelial cells or cell population of the present invention are characterized by substantially not eliciting unintended biological response after administration into a living body, including cytokine profile of the serum. For a conventional low quality cell, it is demonstrated that an inflammatory cytokine is not yet elicited 2 days after infusion, leading to a result as shown in Figs. 61-A to 61-B.

On the other hand, it is understood that the corneal endothelial property possessing functional cells of the invention, functional mature differentiated corneal endothelial cells, or cell population do not elicit an abnormality or inflammatory cytokine after two days, after one week, or thereafter as shown in Figs. 62 and 63.

Examples of "unintended" cytokine profiles used herein include, but are not limited to, RANTES, PDGF-BB, IP-10, MIP-1b, VEGF, EOTAXIN, IL-1ra, IL-6, IL-7, IL-8, IL-0, IL-10, IL-12 (p70), IL-13, IL-17, FGFbasic, G-CSF, GM-CSI, IFN-gamma, MCP-1, MIP-1a, TNF-alpha, and the like. "Unintended cytokine profile" is a profile indicating that a cell is not the corneal endothelial property possessing functional cell of the invention or "unintended" when production thereof is detected above normal.

As used herein, "unintended biological response" refers to eliciting at least one of the above-described "unintended" cytokine profiles at a level beyond the generally elicited level.

In one aspect, the present invention provides a cell bank comprising the corneal endothelial property possessing functional cells or cell population of the present invention. A cell bank refers to an organization or system for holding "cells" (generally cultured cells) that are created or collected through research or the like and providing the cells to other researchers or businesses.

In another aspect, the present invention provides a product comprising the corneal endothelial property possessing functional cells of the invention or cell population. Such a product may be in any form such as cellular processed products and the like prepared for administration to humans and the like, but the product is not limited thereto. It is desirable that such a cell product preferably has not undergone unintended transformation, has no or little effect from physiologically active substances produced by cell/tissue, has no or little effect on a normal cell or tissue, has no or little possibility of forming a heterotopic tissue, have no or little possibility of inducing an undesired immune reaction, has no or little possibility of tumorigenesis or oncogenesis, has been subjected to safety assessment as defined in gene therapy product guidelines in case gene transfer has been performed, and has cleared general toxicity test or the like.

In another aspect, the present invention provides a method of preserving the corneal endothelial property possessing functional cells of the invention, functional mature differentiated corneal endothelial cells, or cell population comprising passaging the cells or the cell population by exchanging a medium. In this regard, the present invention discovered that a cell functional property is maintained and preserved by such exchanging of a medium. Any medium can be used for the medium used herein, while it is advantageous to use, preferably, an ingredient or medium used in the cell manufacturing method explained herein.

In another embodiment, the present invention provides a method of delivering the corneal endothelial property possessing functional cells of the invention, functional mature differentiated corneal endothelial cells, or cell population, comprising implementing the method of preserving the corneal endothelial property possessing functional cells of the invention, functional mature differentiated corneal endothelial cells, or cell population.

Sorting is a typical procedure for selecting out the corneal endothelial property possessing functional cell of the invention or functional mature differentiated corneal endothelial cell. Examples of other methods that can be used include methods involving selective apoptosis induction (miRNA switching) or necrosis induction (glucose starvation or the like) to a non-intended cell by utilizing the difference in cell properties of a cell of interest and non-intended cell. However, the present invention generally enhances the purity of the corneal endothelial property possessing functional cells of the invention or functional mature differentiated corneal endothelial cells.

### (Method of manufacturing human functional corneal endothelial cell capable of eliciting a human corneal functional property when infused into an anterior chamber of a human eye)

In one aspect, the present invention provides a method of manufacturing a human human functional corneal endothelial cell capable of eliciting a human corneal functional property when infused into an anterior chamber of a human eye (the corneal endothelial property possessing functional cell of the invention) or functional mature differentiated corneal endothelial cell, comprising a step of maturing and differentiating a corneal endothelial tissue-derived cell or a corneal endothelial progenitor cell. This method may be performed after a dedifferentiation step in addition to the maturing and differentiating step. The corneal endothelial property possessing functional cell of the invention, as described elsewhere herein, encompasses "functional mature differentiated corneal endothelial cell" having a corneal endothelial functional property as such without further processes and "functional mature differentiated corneal endothelial cell", which lacks some of the functions, but is used similarly or exerts the same function as a functional mature differentiated corneal endothelial cell after cell infusion.

In another aspect, the present invention provides a method of manufacturing a human functional corneal endothelial cell capable of eliciting a human corneal functional property when infused into an anterior chamber of a human eye (corneal endothelial property possessing functional cell of the invention) or functional mature differentiated corneal endothelial cell, comprising a step of culturing a corneal endothelial tissue-derived cell or a corneal endothelial progenitor cell under actin depolymerization conditions. Maturation and differentiation are accomplished by culturing with a step comprising actin depolymerization, resulting in the cell being able to express a corneal functional property when infused into an anterior chamber of a human eye.

As used herein, "maturation and differentiation" may be strictly different from the meaning that is commonly used in the art. That is, "maturation and differentiation" as used herein refers to becoming a cell confirmed to be differentiated to exert a corneal endothelial function, which forms a small hexagonal cobble-stone shape that is most suitable for cell infusion and uses an energy metabolism system by mitochondrial function in the context of corneal endothelium.

The present invention was completed by discovering that a corneal endothelial cell or cell differentiated as such or progenitor cell thereof, when exposed to actin depolymerization inducing conditions, matures and differentiates in the strict meaning described above.

In one embodiment, the actin depolymerization or actin depolymerization accomplishing conditions used in the present invention is accomplished by at least one agent selected from the group consisting of a ROCK inhibitor, HDAC inhibitor, actin depolymerization inhibitor, PPARgamma inhibitor, MMP2 inhibitor, p53 activator, and miRNA.

In a specific embodiment, examples of the actin depolymerization inhibitor used in the present invention include, but are not limited to, latrunculin A, swinholide A, and the like. In this regard, latrunculin A can be used, for example, at about 0.4 .micro.M, and swinholide A can be used at about 0.1 .micro.M.

Examples of HDAC inhibitor include Trichostatin A (TSA), vorinostat and the like. Trichostatin A can be used at about 0.5 .micro.M. Examples of PPARgamma inhibitor includes rosiglitazone, pioglitazone, and the like. Examples of MMP2 inhibitor includes resveratrol and the like. Examples of p53 activator include those used in cancer research. Examples of miRNA include, but are not limited to, those associated with activation in a human functional corneal endothelial cell capable of eliciting a corneal functional property when infused into an anterior chamber of a human eye herein, such as miRNA 34, 1246, 1273, and 4732.

In another specific embodiment, an agent used in actin depolymerization or condition for achieving actin depolymerization is present in a medium in the aforementioned step at a concentration effective at accomplishing actin depolymerization. Examples of such a concentration include, but are not limited to, about 1-30 .micro.M for the ROCK inhibitor Y-27632 such as about 10 .micro.M, and about 100-2000 nM such as 500 nM for HDAC inhibitor Trichostatin A. Those skilled in the art can appropriately change the concentration by measuring the cell function of a resulting cell (e.g., by using a surrogate marker) or examining a cell indicator.

In one further aspect, the method of manufacturing the corneal endothelial property possessing functional cell of the invention or functional mature differentiated corneal endothelial cell further comprises a step of culturing the corneal endothelial tissue-derived cell or corneal endothelial progenitor cell under steps where a cell enters into epithelial-mesenchymal transition-like transformation, proliferation, maturation and differentiation.

Such a condition where a cell that has undergone epithelial-mesenchymal transition-like transformation grows, matures, and differentiates may be accomplished, for example, by culturing in the absence of a transforming growth factor beta (TGF-beta) signaling inhibitor (e.g., 4-[4-(1,3-benzodioxole-5-yl)-5-(2-pyridinyl)-1H-imidazole-2-yl]-benzamide] (SB431542)). It is known that a cultured corneal endothelial tissue derived cell tends toward cell state transition (CST) to senescent phenotype, EMT, and fibroblastic morphology, and it was clearly shown that blocking TGF-beta signals induce a morphological change in a cultured corneal endothelial tissue derived cell. It is elucidated that CD44, CD24, IL-8, and IGFBP3 are upregulated and collagen 4A1, 4A2, and 8A2 are downregulated. It was revealed that culturing in the absence of a transforming growth factor beta (TGF-beta) signaling inhibitor results in a cell that has undergone epithelial mesenchymal transition-like transformation growing, maturing, and differentiating to enhance the quality of a functional mature differentiated corneal endothelial cell. It is understood that the present invention may or may not comprise a step of culturing under a condition where a cell that has undergone epithelial-mesenchymal transition-like transformation grows, matures, and differentiates (e.g., culturing in the absence of a transforming growth factor beta (TGF-beta) signaling inhibitor).

In still another aspect, the method of manufacturing the corneal endothelial property possessing functional cell of the invention or functional mature differentiated corneal endothelial cell further comprises a step of culturing the corneal endothelial tissue-derived cell or corneal endothelial progenitor cell under a condition where cellular senescence is suppressed.

Such a condition where cellular senescence is suppressed can be accomplished by culturing in the presence of a p38 MAP kinase inhibitor. Examples of p38 MAP kinase inhibitors include, but are not limited to, 4-(4-fluorophenyl)-2-(4-hydroxyphenyl)-5-(4-pyridyl)-1H-imidazole (SB-202190), trans-4-[4-(4-fluorophenyl)-5-(2-methoxy-4-pyrimidinyl)-1H-imi dazole-1-yl]cyclohexanol (SB-239063), 4-(4-fluorophenyl)-2-(4-methylsulfinyl phenyl)-5-(4-pyridyl)-1H-imidazole (SB-203580), and 4-(4-fluorophenyl)-5-(2-methoxypyrimidine-4-yl)-1-(piperidine-4-yl)imidazole (SB-242235), and the like.

In a preferred embodiment, the method of manufacturing the corneal endothelial property possessing functional cell of the invention or functional mature differentiated corneal endothelial cell cultures under a condition of exposing a cell to actin depolymerization, or maturing and differentiating, under a condition where a cell that has undergone epithelial-mesenchymal transition-like transformation grows, matures, and differentiates, and a condition where cell senescence is suppressed or in the presence of a p38 MAP kinase inhibitor. Such a combination correctly directs maturation and differentiation to suppress or reverse epithelial mesenchymal transition-like state transition to suppress senescence. Thus, a high quality functional mature differentiated corneal endothelial cell can be advantageously manufactured.

When a corneal endothelial cell is manufactured by a conventionally known manufacturing method, SPs defined by a cell surface CD marker are frequently expressed at notably different proportions. In addition, it was revealed that the E-ratio defined by the proportion of subpopulation with CD44 negative CD166 positive CD24 negative CD26 negative CD105 negative is 0.2-19% or 0.1-31.2%. It was also confirmed that CD44 expression decreases as a cultured corneal endothelial cell differentiates into a functional mature differentiated corneal endothelial cell. In the presence of a SP expressing either CD24 or CD26 in cells, there is a possibility of the presence of some type of a SP with notable karyotype abnormality such as sex chromosome loss, trisomy, or translocation, such that the presence thereof is not suitable for cell infusion therapy. At the maximum, the proportion of CD24 positive cells reached 54.3%-96.8%, and the proportion of CD26 positive cells reached 44.2%; CD44 strongly positive expressing cells were observed to be present at the highest proportion exceeding 80%. The outward phenotype was non-fibroblastic, and when observed with a phase contrast microscope, the cells had a characteristic polygonal shape and monolayer structure due to contact obstruction. Surprisingly, both ZO-1 and Na⁺/K⁺ ATPase, which are well known HCEC markers, were stained for CD24 positive, CD26 positive or CD44 strongly positive subpopulations. In view of the above, the present invention discovered that heterogeneous SPs present in cHCECs may not be sufficiently distinguished only by morphological judgment. The method of the present invention achieves an effect of decreasing the proportion of such unsuitable cells and increasing proportion of the corneal endothelial property possessing functional cells of the invention and human functional mature differentiated corneal endothelial cells that are suitable for infusion. A human bone marrow derive mesenchymal stem cell medium not only stimulates HCEC growth by controlling G1 proteins in a cell cycle, but also maintains a differentiation phenotype required for the endothelial function of cHCECs. Thus, use thereof was recommended for maintaining cHCECs (Nakahara M, et al., PLoS One. 2013; 8:e69009). However, the present invention revealed that a medium comprising a bone marrow derived mesenchymal stem cell medium and a medium that is free thereof do not have a difference in increasing the proportion of mature differentiated cell functional cells.

Although not wishing to be bound by any theory, the reason a corneal endothelial cell can mature and differentiate by culturing under actin depolymerizing conditions in the present invention is the following.

That is, under conditions for maturing and differentiating a specific corneal endothelial cell of the present invention, HDAC inhibition, increase in miRNA34 expression, and/or suppression of CD44 expression in a certain pathway suppresses actin polymerization, also suppresses RhoA, and suppresses tubulin and actin polymerization in another pathway, resulting in action of generating a pseudopod on a cell. In yet another pathway, CD44 activates RhoA through MMP2. Thus, use of an MMP2 inhibitor is also expected to achieve the same effect. Hence, the corneal endothelial property possessing functional cell of the invention or functional mature differentiated corneal endothelial cell can be similarly manufactured by suppressing MMP2.

As used herein, a "corneal endothelial tissue derived cell or corneal endothelial progenitor cell", as defined elsewhere herein, refers to a cell that becomes the corneal endothelial property possessing functional cell of the invention or functional mature differentiated corneal endothelial cell from a cell derived from corneal endothelial tissue and differentiation via a dedifferentiating step, respectively. Such a cell encompasses any cell such as cells differentiated into corneal endothelial cell-like cells, from iPS cells, ES cells, or the like and progenitor cells before differentiation into corneal endothelial cells, in addition to cells obtained from a donor corneal endothelium, as well as intermediately differentiated corneal endothelial cells defined herein.

In the manufacturing method in the present invention, a corneal endothelial tissue-derived cell that can be used as a starting material is collected from a living body. Alternatively, starting materials that can be used may be corneal endothelial progenitor cells, such as cells differentiated from stem cells or progenitor cells. Examples of such differentiated cells may include, but are not limited to, cells differentiated from various stem cells (e.g., induced pluripotent stem cells (iPS cells), embryonic stem cells (ES cells), fertilized eggs, and somatic stem cells). Thus in a specific embodiment, examples of the corneal endothelial tissue-derived cells or corneal endothelial progenitor cells used (as a starting material) in the present invention include, but are not limited to, pluripotent stem cells, mesenchymal stem cells, corneal endothelial progenitor cells collected from a corneal endothelium, corneal endothelial cells collected from a corneal endothelium, corneal endothelial progenitor cells and corneal endothelium-like cells produced by direct programming method and the like. In this regard, examples of pluripotent stem cells include, but are not limited to, induced pluripotent stem cells (iPS cells), embryonic stem cells (ES cells) and the like. Thus, it is understood that the corneal endothelial cells or progenitor cells thereof used (as a starting material) in the present invention include cells prepared by differentiating induced pluripotent stem cells (iPS cells), embryonic stem cells (ES cells) or the like into corneal endothelium-like cells. Techniques of differentiating induced pluripotent stem cells (iPS cells), embryonic stem cells (ES cells) or the like into corneal endothelium-like cells are known in the art, such as the AMED method (Ueno et al supra), WO 2013/051722 (KEIO UNIVERSITY, and the like, but the techniques are not limited thereto.

When a cell that is not differentiated into a corneal endothelium-like cell is used, it is preferable to comprise a step of differentiating or maturing and differentiating into a corneal endothelium-like cell.

Methods of differentiating a stem cell such as a pluripotent stem cell r mesenchymal stem cell into a corneal endothelium cell-like cell are known in the art, such as the AMED method (Ueno et al. supra), WO 2013/051722 (KEIO UNIVERSITY) and the like, but the methods are not limited thereto.

Cell seeding densities can be associated with the quality of a manufacturing method. For instance, higher cell seeding density leads to less decrease in the ratio of the corneal endothelial property possessing functional cells of the invention or functional mature differentiated corneal endothelial cells in the next passage. A group with a ratio of functional mature differentiated corneal endothelial cells higher than 90% exhibits a very rapid increase (i.e., growth rate is high) in the number of cells at a cell seeding density of 200 cells/mm². Thus, it is understood that a cell seeding density of 200 cells/mm² or greater is advantageous. In a preferred embodiment, culture in the manufacturing method of the present invention is advantageously performed at a culture density of about 200-about 1000 cell/mm². More preferably, about 400 to about 800 cells/mm² or the like is more advantageous. Examples of the lower limit of culture density includes, but are not limited to, about 100 cells/mm², about 150 cells/mm², about 200 cells/mm², about 250 cells/mm², about 300 cells/mm², about 350 cells/mm², about 400 cells/mm², and the like. Examples of the upper limit thereof include, but are not limited to, about 800 cells/mm², about 850 cells/mm², about 900 cells/mm², about 950 cells/mm², about 1000 cells/mm², about 1100 cells/mm², about 1200 cells/mm², about 1300 cells/mm², about 1400 cells/mm², about 1500 cells/mm², about 1600 cells/mm², about 1700 cells/mm², about 1800 cells/mm², about 1900 cells/mm², about 2000 cells/mm², and the like. It is understood that a combination of any of the upper limits and lower limits can be used.

In one embodiment, the manufacturing method in the present invention further comprises a step of testing a cell after the culture using at least one marker for identifying the corneal endothelial property possessing functional cell of the invention or functional mature differentiated corneal endothelial cell. Markers for identifying the corneal endothelial property possessing functional cell of the invention or functional mature differentiated corneal endothelial cells are explained elsewhere herein. It is understood that any of such markers or a combination thereof can be used. This is because the quality can be maintained on or above a certain level by assaying a manufactured cell.

In another embodiment, the manufacturing method of the present invention may further comprise a step of selectively propagating in cultures a fraction determined to be the corneal endothelial property possessing functional cell of the invention or functional mature differentiated corneal endothelial cell after the testing. This is because a cell tested to be of good quality and a cell that is not can be separated to select, preferably isolate a cell of good quality to further enhance the quality of provided cell. An approach known in the art can be used for such cell sorting, such as fluorescence-activated cell sorting (FACS) as well as non-intended cell selective apoptosis induction (miRNA switching or the like), necrosis induction (glucose starvation or the like) utilizing the difference in cell properties of a cell of interest and non-intended cell, and the like.

In yet another embodiment, the manufacturing method used in the present invention further comprises a step of monitoring cell subpopulation composition during the culturing. Such monitoring can be performed by using at least one marker for identifying the corneal endothelial property possessing functional cell of the invention or functional mature differentiated corneal endothelial cell or by measuring mitochondrial function, oxygen consumption and pH of culture or the like. The quality of the manufacturing method itself can be controlled by such monitoring in the manufacturing process. The monitoring can be materialized by, for example, tracking at least one item selected from the group consisting of mitochondrial function, oxygen consumption and pH of a culture solution, amino acid composition, proteinaceous product, soluble miRNA, cell density with a noninvasive engineering approach, cell size, and cell homogeneity thereof.

When deterioration is observed in the performed manufacturing method as a result of the above-described quality control, the manufacturing method being performed may be altered as needed. Examples of such alteration may include, but are not limited to, enhancement of maturation and differentiation or actin depolymerization conditions (e.g., enhancement of ROCK inhibition), enhancement of growth, maturation and differentiation of a cell with epithelial mesenchymal transition-like transformation, such as further enhancement in inhibition of transforming growth factor beta (TGF-beta) signaling, enhancement in suppression of cell senescence, such as enhancement of p38 MAP kinase inhibition, a combination thereof and the like.

In one embodiment, the manufacturing method in the present invention comprises a step of subculturing. Subculturing can exponentially grow the corneal endothelial property possessing functional cell of the invention or functional mature differentiated corneal endothelial cell. In this regard, the number of cells expands about 3-fold by a single subculture. The factor of expansion can be increased or decreased by appropriately altering culture conditions based on conditions known in the art. Upon passage, it is preferable that subculturing is performed at the aforementioned advantageous cell seeding density. Further, it is preferable that the ratio of the corneal endothelial property possessing functional cells of the invention or functional mature differentiated corneal endothelial cells is increased as much as possible upon passage, such as 90% or higher.

In a specific embodiment, the manufacturing method in the present invention comprises a step of adding a ROCK inhibitor or other actin depolymerization agents, such as HDAC inhibitor, actin depolymerization inhibitor, PPARgamma inhibitor and MMP2 inhibitor, p53 activator, and miRNA at the time of subculture. A ROCK inhibitor or other actin depolymerization agent can be added at a time other than during subculture to maintain the concentration at or above a certain level. At such a concentration, very little improvement is observed with about 1 .micro.M and little is observed at about 5 .micro.M (=E-ratio, increase in the proportion of intermediately differentiated corneal endothelial cells) for Y-27362. More of an effect is exhibited at about 10 .micro.M or more. For actin polymerization inhibitor, latrunculin A is about 1-about 50 nM and is shown to be usable even at about 200 .micro.M, and swinholide A is about 1 nM or less and is even shown to be usable at about 3 nM. The details thereof are explained elsewhere herein including the Examples and the like.

The method of manufacturing the corneal endothelial property possessing functional cellof the invention or functional mature differentiated corneal endothelial cell can be performed in the presence or absence of a cell conditioning medium. In conventional methods, mesenchymal stem cell conditioning media (for example, see Nakahara, M. et al. PLOS One (2013) 8, e69009) or the like were often used, but it was revealed that a cell conditioning medium may or may not be present when manufacturing a functional cell under the conditions of the present invention. Thus, the present invention encompasses an embodiment of culturing in the absence of a cell conditioning medium in the method of manufacturing the corneal endothelial property possessing functional cell of the invention or functional mature differentiated corneal endothelial cell.

In another aspect, the present invention provides a method of preserving functional mature differentiated corneal endothelial cell comprising a step of continuously culturing the corneal endothelial property possessing functional cell of the invention, functional mature differentiated corneal endothelial cell, or cell obtained by the manufacturing method of the present invention for cell function maturation after a cell density of cultured cells reaches a saturation density, after manufacture. Preferably, after cell function maturation, culturing is performed, for example, for 1 week or longer with only medium exchange for preserving cultured cells. Such medium exchange is advantageously performed after the cells reach the saturated cell density and maturation of cell function thereafter in the aforementioned step of further culturing the cells. It was discovered that the functionality and quality of the cells manufactured in the present invention, when manufactured, can be maintained/preserved only by changing media without passage unlike normal culture conditions. Such preservation is typically accomplished by at least about one week to six months of exchanging media, such as by continuing culture for about 2-4 weeks. Although the upper limit is not particularly limited, the inventors have discovered that the state of a functional mature differentiated corneal endothelial cell can be continuously preserved from continued culture for at least 6 months or longer, such as more than about 200 to about 1 year.

In one embodiment, the manufacturing method in the present invention can further comprise a step of testing a cell function after the culturing by using at least one cell indicator or surrogate marker for identifying the human functional corneal endothelial cell.

In one exemplary embodiment, a manufacturing method can be carried out as follows. That is, a single layer corneal endothelial layer is stripped with the Descemet's membranes from a cornea, which was obtained from a US eye bank SightLife Inc. (advantageously in compliance with the safety standard of official institutions such as the FDA and Ministry of Health, Labour and Welfare). Corneal endothelial cells are separated overnight by treatment with collagenase. The cells are cultured in a culture medium (e.g., Nancy medium (Cheng Zhu and Nancy C. Joyce, Invest Ophthalmol Vis Sci 2004 1743; Gary S. L. Peh et al., PLoS ONE 2012 e28310 (Table 1); US6541256) comprising a suitable supplemented component (e.g., 8% FBS) while using a suitable medium (e.g., Opti-MEM) as the basal medium. Primary culture (P0 culture) is started by seeding endothelial cells from left and right eyes of the same donor in a collagen I plate with a suitable scale (e.g., 2 well/6 well). A suitable ingredient for maturation and differentiation for inducting differentiation into a mature differentiated cell (10 .micro.M of Y-27632 (ROCK inhibitor)) is added to the culture to induce differentiation into mature differentiated cells. An ingredient suitable for senescence prevention or state transition suppression of cells (e.g., 10 .micro.M of SB203580) is added during the culture period. After the cells reaches confluence from a suitable period of culturing (e.g., after about 4-8 weeks), the cells are passaged. After reaching confluence, the same quality is retained over several weeks only by changing the medium. In passaging, the cells are detached from the plate with an appropriate detaching reagent (e.g., TrypLE) and washed with the medium, and then seeded to a T25 collagen I flask at a suitable level of cell density (e.g., 400 cells/mm² or greater). Up to P2 (passage twice, same hereinafter) or P3 culture cells can be used to obtain good results. It is understood that cultured cells up to P5-P6 can also be used to obtain cells with sufficient therapeutic effect.

When cultured by the method of the present invention, the purity and function of the high quality functional mature differentiated corneal endothelial cells of the present invention (i.e., cells of interest considered to exhibit efficacy in infusion) do not change, as long as corneal endothelial cells are continuously cultured by only exchanging media without further passage for 2-8 weeks with an incubator kept at 37.degrees.C after the corneal endothelial cell culture has reached confluence. The proportion of non-intended cells also does not vary. Thus, the manufacturing method of the present invention is also recognized as an excellent manufacturing method in practical terms. When a cell for clinical use is manufactured, it is preferable that a medium free of metavanadate (MVA), which is generally contained in Opti-MEM, is used and a serum lot or additive added to the medium are subjected to a thorough lot test in advance with respect to quality thereof.

### (Pharmaceutical application of human functional corneal endothelial cell capable of elicitingeliciting a human corneal endothelial functional property when infused into an anterior chamber of a human eye)

In another aspect, the present invention provides a medicament comprising a functional corneal endothelial cell capable of eliciting a human corneal endothelial functional property when infused into an anterior chamber of a human eye (the corneal endothelial property possessing functional cell of the invention) or functional mature differentiated corneal endothelial cell. The corneal endothelial property possessing functional cell of the invention, as described elsewhere herein, encompasses functional mature differentiated corneal endothelial cells having a corneal endothelial functional property as such without further process and intermediately differentiated corneal endothelial cell, which lacks some of the functions, but is used similarly or exerts the same function as a functional mature differentiated corneal endothelial cell after cell infusion.

As the corneal endothelial property possessing functional cell of the invention or functional mature differentiated corneal endothelial cell used in the present invention, any of the corneal endothelial property possessing functional cell of the invention, functional mature differentiated corneal endothelial cell, or a cell population comprising a cell subpopulation of the corneal endothelial property possessing functional cell of the invention, functional mature differentiated corneal endothelial cell, which are provided in the present invention can be used.

As used herein, "cell subpopulation" refers to a population of homogeneous cells within a certain range, which were selectively propagated in cultures with a cell surface marker or the like and are then deemed to have a property within the range. "Cell population" refers to any population of cells, which may consist of one "cell subpopulation" or comprise multiple "cell subpopulations", or a population comprising any cell unrelated to a particular cell subpopulation.

It is understood that any embodiment explained in the section of (Human functional corneal endothelial cell capable of eliciting a human corneal endothelial functional property when infused into an anterior chamber of a human eye) in the present invention or a combination thereof can be used for the corneal endothelial property possessing functional cell, functional mature differentiated corneal endothelial cell of the present invention, or cell population used in the medicament of the present invention.

In a specific embodiment, the medicament of the present invention comprises a cell population with the corneal endothelial property possessing functional cells of the invention or functional mature differentiated corneal endothelial cells which are present at a higher ratio. As such a cell population, any embodiment described in the section of (Human functional corneal endothelial cells capable of eliciting a human corneal functional property when infused into an anterior chamber of a human eye) can be used.

In a more preferred embodiment, the medicament of the present invention comprises a cell population in which at least about 75%, at least about 80%, at least about 85%, at least about 90%, or at least about 95% of cells are the corneal endothelial property possessing functional cells of the invention. In this embodiment, cells comprising a cell functional property comprising CD166 positive and CD133 negative and having, as needed, CD44 negative to CD44 intermediately positive, preferably CD44 negative to CD44 weakly negative, are concentrated. It is confirmed that a cell density (e.g., about 1000 cells/mm² or greater, preferably about 2000 cells/mm², and generally about 2300 cells/mm² in cells integrated into the corneal endothelial surface), which is considered a benchmark for successfully corneal cell infusion therapy, can be more reliably accomplished by the presence of highly pure functional mature differentiated corneal endothelial cells.

In another embodiment, the medicament of the present invention comprises a cell population with a ratio of functional mature differentiated corneal endothelial cells that is present at a ratio higher than a naturally-occurring ratio. As such a cell population, any example described in the section of (Corneal endothelial functional capable of eliciting a human corneal functional property when infused into an anterior chamber of a human eye) can be used.

In a more preferred embodiment, the medicament of the present invention comprises a cell population with at least about 75%, at least about 80%, at least about 85%, at least about 90%, or at least about 95% of cells being functional mature differentiated corneal endothelial cells. In this embodiment, cells contained in the cell population having CD166 positive, CD133 negative, and CD44 negative to weakly positive (preferably CD44 negative) or CD166 positive, CD133 negative, and CD200 negative are selected out. It is confirmed that a cell density (e.g., about 2300 cells/mm² or a higher level), which is considered a benchmark for successful corneal cell infusion therapy, can be more reliably accomplished by the presence of high levels of the functional mature differentiated corneal endothelial cells. In a more preferred embodiment, the medicament of the present invention comprises a cell population with at least about 95% or greater, at least about 96% or greater, at least about 97% or greater, at least about 98% or greater, or at least about 99% or greater of cells being functional mature differentiated corneal endothelial cells. It is demonstrated that a therapeutic result which exceeds about 2300 cells/mm² (e.g., about 3000 cells/mm² or greater) can be achieved in not even one month after infusion by providing such a cell population comprising functional mature differentiated corneal endothelial cells. Thus, a fast and high quality therapeutic technique that was not available is provided. The medicament of the present invention is demonstrated as achieving excellent clinical results by 6 month after surgery. It was also discovered that such an effect is correlated with the E-ratio and a ratio of corneal endothelial property possessing functional cells of the invention. Thus, it is understood that a stable and excellent clinical result can be provided by the medicament of the present invention. It is discovered in particular that the E-ratio or the ratio of corneal endothelial property possessing functional cells of the invention can be raised to achieve a therapeutic effect earlier. It is understood that a clinical effect is achieved to the extent that the effect can be observed after 3 months, and in some cases after 1 month. Such an effect can be achieved by raising the E-ratio or the ratio of corneal endothelial property possessing functional cells of the invention, or by selection of a subpopulation based on the E-ratio or the ratio of corneal endothelial property possessing functional cells, method of manufacturing cells that enable an increase in the E-ratio or the ratio of corneal endothelial property possessing functional cells of the invention, or the like. Such an effect can be achieved by the disclosure of the present invention. In particular, as demonstrated in the Examples, for what was a corneal endothelial cell density (ECD) of about 500 cells/mm², a density exceeding at least about 1000 cells/mm² was accomplished, a density exceeding about 2000 cells/mm² in average was accomplished, and a density exceeding about 2300 cells/mm² was accomplished in nearly half of such cases. Thus, it was revealed that a therapeutic effect is achieved beyond the level that could be achieved previously. In addition, when the E-ratio is further increased to 90% or higher, a result exceeding 3000 cells/mm² in average was already accomplished after 1 month. Results comparable to normal subjects were achieved, such that the results are notably improved.

The medicament of the present invention also notably improves other therapeutic assessment items such as corneal thickness, vision, and the like. For instance, when assessment of corneal thickness is studied, a therapeutic effect is achieved earlier compared to conventional methods. An effect was already achieved after 3 months. The corneal thickness is sufficiently reduced even after one month by raising the E-ratio, such that a notable improvement is observed.

An effect based on controlling the E-ratio or the ratio of corneal endothelial property possessing functional cells of the invention is observed even at visual inspection level. When neither the E-ratio nor the ratio of corneal endothelial property possessing functional cells is controlled of the invention, opacity was observed even after 3 months. Meanwhile, when the E-ratio or the ratio of corneal endothelial property possessing functional cells of the invention was controlled, corneal edema was cleared in 3 months. Further increasing the ratio and raising the E-ratio allowed edema to be cleared in 1 month.

Further, the medicament of the present invention also notably improves other items, such as vision, stromal edema and total score thereof. Further, a severe adverse event was not observed and a non-severe adverse event was hardly observed, such that the medicament of the present invention is understood to provide excellent therapeutic result.

As a notable case achieved by the medicament of the present invention, cultured corneal endothelial cell infusion with combined use of a Rho kinase inhibitor can be performed on a patient with bullous keratopathy. An embodiment of injecting a corneal endothelial cell collected and cultured from a cornea provided by an organization such as the US eye bank with a Rho kinase inhibitor on the back side of a cornea called the anterior chamber is an example for a therapeutic method using the medicament of the present invention. For instance, an embodiment of attempting adhesion of an infused cell to the endothelial surface in a face-down posture for typically 3 hours or longer after injection is shown.

It is preferable that a possible complication is avoided as much as possible by carefully observing the progress for post-surgery inflammation or the like. In case of a severe side effect, measures can be taken by immediately discontinuing therapy to take measures matching the side effect. The therapeutic period is typically 24 weeks, but the period can be extended or reduced depending on progress in therapy. For instance, the present invention has examples where 3000 cells/mm² is exceeded one month after therapy with a corneal endothelial specular microscopy. Thus, progress can be observed after a certain period has passed to take measures. Further, it is generally desirable to observe progress to confirm safety and therapeutic effect after the end of the period. Therapeutic effects can be judged by assessment of vision, transparency of the cornea, corneal endothelial cell density, corneal thickness and the like by visual observation. Those skilled in the art can determine such specific therapeutic forms via a suitable test as needed by using findings known in the art for indication, infusion vehicle, number of cells to be infused and the like for a target patient.

In one specific embodiment, the medicament of the present invention is used for treating a corneal endothelial dysfunction, disorder, or disease. Such a corneal endothelial dysfunction, disorder, or disease comprises, but is not limited to, at least one selected from the group consisting of corneal endothelial disorder Grade 3 and corneal endothelial disorder Grade 4 (typically bullous keratopathy) (e.g., Fuchs endothelial corneal dystrophy, PEX-BK (pseudoexfoliation bullous keratopathy; bullous keratopathy involving pseudoexfoliation syndrome), post-laser iridotomy bullous keratopathy, post-cataract surgery bullous keratopathy (including pseudophakic or aphakic bullous keratopathy), post-glaucoma surgery bullous keratopathy, and post-trauma bullous keratopathy, bullous keratopathy of unknown cause after multiple surgeries, post-corneal transplantation graft failure, congenital corneal endothelial dystrophy, and congenital anterior chamber angle hypoplasia syndrome. The grade system used herein is based upon the severity classification of corneal endothelial disorders, which is based on Japanese Journal of Ophthalmology 118: 81-83, 2014. For instance, an example of bullous keratopathy includes post-laser iridotomy bullous keratopathy, which involves a surgery that opens a hole with laser on the iris of a patient with ocular pressure which is difficult to control only with a glaucoma therapeutic agent to improve the flow of aqueous humour. Meanwhile, it is understood that corneal endothelium is hit by flowing water thereof to damage the endothelium. The medicament of the present invention is considered as exhibiting a notable effect. Fuchs corneal dystrophy is a congenital genetic disease considered to affect 4-5% of 40-50 year olds or older individuals in Europe and the US. The endothelium in the center of the cornea falls off to exhibit opacity. Fuchs corneal dystrophy is the leading cause of corneal transplantation in Europe and the US. The medicament of the present invention is also considered to exhibit a notable effect on Fuchs corneal dystrophy. Further, the medicament is also effective for bullous keratopathy after multiple operations with an unknown cause called Multiple OP-BK. Typical example of such a multiple operation includes an operation with concurrent vitreoretinal operation and cataract+intraocular lens insertion generally called "triple operation" and the like.

The medicament of the present invention can be administered to a subject in any manner, but it is desirable that a cell contained in the medicament of the present invention is administered into the anterior chamber in a preferred embodiment. A technique of infusing a cultured corneal endothelial cell into the anterior chamber is established. Although not wishing to be bound by any theory, this is because the concept of regenerating corneal endothelia by intra-anterior chamber infusion is (1) minimally invasive, (2) uses no artificial material, and (3) allows use of a highly functional corneal endothelial cell from a young donor with little senescence as a master cell. Further, this is because a corneal endothelial function is most efficiently regenerated by infusion of the cell into the anterior chamber. This is also because the process of the present invention has revealed that the safety and clinical POC has been established in human applications by studies based on the guidelines for clinical studies using a human stem cell (exploratory clinical studies) for ex vivo culture expansion and then infusion of cell suspension into the anterior chamber of a patient (with bullous keratopathy or the like).

In addition to a cell, the medicament of the present invention may be administered in conjunction with an additional agent. As such an additional agent, agents that are generally used in ophthalmic therapy (e.g., steroid agent, antimicrobial, antibacterial or NSAID) may be used. In addition to a ROCK inhibitor, an agent used under the condition of maturing and differentiating the specific corneal endothelial cell of the present invention, which can be used as a medicament, can also be included mainly for maintaining or enhancing the quality of a cell.

Agents that are used concomitantly include steroid agents. In this regard, adrenocortical steroid agent (e.g., methylprednisolone (Solu-Medrol(R)), betamethasone (Rinderon(R)), fluorometholone (Flumetholon(R)), dexamethasone (Decadron(R) or the like), prednisolone (Predonine(R)) or the like) for suppressing a rejection and controlling post-operation inflammation. To prevent infections, antibiotics are administered. Examples of such antibiotics include, but are not limited to, flomoxef sodium (Flumarin(R)), cefcapene pivoxil hydrochloride (Flomox(R), gatifloxacin (Gatiflo(R)) and the like), and the like. To prevent inflammations, NSAIDS can be administered. Examples of such NSAIDs include indomelol eye drops (generic name: indomethacin), Niflan eye drops (generic name: planoprofen), Diclod eye drops (generic name: diclofenac sodium), Bronuck eye drops (generic name: bromfenac sodium hydrate), Nevanac suspension eye drops (generic name: nepafenac) and the like.

Examples of ROCK inhibitors used as a combined agent include compounds disclosed in US Patent No. 4678783, Japanese Patent No. 3421217, WO 95/28387, WO 99/20620, WO 99/61403, WO 02/076976, WO 02/076977, WO 2002/083175, WO 02/100833, WO 03/059913, WO 03/062227, WO 2004/009555, WO 2004/022541, WO 2004/108724, WO 2005/003101, WO 2005/039564, WO 2005/034866, WO 2005/037197, WO 2005/037198, WO 2005/035501, WO 2005/035503, WO 2005/035506, WO 2005/080394, WO 2005/103050, WO 2006/057270, WO 2007/026664, and the like. Such compounds can be manufactured by the method described in each disclosed document. Examples thereof include 1-(5-isoquinolinesulfonyl)homopiperazine or a salt thereof (e.g., fasudil or fasudil hydrochloride), (+)-trans-4-(1-aminoethyl)-1-(4-pyridylcarbamoyl)cyclohexanecarboxamide or a salt thereof (e.g., Y-27632 ((R)-(+)-trans-(4-pyridyl)-4-(1-aminoethyl)-cyclohexanecarboxamide dihydrochloride monohydrate), and the like), and preferably comprising Y-27632.

Such an addition agent may be comprised in the cell medicament of the present invention as a medicament, or provided in a separately administered form. In a separately provided or administered form, the additional agent is provided as a kit or combined agent. When used as a kit or combined agent, a package insert or the like that describes the usage method thereof may also be combined.

The medicament, pharmaceutical composition or agent (therapeutic agent, prophylactic agent or the like) of the present invention can be provided as a kit. In a specific embodiment, the present invention provides an agent pack or kit comprising one or more containers filled with one or more ingredients of the composition or medicament of the present invention. In some cases, information showing approval for manufacture, use or sale for human administration by a government agency can be shown on such a container in a form defined by the government agency restricting the manufacture, use or sale of a medicament or biological product.

The corneal endothelial property possessing functional cell or functional mature differentiated corneal endothelial cell of the present invention contained in the medicament of the present invention can be contained at a density of, for example, about 5 × 10⁴ cells/300 .micro.L to about 2.0 × 10⁶ cells/300 .micro.L, or about 2 × 10⁵ cells/300 .micro.L to about 5× 10⁵ cells/300 .micro.L. A suitable cell density is not limited thereto. Each of the upper limit and lower limit thereof can be appropriately varied. Examples of the lower limit include about 5 × 10⁴
cells/300 .micro.L, about 1 × 10⁵ cells/300 .micro.L, about 1.5 × 10⁵
cells/300 .micro.L, about 2 × 10⁵ cells/300 .micro.L, about 2.5 × 10⁵
cells/300 .micro.L, about 3 × 10⁵ cells/300 .micro.L, and the like. Examples of the upper limit include about 3 × 10⁶ cells/300 .micro.L, about 2.5 × 10⁶
cells/300 .micro.L, about 2.0 × 10⁶ cells/300 .micro.L, about 1.5 × 10⁶
cells/300 .micro.L, about 1.0 × 10⁶ cells/300 .micro.L, and the like. Any combination thereof may be used.

The medicament of the present invention may further comprise a cell infusion vehicle. Such a cell infusion vehicle may be provided after mixing with the cell of the present invention, or separately. In a separately provided or administered form, such a solution is provided as a kit or combined agent. When used as a kit or combined drug, a package insert or the like that describes the usage method thereof may also be combined.

As used herein, "kit" refers to a unit generally providing portions to be provided (e.g., inspection drug, diagnostic drug, therapeutic drug, antibody, label, manual and the like) into two or more separate sections. This form of a kit is preferred when a composition that should not be provided in a mixed state and is preferably mixed immediately before use for safety or the like is intended to be provided. Such a kit advantageously comprises an instruction or manual describing how the provided portions (e.g., inspection drug, diagnostic drug, or therapeutic drug) are used or how a reagent should be handled. When the kit is used herein as a reagent kit, the kit generally comprises an instruction describing how to use an inspection drug, diagnostic drug, therapeutic drug, antibody and the like.

As used herein, "instruction" is a document with an explanation of the method of use of the present invention for a physician or other users. The instruction has an instructive description of the detection method of the present invention, method of use of a diagnostic agent, or administration of a medicament or the like. Further, an instruction may have a description instructing oral administration or administration to the esophagus (e.g., by injection or the like) as a site of administration. The instruction is prepared in accordance with a format defined by the regulatory agency of the country in which the present invention is practiced (e.g., the Ministry of Health, Labour and Welfare in Japan, Food and Drug Administration (FDA) in the U.S. or the like), with an explicit description showing approval by the regulatory agency. The instruction is a so-called package insert and is typically provided in, but not limited to, paper media. The instructions may also be provided in a form such as electronic media (e.g., web sites provided on the Internet or emails).

For "cell infusion vehicle" used herein, any solution can be used as long as a cell can be maintained. Cell infusion vehicles include those which can be used as an intraocular irrigating solution or the like. Examples of solutions used as a cell infusion vehicle include Opti-MEM, additive added form thereof, Opeguard-MA, Opeguard-F and the like.

The cell infusion vehicle used in the present invention may further comprise at least one of albumin, ascorbic acid (or ascorbate), and lactic acid (or lactate). This is because addition of these components facilitates cell maintenance. Preferably, all of these components are added to a cell infusion vehicle. This is because therapeutic results of solution using them are demonstrated to be excellent. In a preferred embodiment, a solution using Opeguard-MA(R) and at least one, two or all three of albumin, ascorbic acid, and lactic acid is used.

Based on the knowledge obtained herein, patients can be classified using an expression property of a cell surface marker or the like, cytokine, miRNA, metabolite or the like as an indicator and appropriately prepare the corneal endothelial property possessing functional cell or functional mature differentiated corneal endothelial cell of the present invention according to the pathological condition of classified patients to provide suitable therapy.

### (Quality control or process control of produced cell, cell medium, cell infusion vehicle or the like)

In another aspect, the present invention provides a technique directed to quality control or process control of produced corneal endothelial property possessing functional cells of the invention or functional mature differentiated corneal endothelial cells, cell medium for these cells (culture solution), cell infusion vehicle (suspension) or the like. In one aspect, the present invention provides a method of quality control or process control of a cultured human functional corneal endothelial cell capable of eliciting a human corneal endothelial functional property, corneal endothelial property possessing functional cell of the invention or functional mature differentiated corneal endothelial cell, comprising the step of measuring at least one cell indicator selected from the group consisting of: a cell surface marker; a proteinaceous product and a related biological material of the product; a SASP related protein; miRNA (e.g., secreted (soluble) miRNA or intracellular miRNA); an exosome; a cellular metabolite comprising an amino acid and a related biological material of the metabolite; cell size; cell density and the presence of an autoantibody reactive cell. Each of the cell indicators, i.e., cell surface markers; proteinaceous products and related biological materials of the products; SASP related proteins; miRNA (e.g., secreted (soluble) miRNA or intracellular miRNA); exosomes; cellular metabolites and related biological materials of the metabolites; cell sizes; cell densities and the presence of an autoantibody reactive cell are explained in detail elsewhere herein. Such a technique of quality control or process control using cell indicator was not provided previously. It was revealed that cells used in infusion therapy are mixtures of heterogeneous cell subpopulations and cells of interest are limited to a portion thereof by providing such a technique using cell indicators for revealing the properties in detail. It was also revealed that cultured human corneal endothelial cells are comprised of subpopulations of functional mature differentiated corneal endothelial cells, intermediately differentiated corneal endothelial cells, unintended cells and the like. It was also revealed that karyotype abnormalities occur subpopulation selectively, and there are autoantibodies that reacts subpopulation selectively. It was revealed that the corneal endothelial property possessing functional cell of the invention or functional mature differentiated corneal endothelial cell do not have such an abnormality and have low expression of cell degeneration associated antigens or HLA class I antigens associated with immunological rejection relative to other subpopulation. Since it is revealed that a non-intended cell has high cytokine (SASP) production associated with cell senescence such that the amount of SASP in the anterior chamber environment where a cell is infused greatly varies depending on the patient, these cell indicators can be used to perform the quality control or process control of the functional mature differentiated corneal endothelial cell of the present invention. The technique of the present invention is the first to be able to perform such quality control or process control, which was completely unimaginable from conventional knowledge.

It was discovered that quality control or process control can be performed by assessing the purity of the corneal endothelial property possessing functional cells of the invention or functional mature differentiated corneal endothelial cells, proportion of coexisting non-intended cells, SASP related proteins secreted from infused cells or endogenous to the cell infusion site, miRNAs, metabolites and the like on the cell side, and assessing a host factor such as an autoantibody on the living body side.

In addition, a method of assessment or process control that can identify the corneal endothelial property possessing functional cell of the invention or functional mature differentiated corneal endothelial cell was developed. For example, the present inventors discovered that secreted miRNAs, tricarboxylic acid (TCA) cycle metabolites vs glycolytic system metabolites and the like can be effectively utilized for the identification of the corneal endothelial property possessing functional cell of the invention or functional mature differentiated corneal endothelial cell and unintended cell in culture cells. Thus, the present invention provides, for example, a method of assessing purity by secreted miRNA or tricarboxylic acid (TCA) cycle metabolite vs glycolytic system metabolite, and equipment for use therein.

The present invention also provides a method of tracking metabolism which can track and assess the production process of the corneal endothelial property possessing functional cell of the invention or functional mature differentiated corneal endothelial cell and equipment for use therein.

In addition, the present invention provides a method of quality control or process control of the corneal endothelial property possessing functional cell of the invention or functional mature differentiated corneal endothelial cell, comprising evaluating a cell area and a distribution thereof.

In a specific embodiment, at least three of the cell indicators of the present invention are used. By using at least three cell indicators, the quality of the corneal endothelial property possessing functional cell of the invention or functional mature differentiated corneal endothelial cell can be properly assessed, and process control can be implemented.

In one preferred embodiment, the cell indicators used in the present invention comprise cell size, cell density or a combination thereof. The functionality of a corneal endothelium can be evaluated to a considerably degree by evaluating cell size, cell density or a combination thereof.

In a specific embodiment, the cell indicators used in the present invention comprise a combination of: at least one of cell surface marker, cellular proteinaceous product and related biological material of the product; at least one of miRNA (intracellular miRNA, secreted miRNA, and the like); and at least one of cellular metabolite and related biological material of the metabolite. This is because use of these three types of cell indicators can more reliably exclude a transformed cell, exclude an unintended cell, and identify an intermediately differentiated corneal endothelial cell. Further, by using secreted miRNA, noninvasive quality control or process control can be performed with a combination of each indicator with a product from a cell (proteinaceous product and metabolite).

The method of quality control or process control of the present invention further comprises identifying a subpopulation of functional mature differentiated corneal endothelial cells by a cell indicator and/or a corneal functional property.

For example in one embodiment, the present invention can perform quality control or process control by measuring at least one of the cell indicators (e.g., cell surface marker) of the corneal endothelial property possessing functional cell of the invention or functional mature differentiated corneal endothelial cell. For cell indicators such as cell surface markers, it is understood that any embodiment discussed in detail herein in (Human functional corneal endothelial cells capable of eliciting a human corneal endothelial functional property when infused into an anterior chamber of a human eye) or the like can be used alone or in combination.

In one embodiment of the quality control or process control technique of the present invention, a corneal functional property comprises expression of a cell surface antigen comprising CD166 positive and CD133 negative on a cell surface. Preferably, the property may be characterized by the cell surface antigen comprising CD166 positive, CD133 negative, and CD44 negative to intermediately positive, or CD166 positive, CD133 negative, and CD44 negative to CD44 weakly positive. Further, the property may be characterized by the cell surface antigen comprising CD166 positive, CD133 negative, CD44 negative to CD44 weakly positive, and CD90 negative. Alternatively in another embodiment, the property is characterized by the cell surface antigen comprising CD166 positive, CD133 negative, and CD200 negative.

In one specific embodiment, quality control or process control can be performed by selecting a plurality of indicators from each of proteinaceous product and related biological material of the product, secreted miRNA, and cellular metabolite comprising an amino acid and related biological material of the metabolite to examine a variation in a profile of each indicator to determine homogeneity of cells having a cell indicator comprising CD166 positive, CD133 negative, CD44 negative to CD44 weakly positive and CD90 negative to weakly positive.

In one embodiment, the cellular proteinaceous product and related biological material of the product used in the present invention includes, but is not limited to, those with (A) elevated expression in a functional mature differentiated corneal endothelial cell and (B) decreased expression in a functional mature differentiated corneal endothelial cell.

In one embodiment, any miRNA marker described herein in the section of (Human functional corneal endothelial cells capable of eliciting a human corneal endothelial functional property when infused into an anterior chamber of a human eye) or the like can be used as the miRNA markers used in the preset invention.

In one embodiment, the exosome used in the present invention may have (A) a cell indicator with decreased expression in a functional cell, and more specifically, comprises at least one indicator selected from the group consisting of CD63, CD9, CD81, HSP70 and the like.

In one embodiment, any cell metabolite and related biological material of the metabolite described herein in the section of (Human functional corneal endothelial cells capable of eliciting a human corneal endothelial functional property when infused into an anterior chamber of a human eye) or the like can be used as the cell metabolite and related biological material of the metabolite used in the present invention.

In another embodiment, the cell size used in the present invention has a mean cell area of about 250 .micro.m² or less, about 240 .micro.m² or less, about 230 .micro.m² or less, about 220 .micro.m² or less, about 210 .micro.m² or less, or about 200 .micro.m² or less. Alternatively, the mean cell area may be about 300 .micro.m² or less, about 290 .micro.m² or less, about 280 .micro.m² or less, about 270 .micro.m² or less, or about 260 .micro.m² or less. The functional mature differentiated corneal endothelial cell of the present invention achieved a decrease in size to the same level as a relatively small cell with functionality. Further, it was revealed that a cell is functional by achieving the size defined in the present invention and the size is correlated with quality. Thus, the quality of the functional mature differentiated corneal endothelial cell of the present invention can be controlled by determining whether the size is as defined in the present invention.

In a further embodiment, the mean cell density at saturated cell culture of cell population used in the present invention is at least about 2000 cells/mm² or greater. It can be determined whether the cell population of the present invention can be used in therapy by observing that the mean cell density at saturated cell culture is at least about 1500 cells/mm² or greater, at least about 1600 cells/mm² or greater, at least about 1700 cells/mm² or greater, at least about 1800 cells/mm² or greater, at least about 1900 cells/mm² or greater, or at least about 2000 cells/mm² or greater. Alternatively, the mean cell density at saturated cell culture of the cell population is at least 2100 cells/mm² or greater, at least 2200 cells/mm² or greater, at least 2300 cells/mm² or greater, at least 2400 cells/mm² or greater, or at least 2500 cells/mm² or greater, but not limited thereto. The upper limit can be any materializable value, but examples of materializable upper limit include about 3000 cells/mm² or higher, about 3100 cells/mm², about 3200 cells/mm², about 3300 cells/mm², about 3400 cells/mm², about 3500 cells/mm², about 3600 cells/mm², about 3700 cells/mm², about 3800 cells/mm², about 3900 cells/mm², about 4000 cells/mm² and the like. It is understood that any combination of such upper limit and lower limit is used as the preferred range of cell density at saturated cell culture of the cell population of the present invention.

For example, process control can be performed by quantifying proteinaceous product (e.g., cytokines or the like) in culture at any point during subculture by ELISA. The morphology of a cell can be inspected at the same time. Further, some of the cells can be taken out at a certain time before infusion (e.g., 1 day before, 1 week before, 2 weeks before, 3 weeks before, or the like, any point between immediately before to three weeks before infusion or the like, or before three weeks or more or the like) to inspect whether cell surface traits meet the specification standard by FACS. Alternatively, this can be performed during subculture. It is common that a difference is not recognized at a level diverging from the specification value among multiple flasks in the range of the same lot. Thus, it is sufficient that FACS analysis is performed for any one flask or scale down plate when there are multiple plates.

Alternatively, a quality specification test (cell function confirmation test) can be additionally or separately performed. For instance, immunostaining test (Na⁺/K⁺ ATPase, ZO-1) can be performed as a quality specification test (cell function confirmation test). For instance, cells seeded at the same cell density and cultured on plates (example thereof includes a 24-well plates) can be used for such an immunostaining test. Human corneal endothelial cells obtained by culturing in another scale can be incorporated into a method of evaluation when evaluating a suspension cell for infusion into humans.

In one exemplary embodiment of the present invention, quality control or process control can be performed as follows. That is, the corneal endothelial property possessing functional cell of the invention or functional mature differentiated corneal endothelial cell is expected to be injected as a cell suspension into the anterior chamber of a human eye. Culture cells vary not only in shapes (size, morphology, or the like), but also in terms of all aspects such as a wide range of cellular traits (adhesiveness, soluble product producing capability, cell surface trait, expression of intracellular functional molecule). Meanwhile, a non-cell strain primary culture cell exhibits various transformations (epithelial mesenchymal transition, cell senescence and the like) and various normal plasticity depending on the culture. Unlike low molecule compounds, a cellular medicament requires many a priori attempts to determine quality specification. Thus, the method of quality control or process control of the present invention is important. A typical example of a manufacturing method of the corneal endothelial property possessing functional cell of the invention or functional mature differentiated corneal endothelial cell targeted by the present invention includes a method comprising adding, for example, 10 .micro.M Y-27632 (ROCK inhibitor) to induce differentiation into a mature differentiated cell, adding for example 10 .micro.M of SB203580 for suppressing cell state transition during culture period, continuing culture by exchanging the medium over several weeks after culture cells reach cell saturation (confluence) in terms of cell density for maturation and differentiation of cells, and the like. At this time, the present invention provides a method using multiple types (e.g., 9 types) of cell surface traits to objectively and reproducibly define the purity of the corneal endothelial property possessing functional cells of the invention or functional mature differentiated corneal endothelial cells. As a method of process control, this can be accomplished by selecting some of the culture container in the process of culturing and using the culture solution at the time of culture solution exchange to set process control/specification item and determine a standard value. Quality evaluation or process control can be performed for culture cell density at each passage, cell size, and size distribution by defining a specification value. The present invention provides a standard for cell metabolite and miRNA that can estimate contamination of a non-intended cell as a noninvasive process control/specification testing method. Thus, quality evaluation or process control can be achieved in each situation by using the various standard values.

In another aspect, the present invention provides a method of detecting a corneal endothelial nonfunctional cell (non-intended cell) coexisting with a cultured human corneal endothelial cell comprising a step of measuring at least one cell indicator selected from the group consisting of cell size, cell density and the presence of an autoantibody reactive cell.

In still another aspect, the present invention provides a quality evaluating agent, process controlling agent, or corneal endothelial nonfunctional cell detecting agent for the corneal endothelial property possessing functional cell of the invention or functional mature differentiated corneal endothelial cell, comprising a reagent or means for measuring the cell indicator of the present invention.

Measuring means can be anything that can measure a cell indicator.

The "agent" used herein as a quality evaluating agent, process controlling agent, or corneal endothelial nonfunctional cell detecting agent or the like may be any substance or other element (e.g., energy, radiation, heat, electricity and other forms of energy) as long as the intended objective can be achieved. Examples of such a substance include, but are not limited to, protein, polypeptide, oligopeptide, peptide, polynucleotide, oligonucleotide, nucleotide, nucleic acid (including, for example, DNAs such as cDNA and genomic DNA and RNAs such as mRNA), polysaccharide, oligosaccharide, lipid, organic small molecule (e.g., hormone, ligand, information transmitting substance, organic small molecule, molecule synthesized by combinatorial chemistry, small molecule that can be used as medicine (e.g., small molecule ligand and the like)) and a complex molecule thereof. Typical examples of an agent specific to a polynucleotide include, but are not limited to, a polynucleotide having complementarity with a certain sequence homology (e.g., 70% or greater sequence identity) to a sequence of the polynucleotide, polypeptide such as a transcription factor that binds to a promoter region and the like. Typical examples of an agent specific to a polypeptide include, but are not limited to, an antibody directed specifically to the polypeptide or a derivative or analog thereof (e.g., single stranded antibody), a specific ligand or receptor when the polypeptide is a receptor or ligand, a substrate when the polypeptide is an enzyme and the like.

For cell surface markers (e.g., CD marker), they include, but are not limited to, antibodies. For miRNA, they include, but are not limited to, probes with a complementary sequence. Such measuring means are preferably labeled.

For cellular proteinaceous products and related biological materials of the product, SASP proteins, exosomes, cellular metabolites and related biological materials of the metabolite and the like, means include a measuring kit utilizing an enzymatic reaction.

In one aspect, the present invention provides a method of selectively propagating in cultures a human functional corneal endothelial cell, comprising the steps of: A) providing a sample that possibly comprises a human functional corneal endothelial cell capable of eliciting a human corneal functional property when infused into an anterior chamber of a human eye (or functional mature differentiated corneal endothelial cell); B) determining whether the sample comprises the human functional corneal endothelial cell capable of eliciting a human corneal functional property when infused into an anterior chamber of a human eye (or functional mature differentiated corneal endothelial cell) by using the quality evaluating agent, process controlling agent, or corneal endothelial nonfunctional cell detecting agent of the present invention, wherein it is determined that the sample comprises the human functional corneal endothelial cell capable of eliciting a human corneal functional property when infused into an anterior chamber of a human eye when a result of evaluation with the quality evaluating agent, process controlling agent, or corneal endothelial nonfunctional cell detecting agent indicates that the cell is a human functional corneal endothelial cell capable of eliciting a human corneal functional property when infused into an anterior chamber of a human eye (or functional mature differentiated corneal endothelial cell); and C) selectively propagating in cultures a cell determined to be a human functional corneal endothelial cell capable of eliciting a human corneal functional property when infused into an anterior chamber of a human eye (or functional mature differentiated corneal endothelial cell).

In another aspect, the present invention provides a method of assaying quality of a human functional corneal endothelial cell, comprising the steps of:
A) obtaining information related to a cell indicator of the functional corneal endothelial cell of cells provided as being human functional corneal endothelial cells capable of eliciting a human corneal functional property when infused into an anterior chamber of a human eye (or functional mature differentiated corneal endothelial cell) using the quality evaluating agent, process controlling agent, or corneal endothelial nonfunctional cell detecting agent of the present invention; and
B) determining that the provided cells are human functional corneal endothelial cells capable of eliciting a human corneal functional property when infused into an anterior chamber of a human eye (or functional mature differentiated corneal endothelial cell) based on the information.

In yet another aspect, the present invention provides a method of evaluating quality in preparation of a human functional corneal endothelial cell capable of eliciting a human corneal functional property when infused into an anterior chamber of a human eye (or functional mature differentiated corneal endothelial cell), comprising the steps of: A) obtaining information related to a cell indicator of a functional mature differentiated corneal endothelial cell (or functional mature differentiated corneal endothelial cell) of cells obtained in the preparation using the quality evaluating agent, process controlling agent, or corneal endothelial nonfunctional cell detecting agent of the present invention; and B) determining that the preparation is suitable for preparation of a human functional corneal endothelial cell capable of elicitingeliciting a human corneal endothelial functional property when infused into an anterior chamber of a human eye (or functional mature differentiated corneal endothelial cell) based on the information.

Furthermore, the present invention provides a method of assaying purity of a human functional corneal endothelial cell capable of eliciting a human corneal functional property when infused into an anterior chamber of a human eye (or functional mature differentiated corneal endothelial cell), comprising the steps of: A) providing a sample possibly comprising the human functional corneal endothelial cell capable of eliciting a human corneal functional property when infused into an anterior chamber of a human eye (or functional mature differentiated corneal endothelial cell); B) obtaining information related to a cell indicator of a functional corneal endothelial cell of the cells using the quality evaluating agent, process controlling agent, or corneal endothelial nonfunctional cell detecting agent of the present invention; and C) calculating the purity of the human functional corneal endothelial cell capable of eliciting a human corneal functional property when infused into an anterior chamber of a human eye (or functional mature differentiated corneal endothelial cell) in the sample based on the information.

The present invention provides a method of assaying quality of a medium for a human functional corneal endothelial cell, comprising the steps of: A) culturing cells provided as being a human functional corneal endothelial cell capable of eliciting a human corneal functional property when infused into an anterior chamber of a human eye (or functional mature differentiated corneal endothelial cell) to obtain information related to a cell indicator of the functional corneal endothelial cell of the cells using the quality evaluating agent, process controlling agent, or corneal endothelial nonfunctional cell detecting agent of the present invention; and B) determining that the medium is suitable for manufacture of the human functional corneal endothelial cell capable of eliciting a human corneal functional property when infused into an anterior chamber of a human eye (or functional mature differentiated corneal endothelial cell) based on the information.

The prevent invention provides a method of assaying quality of a cell infusion vehicle for a human functional corneal endothelial cell, comprising the steps of: A) culturing cells provided as being a human functional corneal endothelial cell capable of eliciting a human corneal functional property when infused into an anterior chamber of a human eye (or functional mature differentiated corneal endothelial cell) to obtain information related to a cell indicator of the functional corneal endothelial cell of the cells using the quality evaluating agent, process controlling agent, or corneal endothelial nonfunctional cell detecting agent of the present invention; and B) determining that the cell infusion vehicle is suitable for cell infusion therapy based on the information.

As used herein, "sample that possibly comprises a human functional corneal endothelial cell capable of eliciting a human corneal functional property when infused into an anterior chamber of a human eye (or functional mature differentiated corneal endothelial cell)" refers to any sample obtained by the method of manufacturing human functional corneal endothelial cells capable of eliciting a human corneal endothelial functional property when infused into an anterior chamber of a human eye (or functional mature differentiated corneal endothelial cells) of the present invention or another method. Any sample falls under such a sample as long as the sample possibly comprises a human functional corneal endothelial cell capable of eliciting a human corneal functional property when infused into an anterior chamber of a human eye.

In another aspect, a method of quality control or process control of a cultured human functional corneal endothelial cell capable of eliciting a human corneal functional property when infused into an anterior chamber of a human eye or functional mature differentiated corneal endothelial cell of the present invention comprises the step of examining one or a plurality of the following:
(1) purity test by culture supernatant ELISA, TIMP-1: 500 ng/mL or less, IL-8: 500 pg/mL or less, PDGF-BB: 30pg/mL or greater, MCP-1: 3000 pg/mL or less, (2) purity test by cell FACS, CD166 = 95% or greater, CD133 = 5% or less, CD105 negative-low positive = 95% or greater, CD44 negative-low positive = 70% or greater, CD44 medium-high positive = 15% or less, CD24 = 10% or less, CD26 positive = 5% or less, CD200 = 5% or less, (3) barrier function (ZO-1) positive, (4) pumping function (Na+/K+ ATPase) positive, (5) cell survival rate, 70% or greater with trypan blue stain, (6) cell form, transformed cell cannot be found by visual inspection (7) Claudin10 positive, (8) effector cell (E-ratio) > 50%
(9) non-intended cell, non-intended cell A (CD44 strongly positive cell) < 15%, non-intended cell B (CD26 positive cell) <5 %, non-intended cell C (CD24 positive cell) < 10%, and (10) karyotype abnormality negative.

Indeed, other items such as the following may also be tested: aseptic test (e.g., no bacteria growth is observed by Bacterialert method), mycoplasma (e.g., negative by PCR), endotoxin (e.g., less than 2.3 pg/mL (less than 0.017 EU/mL or the like) by nephelometric time analysis method), virus (e.g., negative by PCR), and residual BSA (e.g., 125 ng/mL or less for final washed solution by ELISA).

These items involve verifying or evaluating any one or more items by the method of selectively propagating in cultures a human functional corneal endothelial cell, method of assaying quality of a human functional corneal endothelial cell, method of evaluating quality in preparation of a human functional corneal endothelial cell capable of eliciting a human corneal functional property when infused into an anterior chamber of a human eye, method of assaying purity of a human functional corneal endothelial cell capable of eliciting a human corneal functional property when infused into an anterior chamber of a human eye, method of assaying quality of a medium for a human functional corneal endothelial cell, or method of assaying quality of a cell infusion vehicle for a human functional corneal endothelial cell of the present invention, which may be implemented three weeks to immediately prior to (e.g., 7 days prior to) cell injection therapy or during subculture or preserving culture with only medium exchange.

The verification may be carried out three weeks to immediately prior to, or preferably 7 day prior to to immediately prior to, cell infusion therapy or during preserved culture only exchanging a medium. During subculture refers to, but is not limited to, before, in the middle of, and after subculture and a period of about 1-7 days or less therefrom. The cell of the present invention, after manufacture, can be preserved only by exchanging the medium. During preserved culture only exchanging a medium refers to a time, or the period around such a time, of exchanging a medium thereupon. Around may be a period of about 1-7 days or less therefrom.

In another aspect, the method of quality control or process control of a cultured human functional corneal endothelial cell capable of elicitingeliciting a human corneal endothelial functional property when infused into an anterior chamber of a human eye or functional mature differentiated corneal endothelial cell of the present invention comprises the step of determining one or a plurality of the following characteristics with respect to a target cell: (1) retention of endothelial pumping/barrier functions; (2) adhesion/attachment to a specific laminin; (3) secreted cytokine profile; (4) produced metabolite profile; (5) saturated cell density upon in vitro culture; (6) spatial size and distribution of cells obtained in culturing; and (8) cell retention in case of cell infusion after freeze damage by cryo treatment by liquid nitrogen on a mouse cornea.

In one embodiment, determination of the (1) retention of endothelial pumping/barrier functions is determined by using a pumping function measuring method or a barrier function measuring method commonly used for corneal endothelial tissue.

In one embodiment, determination of the (2) retention of endothelial pumping/barrier functions is determined by using a pumping function measuring method or a barrier function measuring method commonly used for corneal endothelial tissue.

In one embodiment, determination of the (3) adhesion/attachment to a specific laminin is determined by adhesiveness to laminin 511 (composite of alpha5 chain, beta1 chain, and gamma1 chain), laminin 521 (composite of alpha5 chain, beta2 chain, and gamma1 chain), or a functional fragment thereof and/or increase in integrin expression with respect thereto as an indicator.

In one embodiment, determination of the (4) secreted cytokine profile comprises measuring a production level of a cytokine profile of serum or aqueous humour.

In one embodiment, determination of the (5) produced metabolite profile comprises measuring a production level of metabolite of the cell.

In one embodiment, determination of the (6) produced microRNA (miRNA) profile comprises obtaining total RNA to obtain a micro RNA expression profile.

In one embodiment, determination of (7) saturated cell density upon in vitro culture comprises counting cells in an image of the cells obtained by using an image capturing system.

In one embodiment, determination of the (8) cell retention in case of cell infusion after freeze damage by cryo treatment by liquid nitrogen on mouse cornea comprises: infusing a cell to be determined into an anterior chamber of a human eye of a model made by pre-treatment of a central region of a mouse cornea by freeze damage to remove an endothelial cell; clinically observing characteristic of a corneal; assessing the thickness of the cornea with a pachymeter; histopathologically testing HCEC adhesion with human nuclear staining; and examining whether the cell has a function.

For example, information related to cell indicators can be stored or output in a computer readable state. Data in such a state can be used to determine an additionally provided cell as a functional mature differentiated corneal endothelial cell suitable for cell infusion therapy, selectively propagating in cultures a cell determined as a functional mature differentiated corneal endothelial cell, determine preparation to be suitable for the preparation of a functional mature differentiated corneal endothelial cell suitable for cell infusion therapy, and calculate the purity of functional mature differentiated corneal endothelial cells in a sample.

In an exemplary embodiment, quality test, purity test, function examination and the like for an infused cell can be performed as described below.

### (Quality test, purity test, function examination for cell for infusion)

A cultured corneal endothelial cell was subjected to the following quality control or process control (Quality Control: QC) test. A product having sufficient quality and meeting the delivery specification are used as the corneal endothelial property possessing functional cell of the invention or functional mature differentiated corneal endothelial cell.
1) Process control test
   *Visual inspection (each flask)
   TIMP-1, IL-8, PDGF-betabeta, MCP-1 in the medium (each flask)
   *Live cell count/survival rate
2) Example of delivery specification of final product and testing method

**[Table 1F]**

| Test item | Method | | Standard |
|---|---|---|---|
| Cell morphology | Visual inspection | | No transformed cell is found |
| Cell survival rate | Trypan blue staining | | Surviving cells are 70% or higher |
| Barrier function | Immunostaining (ZO-1) | | Positive |
| Pumping | Immunostaining (Na*/K*ATPase) | | Positive |
| Residual BSA | ELISA of final washed solution | | 125 ng/mL or less |
| | ELISA on culture | | |
| | | supernatant | |
| Purity test | IL-8 | | 500ng/mL or |
| | TIMP1 | | less 500pg/mL |
| | PDGFbb | | or less 30pg/mL |
| | MCP-1 | | or greater |
| | | | 3000pg/mL or |
| | | | less |
| | FACS on cells | | |
| | | CD166 | 100%, |
| | | CD133 | 0% |
| Purity test | | CD105low | 100% |
| | | | 70% or greater |
| | | CD44 low | 5% or less |
| | | CD44 high | 5% of less |
| | | | 5% or less |
| | | CD24 | 0% |
| | | CD26+ | |
| | | CD200 | |
| Aseptic test | Bacterialert test | | No growth of bacteria is observed |
| Mycoplasm | PCR | | Negative |
| Endotoxin | Nephelometric time analysis method | | Less than 2.3pg/ mL,.(less than 0.017EU/mL) |
| Virus | PCR | | Negative |

Delivery was made according to the procedure stipulated in the delivery judgment instruction/record

### (General techniques)

Molecular biological approach, biochemical approach, and microbiological approach used herein are well known and conventional approaches in the art that are described in, for example, Sambrook J. et al.(1989). Molecular Cloning: A Laboratory Manual, Cold Spring Harbor and its 3rd Ed.(2001); Ausubel, F.M.(1987).Current Protocols in Molecular Biology, Greene Pub. Associates and Wiley-Interscience; Ausubel, F.M.(1989). Short Protocols in Molecular Biology: A Compendium of Methods from Current Protocols in Molecular Biology, Greene Pub. Associates and Wiley-Interscience; Innis, M.A.(1990).PCR Protocols: A Guide to Methods and Applications, Academic Press; Ausubel, F.M.(1992).Short Protocols in Molecular Biology: A Compendium of Methods from Current Protocols in Molecular Biology, Greene Pub. Associates; Ausubel, F.M. (1995).Short Protocols in MolecularBiology: A Compendium of Methods from Current Protocols in Molecular Biology, Greene Pub. Associates; Innis, M.A. et al.(1995).PCR Strategies, Academic Press; Ausubel,F.M.(1999).Short Protocols in Molecular Biology: A Compendium of Methods from Current Protocols in Molecular Biology, Wiley, and annual updates; Sninsky, J.J.et al.(1999). PCR Applications: Protocols for Functional Genomics, Academic Press, Bessatsu Jikken Igaku [Experimental Medicine, Supplemental Volume], Idenshi Donyu Oyobi Hatsugen Kaiseki Jikken Ho [Experimental Methods for Transgenesis & Expression Analysis], Yodosha, 1997, Ekusosomu Kaiseki Masuta Lessun [Exosome analysis master lesson], (Jikken Igaku Bessatsu Yodosha) [Experimental Medicine, Supplemental Volume, Yodosha], Riaru Taimu PCR Kanzen Jikken Gaido [Real time PCR complete experimental guide] (Jikken Igaku Bessatsu Yodosha) [Experimental Medicine, Supplemental Volume, Yodosha], Idenshi donyu Purotokoru [Transgenesis protocol] (Jikken Igaku Bessatsu Yodosha) [Experimental Medicine, Supplemental Volume, Yodosha], RNAi Jilkken Naruhodo Q&A [RNAi I understand Q&A] (Yodosha) and the like, the relevant portions (which can be the entire document) of which are incorporated herein by reference.

DNA techniques and nucleic acid chemistry are described in, for example, Gait, M.J.(1985). Oligonucleotide Synthesis:A Practical Approach, IRL Press; Gait, M.J.(1990). Oligonucleotide Synthesis: A Practical Approach, IRL Press; Eckstein, F.(1991). Oligonucleotides and Analogues: A Practical Approach, IRL Press; Adams, R.L. et al. (1992). The Biochemistry of the Nucleic Acids, Chapman&Hall; Shabarova, Z. et al.(1994). Advanced Organic Chemistry of Nucleic Acids, Weinheim; Blackburn, G.M.et al.(1996). Nucleic Acids in Chemistry and Biology, Oxford University Press; Hermanson, G.T.(1996).Bioconjugate Techniques, Academic Press, and the like, the relevant portions of which are incorporated herein by reference.

As used herein, "or" is used when "at least one or more" of the matters listed in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present invention has been described while showing preferred embodiments to facilitate understanding. The present invention is described hereinafter based on Examples. The aforementioned description and the following Examples are not provided to limit the present invention, but for the sole purpose of exemplification. Thus, the scope of the present invention is not limited to the embodiments and Examples specifically described herein and is limited only by the scope of claims.

While the present invention is further explained hereinafter with Examples, the present invention is not limited thereto. All experiments related to humans were conducted in compliance with the Declaration of Helsinki, and other government and university regulations. Animals were raised and handled in accordance with the ARVO statement (the ARVO Statement for the Use of Animals in Ophthalmic and Vision Research) for use of animals in visual and ophthalmic research. For reagents, the specific products described in the Examples were used. However, the reagents can be substituted with an equivalent product from another manufacturer (Sigma, Wako Pure Chemical, Nacalai Tesque, Abcam, Santa Cruz Biotechnology, R & D Systems, Abnova, AssayPro, Origene, Biobyt, Biorad, Cell Signaling Technology, GE Healthcare, IBL, or the like).

### (Abbreviations)

The following abbreviations are appropriately used.
SP: Subpopulation
cHCEC: cultured human corneal endothelial cell
CST: cell state transition
EMT: epithelial-mesenchymal transition
E-ratio: number obtained by dividing the total number of functional mature differentiated corneal endothelial cells and mature differentiated corneal endothelial functional progenitor cells by all cells (ratio of cells of interest)
ECM: extracellular matrix
CSC: cancer stem cell
ECD: corneal endothelial cell density
ug and uL represent .micro.g and .micro.L, respectively.

### (Example 1: Cell Homogeneity Indispensable for Cell Infusion Therapy for Cultured Human Corneal Endothelial Cell)

The aim of this Example was to identify a subpopulation (SP), suitable for cell infusion therapy and devoid of cell state transition (CST) among heterogeneous cultured human corneal endothelial cells (cHCECs). This is because cHCECs, which are expected to serve as an alternative to donor corneas for the treatment of corneal endothelial dysfunction, have an inclination towards cell state transition (CST) into a senescent phenotype, thereby hampering the application in clinical settings.

The presence of subpopulations in cHCECs was confirmed on the basis of surface CD marker expression by flow cytometry. CD markers effective for distinguishing distinct subpopulations were selected by analysis based on established cHCECs with small mean cell area and the high cell density in cultures. The contrasting features among three typical cHCEC subpopulations were also confirmed by PCR-array for the extracellular matrix (ECM). Combined analysis of CD markers clearly identified the subpopulation (effector cells) adaptable for cell infusion therapy among diverse subpopulations. ZO-1 and Na⁺/K⁺ ATPase, CD200 and HLA expression were compared among heterogeneous subpopulations. This experiment is summarized below.

### (Materials and Methods)

### Human corneal endothelial cell donors

The human tissue used was handled in accordance with the ethical tenets set forth in the Declaration of Helsinki. HCECs were obtained from 20 human cadaver corneas and were cultured before performing karyotype analysis. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the state in which the donor consent was obtained and the tissue was recovered.

The donor ages ranged from 2 to 75 years (average 43.7 +/- 26.4). The donors included 9 males and 11 females. All donor corneas were preserved in Optisol GS (Chiron Vision, Irvine, CA, USA) and imported by airplane for research purposes. According to the donor information, all donor corneas were considered healthy with no corneal disease and all donors had no past history of chromosomal abnormality.

### Cultures of HCECs

Unless specifically stated otherwise, the HCECs were cultured according to published protocols, with some modifications. Briefly, the Descemet's membranes with the corneal endothelial cells were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with OptiMEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp., St. Louis, MO, USA), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. Conditioned medium was prepared as previously described (Nakahara, M. et al. PLOS One (2013) 8(7), e69009). The HCECs were cultured using the conditioned medium at 37.degrees.C in a humidified atmosphere containing 5% CO₂. The culture medium was changed twice a week. The HCECs were subcultured at ratios of 1:3 using 10x TrypLE Select (Life Technologies) for 12 minutes at 37.degrees.C when they reached confluence. The HCECs at passages 2 through 5 were used for all experiments.

### Phase Contrast Microscpy

Phase contrast microscope images were taken by an inverted microscope system (CKX41, Olympus, Tokyo, Japan). For the cell area distribution analysis, the cHCECs were washed with PBS (-) three times and the phase contrast images were acquired by a BZ X-700 Microscope system (Keyence, Osaka, Japan). The cell area distributions were quantified by BZ-H3C Hybrid cell count software (Keyence).

### Immunofluorescent Staining

The HCECs were cultured at a density of 1 × 10⁵ cells/well in a 24-well cell culture plate coated with FNC Coating Mix and were maintained for 3 to 4 weeks for immunofluorescence analysis. Cells were fixed in 95% ethanol supplemented with 5% acetic acid for 10 minutes at room temperature and incubated for 30 minutes with 1% BSA. Samples were incubated overnight at 4.degrees.C with antibodies against CD73 (1:300; BD Pharmingen Stain Buffer), CD166 (1:300; BD Pharmingen Stain Buffer), ZO1 (1:300; Zymed Laboratories, South San Francisco, CA, USA), and Na⁺/K⁺-ATPase (1:300; Upstate Biotec, Lake Placid, NY, USA). After washing with PBS, either Alexa Fluor 488-conjugated goat anti-mouse IgG (Life Technologies) or Alexa Fluor 594-conjugated goat anti-rabbit IgG (Life Technologies) was used as the secondary antibody at a 1:1000 dilution. Nuclei were stained with DAPI (Vector Laboratories, Burlingame, CA, USA). The cells, cultured in a 48-well cell culture plate, were directly examined by fluorescence microscopy (BZ-9000; Keyence, Osaka, Japan).

### Fluorescent Staining of IgG and IgM

First, HCECs were fixed by methanol. The cells were washed twice with PBS, permeabilized with PBS-0.2% Tx-100 for 15 minutes at room temperature, and were blocked with PBS of 1% BSA for 1 hour or longer at room temperature. 250 .micro.L of serum of a healthy individual diluted 5 or 25 fold with PBS of 1% BSA was later added to the wells and incubated overnight at 4.degrees.C. The cells were then washed four times with PBS of 0.2% Tx-100. PBS of 1% BSA comprising Alexa Fluor 488-labeled anti-human IgG (5 ug/mL) and Alexa Fluor 647-labeled anti-human IgM (5 ug/mL) were added (250 uL/well). The cells were then incubated for 1 hour at room temperature. The cells were washed twice with PBS of 0.2% Tx-100 and washed once with PBS. After nuclei were stained with DAPI (5 ug/mL) for 15 minutes at room temperature and washed with PBS, the nuclei were examined with an inverted fluorescence microscope (BZ-9000).

### BD Lyoplate Screening

Screening of surface markers was conducted by assessing the expression of markers through the Human Cell Surface Marker Screening Panel (BD Biosciences, San Jose, CA, USA) according to the manufacturer's protocol. Briefly, cultured HCECs were incubated with 242 primary antibodies and isotype IgGs (BD Biosciences) at the dilution indicated by the manufacturer's protocol for 30 minutes at 4.degrees.C. The cells were washed with PBS containing 1% BSA and 5 mM EDTA and then incubated with AlexaFluor 647-conjugated secondary antibodies (1:200 dilution, BD Biosciences) for 30 minutes at 4.degrees.C. The cells were washed again with PBS containing 1% BSA and 5 mM EDTA and analyzed by flow cytometry using a BD FACSCant II instrument (BD Biosciences) and Cell Quest Pro software (BD Biosciences).

Flow cytometry analysis of cultured HCECs

HCECs were collected from the culture dish by TrypLE Select treatment as described above and suspended at a concentration of 4 × 10⁶ cells/mL in FACS buffer (PBS containing 1% BSA and 0.05% NaN₃). An equal volume of antibody solution was added and incubated for 2 hours at 4.degrees.C. The antibody solutions comprised the following: FITC-conjugated anti-human CD26 mAb, PE-conjugated anti-human CD166 mAb, PerCP-Cy5.5 conjugated anti-human CD24 mAb, PE-Cy7-conjugated anti-human CD44 (all from BD Biosciences), and APC-conjugated anti-human CD 105 (eBioscience, San Diego, CA, USA). After washing with FACS buffer, the HCECs were analyzed with FACS Canto II (BD Biosciences).

### Cell sorting

For cell sorting experiments, HCECs were collected and stained with FITC-conjugated anti-human CD24 mAb and PE-Cy7-conjugated anti-human CD44 mAb (BD Biosciences) as described above. After washing with FACS buffer, the cells were re-suspended in FACS buffer. The CD24-negative/CD44-positive and CD24-negative/CD44-positive cells were sorted using a BD FACSJazz cell sorter (BD Biosciences) and seeded at a density of 4.2 × 10⁴ cells on a 24-well cell culture plate for subsequent analysis. Each sequence used was a sequence accompanying these products.

### Examination of immunity related surface antigen

Alexa647-conjugated anti-HLAI antibody (Santa Cruz [#SC-32235 AF647]), FITC-conjugated pan anti-HLAII antibody (BD Biosciences [#550853]) and PE-Cy7-conjugated anti-PDL1 antibody (BD Biosciences [#558017]) were used for fluorescent labeling of HLA-I, HLA-II, and PDL1.

### PCR array

Total RNA was extracted from cultured HCECs using the miRNeasy Mini kit (QIAGEN strasse1 40724 Hilden Germany).The cDNA synthesis was performed with 100 ng total RNA for 96-well plate format using RT² First Strand kit (Qiagen). Expression of endothelial mRNAs was investigated using the RT² Profiler PCR-Array Human Extracellular Matrix and Adhesion Molecules (Qiagen) according to the manufacturer's recommended protocol, and analyzed using RT² Profiler PCR Array Data Analysis Tool version 3.5.

### Measurement of autoantibody

Autoantibodies were measured in accordance with the following protocol.
∗HCECs were fixed with methanol.
∗ Washed twice with PBS
∗ Permeabilized with PBS-0.2% Tx-100 (room temperature, 15 minutes)
∗Blocked with 1% BSA/PBS (1 hour or longer at room temperature)
∗ Added 250 .micro.L of serum of a healthy individual diluted 5 or 25 fold with 1% BSA/PBS to wells
∗ 4.degrees.C, incubated overnight
∗ Washed 4 times with PBS-0.2% Tx-100
∗Added 1% BSA/PBS comprising Alexa Fluor 488-labeled anti-human IgG (5 .micro.g/mL) and Alexa Fluor 647-labeled anti-human IgM (5 .micro.g/mL) (250 .micro.L/well)
∗ Incubated for 1 hour at room temperature
∗ Washed twice with PBS-0.2% Tx-100
∗ Washed once with PBS
∗ Nuclei were stained with DAPI (5 .micro.g/mL) (room temperature, 15 minutes).
∗ Washed once with PBS
∗Examined with inverted fluorescence microscope (BZ-9000)

### Statistical Analysis

The statistical significance (P value) of mean values for 2-sample comparisons was determined by the Student's t-test. The statistical significance for the comparison of multiple sample sets was determined with the Dunnett's multiple-comparisons test. Values in the graphs represent the mean +/- standard error.

### (Results)

### Phenotypic variations among cultures

cHCECs from 5yearold donors, one young and two newborn donors were cultured according to the method of Okumura et al. (Okumura N, et al., Invest Ophthal Vis Sci. 2014; 55:7610-8.), and surface expressions of CD44, CD166, CD24 and CD105 were characterized (Table 1Ga). The other sets of analysis of cHCECs from other donors for CD44, CD166, CD 105 and LGR5, instead of CD24, were summarized in Table 1Gb.

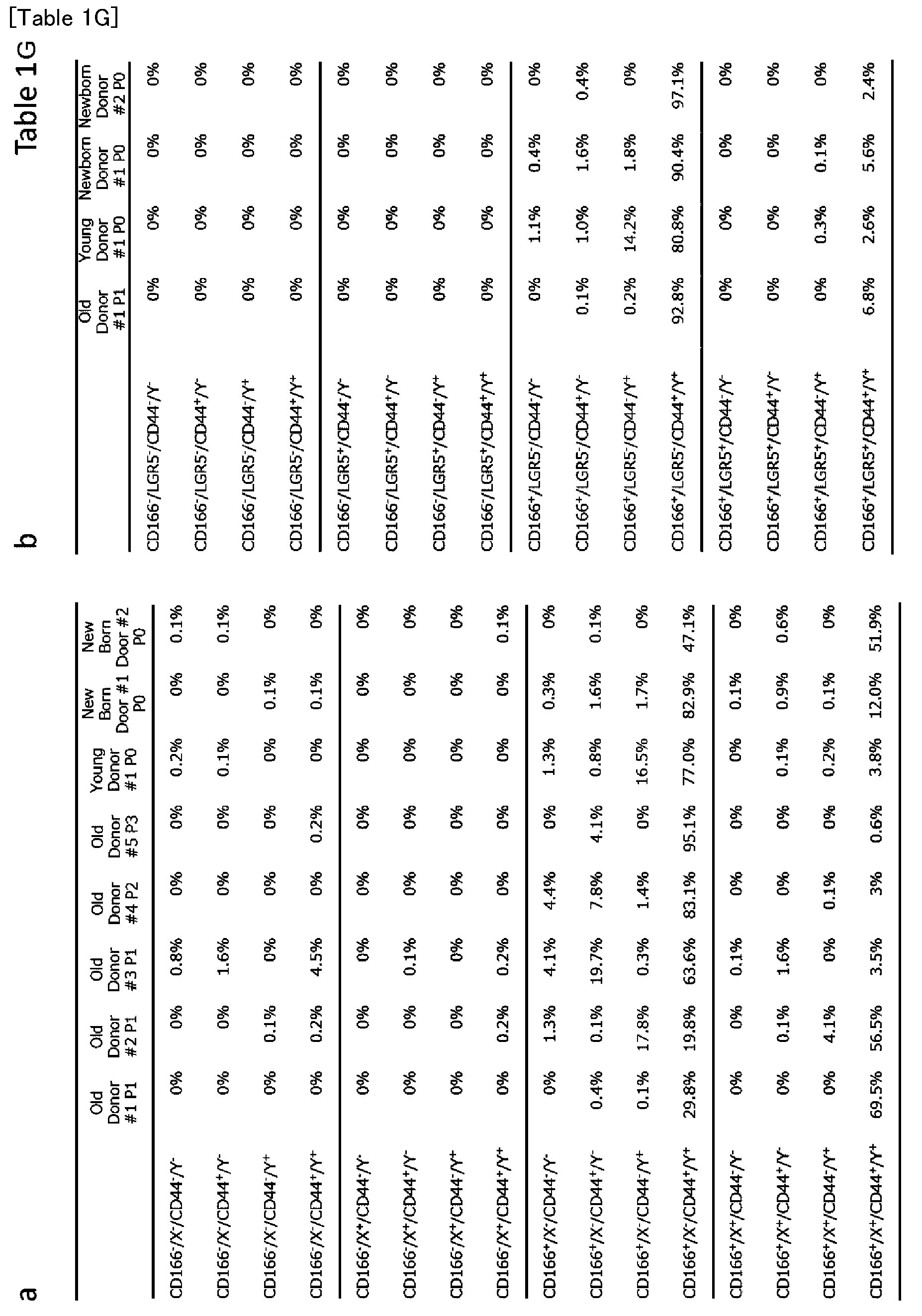

It was found that depending on the difference of donors, there is a great distinction in the proportions of subpopulations defined with the surface markers was evident. The subpopulation with the highest proportion was the CD166+CD24-CD44+CD105+ subpopulation, while the subpopulation present at the highest proportion in cHCECS from old donors #1 and #2 was the subpopulation with CD166+CD44+CD105+CD24-. Table 1b shows that the CD166+CD44+CD105+LGR5- subpopulation is present at the highest proportion. Even primary cHCECs exhibited various phenotypes during cultures under the same culture condition. The origin of any corneal endothelial tissue cultured by a conventional method leads to various cells by culturing, resulting in cultured cells with different size, form, cell density, and homogeneity. Such heterogeneity can be overcome by following categorization by subpopulations in this Example.

To confirm the heterogeneity depending on the number of passages, many cultures were monitored by flow cytometry for the changes of subpopulation compositions. Representative results are shown in Fig. 1 (A-B), together with phase contrast microscope images. Fig. 2 (A-B) shows a representative FACS analysis (Fig. 2-A correspond to Fig. 1-A and Fig. 2-B to Fig. 1-C). In summary, differences in both the number of passages and donors exhibited great variations in the proportion of subpopulations. In view of the above, it was determined that the so-called "cultured corneal endothelial cells" encompass heterogeneous subpopulations and a specific subpopulation thereamong is a functional mature differentiated corneal endothelial effector cells to proceed with the following analysis.

### ZO1 and Na+/K+ ATPase expression does not guarantee homogeneity

Both ZO1 and Na⁺/K⁺ ATPase that are well known as markers of HCECs were not only stained for CD44- subpopulations, but also for CD44++CD24- and CD44++CD24+ subpopulations with stratified and fibroblastic morphology (Fig. 3). CD44+++CD24+ subpopulations with typical stratified and fibroblastic morphology was devoid of these expression (Fig. 3).

### CD200 and HLA class I expression among subpopulations

The expression of CD200, claimed previously as a marker of HCECs, was surprisingly restricted to one CD44++ subpopulation in vulnerable cHCECs that had undergone CST (Fig. 4c). Importantly, CD44- cells did not show any sign of CD200 expression (Fig. 4a), and more interestingly some subpopulations that were highly CD44 positive did not express CD200. In regard to the immunogenicity of cHCECs which may be infused into allogeneic hosts, the inventors found that subpopulations differed in the expression of surface HLA class I antigen, showing a decrease in parallel with the decrease of CD44 and CD26 expression (Fig. 5).

Analysis of HLA-I, HLA-II, and PDL1 expression in cHCECs revealed that HLA-I and PDL1 were positive for each cultured human corneal endothelial cells, but HLAII was mostly negative for the functional mature differentiated corneal endothelial cell of the present invention, as shown in Fig. 10-B and Fig. 5.

As shown in Fig. 10-C, images from a fluorescence microscope and a phase contrast microscope of cultured human corneal endothelial cells comprising cells that have undergone cell state transition show that IgG and IgM are bound to cultured human corneal endothelial cells that have undergone cell state transition. This demonstrates that a natural antibody against a cell state-transitioned cell is present in the serum. In this manner, it was elucidated that substantial absence of autoantigens occurs subpopulation-specifically for the functional cell of the present invention. As shown in this Example, a specific subpopulation can be monitored to select a subpopulation that is substantially free of autoantibodies. Autoantibodies can be measured by a method known in the art.

Flow cytometry analysis was further performed to define the subpopulation adaptable for cell infusion into the anterior chamber. The phenotypes of cHCECs were examined with respect to CD166, CD133, CD105, CD90, CD44, CD26, CD24, HLA-ABC, HLA-DR/DP/DQ and PD L1. At the same time, the inventors confirmed the expression of these markers in freshly extracted corneal tissues and found that they were negative for CD133, CD105, CD90, CD44, CD26, CD24, and HLA-DR/DP/DQ (Fig. 6-B). Based on this immunocytological study, the inventors defined subpopulations with CD133, CD105, CD90, CD44, CD26, CD24, and HLA-DR/DP/DQ negative and CD166 and HLA-ABC positive as effector cells ensuring the application to cell infusion therapy. A representative immunocytological staining is depicted in Fig. 6-B. In the inventors' preliminary, but extensive experiments, fresh human corneal endothelium showed no sign of the presence of CD24, CD26, CD44, CD90 and CD133, but clearly exhibited strongly expression of CD166. This indicates that the phenotypes of aforementioned cHCEC subpopulation, with complete absence of aneuploidy, are consistent with those of HCEC subpopulation in fresh corneal endothelial tissues.

### Highly purified CD44-subpopulation elicited phenotype without CST

CD44 contributes to the maintenance of stem cell features, and the functional contribution of CD44 relies on its communication skills with neighboring molecules, adjacent cells and the surrounding matrix. Accordingly, CD44 negative (gate B in Fig. 7) and positive (gate A) subpopulations were sorted by BD FACSJazz cell sorter and purified subpopulations were cultured in 24 well plates followed by additional 17 days of culture. The subpopulation obtained as a CD44+ subpopulation proliferated rapidly with spindle like morphology and it showed weak irregular staining of Na⁺/K⁺ ATPase, whereas a subpopulation obtained as a CD44- subpopulation showed relatively slow growth and evident expression of Na⁺/K⁺ ATPase (Fig. 7). The results further support the newly introduced concept of the presence of an effector cell in subpopulations suitable for cell infusion therapy.

### Discrimination of subpopulations by PCR array for ECM

### Expression of endothelial cell mRNAs from effector cells and two other subpopulations with CST were investigated using the RT² Profiler PCR-Array Human Extracellular Matrix and Adhesion Molecules (Qiagen). The contrasting features among three typical cHCEC subpopulations were confirmed. The cluster (heat map) analysis demonstrated the clear distinction among these three subpopulations as shown in Fig. 8, again indicating the presence of effector subpopulations with distinct EMA gene signatures.

### Proof of presence of specific subpopulation as effector cells in the context of CD marker

Cell surface markers for CD44- and CD44+ cHCECs were assessed by screening for the expression of 242 cell surface antigens by flow cytometry (Lyoplate, BD Biosciences). The expression profiles of CD markers are shown in Fig. 9 and Table 2.

**[Table 2]**

| **Specificity** | **Functional cell** | **Unintended cell** |
|---|---|---|
| **CD59** | +++ | +++ |
| **CD147** | +++ | +++ |
| **CD81** | +++ | +++ |
| **CD73** | +++ | ++ |
| **CD49c** | +++ | ++ |
| **CD166** | +++ | +++ |
| **CD56** | ++ | + |
| **CD54** | ++ | ++ |
| **B2-uGlob** | ++ | ± |
| **CD47** | ++ | + |
| **CD46** | ++ | ++ |
| **CD141** | ++ | ++ |
| **CD151** | ++ | + |
| **CD98** | + | + |
| **CD165** | + | + |
| **CD340(Her2)** | + | ± |
| **CD58** | + | ± |
| **CD201** | + | ± |
| **CD140b** | ± | - |
| **EGF-r** | + | ± |
| **CD63** | + | ± |
| **CD9** | ± | ++ |
| **CD49b** | ± | + |
| **CD227** | ± | - |
| **CD90** | ± | ++ |
| **CD44** | ± | +++ |

The value of median fluorescence intensity of each marker/median fluorescence intensity of negative control (staining by isotype control antibody) is 30 or greater: +++
10 or greater and less than 30: ++
5 or greater and less than 10: +
less than 5: -

The markers exhibiting low level expression in both groups are not shown. The protein expression of CD73, CD26, CD105 were only observed in CD44+ subpopulations, but completely absent in CD44- subpopulations. On the other hand, CD166, which was used as a representative marker for an HCEC derived cell, was observed in most subpopulations, irrespective of the expression intensity of CD44. The observation was consistent with results of Okumura et al. describing that CD166 was expressed in both normal and fibroblastic cells. CD markers effective for distinguishing distinct subpopulations were selected by analyzing those on established cHCECs with small means cell area and the high cell density. Combined analysis of CD markers clearly identifies the subpopulation (effector cells) adaptable for therapy.

### Factors of this Example influencing the proportion of effector cells in cHCECs

In the absence of a practical scientific index to define subpopulations, the only way to qualify variations from cultures to cultures is microscopic observation of morphology. However, the inventors have developed a method to quantify the proportion of effector subpopulation in cultures (E-Ratio). The method clarifies the relation of donor ages, donor endothelial cell density and the time period between death to preservation time (D-P), with the ratio of effector cells (E-Ratio) in cHCECs (Fig. 10-A). It turned out that only the donor age has a statistically significant correlation with E-Ratio.

### Factors affecting E-Ratio

As mentioned above, CD44 plays diverse critical roles in CST, in maintenance of stem cell features, and in induction of a cancer stem cell (CSC). It is thus important to investigate factors determining the CD44 expression on cHCECs. During extended culture in primary culture, CD44 expression gradually decreased (Fig. 11-A), indicating the linkage of the reduction of CD44, with the progression to a mature differentiated type. Even at the 6th passage, the addition of Y-27632 throughout 35 days of culture strikingly increased the E-Ratio from 1.2% to 52.3%, although no morphological change could be recognized. The addition of Y-27632 during the culture for 47 days also increased the E-ratio. This was also confirmed with the clear narrowing of the distribution of cell areas, decrease of the average cell area from 258 to 216 .micro.m², and the increase of cultured cell density from 2229 to 2582 cells/mm² (Fig. 12 (A-B)).

### (Summary)

Flow cytometry analysis identified the effector cell expressing CD166+CD105-CD44-CD24-CD26-, but not CD200-. The presence of other subpopulations with CD166+CD105-CD44+++ (one of CD24 and CD26 is + and other is -) were also confirmed. PCR array revealed the three completely distinct expression profiles of ECM. Some of these subpopulations expressed ZO1 and Na⁺/K⁺ ATPase at comparable levels with effector cells, while only one of these subpopulations expressed CD200, but not on effector cells. HLA expression was reduced in the effector subpopulation. The proportion of effector cells (E-ratio) was inversely proportional to the age of donors and decreased during extended passages of cultures. The presence of ROCK inhibitor increased the E ratio in cHCECs. The average area of effector cells was about 200-220 .micro.m², and the cultured cell density exceeded 2500 cells/mm².

### (Conclusions)

The specified cultured effector cells sharing the surface phenotypes with matured HCECs in corneal tissues may serve as an alternative to donor corneas for the treatment of corneal endothelial dysfunctions.

Cultured cells varied not only morphologically, but also greatly in terms of the composition of subpopulations in cHCECs from cultures to cultures (Table 1G) even under ideal culture protocol. This may be ascribed to the variation in donor age (Senoo, T., Joyce, N.C., 2000. Invest. Ophthalmol. Vis. Sci. 41, 660e667; Zhu, C., Joyce, N.C., 2004. Invest. Ophthalmol. Vis. Sci. 45, 1743e1751) as well as to the difference in the number of culture passages, while this was also the case for mesenchymal stem cell (MSC) culture in regard to stem cell markers CD29, CD49e, CD73, CD90, CD105 and CD166. Cornea donor ages exhibited a significantly inverse correlation with the frequency of effector cells (Fig. 10-A), but a positive correlation with karyotype aneuploidy (Miyai T, et al. Mol Vis. 2008; 14:942-50).

No HCEC specific cell surface markers have been defined prior to the disclosure of the present invention. A combination of CD markers to quantitatively assess high quality subpopulations had not been so far provided. The inventors applied the combination of CD markers related to cancer stem cells (CSCs) for clinical use due to frequent EMT occurrences in HCEC cultures.

A multifunctional CD44 regulates diverse functions in many cells including stem cell behavior such as self-renewal and differentiation and detects changes in ECM in response to changes in cell-cell and cell-ECM interactions, cell trafficking, homing and signal transduction events, enabling the flexible responses to tissue environment (Karamanos NK, et al. FEBS J. 2011; 278:1429-43; Williams K et al., Exp Biol Med (Maywood). 2013; 38:324-38). It was revealed that an effector cell can be identified by the expression of CD44-CD166+CD133-CD105-CD24-CD26-, but not by the expression of CD200. The presence of other subpopulations with CD44+ to +++ CD166+ CD133-CD105- (one of CD24 and CD26 is + and the other is -) was also confirmed. In addition, depending on the culture condition variations, such as the presence of Y 27632 and the extension of culture periods, CD44 expression was likely reduced, resulting in the increase of E-ratios. Future study is required to determine the role served by CD44 in differentiation pathways to matured HCECs. Downstream signaling factors of CD44 include RhoA and MMP 2, which are required for the organization of tubulin and actin cytoskeleton and the formation of cellular pseudopodia (Lin L, et al., Oncol Rep. 2015; 34:663-72).

EMT is abnormally induced in many diseases. Cultured HCECs have an inclination towards CST into EMT. Through the process of EMT, cell-to-cell adhesion is reduced. A cell undergoing EMT frequently gains a stem cell-like property, including that induced by TGF-beta. It is well known that EMT is closely involved in the generation of cancer stem cells or their niches during phenotypic conversion (Krawczyk N, et al., Biomed Res Int. 2014; 2014:415721. Epub 2014 May 8). This had prompted the inventors to discover that the presence of heterogeneous subpopulations in term of CD44 can be investigated and distinguished.

Considering the phenotypic heterogeneity due to genetic instability of cHCECs, the selection of a combination of markers including CSC markers may allow appropriate assessment of the quality of the subpopulation most suitable for cell therapy.

McGowan et al reportedly have successfully identified corneal endothelial stem cells, but they were never isolated for culture (McGowan, S.L., et al., Mol. Vis. 13, 1984e, 2000).

CD44 is the hallmark feature that distinguishes differentiated cHCECs from either undifferentiated cHCECs or cHCECs that have undergone CST. CD44 plays a critical role as a major adhesion molecule of ECM in the TGF-beta mediated mesenchymal phenotype induction. Loss of CD44 abrogated these changes (Nagano O, et al., Cancer Sci. 2004; 95:930-935). Loss of CD44 increases the flux to mitochondrial respiration and inhibits entry into glycolysis. Such metabolic changes induced by loss of CD44 results in notable depletion of reduced glutathione (Tamada M et al., Cancer Res. 2012; 72:1438-48). HDAC1 regulates the activation of miRNA-34a/CD44 axis and the downstream factors of CD44 including RhoA and MMP 2 (Lin L, et al., Oncol Rep. 2015; 34:663-7245).

This study demonstrated that cHCECs are comprised of various cells, and a specific cHCEC subpopulation with surface expression of CD44-, CD166+, CD133-, CD105-, CD24-, CD26- can be reproducibly cultured without CST or EMT. The combination of CD markers described above can qualitatively assess subpopulations showing the inclination to mitochondria dependent OXHOS, but not anaerobic glycolysis. Thus identified subpopulation shows no sign of karyotype abnormality, thereby open the way to provide the cultured cells in a safe and stable manner for therapy, namely infusion of cHCECs into the anterior chamber in the form of a cell suspension for treating bullous keratopathy.

### (Example 2: Aneuploidy of cultured human corneal endothelial cells is dependent on the presence of heterogeneous subpopulations with distinct differentiation phenotypes)

The purpose of this Example is to clarify whether a specific subpopulation (SP) in heterogeneous cHCECs would exhibit aneuploidy while others do not.

In this Example, subpopulations present in cHCECs were analyzed based on surface CD antigen expression levels by flow cytometry. The analyzed CD antigens are CD166, CD105, CD44, CD26 and CD24. Cytogenetic examination was performed for 23 lots of cHCECs, either as whole cell preparations (bulk) consisting of some cell subpopulations or as a semi-purified subpopulation by magnetic bead cell sorting (MACS) with distinct surface CD markers. The donors of HCECs ranged from 9 to 69 years old and the culture passages used were from primary to fifth passage.

### (Materials and Methods)

### Human corneal endothelial cell donors

The human tissue used was handled in accordance with the ethical tenets set forth in the Declaration of Helsinki. HCECs were obtained from 23 human cadaver corneas and were cultured before performing karyotype analysis. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the state in which the donor consent was obtained and the tissue was recovered.

The donor ages ranged from 9 to 69 years. The donors included 14 males and 7 females. All donor corneas were preserved in Optisol-GS (Chiron Vision, Irvine, CA, USA) and imported by airplane for research purposes. According to the donor information, all donor corneas were considered healthy with no corneal disease and all donors had no past history of chromosomal abnormality.

### Cultures of HCECs

The HCECs were cultured according to published protocols, with some modifications (Nayak SK, Binder PS. Invest Ophthalmol Vis Sci. 1984; 25:1213-6).A total of 30 human donor corneas at distinct ages were used for the experiments. Briefly, the Descemet's membranes with the corneal endothelial cells were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. Conditioned medium was prepared as previously described (Nakahara, M. et al. PLOS One (2013) 8, e69009). The HCECs were cultured using the conditioned medium at 37.degrees.C in a humidified atmosphere containing 5% CO_{2.} The culture medium was changed twice a week. The HCECs were subcultured at ratios of 1:3 using 10x TrypLE Select (Life Technologies) for 12 minutes at 37.degrees.C when they reached confluence. The HCECs at passages 2 through 5 were used for all experiments.

### Flow Cytometry

Screening of cell surface markers was conducted by assessing the expression of markers through the Human Cell Surface Marker Screening Panel (BD Biosciences, San Jose, CA, USA) according to the manufacturer's protocol. Briefly, cultured HCECs were incubated with 242 primary antibodies and isotype IgGs (BD Biosciences) at the dilution indicated by the manufacturer's protocol for 30 minutes at 4.degrees.C. The cells were washed with PBS containing 1% BSA and 5 mM EDTA and then incubated with AlexaFluor 647-conjugated secondary antibodies (1:200 dilution, BD Biosciences) for 30 minutes at 4.degrees.C. The cells were washed again with PBS containing 1% BSA and 5 mM EDTA and analyzed by flow cytometry using a BD FACSCant II instrument (BD Biosciences) and Cell Quest Pro software (BD Biosciences).

### Flow cytometry analysis of cultured HCECs

cHCECs were collected from the culture dish by TrypLE Select treatment as described above and suspended at a concentration of 4 x 10⁶ cells/mL in FACS buffer (PBS containing 1% BSA and 0.05% NaN₃). An equal volume of antibody solution was added and incubated for 2 hours at 4.degrees.C. The antibody solutions comprised the following: FITC-conjugated anti-human CD26 mAb, PE-conjugated anti-human CD166 mAb, PerCP-Cy5.5 conjugated anti-human CD24 mAb, PE-Cy7-conjugated anti-human CD44 (all from BD Biosciences), APC-conjugated anti-human CD105 (eBioscience, San Diego, CA, USA). After washing with FACS buffer, the HCECs were analyzed with FACS Canto II (BD Biosciences).

### Isolation of HCEC subpopulations by MACS

The HCECs were detached with TrypLE Select as described above and CD44-HCEC subpopulations (effector subpopulation) were isolated using anti-human CD44 microbeads (Miltenyi Biotec, Bergisch Gladbach, Germany) and the program depl05 of an autoMACS Pro separator (Miltenyi Biotec). The purity of the isolated effector subpopulation was higher than 95% in all cases as demonstrated by flow cytometry.

### Karyotyping

Cytogenetic examination was performed at several passaged cells from 21 donors as shown in Table 3. In the early stage of this study, cytogenetic examination was performed only for the bulk culture cells without cell sorting. Standard cytogenetic harvesting, fixation, and G-bands after trypsin and Giemsa (GTG banding) techniques were used for HCECs. After incubation with 0.06 .micro.g/ml colcemid for 16 hours to arrest cell mitosis, HCECs were detached using 0.05% trypsin/ EDTA. HCECs were treated with 0.075 M KCl and then fixated with Carnoy's fixative (a 3:1 mixture of methanol and glacial acetic). HCEC solution was dropped onto a slide glass and air-dried. HCECs were then treated with 0.005% trypsin for 7 minutes and stained with 6% Giemsa stain solution for 3.5 minutes. The number of chromosomes was analyzed in 50 cells for each HCEC preparation. A detailed karyotype was analyzed in 20 cells for each HCEC preparation. The standard International System for Human Cytogenetic Nomenclature (ISCN) 1995 and definitions thereof were followed. The frequency of loss or gain of individual chromosomes was examined. The frequency of aneuploidy per sample (the number of abnormal cells divided by the total number of cells examined at metaphase) was tested. All analyses were carried out in Nippon Gene Research Laboratories (NGRL Sendai, Japan).

### (Results)

### General features of karyotypes of cHCECs

The karyotypes of 21 cultured HCECs from donors between 9 and 69 years of age were analyzed. Cultured cells varied not only morphologically, but also greatly in terms of the composition of subpopulations in cHCECs in size and morphology from cultures to cultures despite identical culture protocol. This may be ascribed to the variation in donor age as well as to the difference in the number of culture passages. Cornea donor ages exhibited a significantly inverse correlation with the frequency of effector cells, but a positive correlation with karyotype aneuploidy (Miyai T, et al. Mol Vis. 2008; 14:942-50).

In the early stage of this Example, cytogenetic examination was performed only for the bulk culture cells without cell sorting. The number of chromosomes was analyzed in 50 cells for each HCEC preparation. A detailed karyotype was analyzed in 20 cells for each HCEC preparation (Fig. 13).

As reported previously, HCECs show both sex chromosome loss and trisomy, which were also observed in this study (Fig. 13, Table 3).

**[Table 3]**

| Let #. | Age | Gender | Passage | Donor ECD (cells/mm²) | Abnormality | | | | |
|---|---|---|---|---|---|---|---|---|---|
| | | | | | | 45 | 46 | 47 | 48 |
| 1 | 69 | male | 6 | 3161 | No nuclear staining | | | | |
| 2 | 63 | male | 4 | 3265 | Deletion of Y chromosome (100%) | 50 | - | - | - |
| 3 | 68 | male | 1 | 2830 | Deletion of Y chromosome (98%) | 49 | 1 | - | - |
| 4 | 67 | female | 0 | 2718 | Trisomy on chromosome 20 (34%) | - | 33 | 17 | - |
| 5 | 69 | male | 0 | 2654 | Deletion of Y chromosome (100%) | 50 | - | - | - |
| 6 | 56 | male | 0 | 2612 | Deletion of Y chromosome (98%) | 49 | 1 | - | - |
| 7 | 67 | female | 3 | 2718 | No nuclear staining | | | | |
| 8 | 23 | male | 3 | 3504 | Few mitosis, analysis on 14cells | - | 14 | - | - |
| 9 | 23 | male | 2 | 3194 | Deletion of Y chromosome (36%) | 18 | 32 | - | - |
| 10 | 58 | female | 3 | 2630 | | - | 50 | - | - |
| 11 | 29 | female | 3 | 3007 | Deletion of X chromosome (22%) | 11 | 39 | - | - |
| 12 | 9 | male | 5 | 3507 | | - | 50 | - | - |
| 13 | 42 | male | 4 | 3111 | Trisomy on chromosome 7 (79%) | - | 26 | 24 | - |
| 14 | 15 | female | 3 | 3733 | | - | 50 | - | - |
| 15 | 22 | female | 3 | 2813 | Trisomy on chromosome 6, 20 (18%) | - | 41 | 8 | 1 |
| 16 | 16 | male | 2 | 3433 | | - | 50 | - | - |
| 17 | 28 | female | 3 | 3504 | | - | 50 | - | - |
| 18 | 19 | male | 3 | 3062 | | - | 50 | - | - |
| 19 | 23 | male | 2 | 3833 | Translocation (5%), | - | 50 | - | - |
| 20 | 29 | female | 3 | 3413 | | - | 50 | - | - |
| 21 | 29 | female | 3 | 3309 | Trisomy on chromosome 12(4%) | - | 48 | 2 | - |

Karyotyping of 21 cHCEC bulk culture from donors between 9 and 69 years of age was analyzed in regard to culture passages and donor variations. The donor information is summarized together with the results of karyotype analysis (Table 3). No description means the insufficient proliferation to obtain metaphase cHCECs before cytogenetic examination. As mentioned above, cHCECs exhibited the same frequent of karyotype abnormalities such as sex chromosome loss and trisomy as the report of Miyai et al. However, translocation was seen in only one (donor #13) of the aforementioned 21 cases, which was not observed in the previous study. In the current study, bulk cultured HCECs demonstrated aneuploidy in most cases, regardless of the number of passages from primary culture to fifth passages. This does not exclude the conclusion of previous studies because the number of cell culture passages in the current study was limited to at most 5 passages. Although statistical analysis was not carried out, donor corneal endothelial cell density (ECD) did not directly affect the frequency of aneuploidy. In contrast to the ECD of donors, the lower cell density of cHCECs at the time of analysis may have some correlation with the frequency of aneuploidy. Among 8 cHCECs with a cell density below 1000 cells/mm², 7 exhibited evident aneuploidy (almost reaching 90 %). Conversely, none of the four cHCECs with a cell density greater than 1000 cells/mm² exhibited abnormality. This indirectly indicates that the quality or efficiency of cultures of cHCEC or the presence of CST may have correlation with the observed aneuploidy.

### Donor age and karyotype aneuploidy

In line with the previous report (Mimura T et al., Invest Ophthalmol Vis Sci. 2004; 45: 2992-299710), the frequency of aneuploidy showed a clear inverse correlation with the age of HCEC donors. 10 of 14 HCECs from young donors (incidentally defined as those below 29) exhibited normal karyotype (71%), while the remaining 4 exhibited either sex chromosome loss or trisomy. In cases of HCECs from donors 30 to 69 years of age, 6 out of 8 exhibited aneuploidy (75%) with only 25% exhibiting normal karyotypes. Considering the generally accepted concept of aging increasing genetic instability and decreasing expression of DNA repair genes, the increase of the aneuploidy is conceivably appropriate.

However, the most relevant issue is the presence of karyotype abnormality in 29% of cHCECs from young donors under the age of 29. Figs. 14a-14d show typical examples of karyotyping. Aneuploidy is absence in cHCECs from elder donor #10 (age 58, female, culture passage P3), from young donors #19 (age 23, male, P2), #9 (age 23, male, P2) and #14 (age 15, female P3). The presence of karyotype abnormality in 29% of cHCECs from young donors under the age of 29 indicates that some unknown intrinsic factors latent in HCEC cultures may facilitate karyotype aneuploidy, either irrespective of or in addition to donor age. In this regard, the inventors have investigated the composition of cHCECs in detail.

### Analysis of subpopulations in cHCECs by flow cytometry

cHCECs were heterogeneous in size and morphology and composed with distinct subpopulations depending on the culture conditions (Example 1). To explore unknown intrinsic factors latent in HCEC cultures, flow cytometry analysis were applied to cHCECS in the context of surface CD antigen markers. The cHCECs with high content of CD44- subpopulation exhibited hexagonal morphology and no sign of CST, whereas cultures containing subpopulations with either CD44+++ , CD24+ or CD26+ expression exhibited irregular CST-like morphology (Figs. 15 and 16). It is evident from this analysis that the phenotypic features of cHCECs vary greatly from cultures to cultures, accompanied with the variation in composite subpopulations in cHCECs. This variance may exceed the influence of factors described above such as the age of donor, the number of passages of the donor ECD in regard to the frequency of aneuploidy in cHCECs.

### Composition of cHCECs greatly affects aneuploidy

The cHCEC subpopulation with CD44-, CD166+, CD105-, CD24-, CD26- was purified by magnetic bead cell sorting (MACS). Due to the heterogeneous expression of CD44 in subpopulations shown in Fig. 16, CD44 magnetic beads were mainly used to separate subpopulations. CD44-, CD166+, CD105-, CD24-, and CD26-subpopulations separated by CD44 magnetic beads were semi-purified to a purity over 90%. The subpopulations either with expression of (CD44+++, CD166+, CD24-, CD26+) or (CD44+++, CD166+, CD24+, CD26-) were also semi-purified to a purity over 70%.

As shown in Fig. 17, the first subpopulation with CD44-, CD166+, CD105-, CD24-, CD26- did not show any aneuploidy in 150 cells. To the contrary, subpopulations with CD44+++, CD166+, CD24-, CD26+ elicited the loss of sex chromosome in 100% of cells (60 cells), whereas subpopulations with CD44+++, CD166+, CD24+, CD26-exhibited frequent trisomy on chromosomes 6, 7, 12 and 20.

Cornea donor ages and the frequency of CD44-, CD166+, CD105-, CD24-, CD26-subpopulations had a significantly inverse correlation, whereas Cornea donor ages had a positive correlation to karyotype aneuploidy.

### (Summary)

Flow cytometry analysis demonstrated the presence of at least three cHCECs subpopulations. A specific cHCEC subpopulation, purified by MACS, with surface expression of CD166+, CD105-, CD44-, CD24-, CD26- did not exhibit any kind of aneuploidy in 150 cells. In contrast, CD166+, CD44+++, CD24-, CD26+ cHCEC subpopulation exhibited the loss of sex chromosome in 100% of cells (60 cells), whereas CD166+, CD44+++, CD24+ ,CD26- subpopulation exhibited, albeit partly, trisomy on chromosomes 6, 7, 12 and 20.

Conclusion: This Example presents new findings that the observed aneuploidy during HCEC culture is closely linked to specific subpopulations present in cHCECs, and only a specific subpopulation sharing the surface phenotype with matured HCECs present in corneal tissues does not exhibit the karyotype abnormality.

### Discussion

Recent progress has raised the possibility of new therapeutic modalities based on tissue engineering techniques for various diseases (Okano H Nakamura M Yoshida K. Circ Res. 2013; 112: 523-533., Tabar V Studer L. Nat Rev Genet. 2014; 15: 82-92.) cHCECs expanded in in vitro culture can be a mixture of various subpopulations with distinct CST. This has been an obstacle for clearly defining the features of cHCECs. Since HCECs can be grown in culture, injection therapy of cHCECs to treat corneal endothelial dysfunctions has been explored. A cultured cell has a potential risk of undergoing karyotype changes in general. Thus, the quality of cHCECs should be carefully monitored for clinical settings, where safety and stability of medication must be strictly controlled.

Currently, no HCEC specific cell surface antigen has been reported. Glypican-4 and CD200 were proposed as HCEC markers to distinguish HCECs from corneal stromal fibroblasts (Cheong YK et al., Invest Ophthalmol Vis Sci. 2013; 54: 4538-4547). However, the practical problem of HCEC culture is the presence of vulnerable subpopulations that have undergone CST which is clearly distinct in cHCECs. CD200 reported by Cheong et al. could not distinguish differentiated HCECs, but was expressed rather on the subpopulation of cHCECs that had undergone certain CST. It was revealed that conventional CD markers can distinguish non-fibroblastic phenotype cells retaining normal functions from a cell that has undergone fibroblastic changes, but struggle to identify the functional mature differentiated corneal endothelial cell of the present invention. This Example clearly showed the presence of subpopulations in non-fibroblastic cells with karyotype abnormality. To identify the quality of cHCECs that is sufficient for clinical settings, more detailed analysis would be needed to distinguish subpopulations that have undergone CST other than fibroblastic changes in cHCEC. The aim of this study is to identify the cell-surface CD antigens expressed on cHCEC subpopulations with or without aneuploidy to clarify whether aneuploidy currently observed in cHCECs is dependent on the presence of subpopulations with distinct differentiation phenotypes.

In this Example, cultured HCECs exhibited aneuploidy in most cases, despite the use of cells from primary culture to fifth passages. The aneuploidy observed in cHCECs may have been induced by cell division in culture. Consistent with the observation of Miyai et al. (Miyai T, et al., Mol Vis. 2008; 14:942-50), most abnormal karyotypes observed in cHCECs may have been induced at a very early stage of culture, because of the presence of abnormalities even in cells of primary culture (#4, #5, and #6 in Table 3). Although statistical analysis has not been carried out, donor age and the frequency of aneuploidy may have a significantly positive correlation as pointed out by Miyai et al.

In this Example, cHCECs tended to have a mosaic of sex chromosome monosomy and chromosome 6, 7, 12 and 20 trisomy. Previous studies reported that sex chromosomes showed age-dependent loss in peripheral lymphocytes, bone marrow cells, corneal keratocytes [Stone JF, et al., Mutat Res. 1995; 338: 107-13; Pettenati MJ, et al., Hum Genet. 1997; 101:26-9], and corneal endothelia. Thus, loss of sex chromosomes is an age-dependent phenomenon that is not dependent on the cell type. The results in this Example is very consistent with the results of the studies of Miyai et al (supra). at least in term of the loss of a sex chromosome, but not in regard to the presence of trisomy on chromosomes 6, 7, 12 and 20, because the previous report describes only chromosome 8 trisomy. Chromosome 8 trisomy mosaic syndrome is associated with corneal opacity [Miyata K, et al., Cornea. 2001; 20:59-63]. Further detailed study is required to elucidate this discrepancy in the location of trisomy. The results in this Example also indicate that cHCECs for clinical therapies should be obtained from young donors. Moreover, the results show that careful examination of karyotype in cHCECs is crucial before clinical application.

Flow cytometry analysis demonstrated that a specific cHCEC subpopulation, semi-purified by MACS, with expression of surface phenotypes of CD166+, CD105-, CD44-, CD24-, and CD26- did not show any kind of aneuploidy in 150 cells. Even in this subpopulation, the presence of CD90+ and - subpopulations was indicated by further analysis of the inventors, but it does not appear to affect the result of karyotyping. This Example is the first finding directly indicating the presence of cHCEC subpopulation without karyotype aneuploidy, making it possible to adapt the subpopulation for clinical settings. In contrast, cHCEC subpopulation with the surface expression of CD166+, CD44+++, CD24-, CD26+ exhibited loss of sex chromosome in 100% of cells, while subpopulation with the surface expression of CD166+, CD44+++, CD24+, CD26- exhibited, albeit partly, trisomy on chromosomes 6,7,12 and 20. In preliminary, but extensive experiments by the inventors, a fresh human corneal endothelium showed no sign of the presence of CD24, CD26, CD44 or CD90, but uniformly expressed CD166. This indicates that the phenotypes of cHCEC subpopulation shown herein without aneuploidy appears consistent with those of HCEC subpopulations in fresh corneal endothelium tissues.

This Example presents new findings that aneuploidy present in cHCEC culture reported thus far occurs in limited subpopulations of cHCECs in a very limited scale, but the biochemically refined subpopulation sharing the surface phenotype with mature differentiated HCECs, present in fresh corneal tissues, is free of karyotype abnormality.

### (Example 3: Production of Homogeneous Cultured Human Corneal Endothelial Cells Indispensable for Cell Infusion Therapy)

The Example of this study is to establish culture protocols to reproducibly produce at most a homogeneous subpopulation (SP) with matured HCEC functional characteristics and in devoid of CST for cell therapy.

In this Example, the presence of subpopulations in cHCECs was confirmed in term of surface CD marker expression level by flow cytometry. Analysis was performed on CD markers that can specify definitively the subpopulation (effector cells) of interest most suitable for cell therapy among diverse subpopulations. The culture processes were assessed in the context of the proportion of effector cell subpopulation (E-ratio).

### (Materials and Methods)

### (Reagents & antibody)

HCECs were collected from the culture dish by TrypLE Select treatment as described above and suspended at a concentration of 4 x 10⁶ cells/mL in FACS buffer (PBS containing 1% BSA and 0.05% NaN₃). An equal volume of antibody solution was added and incubated for 2 hrs at 4.degrees.C. The antibody solutions were prepared by mixing the following antibodies: FITC- or PE-conjugated anti-human CD26 mAb, PE-conjugated anti-human CD166 mAb, PerCP-Cy5.5 conjugated anti-human CD24 mAb, PE-Cy7 or PerCP-Cy5.5-conjugated anti-human CD44 (all from BD Biosciences), and APC-conjugated anti-human CD105 (eBioscience, San Diego, CA, USA). After washing with FACS buffer, the HCECs were analyzed with FACS Canto II (BD Biosciences).

### Human corneal endothelial cell donors

The human tissue used was handled in accordance with the ethical tenets set forth in the Declaration of Helsinki. HCECs were obtained from 20 human cadaver corneas and were cultured before performing karyotype analysis. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the particular state in which the donor consent was obtained and the tissue was recovered. The donor age ranged from 2 to 75 years (average 43.7+/-26.4). Nine males and 11 females were included. All donor corneas were preserved in Optisol GS (Chiron Vision, Irvine, CA, USA) and imported by airplane for research purposes. The donor information showed that all donor corneas were considered healthy without corneal disease and all donors had no past history of chromosomal abnormality.

### Cell Cultures of HCECs

Unless noted otherwise, the HCECs were cultured according to published protocols, with some modifications. A total of 30 human donor corneas at distinct ages were used for the experiments. Briefly, the Descemet's membranes with corneal endothelial cells were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. A conditioned medium was prepared as previously described (Nakahara, M. et al. PLOS One (2013) 8, e69009). The HCECs were cultured using the conditioned medium at 37.degrees.C in a humidified atmosphere containing 5% CO₂. The culture medium was changed twice a week. The HCECs were subcultured at ratios of 1:3 using 10x TrypLE Select (Life Technologies) for 12 minutes at 37.degrees.C when they reached confluence. The HCECs at passages 2 through 5 were used for all experiments.

### Trichostatin A (TSA) treatment of HCECs

HCECs with treated with Trichostatin A as follows. The Descemet's membranes with the corneal endothelial cells were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The Trichostatin A-containing medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), 50 .micro.g/mL of gentamicin, and 0.5 .micro.M of Trichostatin A. The HCECs were cultured using the Trichostatin A-containing medium at 37.degrees.C in a humidified atmosphere containing 5% CO₂. The culture medium was changed twice a week. The HCECs were subcultured at ratios of 1:3 using 10x TrypLE Select (Life Technologies) for 12 minutes at 37.degrees.C when they reached confluence. The cells were cultured for 30 days. The HCECs from the second passage were used for observation with a fluorescence microscope.

### Phase Contrast Microscopy

Phase contrast images ware taken by an inverted microscope system (CKX41, Olympus, Tokyo, Japan). For the cell area distribution analysis, the cHCECs were washed with PBS (-) three times and phase contrast images were acquired by a BZ X-700 Microscope system (Keyence, Osaka, Japan). The area distributions were quantified by BZ-H3C Hybrid cell count software (Keyence).

### Immunofluorescent Staining

The HCECs were cultured at a density of 1 × 10⁵ cells/well in a 24-well cell culture plate coated with FNC Coating Mix and were maintained for 3 to 4 weeks for immunofluorescence analysis. Cells were fixed in 4% paraformaldehyde or 95% ethanol supplemented with 5% acetic acid for 10 minutes at room temperature and incubated for 30 minutes with 1% BSA. Samples were incubated overnight at 4.degrees.C with antibodies against CD73 (1:300; BD Pharmingen Stain Buffer), CD166 (1:300; BD Pharmingen Stain Buffer), ZO1 (1:300; Zymed Laboratories, South San Francisco, CA, USA), and Na⁺/K⁺-ATPase (1:300; Upstate Biotec, Lake Placid, NY, USA). After washing with PBS, either Alexa Fluor 488-conjugated goat anti-mouse IgG (Life Technologies) or Alexa Fluor 594-conjugated goat anti-rabbit IgG (Life Technologies) was used as the secondary antibody at a 1:1000 dilution. Nuclei were stained with DAPI (Vector Laboratories, Burlingame, CA, USA). The cells, cultured in a 48-well cell culture plate, were directly examined by fluorescence microscopy (BZ-9000; Keyence, Osaka, Japan).

### Flow cytometry analysis of the cultured HCECs

HCECs were collected from the culture dish by TrypLE Select treatment as described above and suspended at a concentration of 4 × 10⁶ cells/mL in FACS buffer (PBS containing 1% BSA and 0.05% NaN₃). An equal volume of antibody solution was added and incubated for 2 hrs at 4.degrees.C. The antibody solutions comprised the following: FITC-conjugated anti-human CD26 mAb, PE-conjugated anti-human CD166 mAb, PerCP-Cy5.5 conjugated anti-human CD24 mAb, PE-Cy7-conjugated anti-human CD44 (all from BD Biosciences), and APC-conjugated anti-human CD105 (eBioscience, San Diego, CA, USA). After washing with FACS buffer, the HCECs were analyzed with FACS Canto II (BD Biosciences).

### Results

Subpopulation composition variances in cHCECs did not ascertain the culture quality HCECs from donors different in ages were cultured according to the method of Okumura et al (Okumura N, et al., Invest Ophthalmol Vis Sci. 2014; 55: 7610-8), and surface expression of CD44, CD166, CD24, CD26 and CD105 was characterized (Fig. 19). The representative HCECs are summarized in Figs. 18-20, together with the phase contrast microscope pictures. At a first glance, it is quite evident that cHCECs contains a variety of subpopulations. The E-ratio defined by the proportion of subpopulations CD44-CD166+CD24-CD26-CD105- varied (Fig. 19). As described elsewhere, CD44 expression decreased in accordance with the differentiation of cHCECs to matured cHCECs. The presence of subpopulations expressing either CD24 or CD26 in some cultures may indicate the presence of some subpopulations with remarkable karyotype abnormality, such as sex chromosome loss, trisomy or translocation. In this context, most of these cells are not suitable for infusion in clinical settings. The proportion of CD24+ cells ranged up to 54.3%-96.8% and those of CD26+ cells up to 44.2%. CD44+++ expressing cells were present at the highest ratios of over 80%. While visible phenotypes were non-fibroblastic, phase contrast microscopy revealed the characteristic polygonal contact-inhibited shape and monolayer. Both ZO1 and Na⁺/K⁺-ATPase that are well known as HCEC markers were surprisingly stained for CD24+, CD26+ or CD44+++ subpopulations. This suggests a possibility that the morphological judgement alone may not be sufficient to distinguish heterogeneous subpopulations present in cHCECs. This prompted the inventors to reassess the culture protocols to precisely assess the culture processes in the context of the proportion of effector cell subpopulation (E-ratio).

### High quality cHCECs can be provided by utilizing TGF-beta signaling

Cultured HCECs have an inclination towards cell state transition (CST) into a senescence phenotype, EMT, and a fibroblastic cell morphology. In view of the pluripotent functions of TGF-beta and its existence in aqueous humours, the inventors postulated that TGF-beta may function to interfere with the acquisition of an invasive phenotype into the extracellular matrix (ECM). To the contrary, TGF-beta can promote and maintain EMT in a variety of biological and pathological systems. However, the same growth factor is the key signaling molecule for EMT, and the role of TGF-beta as a key molecule in the development and progression of EMT is well studied (Wendt MK, et al., Future Oncol 5: 1145-1168). In this regard, it was conjectured that the functionality of mature differentiated corneal endothelial cells can be maintained or enhanced by maintaining TGF-beta signaling. For this reason, examination of culture results under TGF-beta signaling inhibitor-free conditions made it possible to confirm that the functionality of cells within the human cornea is maintained (Fig. 22-A). Further, HCECs, when cultured in a medium supplemented with a histone deacetylase (HDAC) inhibitor Trichostatin A, can be promoted to differentiate into mature cHCECs (Fig. 22-D).

### The seeding cell density influences the E-ratios in cHCECs

As shown in this Example, the quality of cHCECs can be monitored mostly by the E-ratio and the proportion of CD44+++ cells. Before finalizing the protocol of culturing HCECs to the homogeneity for clinical use, the inventors investigated the influence of the seeding cell density on E-ratios and the proportion of CD44+++ cells. To reach the correct conclusion, the inventors investigated three lots of cHCECs, of which E-ratios were different, namely 54.0% (culture passage 2), 77.3% (passage 1) and 93.4% (passage 2). In all groups, higher the seeding cell density lead to less reduction of E-ratio in subsequent passages. The group with high E-ratios before culture exhibited the lowest reduction of E-ratios. The group with E-ratio higher than 90% showed a very sharp increase in the cell number (namely high proliferation rate) at the seeding cell density of 200 cells/mm² and reached a cell density comparable to those of groups started from a seeding cell density of 750 and 1000 cells/mm². Surprisingly, the proportion of CD44+++ cells of this group was from 0.7 to 24%, compared with that of 1.2% in the group with a seeding density of 750 cells/mm². However, the increase in the proportion shifting from CD44- to CD44++ was prominent in the groups with a lower seeding cell density.

### Culture protocol for near-homogeneity of HCECs for clinical use

Taken the observations mentioned above altogether, the culture protocol for maximum homogeneity of HCECs for clinical use was tentatively determined as follows. The condition used was: age of donor is between ages 7 to 29; the seeding cell density after passage 1 is higher than 400 cells/mm²; and a TGF-beta signaling is not inhibited (typically an inhibitor is not used). In addition, the addition of ROCK inhibitor Y-27632 was changed to every three days throughout the entire culture period in order to accomplish efficient differentiation to CD44 subpopulation. Under this culture condition, Y-27632 dramatically induced the differentiation of cHCECs to matured state with reduced expression of CD44. The downstream factors of CD44 include RhoA, the target of Y-27632. The continuous addition of Y-27632 dramatically increased the E-ratios and reduced the proportion of CD24+ or CD26+ subpopulations as illustrated in Figs. 21, 22B, and 22C. E-ratios were about 90 % or higher and the contaminating subpopulations were found scarcely in cHCECs. Under these conditions, cHCECs with high quality were observed at a high frequency, even at fifth passage or from donors who were 57 to 71 years old.

Flow cytometry analysis identifying a CD44-CD166+CD133-CD105-CD24-CD26-effector cells a convenient and reliable method for standardizing the culturing procedures. To ensure reproducible production of cHCECs with E-ratios over 90% and without karyotype abnormality, the donor age was preferred to be under 29. The E-ratios greatly varied from culture to culture, depending on the culturing conditions such as the presence of additives such as a ROCK inhibitor. It was also found that a higher quality mature differentiated corneal endothelial functionality can be maintained or enhanced by utilizing TGF-beta signaling. The seeding cell density during subcultures was also critical for maintaining a high E-ratio for extended passages. The continuous presence of ROCK inhibitor Y-27632 throughout the culture period further improved the E-ratio. The presence of HDAC inhibitor Trichostatin A also improved the E-ratio.

Conclusion: The detailed culture conditions to reproducibly produce cHCECs with high E-ratios were determined to confirm that homogeneous cHCECs with matured functions that are suitable for the treatment of corneal endothelial dysfunction can be provided.

### Discussion

The inventors defined the cHCEC subpopulation which is CD133, CD105, CD90, CD44, CD26, CD24, HLA-DR, DQ negative and CD166, HLA-ABC and PD L1 positive as effector cells ensuring safe and stable application to regenerative medicine. For reproducible production of cHCECs with E-ratios over 90% and with no karyotype abnormality, recommended conditions are the continuous presence of ROCK inhibitor Y-27632 throughout the culturing period, the presence of an HDAC inhibitor Trichostatin A, and no TGF-beta signaling inhibition.

CD44 contribute to the maintenance of stem cell features, and the functional contribution of CD44 relies on its communication skills with neighboring molecules, adjacent cells and the surrounding matrix (Zoller M. Front Immunol. 2015; 6: 1-25). CD44 is the hallmark feature to distinguish differentiated cHCECs from either undifferentiated cHCECs or cHCECs that have undergone CST. Thus, investigation of the factors regulating the levels of CD44 expression on cHCECs is deeply related to the determination of culture protocols to obtain the final product with E-ratio over 90%. A multifunctional CD44 regulates diverse functions in many cells including stem cell behavior such as self-renewal and differentiation and detects changes in ECM in responses to changes in cell-cell and cell-ECM interactions, cell trafficking, homing and signal transduction events, enabling flexible responses to tissue environment (Williams K, et al., Exp Biol Med (Maywood). 2013; 38: 324-38). Downstream signaling factors of CD44 include RhoA and MMP 2, which are required for the organization of tubulin and actin cytoskeleton and the formation of cellular pseudopodia (Lin L, et al., Oncol Rep. 2015; 34: 663-72). Loss of CD44 leads to changes thereof (Nagano O, et al., Cancer Sci. 2004; 95: 930-935). CD44 ablation increased flux to mitochondrial respiration and inhibited entry into glycolysis. The activation of miRNA-34a/CD44 axis regulates the downstream factors of CD44, such as RhoA and MMP 2 (Lin L, et al., Oncol Rep. 2015; 34: 663-72). This signaling pathway may, albeit in part, participate in the reduction of CD44 expression on cHCECs by Y-27632. The inventors previously confirmed the up-regulation of miR29 in parallel with that of CD44 expression among distinct subpopulations, i.e., this miR was the most downregulated in effector cells with a CD44-phenotype among cHCECs subpopulations. Interestingly, this miR reportedly has the EMT promoting effect (Rostas JW 3rd, et al., Mol Cancer. 2014; 13:200).

At the beginning of this Example, cultured HCECs exhibited unstable phenotypes with varying expression of CD44 and CD24 with CST (Figs. 18-20) and elevated levels of MMP2.

As mentioned above, CD44 is also associated with the miR34/RhoA/MMP2 pathway. The only miR capable of distinguishing a CD44- effector cell from CD44++ to CD44+++ subpopulations was identified as miR34a.

The detailed culture protocols to reproducibly produce cHCECs with high proportion of effector cells were developed by utilizing the recently developed index E-ratio, thereby enabling the provision of cHCECs with matured functions to serve as a cell preparation for cell injection therapy for tissue damage to a corneal endothelial cell due to Fuchs endothelial corneal dystrophy, trauma, or surgical intervention.

According to the findings obtained in relation to the manufacturing method of this Example, an undifferentiated proliferative cell differentiates by actin depolymerization to be a functional mature differentiated cell (effector cell), and dedifferentiates to be an undifferentiated proliferative cell. Meanwhile, when the cell becomes fibroblastic or senescent, the cell becomes a senescent resting state cell or fibroblastic cell. Such a cell can be converted into a functional mature differentiated corneal endothelial cell by subjecting to culturing under conditions where a cell that has undergone epithelial-mesenchymal transition-like transformation grows, matures, and differentiates again.

### (Example 4: Metabolic Plasticity in Cell State Homeostasis and Differentiation of Cultured Human Corneal Endothelial Cells)

The purpose of this Example was to clarify whether cHCECs, heterogeneous in their differentiation state, would exhibit distinctive energy metabolism, aiming to explore a method to correctly sort cHCECs adaptable for regenerative medicine.

The presence of subpopulations in cHCECs was investigated in the context of surface CD marker expression. Metabolic extracts from cHCECs or corresponding culture supernatants of cHCECs with distinctive cellular phenotypes were prepared and analyzed using a capillary electrophoresis (CE)-connected CE-MS/MS system (HMT, CARCINOSCOPE). Concentrations of metabolites were calculated by normalizing the peak area of each metabolite with respect to the area of the internal standard and by using standard curves.

### Materials and Methods

### Human corneal endothelial cell donors

The human tissue used in this Example was handled in accordance with the ethical tenets set forth in the Declaration of Helsinki. HCECs were obtained from 20 human cadaver corneas and were cultured before performing karyotype analysis. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the state in which the donor consent was obtained and the tissue was recovered.

The donor age ranged from 2 to 75 years (average 43.7 +/- 26.4). Males and females were both included. All donor corneas were preserved in Optisol-GS (Chiron Vision, Irvine, CA, USA) and imported by airplane for research purposes. According to the donor information, all donor corneas were considered healthy without corneal disease and all donors had no past history of chromosomal abnormality.

### Cell Cultures of HCECs

Unless noted otherwise, the HCECs were cultured according to published protocols, with some modifications. Human donor corneas at the distinct ages were used for the experiments. Briefly, the Descemet's membranes with the CECs were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. Conditioned medium was prepared as previously described (Nakahara, M. et al. PLOS One (2013) 8, e69009). The HCECs were cultured using the conditioned medium at 37.degrees.C in a humidified atmosphere containing 5% CO₂, and the culture medium was changed twice a week. The HCECs were subcultured at ratios of 1:3 using 10x TrypLE Select (Life Technologies) for 12 minutes at 37.degrees.C when they reached confluence. The HCECs at passages 2 through 5 were used for all experiments.

### Flow cytometry analysis of cultured HCECs

HCECs were collected from the culture dish by TrypLE Select treatment as described above and suspended at a concentration of 4 x 10⁶ cells/mL in FACS buffer (PBS containing 1% BSA and 0.05% NaN₃). An equal volume of antibody solution was added and incubated for 2 hours at 4.degrees.C. After washing with FACS buffer, the HCECs were analyzed with FACS Canto II (BD Biosciences).

### Immunocytochemical assessment of c-Myc expression

HCECs cultured in 6 well plates were washed with PBS and fixed with 4% PFA for 15 minutes, then washed with PBS, followed by treatment with 0.1% TritonX100 in PBS for 5 minutes at room temperature. Blocking was carried out with 1% BSA in PBS for 5 minutes and the cells were stained with rabbit anti-c-Myc antibody (Ab) (Cell Signaling #5605) at 4.degrees.C overnight. After washing with PBS three times, it was stained with Alexa488 conjugated anti-rabbit IgG (1:1000, Invitrogen A1 1034) as secondary Ab. Cell nuclei were stained with DAPI (Vector Laboratories, Inc., Burlingame, CA).

### Fluorescence analysis of 2-NBDG uptake

Glucose uptake by bulk cultured HCECs analyzed by flow cytometry. Briefly, detached cHCECs were incubated with 600 .micro.M 2NBDG for 5, 10, 30 minutes at 37.degrees.C, after culture medium was replaced with fresh glucose deleted culture medium for 15 minutes. The cells were then washed twice with cold FACS buffer (PBS containing 1% BSA), re-suspended in ice-cold FACS buffer and subjected to flow cytometry. Samples were analyzed using BD FACS Canto II (BD Biosciences) at FITC range (excitation 490 nm, emission 525 nm band pass filter). The mean fluorescence intensity of different groups was analyzed by BD FACS Diva software and corrected for auto-fluorescence from unlabeled cells.

### Glucose Starvation

HCECs were cultured according to the procedures described above. The HCECs obtained from a single donor cornea were seeded in one well of a Type I collagen-coated 6-well plate (Corning Inc., Corning, NY, USA). The culture medium was prepared according to published protocols.

Cultured HCECs were exposed, for different time periods, to glucose-depleted culture conditions either supplemented with and without lactate (10mM unless noted otherwise). The exposed HCECs were assessed in terms of morphology, surface markers, and gene expressions of HCEC-related functional markers such as collagen 4A1, 4A2, and 8A2.

### Na⁺/K⁺-ATPase

To assess the partial elimination of subpopulations of cHCECs in bulk cultures, immunohistochemical staining of Na⁺/K⁺-ATPase was performed before and after glucose starvation. Na⁺/K⁺-ATPase was used as a function related marker of the HCECs. The cells were fixed with ice-cold methanol for 10 minutes, and then permealized with PBS(-) containing 0.1 % Triton X-. After blocking of nonspecific reactivity with 1% BSA in PBS(-) for 1 hour at room temperature, the Na⁺/K⁺-ATPase staining was performed with 2 .micro.g/mL of Na⁺/K⁺-ATPase monoclonal antibodies (Millipore, Temecula, CA, USA), followed by Histofine MAX-PO (MULTI) (Nichirei Biosciences, Tokyo, Japan). After washing with PBS (-) containing 0.1 % Triton X-100, cells were developed with Histofine Simple Stain DAB Solution (Nichirei Biosciences) and counterstained with hematoxylin (Merck, Darmstadt, Germany). Finally, cells were mounted with Histofine aqueous mounting medium (Nichirei Biosciences) and observed under a bright field microscope.

### Quantitative RT-PCR

Total RNA was extracted from cultured HCECs using the miRNeasy Mini kit (QIAGEN strasse1 40724 Hilden Germany). The cDNA was synthesized using High Capacity cDNA Reverse Transcription kit with RNase Inhibitor (Applied Biosystems, Foster City, CA, USA). The PCR reactions were performed using TaqMan Fast Advanced Master Mix (Applied Biosystems) and TaqMan Gene Expression Assays, Inventoried (Applied Biosystems), under the following conditions. Each sequence used was a sequence accompanying these products. The primer IDs used are shown below:

**[Table 4]**

| | Assay ID | | Assay ID | | Assay ID |
|---|---|---|---|---|---|
| MMP1 | Hs00899658_m1 | CD44 | Hs01075862_m1 | CDH2 | Hs00983056_m1 |
| MMP2 | Hs01548727_m1 | CD166 | Hs00977641_m1 | VIM | Hs00185584_m1 |
| MMP4 | Hs01128097_m1 | CD105 | Hs00923996_m1 | CDKN1B | Hs01597588_m1 |
| TIMP1 | Hs00171558_m1 | CD24 | Hs03044178_m1 | CDKN1C | Hs00175938_m1 |
| BMP2 | Hs00154192_m1 | COL3A1 | Hs00943809_m1 | IGFBP3 | Hs00365742_g1 |
| SPARK | Hs00234160_m1 | COL4A1 | Hs00266237_m1 | SNAIL1 | Hs00195591_m1 |
| TGFβ1 | Hs00998133_m1 | COL4A2 | Hs01098873_m1 | SNAIL2 | Hs00950344_m1 |
| TGFβ2 | Hs00234244_m1 | COL8A2 | Hs00697025_m1 | IL1B | Hs01555410_m1 |
| FN1 | Hs00365052_m1 | | | 18S | Hs99999901_s1 |
| SERPINB2 | Hs01010736_m1 | | | | |

Activation of enzyme at 95.degrees.C for 20 seconds, 40 cycles of denature at 95.degrees.C for 1 second and annealing/elongation at 60.degrees.C for 20 seconds were performed. The StepOnePlus Real-Time PCR system (Applied Biosystems) was used for PCR amplification and analysis.

The levels of MMP1, MMP2, MMP4, TIMP1, BMP2, SPARC, TGF-beta1, TGF-beta2, FN1, SERPINB2, CD44, CD166, CD105, CD24, Col3A1, Col4A1, Col4A2, Col8A2, CDH2, VIM, CDKN1B, CDKN1C, IGFBP3, SNAIL1, SNAIL2 and IL1B were normalized to that of 18S rRNA. Results were presented as .delta..delta.Ct (relative units of expression).

### Statistical Analysis

The statistical significance (p value) of mean values for 2-sample comparisons was determined by the Student's t-test. The statistical significance for the comparison of multiple sample sets was determined with the Dunnett's multiple-comparisons test. Values shown on the graphs represent the mean +/- SE.

### Measurement of metabolites in cHCECs

Metabolic extracts of intracellular metabolites were prepared from cHCEC culturing 6 well plates or 24 well plates with methanol containing Internal Standard Solution (Human Metabolome Technologies; HMT, Inc., Tsuruoka, Japan). The medium was aspirated from the well and cells were washed twice with 5% mannitol solution (1.5 mL first and then 0.5 mL for 6 well plates or 0.3 mL first and then 0.1 mL for 24 well plates). The cells were then treated with 600 .micro.L (6 well plates) or 200 .micro.L (24 well plates) of methanol and left at rest for 30 seconds in order to inactivate enzymes. Next, the cell extract was treated with 10 .micro.L (6 well plates) or 140 .micro.L (24 well plates) of Milli-Q water containing internal standards (H3304-1002, Human Metabolome Technologies, Inc., Tsuruoka, Japan) and left at rest for another 30 seconds. The extract was obtained and centrifuged at 2,300 x g and 4oC for 5 minutes and then all of the upper aqueous layer was centrifugally filtered through a Millipore 5-kDa cutoff filter at 9,100 x g and 4oC for 120 minutes to remove proteins. The filtrate was centrifugally concentrated and re-suspended in 50 .micro.L of Milli-Q water for CE-MS analysis.

### Measurement of metabolites in culture medium

20 .micro.L of medium and 80 .micro.L of Milli-Q water containing internal standards (H3304-1002, Human Metabolome Technologies, Inc., Tsuruoka, Japan) were mixed thoroughly. The mixture was centrifugally filtered through a Millipore 5-kDa cutoff filter at 9,100 x g and 4oC for 120 minutes to remove proteins and macromolecules. The filtrate was diluted 5-fold with Milli-Q water for CE-MS analysis.

### Data analysis of metabolome analysis

Cationic compounds were measured in the positive mode of CE-TOFMS and anionic compounds were measured in the positive and negative modes of CE-MS/MS according to the methods developed by Soga, et al (Soga, D. et al., T. Soga, et al., Anal. Chem. 2002; 74: 2233-2239 Anal. Chem. 2000; 72:1236-1241; T. Soga, et al., J. Proteome Res. 2003; 2: 488-494). Peaks detected by CE-TOFMS and CE-MS/MS were extracted using automatic integration software (MasterHands, Keio University, Tsuruoka, Japan (M. Sugimoto, et al., Metabolomics, 2009; 6: 78-95) and MassHunter Quantitative Analysis B.04.00, Agilent Technologies, Santa Clara, CA, USA, respectively) in order to obtain peak information including m/z, migration time (MT), and peak area. The peaks were annotated with putative metabolites from the HMT metabolite database based on their MTs in CE and m/z values determined by TOFMS. The tolerance range for the peak annotation was configured at +/- 0.5 minutes for MT and +/- 10 ppm for m/z. In addition, concentrations of metabolites were calculated by normalizing the peak area of each metabolite with respect to the area of the internal standard and standard curves, which were obtained by three-point calibrations.

Hierarchical cluster analysis (HCA) and principal component analysis (PCA) were performed by proprietary software of the inventors, PeakStat and SampleStat, respectively. Detected metabolites were plotted on metabolic pathway maps using VANTED (Visualization and Analysis of Networks containing Experimental Data) software (B. H. Junker, et al., BMC Bioinformatics. 2006; 7: 109). Metabolome measurements were carried out through a service at a facility of Human Metabolome Technologies, Inc., Tsuruoka, Japan.

### (Results)

### C-Myc expression and glucose uptake by bulk cultured cells

Bulk cultured cHCECs with no grouping into subpopulations were immunocytochemically stained with anti c-Myc antibodies. C-Myc expression was confirmed at a position of morphologically transformed cell-like shapes in phase contrast microscopy (Fig. 23). This indicates that at certain culture conditions, there are at least two subpopulations with or without c-Myc expression in cHCECs. Considering the role of c-Myc in glycolysis, the uptake of glucose in bulk cultured cHCECs was investigated by flow cytometry, and the considerable uptake in cHCECs was confirmed, although no difference in the degree of glucose uptake was confirmed (single peaks of 2-NBDG uptake at all incubation times) (Fig. 23). It has been demonstrated that 2NBDG are uptaken into many cell types by a glucose transporter. This immediately raises the question whether glucose starvation cause the partial, but selective elimination of the subpopulations of cHCECs in bulk cultures.

### Phenotype and morphological changes by glucose starvation

To assess the selective elimination of subpopulations of cHCECs in bulk cultures, cHCECs were cultured in glucose starved DMEM without FBS in the presence of lactate. The absence of FBS was intended to prevent the carryover of glucose from FBS. After passages to P3 under normal culture conditions, the cultured cells were incubated in DMEM with glutamine, but without glucose, in the absence or presence of up to 10mM lactate for 72 hours, then the resultant cHCECs were further cultured at three distinct dilution passages (1:3, 1:9, 1:30), under normal conditions for 4 weeks. The morphological changes detected by phase contrast microscopy (Fig. 24-A) demonstrated the elimination of some subpopulations in cHCECs, indicating the presence of glycolytic energy metabolism even under uniform glucose uptake (Fig. 23). To assess the partial elimination of subpopulations of cHCECs in bulk cultures, immunohistochemical staining of Na⁺/K⁺-ATPase was performed before and after glucose starvation. Na⁺/K⁺-ATPase was used as a function related marker of the HCECs. The efficacy of recovery from the elimination effect was clearly dependent on the concentration of lactate added (Fig. 24-B). The partial elimination of cHCECs by glucose starvation was also confirmed with use of another culture lot of HCECs. The starvation was performed at passage P3 for 72 hours and followed by morphologically recovery for 4 weeks at 1:3 dilution.

Alteration of EMT, cell senescence and fibrosis related genes by glucose starvation The partial selective elimination of cHCEC subpopulations by starvation of glucose was reproducibly confirmed, irrespective of the morphological diversity from cultures to cultures, indicating the presence of subpopulations distinct in their glycolytic energy metabolism. To deepen the understanding of the elimination effects, RNAs were extracted from the pre-starved cHCECs and those starved for 72 hours. Expression of genes related to CST, such as EMT, cell senescence and fibrosis, was analyzed by quantitative real-time PCR (Fig. 25(A-H)). The most impressive upregulation after glucose starvation was associated with MMP1, MMP2, BMP2 and TGF-beta1, while TGF-beta2 showed a decrease (Fig. 25(A-H)).

In the same experiment with other culture and starvation conditions, upregulation was confirmed for MMP1, MMP2, MMP4, TIMP1, BMP2 and TGF-beta1, while TGF-beta2 showed a decrease. In contrast, most of the genes associated with EMT and fibrosis were uniformly downregulated. The downregulated genes include SPARC, TGF-beta2, IL-1beta, CD44, CD24, CDH2, VIM, SNAIL1, SNAIL2, IGFBP 3, 4, 7 (Fig. 25(A-H)). Several isoforms of collagen were also downregulated (3A1, 4A1, 8A2) and CDKN1s showed a decreasing trend.

### Metabolomic profile clustering among heterogeneous cHCECs

To identify intracellular metabolite variations among different cHCECs, CE-MS analysis was performed to profile the levels of small molecule metabolites. Three lots of cHCECs (C16P6, C21P3 and 164P1) were analyzed, and proteins were extracted 2 or 3 days after medium changes at sub-confluent cell densities (7.22 X 10⁵, 9.85 × 10⁵ and 3 × 10⁵, respectively). Normalized intracellular metabolite signal positioning is relatively depicted in Fig. 26-A as PCA analysis, together with typical metabolites in PCA1 and 2 components. Hierarchical clustering (HCA) of normalized metabolite intensities showed clear separation among three lots of cHCECs (Fig. 26-A). The morphological differences of these cHCECs are shown together with flow cytometry analyze (Figs. 26-E to 26-G). Despite the superficially similar microscopic features, the HCA differed greatly especially between C16, C164 and C21. The distinction by flow cytometry analysis well coincided with the result. Nevertheless, metabolite profiles were distinct between C164 and C16, possibly due to the presence of CD44+++ cell populations in C16 (Fig. 26-D). These clustering results are consistent with the possibility that metabolic reprogramming is required both during the differentiation/maturation process and during the acquisition of CST such as EMT or fibrosis.

Lactate /pyruvate ratios were higher in C164 than C16 and C21, and the content of GSH, GSSG as well as total glutathione, which is the markers of intracellular redox status, was dramatically lower in C16 and C21 compared with C164. The lower GSH levels in transformed cells suggest that reducing antioxidant activity occurs in the process of CST.

In summary, the observations suggest that C16 and C21 are relatively prone to use anaerobic glycolysis than C164, perhaps due to exposure to cell stress.

### Metabolomic profiling clustering of secreted metabolites

The aim of this Example is to provide a practical, non-invasive, and sensitive way to monitor the conformity of cHCEC quality for regenerative therapy as an innovative medicine, instead of the invasive way to follow surface CD markers. Comprehensive survey of the secretory metabolites in medium accurately categorized cHCEC subpopulations distinct in the expression levels of surface CD44 antigens. Fig. 27 characterizes metabolite changes in conditioned media. Hierarchical clustering of metabolomic profiles of the culture media identified subsets of metabolites that correlated with the presence of CST. The clusters were divided at least into four metabolite subsets: metabolites that increased in all cHCECs (# 66, #72, #55); metabolites that increased mainly in #72 and # 55, but not in #66; metabolites that decreased the most in #66; and metabolites that are present more or less in three groups (Fig. 27-A). Several intermediates in glycolytic systems were higher in #72 and # 55 than in #66, which is consistent with the "Warburg effect" of increased glycolysis rate. The Warburg effect includes increased lactate production. Indeed, the lactate/pyruvate ratios were higher in #72 and # 55 than tin #66 (Fig. 27-B). It is of note that these three lots exhibited good morphology under microscopic inspection, indicating the most usefulness of comprehensive survey of secretory metabolites in a medium.

### Fine distinction of secreted metabolites among quality controlled cHCECs without CD44+++, CD24+ and/or CD26+ subpopulations

To validate the usefulness to monitor extracellular metabolites during culture of HCECs for cell injection therapy, a medium of HCECs produced under the GMP condition and without CD44+++, CD24+ or CD26+ cells was analyzed. Four cHCECs differed in their subpopulation composition in the context of CD44- to CD44+ versus CD44++ subpopulation were investigated. Consistent with the clustering described above, HCA identified four metabolite subsets as described above (Figs. 28-A to 28-E). The four lots exhibited a similar metabolite profiling, but C23 exhibited a strong disposition to mitochondria dependent OXHOS instead of anaerobic glycolysis, as verified with lowest production of lactate, the lowest lactate/ pyruvate ratio and the highest production of TCA cycle intermediates such as citrate/isocitrate and cis-aconitate. Interestingly Gin consumption was highest in C24, while it was the lowest in C21, indicating the possible difference in glutaminolysis in the two. There was no big difference in the distinction in amino acid metabolism and redox status. This result suggested quantitative differences in secreted metabolites among these four lots. Overall, the data reinforces the conclusion from the analysis of secreted metabolites. TCA cycle metabolism alterations accompany high quality of cHCECs suitable for cell therapy. To confirm the reduction of glycolysis metabolites and the disposition to mitochondria dependent OXHOS in the most ideal cHCECs C23, the inventors propose a simple method of monitoring metabolites in a media by the citrate/lactate ratio. Fig. 28-H are graphs showing the differences in the citrate/lactate ratios among # 66, #55 and #72, different in the content of CD44+++ subpopulations, and those among C21, C22, C23 and C24, which differs only in the proportion of CD44- to CD44+ versus CD44++. The quality can be monitored by the ratio of citrate to lactate in cHCEC culture supernatants.

### (Summary)

Cultured HCECs were examined in detail with respect to energy-metabolism-related functional markers C-Myc and CD44. To gain insight on the molecular mechanisms underlying phenotype switching, the propensity of metabolic requirements in cHCECs heterogeneous in subpopulations was investigated. It has been revealed that cHCECs were comprised of subpopulations with distinct metabolic requirements for energy supply. The inventors have succeeded in distinguishing subpopulations in term of their secretory metabolites, and found that cell state transitioned (CST) subpopulations exhibited the disposition to anaerobic glycolysis, instead of mitochondria dependent oxidative phosphorylation. This opens the way for the first time to monitor the disposition of cHCECs in their energy metabolism, leading to safe and stable regenerative medicine using metabolically defined cHCECs in the form of a cell suspension.

### (Conclusions)

The results of this study unravel the existence of subpopulations in cHCECs with distinctive energy metabolisms, and provide the possibility of establishing effective culture conditions to selectively expand matured cHCECs with cobble-stone shapes, disposed to mitochondria dependent oxidative phosphorylation.

Since HCECs can be grown in culture, the cell infusion therapy with cHCEC to treat corneal endothelial dysfunction has been extensively explored. As pointed out by several groups, cultured cells generally have a risk of karyotype changes (Miyai T, et al., Mol Vis. 2008; 14: 942-50). One of the most notable obstacles against the application of cHCECs to cell therapy is the method to validate the cell quality of cHCECs. In this Example, the inventors have succeeded in providing a candidate for noninvasively monitoring cell quality in place of an invasive method to track surface CD markers. Comprehensive survey of the secreted metabolites in culture medium accurately identified cHCEC subpopulations distinct in the expression levels of surface CD44 antigens.

Transition from oxidative metabolism, typical of somatic tissues, into glycolysis is a prerequisite for reprogramming to the pluripotent state. Conversely, redirection of pluripotency into defined lineages requires mitochondrial biogenesis and maturation of efficient oxidative energy generation.

Metabolic state influences cell state. Warburg effect in a cancer cell destined to aerobic glycolysis is a representative example. Nonetheless, the requirement for specific metabolic reprogramming in the maturation and differentiation of HCECs remains to be explored. The findings of the inventors describe for the first time the profound change of energy metabolism states among distinct subpopulations in cHCECs. Cell state transitioned cHCECs switch toward glycolytic metabotype, whereas differentiated cHCECs become more oxidative mitochondria respiratory system dependent. Low lactate production and elevated activation of phosphorylation levels in mature subpopulations of cHCECs suggest the possible application of metabolomics in cell quality control and/or the usefulness thereof as biomarkers for diagnosis, prognosis, and therapeutic efficacy for corneal endothelial dysfunctions, such as Fuchs endothelial corneal dystrophy.

This Example also provides novel insights into the origin of guttae in FECD with cardinal features such as abnormalities of cells, coalescence of multiple guttae, and contour. Considering the need to match the metabolic output with the demands of cellular function for tissue integrity under homeostatic and stress conditions, future elucidation of the origins of guttae in regards to the metabolic changes will provide new insights into the pathogenesis of Fuchs corneal dystrophy (FECD).

In summary, data of the inventors provide a way to monitor the disposition of cHCECs in their energy metabolism, leading to safe and stable regenerative medicine by metabolically defined cHCECs in the form of a cell suspension. At the same time, the new findings of the inventors, albeit very preliminary, provide evidence that may link the metabolic regulation in the corneal endothelium to the development of more efficient targeted therapies to treat patients with bullous keratopathy including FECD.

### (Example 5: MicroRNA Profiles Qualify Phenotypic Features of Cultured Human Corneal Endothelial Cells)

The aim of this Example was to find a noninvasive way to assess and identify CST-free cultured cells adaptable for cell therapy.

The variations of cHCECs in their composition of heterogeneous subpopulations (SPs) were proven in the context of their surface CD markers and morphology. The profiles of miRNA in cultured cells or supernatants were detected by 3D-gene (Toray). The profiles were also analyzed for fresh corneal tissues with distinct endothelial cell densities (ECD) with or without gutatta. To assess the 3D gene results, qPCR was carried out. RNAs were extracted from cHCECs transfected with selected miRNA, and target genes were estimated by PCR array (Qiagen).

### (Materials and Methods)

### Human corneal endothelial cell donors

The human tissue used in this Example was handled in accordance with the ethical tenets set forth in the Declaration of Helsinki. HCECs were obtained from 20 human cadaver corneas and were cultured before performing karyotype analysis. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the state in which the donor consent was obtained and the tissue was recovered.

The donor age ranged from 2 to 75 years (average 43.7 +/- 26.4). Both males and females were included. All donor corneas were preserved in Optisol-GS (Chiron Vision, Irvine, CA, USA) and imported by airplane for research purposes. According to the donor information, all donor corneas were considered healthy without corneal disease and all donors had no past history of chromosomal abnormality. For the analysis of corneal endothelium tissues with gutatta, tissues with endothelial cell density (ECD) less than 2000 cells/ mm² (to 380) were used and compared with tissues at the same age range.

### Cell Cultures of HCECs

Unless described otherwise, HCECs were cultured according to published protocols, with some modifications. Human donor corneas at distinct ages were used for the experiments. Briefly, Descemet's membranes with CECs were stripped from donor corneas and digested at 37.degrees.C with 1 mg/mL collagenase A (Roche Applied Science, Penzberg, Germany) for 2 hours. HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. Conditioned medium was prepared as previously described (Nakahara, M. et al. PLOS One (2013) 8, e69009). The HCECs were cultured using the conditioned medium at 37.degrees.C in a humidified atmosphere containing 5% CO₂. The culture medium was changed twice a week. The HCECs were subcultured at ratios of 1:3 using 10x TrypLE Select (Life Technologies) for 12 minutes at 37.degrees.C when they reached confluence. The HCECs at passages 2 through 5 were used for all experiments.

### Phase Contrast Microscopy

Phase contrast images ware taken by an inverted microscope system (CKX41, Olympus, Tokyo, Japan).

For the area distribution analysis, the cHCECs were washed with PBS (-) three times and phase contrast images were obtained with a BZ X-700 Microscope system (Keyence, Osaka, Japan). The area distributions were quantified by BZ-H3C Hybrid cell count software (Keyence).

### Immunofluorescent Staining

The HCECs were cultured at a density of 1 × 10⁵ cells/well in a 24-well cell culture plate coated with FNC Coating Mix and were maintained for 3 to 4 weeks for immunofluorescence analysis. Cells were fixed in 95% ethanol supplemented with 5% acetic acid for 10 minutes at room temperature and incubated for 1 hour with 1% BSA. Samples were incubated overnight at 4.degrees.C with antibodies against CD73 (1:300; BD Pharmingen Stain Buffer), CD166 (1:300; BD Pharmingen Stain Buffer), ZO-1 (Life Technologies), and Na⁺/K⁺-ATPase (Milford, MA, USA). After washing with PBS, either Alexa Fluor 488-conjugated goat anti-mouse IgG (Life Technologies) or Alexa Fluor 594-conjugated goat anti-rabbit IgG (Life Technologies) was used as a secondary antibody at a 1:1000 dilution. Nuclei were stained with DAPI (Vector Laboratories, Burlingame, CA, USA). The cells, cultured in a 48-well cell culture plate, were directly examined by a fluorescence microscope (BZ-9000; Keyence, Osaka, Japan).

### Flow cytometry analysis of cultured HCECs

HCECs were collected from a culture dish by TrypLE Select treatment as described above and suspended at a concentration of 4 x 10⁶ cells/mL in FACS buffer (PBS containing 1% BSA and 0.05% NaN₃). An equal volume of antibody solution was added and incubated for 2 hours at 4.degrees.C. The antibody solutions contained the following: FITC-conjugated anti-human CD26 mAb, PE-conjugated anti-human CD166 mAb, PerCP-Cy5.5 conjugated anti-human CD24 mAb, PE-Cy7-conjugated anti-human CD44 (all from BD Biosciences), APC-conjugated CD105 (eBioscience, San Diego, CA, USA). After washing with FACS buffer, the HCECs were analyzed with FACS Canto II (BD Biosciences).

### Microarray Analysis (3D-Gene)

MicroRNA extraction:
Corneal endothelial tissues and epithelial tissues were obtained after stripping from donor corneas, and were stored in QIAzol Lysis Reagent (QIAGEN strasse1 40724 Hilden Germany) at -80.degrees.C until total RNA extraction. Total RNA was extracted using the miRNeasy Mini kit (QIAGEN). The quality of purified total RNAs was analyzed by a Bioanalyzer 2100 (Agilent Technologies, Palo Alto, Calif., USA).

### MicroRNA expression profiling

The inventors utilized Toray's 3D-Gene^{™} human microRNA chips (miRBase version 17-19) for microRNA expression profiling. One was 250 ng-500 ng of total RNA derived from both tissue and cell samples labeled with miRCURY LNA^{™} microRNA Power Labeling Kits (Exiqon, Vedbaek, Denmark), the other was all of the miRNA derived from 400 .micro.L supernatant that were labeled. The labeled microRNAs were individually hybridized onto the microRNA chips surface, and were incubated at 32.degrees.C for 16 hours. The washed and dried microRNA chips in an ozone-free environment was scanned using 3D-Gene scanner 3000 (Toray Industries Inc., Tokyo, JAPAN) and analyzed using 3D-Gene Extraction software (Toray). Each sequence used was a sequence accompanying these products.

### Standardized data processing

The digitalized fluorescent signals provided by the software were regarded as raw data. All the standardized data were globally standardized per microarray, such that the median of fluorescence intensity was adjusted to 25. In a case where a bar graph of Tissue, cHCECs and Supernatant, standardized levels were adjusted to match the 100^{th} value from the highest rank.

### PCR Array

Total RNA was extracted from cultured HCECs using miRNeasy Mini kit (QIAGEN strasse1 40724 Hilden Germany). The cDNA synthesis was performed on 100 ng total RNA for a 96-well plate format using RT² First Strand kit (Qiagen). Expression of endothelial mRNAs was investigated using the RT² Profiler PCR-Array (Human Extracellular Matrix and Adhesion Molecules, Human p53 Signaling Pathway, Human Fibrosis, Human Cellular senescence, and Human Epithelial to Mesenchymal Transition (EMT)) (Qiagen) and analyzed using RT² Profiler PCR Array Data Analysis Tool version 3.5.

### Statistical Analysis

The statistical significance (p value) of mean values for 2-sample comparisons was determined by the Student's t-test. The statistical significance for the comparison of multiple sample sets was determined with the Dunnett's multiple-comparisons test. Values shown on the graphs represent the mean +/- SE.

### (Results)

### Distinct miR expression profiles among HCEC cultures

This Example discussed improved cultures of cHCECs with hardly any conspicuous EMT. However it is difficult to distinguish the presence of minute EMT among heterogeneous subpopulations. At first, the inventors compared the profiles of miRs detected by 3D gene (Toray) with a CD44-subpopulation (effector cell) and several cultured cells with unknown composition of subpopulations (2911, 3411, and 3511, all were at passage 1) (Fig. 29-B). There was a slight upregulation in miR29c expression and downregulation in miR378 expression in the latter subpopulations compared with effector cells. Next, effector CD44- subpopulation was compared with cultured cells 67 with evident CST (whose subpopulation compositions are depicted in Figs. 30-A to 30-C) (Fig. 29-C). The comparison also revealed downregulation in the miR378 family. This clearly indicates the presence of at least two different types of CST in cHCECs, even in the absence of conspicuous EMT. The striking reduction of miR378 was confirmed in morphologically distinguishable cultures with poor quality, and the expression levels of the miR378 family were comparable between the cultures C66 and C11.

### MiR expression profiles among subpopulations with distinct CD markers

Aiming to reveal the dependency of expression profiles of miRs on the compositions of subpopulations with distinct CD marker expression levels such as CD44, RNA extracted from FACS defined cHCEC subpopulations (Figs. 30-A to 30-C) were subjected to 3D gene analysis. These three cHCECs, a5, a1 and a2, contain subpopulations mainly comprised of CD44-CD24-CD26-, CD44++CD24-CD26- and CD44+++ CD24- CD26++ subpopulations, respectively. Again, the expression levels of the miR378 family were comparable between a5 and a1, but dramatic decrease was confirmed in a2 along line with elevated CD44 expression. Fig. 31-A illustrates the differences in the expression for each miR378 family subpopulation such as miR378a-3p, 378a-5p and 378f. In addition to the miR378 family, 23, 27, 30, 130 and 181 families exhibited negative correlation with CD44 expression, whereas 29, 31, 193 and 199 families exhibited positive correlation. To make the situation clear, the changes were divided into five classes. In Fig. 31-B, the decrease of miR378 family expression in the opposite direction of CD44 expression becomes gradual with the increase of CD44, such that CST cannot be distinguished between a5 and a1 (it can be distinguished between a5 and a2). It is most suitable that changes in the expression level of miR34 between a5 and a1 is remarkable such that CST can be distinguished between a5 and a1, although not between a1 and a2.

Only some aspects of the findings were subjected to further validation. Q-RT-PCR also demonstrated the observation that miR378 family was most highly expressed. At the same time, miR1246 was downregulated in CD44 positive cells, whereas miR1273 and miR205 were upregulated in those cells (data not shown).

### Distinct miR expression profiles in culture supernatants

The aims of this Example were to find miRs in cultured supernatants which may serve as an alternative tool to noninvasively identify cHCEC subpopulations, adaptable for cell therapy. Using the same three cHCECs as above (i.e., a5, a1 and a2), RNAs were extracted from the corresponding culture supernatants and subjected to 3D gene analysis. Many miRs were selected and the patterns of changes were divided into 6 categories. Among them, patterns with a characteristic tendency are depicted in Fig. 33(A-B).

Unlike the results from cHCECs, miR184 decreased gradually as in the miR378 family in cells, in an inverse manner with the expression of CD44. MiR24-3p/1273e was able to distinguish a5 and a1 from a2, as in miR 23, 27 and 181 families in cells, by their decrease in a2. There were also four miRs that can distinguish a5 and a1 from a2, by their increase in a2, in sharp contrast with miR24-3p/1273e.

### Corneal endothelial tissue with gutatta and miR378 and 146

Considering the commonality among in vitro CST such as EMT, cell senescence and fibrosis, the inventors then compared the miR profiles in fresh corneal endothelium tissues. Scatter plots of miR expression profiles between tissues with normal levels of ECD with those of ECD378 demonstrated the upregulation of miR 378 family more in the corneal endothelium tissue than corneal epithelium tissues (data not shown), but a dramatic decrease in low ECD tissues accompanying advanced gutatta. In contrast, miR146b-5p with a high higher expression level than miR378 family was not reduced. The upregulation of the miR378 family was comparable between corneal endothelial and epithelial tissues. It is of note that the family was also notably upregulated during culture and the highest expression was observed in CD44 negative effector cell subpopulation (Fig. 34(A-G)).

Several miRs were subjected to the further validation of the above findings. Q-RT-PCR also demonstrated that the expression of the miR378 family (a-3p, e and f) was suppressed uniformly in tissues with ECD 795 and 1410 (at this time, no RNA from tissue with ECD 378 was available), whereas miR 200c, 205 and 124-5p were upregulated in those cells.

### Candidate target of miR378

Finally, the inventors performed preliminary transfection of miR378a-3p or 5f mimetics into a CD44+++ cHCEC subpopulation, where the expression thereof was not detected. The cells elicited the gene signature of ECM and adhesion molecules class as shown in Fig. 35-C. The cells exhibited upregulation in many gene signatures of collagen, ITG and MMP families as well as CD44. Fig. 35-C shows heat maps of the gene signatures after transfection of two miR mimetics, as assayed by PCR arrays of senescence, EMT, fibrosis, p53 and EMA. Some of the genes such as CCNF, TWIS1 and GADD45 were upregulated in the senescence array. Many genes showed distinct signatures in the p53 PCR array after transduction, as well as downregulation by an miR378f mimetic of TCF4, TCF3, TGF-beta2, TGF-beta3 and SOX10i in the EMT array. The transfection of miR146 in addition to the miR378 family may add complementary insights to the pathogenesis of BK including FECD.

### (Summary)

MiRNA expression profiles among a variety of morphologically different cHCECs revealed a clear distinction thereamong. The miR34a was identified as miR capable of distinguishing a CD44- subpopulation from subpopulations with CD44++ to CD44+++ phenotypes. The downregulation of miRs in the 378 family paralleled with the upregulation of surface CD44 on cHCECs. Interestingly, upregulated miRs in the 378 family in the corneal endothelium dramatically decreased in tissues with lower ECD with advanced gutatta, providing the new insight on pathogenesis of Fuchs endothelial corneal dystrophy (FECD).

### (Conclusions)

The identified cultured subpopulations sharing the CD44- surface phenotypes with matured HCECs were shown to exhibit the highest expression of miR378. Conversely, subpopulations with upregulated CD44+++ exhibited reduction in miR378. Thus, miRNA in cultured cells or supernatants may serve as an alternative tool to identify cultured cHCECs.

Expansion of cHCECs from a donor corneal endothelium could provide a pragmatic tool for cHCEC cell therapy. cHCECs expanded in an in vitro culture system can be a mixture of subpopulations with distinct CST. A cultured cell tend to change karyotypes. Thus, cHCECs need to be carefully monitored with respect to their quality and purity in the context of heterogeneous subpopulation composition from the viewpoint of clinical settings, where safety should be strictly ensured.

Not only the morphological variations of cultured cells, but the composition of cHCEC subpopulations classified by CD markers varied greatly, even with similar morphological appearances, from cultures to cultures. Combined analysis of CD markers clearly identified the subpopulation (effector cells) adaptable for cell therapy among diverse subpopulations. The inventors proposed a method of quantifying the proportion of effector subpopulation in cultures (E-Ratio) as described herein. The inventors discovered that subpopulation with CD133, CD105, CD90, CD44, CD26, CD24, HLA-DR negative and CD166, HLA-ABC and PD-L1 positive are functional cells, which are completely free of karyotype aneuploidy and have a CD marker profile consistent with that of HCECs in fresh corneal tissues.

The plasticity due to morphological and phenotypical conversions, such as expression of mesenchymal markers and loss of epithelial markers, is collectively referred to as EMT. Cultured HCECs have an inclination towards CST into EMT. Numerous miRNAs were involved in EMT (miR-200, miR-34 and miR-203 families). In the comparison of distinct cultures shown in Fig. 29-A, no evident change in these EMT related miRs was detected, consistent with the inventors' judgement that the culture conditions in this Example exclude such conspicuous EMT. However, as summarized in Table 5, many of the miR378 family were clearly downregulated in cultures with heterogeneous subpopulations or low E-ratios.

**[Table 5]**

| miR378 family expression profiles | | | | | | | | | | | | | | | | | |
|---|---|---|---|---|---|---|---|---|---|---|---|---|---|---|---|---|---|
| **Name** | **C1** | **C2** | **C3** | **C4** | **C5** | **C6** | **C7** | **C8** | **C9** | **66-1** | **66-2** | **66-3** | **C13** | **C14** | **C15** | **C11-1** | **C11-2** |
| **miR-378*** | 0.1 | 9.5 | 0.1 | 6.4 | 0.1 | 10.8 | 8.0 | 0.1 | 7.1 | 26.2 | 20.6 | 37.2 | 0.1 | 16.7 | 12.0 | 25.7 | 29.6 |
| **miR-378** | 22.9 | 27.0 | 67.8 | 32.1 | 17.6 | 212.9 | 153.3 | 60.7 | 26.6 | 733.0 | 656.2 | 710.0 | 75.6 | 85.4 | 139.2 | 425.1 | 481.7 |
| **miR-378b** | 0.1 | 13.1 | 10.4 | 9.1 | 0.1 | 12.3 | 10.2 | 12.0 | 15.6 | 30.0 | 17.4 | 25.6 | 9.2 | 18.0 | 22.6 | 18.6 | 39.7 |
| **miR-378c** | 0.1 | 12.4 | 30.2 | 0.1 | 0.1 | 115.9 | 69.3 | 30.9 | 47.7 | 443.2 | 384.9 | 413.2 | 50.5 | 47.7 | 73.2 | 268.6 | 288.2 |
| **miR-378d** | 0.1 | 8.0 | 26.9 | 9.9 | 6.9 | 113.8 | 65.8 | 37.0 | 9.9 | 407.1 | 321.9 | 336.6 | 49.5 | 47.4 | 84.5 | 245.7 | 237.4 |
| **miR-378e** | 8.6 | 11.2 | 26.7 | 13.4 | 0.1 | 108.1 | 59.2 | 28.0 | 0.1 | 267.0 | 207.7 | 212.3 | 25.5 | 32.8 | 63.5 | 153.5 | 152.9 |
| **miR-378f** | 8.4 | 9.5 | 24.3 | 10.0 | 0.1 | 119.5 | 72.7 | 29.4 | 11.5 | 411.6 | 319.8 | 340.8 | 36.1 | 50.8 | 66.6 | 259.1 | 218.8 |
| **miR-378g** | 17.4 | 13.9 | 29.9 | 6.8 | 8.6 | 114.5 | 65.3 | 28.9 | 17.1 | 292.5 | 237.8 | 234.7 | 32.2 | 42.3 | 53.4 | 173.7 | 157.6 |
| **miR-378h** | 0.1 | 0.1 | 0.1 | 0.1 | 7.0 | 9.4 | 0.1 | 5.9 | 10.2 | 17.3 | 8.2 | 0.1 | 0.1 | 10.3 | 13.1 | 14.6 | 0.1 |
| **miR-378i** | 13.2 | 13.7 | 27.9 | 13.9 | 16.4 | 154.2 | 105.2 | 38.4 | 8.2 | 552.5 | 460.0 | 521.2 | 44.7 | 104.8 | 110.4 | 349.5 | 326.9 |
| **miR-422a** | 24.1 | 40.2 | 49.9 | 30.4 | 30.9 | 207.0 | 108.6 | 69.8 | 104.5 | 656.1 | 473.4 | 530.2 | 94.2 | 142.1 | 140.0 | 421.5 | 424.2 |
| **miR-130a** | 432.9 | 682.6 | 923.4 | 693.0 | 777.5 | 1342.6 | 1322.6 | 1281.2 | 523.1 | 8557.8 | 7834.0 | 8032.3 | 1391.5 | 1946.3 | 2477.1 | 2637.1 | 2468.6 |

In fresh HCE tissues, contrasting features of miR signatures were observed. miRs relating to EMT were upregulated in HCE tissues with low ECD, as was the case for miR146 (also upregulated in tissues with advanced gutatta). The miR378 family was downregulated in cultures with low E-ratios (Fig. 34), in a recent report (Bracken CP, et al., Cancer Res. 2015; 75: 2594-9)

miR-146a affects structural ECM proteins important in the assembly, composition and organization of the ECM.

miR-378performs a metabolic shift leading to a reduction in tricarboxylic acid (TCA) cycle gene expression and an increase in lactate production. CD44 is the hallmark to distinguish differentiated cHCECs from cHCECs which are either undifferentiated or have CST by E-ratios. CD44 ablation increased metabolic flux to mitochondrial respiration and concomitantly inhibited entry into a glycolytic system. Such metabolic reprogramming induced by CD44 ablation resulted in notable depletion of intracellular reduced glutathione (Tamada M, et al., Cancer Res. 2012; 72: 1438-48).

It is noteworthy that miR34a was identified as miR capable to distinguish CD44-effector cells from CD44++ to CD44+++ subpopulations (Fig. 31-B). miR-34a/CD44 axis activates the downstream factors of CD44 including RhoA and MMP-2 (Lin L, et al., Oncol Rep. 2015; 34: 663-72). In 2007, several groups identified the members of the miR-34 family as the most prevalent p53-induced miRNAs. Currently, the members of the miR-34 family are recognized as important mediators of cancer suppression (He L, et al., Nat Rev Cancer. 2007; 7:819-22). This is consistent with the above-described results in Example 2 where only the CD44- effector subpopulation exhibited the absence of karyotype abnormality.

miR378 family exhibited a gradual decrease in parallel with decreases in CD44 expression (Fig. 31-B). miR-378 is known to play a role in mitochondrial energy homeostasis (Eichner LJ, Pet al., Cell Metab. 2010; 12: 352-3611; Carrer M, et al., Proc Natl Acad Sci USA. 2012; 109: 15330-15335). This immediately indicates that the shift from aerobic, oxidative metabolism to glycolytic metabolism is a characteristic feature of some CST in cHCECs (see Example 4). Pyruvate is processed by a TCA cycle through oxidative phosphorylation (OXPHOS). Citrate/lactate ratios can distinguish CD44-effector cells not only from CD44+++ but also from CD44++ subpopulations with slight CST (see Example 4).

Several microRNAs (miRNAs) including miR-378a-5p have also been shown to be involved in senescence while targeting the p53 pathway. Senescence induction could provide further possibilities of the mechanism for miR-378a-5p to be involved in CST regulation in cHCECs.

The following conclusions can be drawn from this Example with respect to therapeutic options for BK or FECD. Remodeling of tissue under chronic stress appears to result, albeit in part, from downregulation of miR-378 and upregulation of the EMT regulating miR200 family. Thus, insufficient adaptation to tissue stress may be a manifestation of the progression of pathogenesis controlled by these miRs.

This Example demonstrated that cHCECs exhibited dysregulated expressions of a hierarchy of miRs clusters, probably due to the presence of CST and senescence. Isoforms of miR378 were downregulated and miR146 isoforms, in contrast, were upregulated in fresh corneal endothelium tissues with low ECD as well as gutatta. The miR profiles detected in the medium were distinct from those in cHCECs. The assay of miRs secreted in the medium could clearly distinguish cell state transitioned CD44+++ cHCECs from CD44 negative effector cells by miR34a. However, the profile of miRs in medium could not distinguish undifferentiated progenitor cells from effector cells, so that the issue needs to be carried over into future studies. In addition to the practical purpose, the new findings presented here warrants further investigation to develop a better understanding of the role of dysregulated expression of miRs in the pathogenesis of BK and FECD.

The upregulation of miR29 in cHCEC subpopulations with CST is also noteworthy in regard to the recent report describing its role in EMT promoting effect through Wnt/beta-catenin signaling (Rostas JW 3rd, et al., Mol Cancer. 2014; 13:20031). The details of downregulation of TCF4 and others by an miR378f CD44+++ cHCEC subpopulation with EMT is described herein in other Examples or the like.

### (Additional miRNAs)

The following was further revealed as a result of an experiment similar to the above for other miRNAs.
(C) a5:a1:a2 exhibits high expression: low expression: low expression:
   (cell-secreted) miR4419b, miR371b-5p, miR135a-3p, miR3131, miR296-3p, miR920, miR6501-3p;
(F) a5:a1:a2 exhibits high expression: low expression: high expression:
   (cell-secreted) miR92b-5p; and
(G) a5:a1:a2 exhibits low expression: high expression: low expression:
   (cell-secreted) miR1246, miR4732-5p, miR23b-3p, miR23a-3p, miR1285-3p, miR5096

The above results are shown in Fig. 35-D. The different expression patterns of secreted miR for each cell subpopulation were classified into 6 categories.

### CD44 expression level and miR profile

While cHCEC cultures prepared by a known method had most of evident EMTs eliminated in this Example, senescent forms were maintained. To eliminate interference by senescent cHCECs, an p38 mitogen-activated protein kinase (p38 MAPK), SB203580, inhibitor was added to the entire culture to control senescent CSTs. Under such a culture protocol, the inventors successfully obtained cultures a5, a1, and a2, which did not have any senescent form at first glance. Interestingly, they were heterogeneous with regard to CD44, CD24, and CD26 expression on the surface (Fig. 35-E). Such cHCECs were used to extract RNA from the corresponding culture supernatant. miRs (miRs of 23a-3p, 184, 1260a, 3130-3p, 23b-3p, 135a-3p, 1246, 3131, 24-3p, 296-3p, 1290, 4419b, 92a-2-5p, 371b-5p, 6501-3p, and 920) exhibiting different expressions among such cHCECs (i.e., a5, a1, and a2) were identified using a volcano plot (Fig. 35-E).

Furthermore, miRs of 92b-5p, 1273e, 1285-3p, 4732-5p, 221-3p, 1273f, 1915-3p, 4745-5p, 1246, 1273g-3p, 1972, 4792, 3937, and 5096 were identified in culture supernatant of high quality cells (effector cells) among cells distinguished by the standard of the quality of the form. Thus, such miRs are candidates for secreted miR used in determination of quality of noninvasive cells.

Analysis of miR in culture supernatant of subpopulations (effector, sub-qualified, CD44+++) by 3D-GEne identified miRs of 23a-3p, 184, 1260a, 3130-3p, 23b-3p, 135a-3p, 1246, 3131, 24-3p, 296-3p, 1290, 4419b, 92a-2-5p, 371b-5p, 6501-3p, and 920 as miRs exhibiting different expression patterns among said subpopulations. Thus, such miRs are candidates for secreted miRs used in distinguishing the quality of noninvasive cells.

### (Example 6: Different binding properties of cultured human corneal endothelial cell subpopulations to Descemet's membrane components)

To clarify the adherent properties of these subpopulations (SP), this Example compared the binding abilities of cultured HCEC subpopulations to major Descemet's membrane components that are distributed on the endothelial surface (i.e., laminin-511, -411, Type IV collagen, and proteoglycans).

In this Example, each subpopulation was prepared by controlling culture conditions or by using magnetic cell separation, and then examined by staining with several cell surface markers. To test the binding abilities of HCEC subpopulations, the cells were added to 96-well culture plates immobilized with collagens, laminins, or proteoglycans, and the plates were then centrifuged. The attached cells were then assessed under a phase contrast microscope.

### (Materials and Methods)

### Human corneal endothelial cell donors

The human tissue used in this Example was handled in accordance with the ethical tenets set forth in the Declaration of Helsinki. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the state in which the donor consent was obtained and the tissue was recovered. All donor corneas were preserved in Optisol GS (Chiron Vision, Irvine, CA, USA) and imported by airplane for research purposes. The donor information showed that all donor corneas were considered healthy without corneal disease.

### Cell Cultures of human corneal endothelial cells

Unless noted otherwise, HCECs were cultured according to published protocols, with some modifications. A total of 30 human donor corneas were used for the experiments. Briefly, the Descemet's membranes with CECs were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. Conditioned medium was prepared as previously described (Nakahara, M. et al. PLOS One (2013) 8, e69009). The HCECs were cultured using the conditioned medium at 37.degrees.C in a humidified atmosphere containing 5% CO₂. The culture medium was changed twice a week. The HCECs were subcultureed at ratios of 1:3 using 10x TrypLE Select (Life Technologies) for 12 minutes at 37.degrees.C when they reached confluence. The HCECs at passages 2 through 5 were used for all experiments.

### The cell attachment assay

Cell attachment to laminins, Type I collagen, proteoglycans and glycoprotein was tested by the centrifugation cell attachment assay described previously (Friedlander et al., 1998. J. Cell Biol. 107: p2329) with some modifications. Laminin-521, -511, - 411, and -332 were purchased from Veritas (Tokyo, Japan). Perlecan, Agrin, Nidogen-1, TSP-1 and Fibulin 5 were purchased from R&D Systems (Minneapolis, MN). Type IV collagen was purchased from BD Biosciences (San Jose, CA, USA).

Each well of a U-shaped 96-well Maxisorp plate (Nunc) was coated with 40 .micro.l of laminin, collagen, proteoglycan or glycoprotein solution overnight at 4degrees Celcius. The wells were washed three times with PBS (-) and blocked with 1% BSA in 0.1 M NaHCO₃ (pH 8.0) for 1 hour at room temperature. The cultured HCECs were suspended in Opti-MEM, Opeguard-MA (Senju Pharmaceutical, Osaka, Japan) or BSS-Plus (Alcon Laboratories, Fort Worth, TX, USA) at a concentration of 1 × 10⁵ cells/mL, and 0.1 mL of the cell suspension was added to each well and incubated for 10 minutes. The plate was then centrifuged at 200 x g for 2 minutes. The bright-field Z stack images were captured by a BZ-9000 microscope (Keyence, Osaka, Japan) at 2x objective magnification and omnifocal images were created from these images using BZ-II Analyzer software. In accordance with the description by Friedlander et al (Friedlander et al., 1998. J. Cell Biol. 107: p2329), the cells were centrifuged into a pellet at the bottom of the well on non-adhesive substrates. As the adhesiveness of the substrate increased, more cells bound to the substrates along the wall of the well. Bond to the substrate is detected in a circular area with a measurable diameter at the bottom or the well. On strongly adhesive substrates, cells were distributed more or less uniformly on the well (Grumet M, Flaccus A, and Margolis RU. J. Cell Biol. 1993; 120:815-824).

### Immunofluorescent Staining

The HCECs were cultured at a density of 1 × 10⁵ cells/well in a 24-well cell culture plate coated with type I collagen and were maintained for 3 to 4 weeks for immunofluorescence analysis. Cells were fixed in ice cold methanol for 10 minutes at room temperature and incubated for 1 hour with 1% BSA. Samples were incubated overnight at 4.degrees.C with antibodies against ZO-1 (Life Technologies) and Na⁺/K⁺-ATPase (Milford, MA, USA). After washing with PBS(-), either Alexa Fluor 488-conjugated goat anti-mouse IgG (Life Technologies) or Alexa Fluor 594-conjugated goat anti-rabbit IgG (Life Technologies) was used as a secondary antibody at a 1:1000 dilution. Nuclei were stained with DAPI (Vector Laboratories, Burlingame, CA, USA). The cells, cultured in a 48-well cell culture plate, were directly examined by a fluorescence microscope (BZ-9000; Keyence, Osaka, Japan).

### Flow cytometry analysis of cultured HCECs

HCECs were collected from a culture dish by TrypLE Select treatment as described above and suspended at a concentration of 4 x 10⁶ cells/mL in FACS buffer (PBS containing 1% BSA and 0.05% NaN₃). An equal volume of antibody solution was added and incubated for 2 hours at 4.degrees.C. After washing with FACS buffer, the HCECs were analyzed with FACS Canto II (BD Biosciences).

### Isolation of HCEC subpopulations by MACS

The HCECs were detached with TrypLE Select as described above and a CD44⁻HCEC subpopulation (effector subpopulation) was isolated using anti-human CD44 microbeads and the program depl05 of an autoMACS Pro separator (Miltenyi Biotec, Bergisch Gladbach, Germany). The purity of the isolated effector subpopulation was higher than 95% in all cases as demonstrated by flow cytometry.

### (Results)

The cultured HCECs bound more strongly to laminin-511 than laminin-411 and -332 In cell injection therapy, one of the important steps for therapeutic efficacy is the attachment of HCECs to the Descemet' membrane. The Descemet' membrane is comprised of mainly type IV collagens, laminins, fibronectins and proteoglycans/glycoproteins [Fitch et al., 1990 and Suda et al., 1981] (Table 6) (Weller JM, Zenel M, Schlotzer-Schrehardt U, Bachmann OB, Tourtas T, Kruse FE. Invest Ophthalmol Vis Sci. 2014; 55: 3700-3708).

**[Table 6]**

| | Descemet's Membrane | |
|---|---|---|
| | Stromal Face | Endothelial Face |
| Type IV Collagen chains | a1, a2 | a3-a6 |
| Laminin chains | None | a4, a5, b1, g1 |
| Possible laminin isoforms | None | 411, 511 |
| Nidogen-1/entactin-1 | - | + |
| Nidogen-2/entactin-2 | - | + |
| Perlecan | - | + |
| Netrin-4/b-netrin | - | + |
| Matrillin-4 | - | ±^{†} |
| Type VIII Collagen | + | - |
| Type XII Collagen | - | + (Short form) |
| SPARC/BM-40/osteonectin | ± | - |
| Type XVIII Collagen | - | ± |
| Thrombospondin-1 | - | + |
| Fibronectin | + | - |
| Vitronectin | + | - |

| | | |
|---|---|---|
| Expression intensity is as follows -, lack of staining; ±,weak staining in some cases, +, distinct staining in most or all cases, † Some cases were negative. | | |

Thus, this Example tested the attachment abilities of HCECs to the Descemet' membrane components listed in Table 6. As a method for assessing the attachment ability of HCECs, this Example performed a centrifugation cell attachment assay [Friedlander et al., 1998. J. Cell Biol. 107: p2329] with some modification (Fig. 36).

The cultured HCECs were detached from culture dishes as described in (Materials and Methods) and cell suspension was prepared at a concentration of 1 × 10⁵ cells/ml. 100 .micro.l of the cell suspension was added to each well of the U-bottomed 96-well plates coated with laminin-511, laminin-411, Type IV collagen or other components of Descemet's membrane listed in Table 6. The plate was incubated for 10 minutes at room temperature and then centrifuged at 200 x g for 2 minutes. The attached cells were assessed under a BZ-9000 microscope system as described in (Materials and Methods).

As shown in Fig. 37, the HCECs were uniformly distributed on the bottom of wells coated with laminin-521 or -511, while HCECs formed pellets at the bottom of the negative control BSA-coated wells. In the laminin-411 or -332 coated wells, the HCECs formed a circular pellet. These results suggest that the HCECs bound more strongly to laminin-521 and -511 than to laminin-411 and -332.

This Example further compared the binding ability of the cultured HCECs to laminin-521 and to laminin -511 by lowering the coating concentration of laminins. As shown in Fig. 38, the cultured HCECs bound to laminin-521 and -511 in a concentration dependent manner, but the difference was not clearly observed between laminin-521 and -511.

### Cultured HCECs bound to Type IV collagen

Next, the inventors examined the binding of cultured HCECs to type IV collagen. A centrifugation attachment assay was similarly performed with type IV collagen-coated plates. As shown in Fig. 39, the cultured HCECs bound to Type IV collagen in a concentration dependent manner.

### Cultured HCECs weakly bound to Agrin, TSP-1 and Perlecan

This Example also examined the binding of cultured HCECs to proteoglycan/glycoproteins that were reported to exist in the Descemet's membrane (i.e., Agrin, Nidgen-1, Fibulin 5, TSP-1 and Perlecan). Binding was not observed when coating concentrations of these proteoglycan/glycoproteins were the same 5 nM as in Fig. 37 (data not shown), but the binding to Agrin, TSP-1 and Perlecan was observed at a coating concentrations of about 400 nM (Fig. 40). These results show that the cultured HCECs bind to these components but with a considerably lower affinity than to laminins.

### Influence of cell suspension infusion vehicle on cultured HCEC binding

In cell injection therapy, the choice of cell suspension infusion vehicle is critical. Therefore, the inventors compared the effect of cell suspension infusion vehicles on the attachment of HCECs to Descemet's membrane components. As the cell suspension infusion vehicle, Opti-MEM, Opeguard-MA and BSS Plus were selected. Opti-MEM (used in Fig. 37-40) is the base medium of HCEC culture. Opeguard-MA and BSS Plus are intraocular irrigating solutions used in clinical routines. The Descemet's membrane component used was laminin-411, because laminin-411, with high affinity for cHCECs, is considered more readily detectable than laminin-511. For both Opti-MEM and Opeguard-MA, the HCECs bound to laminin-411, but no binding was observed in BSS Plus (Fig. 41). This Example further examined whether addition of aqueous humour components (proteins, ascorbic acids and lactic acids) to Opeguard-MA enhances the binding affinity of cultured HCECs to laminin-511 and - 411. As shown in Figs. 42 and 43, enhanced effect was not observed.

Cultured HCEC subpopulations (SP) have different binding properties The tendency to enter into cell state transition (CST) such as epithelial-mesenchymal transition or fibrosis during culture produces different subpopulations (SP) with distinct surface CD markers from HCECs. To date, the inventors have developed a method to clearly distinguish those subpopulations with respect to their cell surface markers. To clarify the adherent properties of these subpopulations, this Example compared the binding abilities of cultured HCEC subpopulations by a centrifugation attachment assay.

Purified HCEC subpopulations exhibiting no signs of CSTs with hexagonal shapes (fully differentiated mature HCEC subpopulations) were prepared by magnetic bead cell sorting (MACS) with human-CD44 magnetic beads by negative selection (as described in (Materials and Methods)). As shown in Fig. 44, the effector subpopulations separated by MACS with CD44 magnetic beads were purified to over 90% purity (as determined by flow cytometry analysis) and these cells expressed Na⁺/K⁺ ATPase and ZO-1 (Fig. 45).

To obtain subpopulations with EMT-phenotype (CD166⁺, CD44⁺⁺⁺, CD24⁻), MACS positive selection with human-CD44 magnetic beads was not performed, but instead subpopulation spontaneously generated in cultures were used (Fig. 46). This is to avoid the influence of direct interaction between CD44 on the cell surface and CD44 magnetic beads on cell attachment abilities.

Both the fully differentiated mature HCEC subpopulation and the subpopulation with EMT-phenotype were found to attach to laminin or collagen coated plates (Fig. 47). Interestingly, properties to bind to laminins were different among these subpopulations. Although the levels of cells attached to the laminin-411 coated plate were the same among the HCEC subpopulations, the fully differentiated mature HCEC subpopulation bound significantly more strongly to laminin-511 than the subpopulation with EMT-phenotype (Fig. 47).

Difference in expression of integrin alpha2 subunit in cultured HCEC subpopulations Previously, Okumura et al. reported that interaction between laminin-511 and cHCECs was facilitated by integrin alpha3beta1 and alpha6beta1 (Okumura et al. (2015) Invest Ophthalmol Vis Sci. 2015; 56:2933-2942). Therefore, this Example tested the expression of integrin alpha3 and alpha6 on these subpopulations. Unexpectedly, expression of these integrin alpha subunits on the subpopulation with an EMT-phenotype was comparable or a slightly higher than that of the matured HCEC SPs (Fig. 48). Interestingly, the integrin alpha2 subunit expression on the subpopulation with an EMT-phenotype was notably higher than that of the matured HCEC SPs (Fig. 48).

### (Summary)

Cultured HCECs bound to laminin-511, laminin-411 and Type IV collagen in a concentration dependent manner. These cells were weakly bound to Perlecan, Agrin and TSP-1. The inventors compared the influence of cell suspension infusion vehicles on cultured HCEC attachment. When the HCECs were suspended in Opti-MEM or Opeguard-MA, these cells bound to laminin, but no binding was observed in BSS Plus. Next, the inventors compared the adherent properties of HCEC subpopulations. Both the fully differentiated mature HCEC subpopulation and the EMT-phenotype subpopulation were found to attach to laminin or collagen coated plates. Interestingly, the properties of binding to laminins were different among these subpopulations. Although the levels of cells attached to the laminin-411 coated plates were the same among the HCEC subpopulations, the fully differentiated mature HCEC subpopulation bound significantly more strongly to laminin-511 than the subpopulation with EMT-phenotype did.

Conclusions: the results in this Exmaple suggest that the binding ability of HCEs to major Descemet's membrane components is distinct among subpopulations of cultured HCECs. Opeguard-MA is useful as a cell suspension infusion vehicle in cell injection therapy as is OptiMEM. Moreover, the simple methods used herein are effective for assessing the interaction between HCECs and extracellular matrix components.

This Example investigated binding properties of cultured HCECs by centrifugation cell attachment assay. The cultured HCECs bound more strongly to laminin-521 and -511 than laminin-411 and -332, suggesting that cultured HCECs have high affinity to laminin alpha5 subunit. These cells also bound to Type IV collagen in a concentration dependent manner. The minimum concentrations necessary for the observed cell binding in this Example are as follows; Laminin-511: 4 pM (3 ng/mL), Laminin-411: 1.25 nM (2.85 .micro.g/mL), and Type IV collagen: 200 ng/mL. Binding to Perlecan, TSP-1 and Agrin was only observed at the concentrations at 400 nM.

HCECs express several integrins (alpha2beta1, alpha3beta1 alpha5beta1, and alpha6beta1). Recently, Okumura et al. reported that HCECs binding to laminin-511 are mediated by integrins alpha3beta1 and alpha6beta1 [Okumura et al. (2015) Invest Ophthalmol Vis Sci. 2015; 56:2933-2942]. Integrins alpha3beta1 and alpha6beta1 have higher affinity to laminin-511 than laminin-332 or -411 [Barczyk et al. (2010) Cell Tissue Res. 2010; 339:269-280]. In this Example, despite having higher affinity for laminin-511, the expression of alpha3 and alpha6 on the fully differentiated mature HCEC subpopulation was lower than that of the subpopulation with an EMT-phenotype. Meanwhile, integrin alpha2 subunit expression was higher in the former subpopulation than that in the latter subpopulation. Additionally, integrin beta1 expression was not notably different between these two subpopulations (data not shown: assessed by Lyoplate (BD Biosciences, San Jose, CA, USA)). It is possible that the high expression of integrin alpha2 subunit produces the alpha2beta1 complex known as the collagen binding integrin [Barczyk et al. (2010) Cell Tissue Res. 2010; 339:269-280], resulting in the lower expression of alpha3beta1 or alpha6beta1 complexes. This clearly indicates the presence of distinction in the ratio of integrin alpha2beta1 vs alpha3beta1 and alpha6beta1 among subpopulations, and the proportion thereof was prominently observed in cultured HCECs.

In "cell infusion therapy", the cell suspension infusion vehicle is important for therapeutic efficacy. In this Example, the effects of three cell suspension infusion vehicles, Opti-MEM, Opeguard-MA and BSS Plus on the attachment of HCECs to laminin were compared. Opeguard-MA was comparable with Opti-MEM. Because the composition of Opti-MEM is not disclosed by the supplier, Opeguard-MA (intraocular irrigating solution used in clinical routines) might be more preferable as the cell suspension infusion vehicle for cell infusion therapy.

### (Example 7: Development of cryo-induced freeze damage murine model to evaluate the cell quality of cultured corneal endothelial cells for cell infusion therapy (human corneal endothelial cells infusion)-development of surrogate endpoint)

The aim of this Example is to determine the surrogate endpoint of HCECs by utilizing a mouse model.

The cHCECs with composition fully characterized by flow cytometry analysis were provided for the examination of the proposed models. The 2 mm central region of BALB/c corneas were subjected to cryo-induced freeze damage to eliminate endothelial cells, then 4 × 10⁴ cHCECs were injected into the anterior chamber of the eye. Corneal features were clinically observed, and the corneal thickness was assessed by a pachymeter. For the cHCEC suspension, Opti-MEM, Opeguard MA, and Opeguard MA supplemented with human albumin, ascorbic acid and lactic acid (Opeguard-F) were compared in the model. The presence of a ROCK inhibitor (Y-27632) was also assessed.

### (Materials and Methods)

### Human corneal endothelial cell donors

The human tissue used in this Eample was handled in accordance with the ethical tenets set forth in the Declaration of Helsinki. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the state in which the donor consent was obtained and the tissue was recovered. All donor corneas were preserved in Optisol GS (Chiron Vision, Irvine, CA, USA) and imported by airplane for research purposes. According to the donor information, all donor corneas were considered healthy without corneal disease.

### Cultures of human corneal endothelial cells

Unless noted otherwise, the HCECs were cultured according to published protocols (Nakahara M, Okumura N, Kay EP, et al. PLoS One 2013; 8:e69009), with some modifications. A total of 4 human donor corneas at distinct ages were used for the experiments. Briefly, the Descemet's membranes with corneal endothelial cells were stripped from donor corneas and digested at 37.degrees.C with 1 mg/mL collagenase A (Roche Applied Science, Penzberg, Germany) for 2 hours. HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. The HCECs were cultured at 37.degrees.C in a humidified atmosphere containing 5% CO₂. The culture medium was changed twice a week. The HCECs were subcultured at ratios of 1:3 using 10x TrypLE Select (Life Technologies) for 12 minutes at 37.degrees.C when they reached confluence. The HCECs at passage 2 were used for all experiments.

### Preparation of cell suspension for injection

HCECs were collected from a culture dish by TrypLE treatment as described above and suspended at a concentration of 6.7 x 10⁶ cells/mL in Opti-MEM or Opeguard-MA (Senju Pharmaceutical, Osaka, Japan) with or without additives. The additives used were 100 .micro.M Y-27632 for Opti-MEM, and 0.024% human serum albumin, 1.06 mM ascorbic acid, 4.5 mM lactic acid, 100 .micro.M Y-27632 for Opeguard-MA.

### Flow cytometry analysis of cultured HCECs

HCECs were collected from a culture dish by TrypLE Select treatment as described above and suspended at a concentration of 4 x 10⁶ cells/mL in FACS buffer (PBS containing 1% BSA and 0.05% NaN₃). An equal volume of antibody solution was added and incubated for 2 hours at 4.degrees.C. The antibody solutions comprised the following: FITC-conjugated anti-human CD26 mAb, PE-conjugated anti-human CD166 mAb, PerCP-Cy5.5-conjugated anti-human CD24 mAb, PE-Cy7-conjugated anti-human CD44 mAb (all from BD Biosciences), and APC-conjugated anti-human CD105 mAb (eBioscience, San Diego, CA, USA). After washing with FACS buffer, the HCECs were analyzed with FACS Canto II (BD Biosciences).

### Animals

Male BALB/c (H-2^{d}) mice (SLC, Osaka, Japan) aged between 8 to 12 weeks were used in this Example. All animals were treated according to the "Statement for the Use of Animals in Ophthalmic and Vision Research" promulgated by the "Association for Research in Vision and Ophthalmology". All experiments were approved by the Committee for Animal Research, Kyoto Prefectural University of Medicine. Before any surgical procedures, all animals were fully anesthetized with an intraperitoneal injection of 3 mg ketamine.

Freeze damage treatment and HCEC injection into anterior chamber of the eye Freeze damage for eliminating endothelial cells was applied to the right eye of each mouse (Han SB, et al., Mol Vis 2013; 19:1222-1230; Koizumi N, et al., Invest Ophthalmol Vis Sci 2007; 48:4519-4526). Briefly, after topical application of mydriatic agent (Mydrin P, Santen, Japan), transcorneal freezing was initiated by gently placing a cryoprobe made of stainless steel (2 mm in diameter) (precooled to -196.degrees.C by liquid nitrogen) on the center of the cornea. No pressure was applied to avoid damage to adjacent tissue including the lens and the trabecular meshwork. The lateral surface of the probe, instead of the tip, was placed on the cornea to maximize the contact surface. The cryoprobe was maintained on the corneal surface until an ice ball formed on the cornea and covered the entire corneal surface (corresponds to a duration of 10 seconds). The defect on the endothelium was shown to be the same size as the reported ice ball (Khodadoust AA, G Invest Ophthalmol 1976; 15:96-101). Immediately after freezing, the cryoprobe was freed from the corneal surface, washed with a balanced salt solution, and the cornea was allowed to naturally thaw. No topical medication was applied during the study period.

After eliminating endothelial cells by freeze damage, a diagonal incision was made at the center of the cornea of host BALB/c mice with a microknife, and 3 .micro.l of aqueous humour was removed with a glass needle. Then, 0.5 - 2 × 10⁴ HCECs in 3 .micro.l of an appropriate solution were injected into the anterior chamber without any leakage (Streilein JW. FASEB J 1987; 1:199-208; Yamada J, et al., Invest Ophthalmol Vis Sci 1997; 38:2833-2843). This cell count was calculated from 5 x 10⁵ cells in case of human. These mice were anesthetized for 3 hours with additional 1 mg ketamine injection per hour for the precipitation of HCECs at the endothelium surface.

### Clinical and Histopathologic Assessment

For clinical assessment, eyes were examined with a slit-lamp biomicroscope and confirmed no technical issues. Corneal thickness was measured by a pachymeter (SP-100, Tomey, Japan) before freeze damage, 24 and 48 hours after HCEC injection.

For histopathologic assessment, eyes were enucleated 48 hours after infusion, then fixed by 4 % paraformaldehyde at 4.degrees.C for 3 days, and dissected to make corneal eyecups. After washing with PBS (-) twice, the eyes were incubated for 5 minutes at room temperature with 0.1% triton X-100 in PBS (-) for permeabilization. Subsequently, after washing with PBS (-) twice, the eyes were incubated with 1 % BSA in PBS (-) for 20 minutes at room temperature. The eyecups were stained with Alexa Fluor 488-conjugated mouse anti-human nuclei antibodies (Merck Millipore, Germany) at a 1:20 dilution for 2 hours at room temperature. After washing twice, the eyecups were flat-mounted by 4 incisions, and stained with DAPI (Vector Laboratories, Burlingame, CA, USA). Sections were assessed with a fluorescence microscope (OLYMPUS, Tokyo, Japan).

### Statistical Methods

Student's paired t-test was used to compare corneal thicknesses. P values of < 0.05 were deemed significant.

### (Results)

The proposed murine model turned out to be effective to differentiate the effect of injected cHCEC distinct in their cellularity. 48 hours after cHCEC injection was the selected time period to monitor the effect of the injected cHCECs. The cHCEC injection significantly improved the corneal thickness (p < 0.01).

### Preparation and selection of functional mature differentiated corneal endothelial cells (effector HCEC)

The inventors, with their collaborators, developed cHCEC-injection therapy. This is a novel treatment modality for bullous keratopathy which involves direct injections of cHCECs into the anterior chamber. It was revealed that the quality of cHCEC composition is critical for the pharmacological implications in this method. For this reason, composition of cHCECs used in this study was assessed.

The cells were detached from a culture dish, and the expression of CD166, CD24, CD44, CD105 and CD26 was analyzed with a FACS Canto II flow cytometer as described in (Materials and Methods). After gating for CD166+CD24- (R1) or CD166+CD24+ (R2), the following five subpopulations were defined: CD166+CD24-CD44- to +CD105+ to - subpopulation (gate 1 = G1), CD166+CD24-CD44++CD105+ subpopulation (G2), CD166+CD24-CD44+++CD105+ subpopulation (G3), CD166+CD24+CD44+CD105+ subpopulation(G4), and CD166+CD24+CD44++CD105+ subpopulation (G5). CD44++CD26+ cells were also detected (Fig. 49(A-B)). Since the inventors hypothesized that the G1 cells were the effector cell subpopulation (Example 1) as they were considered essential for the action as a medicament, the culture flask containing high percentage of G1 cells was used in the following experiments. (The cells of Fig. 49(A-B) were tested in each of Figs. 51-53).

Determination of assessing protocol after HCEC injection in mouse models Considering the simplicity and easy procedures, mice were selected as recipients to utilize the freeze damage model reported by Han et al (Han SB, et al., Mol Vis 2013; 19:1222-1230).An albino BALB/c mouse was selected, since BALB/c mice are mainly used for corneal transplantation studies of allogeneic (Sonoda Y, and Streilein JW. J Immunol 1993; 150:1727-1734) and xenogeneic (Tanaka K, et al., Transplantation 2000; 69: 610-616) combinations. Non-pigmented eyes of these mice facilitate assessment of in vivo observation as well as histological examination. Moreover, BALB/c mice are more resistant to corneal rejection than C57BL/6 mice (Yamada J, and Streilein JW. Transpl Immunol 1998; 6:161-168.) When the inventors have attempted a similar examination in C57BL/6 or C3H mice, several technical difficulties were observed, such as the shallow anterior chamber, floating of iris pigments in the anterior chamber of the eye (data not shown).

The individual difference of endothelial loss after freeze damage was examined first. It was discovered by the histological examination of nucleus stained by DAPI that mice endothelial cells in the cornea with diameters of 1.8 to 2.2 mm were lethally damaged just after freeze damage. Importantly, no healthy endothelial cells were observed in the central region, but all CECs in a peripheral region were healthy (data not shown). The cryo-injured BALB/c eyes (n = 6 each) were then dissected at appropriate periods, flat-mounted, and stained with DAPI. As shown in Fig. 50, most of the mice endothelial cells separated from the Descemet' membrane and only damaged nuclei remained at the central region 24 hours after freeze damage (Fig. 50(A-c)). The peripheral edge of damaged endothelial cells was clearly visible (white arrow). Normal corneal endothelial cells with clear nuclei were observed in the peripheral region of the cryo-injured cornea. At 48 hours after freeze damage, these BALB/c mice derived endothelial cell nuclei disappear such that the Descemet' membrane surface looks stable (Fig. 50(A-f)). However, rapid mice corneal endothelial growth was observed at 72 hours post injury (Fig. 50). From these observations, observation at 48 hours post injury should be the preferred assessment period to compare attached HCECs.

Immediately after freeze damage to BABL/c corneas, 0 to 2.0 × 10⁴ HCECs in Opti-MEM were injected intracamerally (N=4 each). According to the clinical outcome (Fig. 50), the corneas became edematous and opaque at 24 hours and 48 hours post-freeze damage, and recovered most of their transparency at 72 hours even in the non-HCEC injected control eyes. Typical xenogeneic rejection was not observed within 72 hours. The corneal transparency (assessed by iris edge visibility etc.) varied in each cornea, such that the effect of HCEC injection could not be assessed. The corneal thickness of these eyes was measured and the results are shown in Fig. 50(C). The corneal thickness notably increased after 24 hours and gradually recovered. By injecting HCECs intracamerally, the corneal thickness quickly recovered. Especially the corneas injected with 2.0 × 10⁴ HCECs showed significant recovery at 48 hours (p < 0.05).

By considering these observations, the inventors have selected the observation period of 48 hours post freeze damage and injected cell count of 2.0 × 10⁴. The corneal thickness and the histological staining of HCECs were then compared in the following experiments.

Assessment of cHCECs injected using distinct cell suspension infusion vehicles Next, the inventors assessed whether this assay is useful as a surrogate endpoint by comparing the effect of injected cHCECs using distinct cell suspension infusion vehicles (Figs. 51-53).

After injury, 2.0 × 10⁴ cHCECs (population of Fig. 49) were injected intracamerally with 4 types of infusion vehicles, Opti-MEM (a) oand Opeguard MA (b). They were compared with the control injected with Opeguard MA only (c) (n=3 each). Further, to mimic the involvement of diverse factors in the aqueous humour in adherence of cHCECs, Opeguard MA was supplemented with human albumin, ascorbic acid and lactic acid (Opeguard F). Similar experiments were then performed by intracameral injection of cHCEC (population of Fig. 49) with Opti-MEM (a) or Opeguard F (b), and compared with the control of Opeguard F vehicle only (c) (n=3 each). As shown in Fig. 51, the corneal outcome at 48 hours had individual differences, but all eyes maintained a normal anterior chamber and no hyphema. By injecting 2.0 × 10⁴ HCECs, the corneal transparency is considered to show better recovery from the observation of the iris edge visibility. The corneal thickness significantly recovered at 48 hours post freeze damage by the injection of HCECs (Fig. 52). Especially the recovery of corneal thickness was repeatable when using Opeguard (Fig. 52). In contrast to primate corneal endothelial cells, murine corneal endothelial cells proliferate rapidly in vivo. Therefore, to differentiate the adherence of injected cHCECs from that of proliferated host murine corneal endothelial cells, the cHCEC adherence were assessed by anti-human nucleus antibody staining (Kuzma-Kuzniarska M, et al. Differentiation 2012; 83:128-137; Sanchez-Pernaute R, et al. Stem Cells 2005; 23:914-922; Le Belle JE, et al., J Neurosci Res 2004; 76:174-183; Wurmser AE, et al., Nature 2004; 430:350-356) as shown in Fig. 53. Though the human nuclei positive cells were present at the endothelial surface in the two groups injected with HCECs, the aligned HCEC nuclei were the most clearly visible in Opeguard F group (Fig. 53). Better HCEC adherence observed was, in descending order, in the group of Opeguard F, Opti-MEM, and Opeguard MA.

### (Summary)

The 48 hours after HCEC injection was the appropriate time period for all assessments. HCEC injection significantly improved corneal thickness (p < 0.01).

Conclusions: This surrogate mice model established herein is effective for the functional assessment of injected HCECs in vivo. Since several amino acids are contained, the best binding ability of HCECs to the Descemet's membrane of mice in vivo was obtained.

This Example is the first to establish a mouse model for HCEC quality assessment in vivo. Injected HCECs could attach to the corneal inner surface well and played a role in suppressing corneal edema. Moreover, there were notable differences in results among injecting infusion vehicles. Although Opti-MEM is a clinically excellent HCEC injection infusion vehicle, it is currently not approved for human usage. In this Example, Opeguard F, modified from Opeguard MA, exhibited notably excellent HCEC attachment and suppression of corneal edema. The results in this Example may support safer HCEC infusion.

The cHCEC adherence is one of the most important factors for the success of endothelial cells infusion. The cHCEC adherence can be assessed in vitro by centrifuging a 96-well culture plate with immobilized collagens, laminins, or proteoglycans (Example 6 or the like). Theoretically, BALB/c mice reject xenogeneic cHCECs hyper acutely, but hyperacute rejection was not observed in rabbit (Ishino Y, et al. Invest Ophthalmol Vis Sci 2004; 45:800-806; Mimura T, et al. Invest Ophthalmol Vis Sci 2004; 45:2992-2997) and rat study (Mimura T, et al. Exp Eye Res 2004;79:231-237) in endothelial transplantation models. In the actual model used in this Example, many human-nuclei positive cells survived 72 hours post injection. While magnetic fields were often used to attempt to have corneal endothelial cells adhere to a corneal surface by using magnetic field, the results in this Example indicate that HCECs themselves have an ability to attach to the corneal inner surface and the infusion vehicle used for transplantation is an important factor for HCEC adhesion. More details of the influence of the expression of cell adhesion molecules can be understood by examination.

There were significant differences in the results among the infusing infusion vehicles. Opeguard-MA comprising safety reagents is a clinically excellent infusion vehicle. The inventors have selected human serum albumin, ascorbic acid, and lactic acid for the modified Opeguard MA, Opeguard F. These three reagents are components of the aqueous humour and are commercially available as clinical grade materials. In this regard, the Opeguard MA-modified Opeguard F exhibited significantly better HCEC attachment and suppression of corneal edema. It is understood, in view of the results of this Example, that safer cHCEC infusion in human can be achieved.

In conclusion, the inventors established a novel murine model to assess a surrogate endpoint for cHCEC injection therapy in vivo. This in vivo mouse model is also valuable for the assessment of systems associated with cHCEC infusion therapy, such as the effect of combined drugs, cHCEC suspension infusion vehicle, optimal cell count for injection, or the like.

### (Example 8: Gene Signature Based Development of ELISA Assays for Reproducible Qualification of Cultured Human Corneal Endothelial Cells)

This Example developed a method to qualify the function of cHCECs adaptable in clinical settings.

This Example examined diverse genes and miRNA signatures in HCECs from a variety of tissue and donors by 3D-gene (Toray). These signatures were compared with those of more than 20 cHCECs with distinct cell morphology or culture lots. Candidate genes were selected after validation by qRT-PCR, and the genes were assayed by ELISA. After additional screening steps, the final candidate cytokines for qualification were selected.

### (Materials and Methods)

### Human corneal endothelial cell donors

Human tissue used was handled in accordance with the ethical tenets set forth in the Declaration of Helsinki. HCECs obtained from 20 human cadaver corneas were cultured before performing karyotype analysis. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the state in which the donor consent was obtained and the tissue was recovered. All donor corneas were preserved in Optisol GS (Chiron Vision, Irvine, CA, USA) and imported by airplane for research purposes. According to the donor information, all donor corneas were considered healthy without corneal disease and all donors had no past history of chromosomal abnormality.

### Cultures of HCECs

Unless noted otherwise, HCECs were cultured according to published protocols, with some modifications. Briefly, the Descemet's membranes with corneal endothelial cells were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp., St. Louis, MO, USA), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. Conditioned medium was prepared as previously described (Nakahara, M. et al. PLOS One (2013) 8(7), e69009). The HCECs were cultured using the conditioned medium at 37.degrees.C in a humidified atmosphere containing 5% CO₂. The culture medium was changed twice a week. The HCECs were subcultured at ratios of 1:3 using 10x TrypLE Select (Life Technologies) for 12 minutes at 37.degrees.C when they reached confluence. The HCECs at passages 2 through 5 were used for all experiments.

### Phase Contrast Microscopy

Phase contrast images were taken by an inverted microscope system (CKX41, Olympus, Tokyo, Japan).

TotalRNA extraction: Corneal endothelial tissues and epithelial tissues were stored in QIAzol Lysis Reagent (QIAGEN strasse1 40724 Hilden Germany) at -80.degrees.C until total RNA extraction. Cultured HCECs and supernatant of cHCECs were lysed by QIAzol Lysis Reagent and were stored at -80.degrees.C until total RNA extraction. Total RNA was extracted using the miRNeasy Mini kit (QIAGEN). The quality of the purified total RNAs was analyzed by a Bioanalyzer 2100 (Agilent Technologies, Palo Alto, Calif., USA). MicroRNA expression profiling: Toray's "3D-Gene" human miRNA oligo chip (miRBase version 17) was used for microRNA expression profiling. 250 ng-500 ng of total RNA derived from both tissue and cell samples that were labeled with Hy5 using miRCURY LNA(R) microRNA Power Labeling Kits (Exiqon, Vedbaek, Denmark), and labeled total miRNA derived from 400 .micro.L supernatant were prepared. The labeled microRNAs were individually hybridized onto the microRNA chips surface, and were incubated at 32.degrees.C for 16 hours. The microRNA chips washed and dried in an ozone-free environment were scanned using 3D-Gene scanner 3000 (Toray Industries Inc., Tokyo, JAPAN) and analyzed using 3D-Gene Extraction software (Toray).

mRNA expression profiling: Toray's "3D-Gene" Human Oligo chip 25K (version 2.1) was used to obtain an mRNA expression profile. 200 ng-500 ng total RNA were amplified using Amino Allyl MessageAmp^{™} II aRNA Amplification Kit (ambion, CA, USA). The amplified RNAs were labeled with Cy5 using Amersham Cy5 Mono-Reactive Dye (GE Healthcare, UK). The labeled amplified RNAs were individually hybridized onto the microRNA chip surface, and were incubated at 37.degrees.C for 16 hours. The oligo chips washed and dried in an ozone-free environment were scanned using 3D-Gene scanner 3000 (Toray Industries Inc., Tokyo, JAPAN) and analyzed using 3D-Gene Extraction software (Toray).

### Normalized data processing

The digitalized fluorescent signals provided by the software were regarded as the raw data. All normalized data were globally normalized per microarray, such that the median of the signal intensity was adjusted.

### Quantitative RT-PCR

Total RNA was extracted from cultured HCECs using the miRNeasy Mini kit (QIAGEN strasse1 40724 Hilden Germany). The cDNA was synthesized using High Capacity cDNA Reverse Transcription kit with RNase Inhibitor (Applied Biosystems, Foster City, CA, USA).

PCR reactions were performed using TaqMan Fast Advanced Master Mix (Applied Biosystems) and TaqMan Gene Expression Assays, Inventoried (Applied Biosystems) under the following conditions: activation of Enzyme at 95.degrees.C for 20 seconds; and 40 cycles of denaturation at 95.degrees.C for 1 second and annealing/elongation at 60.degrees.C for 20 seconds. The StepOnePlus Real-Time PCR system (Applied Biosystems) was used for PCR amplification and analysis. The levels of gene expression were normalized to that of 18S rRNA. Results were denoted as 2.delta..delta.Ct(relative units of expression).

Each sequence used was a sequence accompanying these products. ID information thereof is shown below.

**[Table 7]**

| | Assay ID | | Assay ID | | Assay ID |
|---|---|---|---|---|---|
| MMP1 | Hs00899658_m1 | CD166 | Hs00977641_m1 | CDH2 | Hs00983056_m1 |
| MMP2 | Hs01548727_m1 | CD105 | Hs00923996_m1 | VIM | Hs00185584_m1 |
| MMP4 | Hs01128097_m1 | CD24 | Hs03044178_m1 | CDKN1B | Hs01597588_m1 |
| MMP9 | Hs00234579_m1 | COL1A2 | Hs01028970_m1 | CDKN1C | Hs00175938-_m1 |
| SPP1 | Hs00959010_m1 | COL3A1 | Hs00943809_m1 | CDKN2C | Hs01568320_m1 |
| TIMP1 | Hs00171558_m1 | COL4A1 | Hs00266237_m1 | NOX4 | Hs00418356_m1 |
| BMP2 | Hs00154192_m1 | COL4A2 | Hs01098873_m1 | HGF | Hs00900070_m1 |
| STEAP1 | Hs00185180_m1 | COL5A1 | Hs00609088_m1 | THBS2 | Hs01568063_m1 |
| SPARK | Hs00234160_m1 | COL8A1 | Hs00156669_m1 | LGR5 | Hs00173664_m1 |
| IL13RA2 | Hs00152924_m1 | COL8A2 | Hs00697025_m1 | IGFBP3 | Hs00365742_g1 |
| TGFβ1 | Hs00998133_m1 | IL6 | Hs00985639_m1 | IGFBP4 | Hs01057900_m1 |
| TGFβ2 | Hs00234244_m1 | IL8 | Hs00174103_m1 | IGFBP7 | Hs00266026_m1 |
| EGFR | Hs01076078_m1 | IL10 | Hs00961622_m1 | ITGB1 | Hs00559595_m1 |
| FN1 | Hs00365052_m1 | IL18 | Hs01038788_m1 | WNT5A | Hs00998537_m1 |
| EGR1 | Hs00152928_m1 | IL33 | Hs01125943_m1 | SNAIL1 | Hs00195591_m1 |
| SERPINB2 | Hs01010736_m1 | TSLP | Hs00263639_m1 | SNAIL2 | Hs00950344_m1 |
| CD44 | Hs01075862_m1 | CDH1 | Hs01023894_m1 | | |

### PCR Array

Total RNA was extracted from cultured HCECs using the miRNeasy Mini kit (QIAGEN strasse1 40724 Hilden Germany). The cDNA synthesis was performed with 100 ng total RNA for 96-well plate format using RT² First Strand kit (Qiagen). Expression of endothelial mRNAs was investigated using the RT² Profiler PCR-Array Human Extracellular Matrix and Adhesion Molecules (Qiagen) according to the manufacturer's recommended protocols, and analyzed using RT² Profiler PCR Array Data Analysis Tool version 3.5.

### Bio-Plex for integrated analysis of cytokines

The culture supernatant of cHCECs was harvested after 4 days of culture and was immediately frozen and stored after harvest at -80.degrees.C until analysis. The cytokine levels of the culture supernatants were analyzed by Luminex X-Map technology (Bio-Plex 200, BioRad, Hercules, CA, USA) and the Bio-Plex Human 27-plex panel kit (BioRad) according to the manufacturer's instructions. The following cytokines were measured: interleukin-1 beta (IL-1beta), IL-1ralpha, IL-2, IL-4, IL-5, IL-6, IL-7, IL-8, IL-9, IL-10, IL-12p70, IL-13, IL-15, IL-17A, basic fibroblast growth factor (b-FGF), Eotaxin, granulocyte colony-stimulating factor (G-CSF), granulocyte macrophage colony-stimulating factor (GM-CSF), interferon-gamma (IFN-gamma), interferon-induced protein-10 (IP-10), monocyte chemotactic protein-1 (MCP-1), macrophage inflammatory protein-1alpha (MIP-1alpha), MIP -1beta, platelet-derived growth factor-BB (PDGF-BB), regulated upon activation normal T-cell expressed and secreted (RANTES), tumor necrosis factor-alpha (TNF-alpha), and vascular endothelial growth factor (VEGF). Standard curves for each cytokines (in duplicate) were generated using the reference cytokine concentrations supplied in this kit and were used to calculate the cytokine concentrations in the culture supernatants.

### Enzyme immunoassay

Cell culture supernatant was harvested from cHCECs and centrifuged at 1580 g at RT for 10 minutes to remove detached cells. Supernatant was collected and filtered through 0.22 .micro.m filters (Millex-GV Millipore).

ELISA was performed using Clusterin (Human) Competitive ELISA kits (Adipogen , Inc., Incheon, Korea), Procollagen type I C-peptide(PIP) EIA kit (TaKaRa., JAPAN), Human CCL2/MCP-1, Human TGF-gamma1, Human TIMP-1, and Human HGF, (R&D System, Inc. USA), BMP2 Human Elisa kit, PDGF-bb Human ELISA Kit, IL6 Human ELISA Kit, and Human IL8 ELISA Kit (Abcam., OFL, UK), Human Osteopontin Assay kit, and Human Osteopontin N-Half Assay Kit (IBL., Gunma, JAPAN), Enzyme-Linked Immunosorbent Assay Kit For Early Growth Response Protein 1 (USCN Life Science INC., China), IP-10 ELISA Kit, Human, IL-1RA ELISA Kit, Human IFN-gamma ELISA Kit, and TNF-alpha Human ELISA Kit (Invitrogen), Collagen TypeIII alpha1 ELISA kit Human (-), and Collagen alpha-2 (VIII) chain ELISA kit Human (-) (CUSABIO).

### Statistical Analysis

The statistical significance (P value) of mean values for 2-sample comparisons was determined by the Student's t-test. The statistical significance for the comparison of multiple sample sets was determined with the Dunnett's multiple-comparisons test. Values shown on the graphs represent the mean +/- standard error.

### (Results)

### Genes and miRNA signatures varied among cultures

Human corneal endothelium (Endo) tissues from 6 elderly donors and 5 corneal epithelia (EP) were analyzed by 3D-gene (Toray) for their mRNA and miRNA signatures. The discrepancy of the gene signatures between Endo and EP was evident, whereas the miR signatures between these two tissues were not notably different for mRNA (Fig. 54-A(a), 54-A(b) and 54-B). The signatures were almost the same among 6 donors. In addition, the changes were not evident in tissues from four generations, both in Endo and Ep, except for the mRNA signatures in neonatal donors.

In contrast, both mRNA and miRNA signatures of cHCECs turned out to be greatly distinct from those in fresh Endo (Fig. 54-A(a), 54-A(b) and 54-B), and the signatures were far apart from those of fresh tissue in miRNA. This indicates the role of epigenetic regulation in cultures.

### Variation in expression of genes relating to CST

Typical cultures clearly different in morphology (Figs. 55-A and 55-B), together with fresh Endo, were subjected to PCR array using the RT² Profiler PCR-Array for EMT, fibrosis and cell senescence. Consistent with the finding that gene expression notably changes in cultures of HCECs, heat map revealed the clear distinction among these three groups. It is of note that even two groups of cHCECs depicted in Figs. 55-A and 55-B exhibited distinct gene expression profiles. Almost 50 genes were commonly elevated in two cultured cells. These included Col3A1, FN1, IGFBP3, 4, and 5, ITGA3 and 5, MMP2, TIMP1, ZEB2, MAP1B, Serpine1, THBS2, TGFbR2, TGFb1, and CD44. Between two cultures in Figs. 55-A and 55-B, many upregulated and downregulated genes were found (data not shown), suggesting differentiated gene expression among cHCEC cultures even under the same culture protocol. From these screening results of varying genes among cultures, 50 candidate genes were selected for further validation by qRT-PCR (32 genes selected by screening and 18 genes considered functionally importance in CST were added thereto) (Table 8).

[Table 8]

**(Table 8) Candidates for 50 Genes by PCR Array and 3D-Gene**

| | | | | | | |
|---|---|---|---|---|---|---|
| **MMP1** | **TGFβ1** | **COL1A2** | **IL6** | **CDH1** | **NOX4** | **IGFBP3** |
| **MMP2** | **TGFβ2** | **COL3A1** | **IL8** | **CDH2** | **HGF** | **IGFBP4** |
| **MMP4** | **EGFR** | **COL4A1** | **IL10** | **VIM** | **THBS2** | **IGFBP7** |
| **MMP9** | **FN1** | **COL4A2** | **IL18** | **CDKN1B** | **LGR5** | **ITGB1** |
| **SPP1** | **EGR1** | **COL5A1** | **IL33** | **CDKN1C** | | **WNT5A** |
| **TIMP1** | **SERPINB2** | **COL8A1** | **TSLP** | **CDKN2C** | | **SNAIL1** |
| **BMP2** | **CD44** | **COL8A2** | | | | **SNAIL2** |
| **STEAP1** | **CD166** | | | | | |
| **SPARC** | **CD105** | | | | | |
| **IL13RA2** | **CD24** | | | | | |

### Validation of selected genes by qRT-PCR

The candidate genes were validated by qRT-PCR by comparing their expression in two cHCECs depicted in Figs. 56-A and 56-B (i.e., 665A2 and 675A2, which were morphologically contrasting). Figs. 56-A and 56-B show part of the results. Gene expressions of CDH2, TGF-beta2 and Col8A2 were clearly upregulated in cHCECs without CST (i.e. 665A2), whereas those of TIMP1, Col3A1, CD44, IL-6, IL-8 and BMP2 were upregulated in cHCECs with CST (i.e. 675A2). In this comparison, VIM, CD166, CD105, CD24 and MMP4 genes were expressed at comparable levels in both cHCECs. The results are summarized in Fig. 56-B (the table does not include the genes expressed at very low levels).

### Validation of selected genes using morphologically graded cHCECs

The results mentioned above clearly show that CST varied more than the level taking into account EMT, senescence and fibrosis. Thus, the expression levels were investigated in more detail. cHCECs were graded into 11 types by assigning points from 0 to 10 in term of the cultured cell morphology (three people participated) (Fig. 57-A). The expression levels of some of the genes investigated are illustrated in Fig. 57-B. One group exhibited upregulated expression in an inverse correlation with the order of the points and the second group exhibited a positive correlation, while the third group hardly had any correlation with the points. Interestingly, even among 10 point cHCECs, CD24 expression was distinct, indicating that this gene is more finely regulated than the other genes shown in Fig. 57-B. Subsequent validation was performed using cHCECs produced at the Cell Processing Center under GMP (Figs. 58-A and 58-B). In this comparison, two cHCECs seemingly distinct in their CST form (C9 and C11), exhibited a contrasting gene expression profiles. For example, CD24 was upregulated only in C9, as well as Col4A1 and 4A2. However, MMP2, TIMP1, IL-6, IL-8 and TGF-beta1 were elevated only in C11. CD44, THBS2, Col3A1 and HGF were upregulated both in C9 and C11.

### Screening of cytokine produced by Bio-Plex human cytokine 27-plex panel

Gene expression generally corresponds to products consisting of a single chain and does not sufficiently reflect the products as a heterodimer. To overcome this issue and to confirm the possible assays by culture supernatants, culture supernatants of diverse cHCECs were analyzed by Bio-Plex human cytokine 27-plex panel (Bio-Rad). Three cHCECs (#82, #84, and #88), different in the frequency of culture passages were provided for analysis. Typical results are illustrated in Fig. 59-A. Most cytokines secreted exhibited a decrease or increase depending on the increase of passage frequency. From this test, IL-6, MCP-1 and IL-8 showed an increase along with deterioration in culture quality. In contrast, IL-1Ralpha, IFN-gamma, IP-10, PDGF-bb and MIP-1beta in the culture showed an increase corresponding with improvement in culture quality.

### ELISA Assays for Reproducible Quality control of cHCECs

Extensive studies have developed a reproducible quality control method of cHCECs and four cytokines (i.e., TIMP1, MCP-1, IL-8 and PDGF-bb) were selected. The final conditions were narrowed down after testing the method can be used in practice for evaluating the quality of 32 lots of cHCECs produced in the Cell Processing Center at the Kyoto Prefectural University of Medicine. The selection was also investigated as to whether the quality evaluating ability thereof corresponds to that by FACS at the time of cell harvesting on the day of cell infusion therapy. The standard values for each cytokine are < 500ng/ml TIMP1, < 500pg/ml IL-8, < 3000 pg/ml MCP-1 and > 30 pg/ml concentration of PDGF. The quality control should be carried out 7 day before cell injection therapy.

The difference in the amount of MCP-1, IL-8, and PDGF-bb secreted in culture supernatant was investigated for cultures with various cultured strains, number of passages, and days of passage (Fig. 59-B to 59-C). It is shown that a difference in the subpopulation ratio results in a different cytokine yield.

### (Summary)

The gene and miRNA signatures of cHCECs among distinct culture lots varied more than those among fresh tissues from donors with different ages. By comparing more than 20 lots of cultures, 32 candidate genes were considered to be associated with a difference in morphological features of cHCECs. The investigation of candidate genes by qRT-PCR revealed the genes that are either upregulated or downregulated in accordance with morphological variances in cHCECs (e.g., features such as EMT or cell senescence). ELISA results by Bio-Plex human cytokine 27-plex panel were further added, and 11 candidate cytokines were selected as suitable for controlling the quality of the function of cHCECs. After considering the presence of these cytokines in the anterior chamber, IL-8, TIMP-1, MCP-1 and PDGF were finally selected and applied in practice for assessment of quality of cHCECs that can actually be used in this cell infusion studies in clinic settings.

### Conclusion

Specific cytokines for properly distinguishing the functional features of cHCECs were determined, making it possible to control the quality of the cHCECs adaptable as an alternative to donor corneas for the treatment of corneal endothelial dysfunctions.

Expansion of cHCECs from a donor corneal endothelium could provide a method for cHCEC cell infusion therapy. Cultured HCECs expanded in in vitro culture systems can be contaminated by cHCECs that have undergone CST. Cultured cells also tend to undergone karyotype change (see Example 2). Thus, the quality of cHCECs should be carefully monitored for clinical use.

Morphological features of cHCECs varied greatly from cultures to cultures even under identical culture protocols. One of the largest obstacles against the application of cHCECs for cell therapy is how to validate the cell quality of cHCECs.

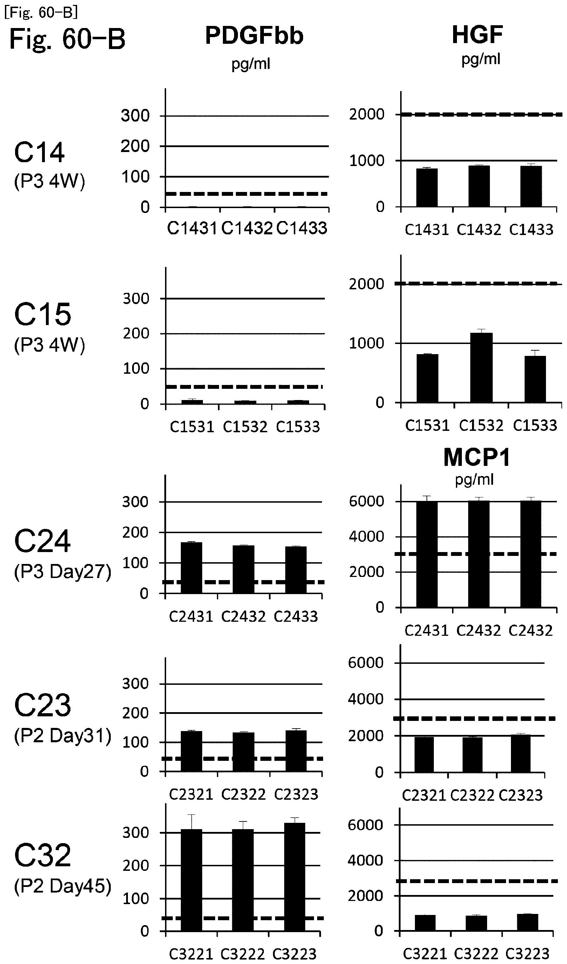

Fibroblastic transformation of cHCECs was readily detected by microscopic observation (Figs. 55-A and 55-B). As a result, a relevant and more difficult quality control is the discrimination of cHCECs that have not undergone CST from those with cellular senescence (Figs. 60-A and 60-B).

EMT is abnormally induced in many diseases. Cultured HCECs have a tendency towards CST into EMT. Cell-to-cell adhesion decreases in the process of EMT. Cells that have undergone EMT frequently gain stem cell-like properties (Krawczyk N, Meier-Stiegen F, Banys M, et al. Biomed Res Int. 2014; 2014:415721.), including those induced by TGF-beta. At the beginning of this study, this EMT-like CST was frequently observed, but after improvement in culture protocols, many cultures tended to exhibit senescent CST.

Senescence is regulated in a stimulus-dependent manner by a signaling network involving the tumor suppressors p53 and pRb (Sheerin AN, et al., Aging Cell. 2012; 11: 234-240). However, senescent cells are metabolically active, thereby propagating the senescence process to neighboring cells, notably via the secretion of a vast number of inflammatory mediators called SASP mediators (Lasry A, Ben-Neriah Y. Cell, 2015; 36:217-228).

In this Example, a candidate of monitoring the cell quality in a non-invasive manner has been successfully provided. Comparative studies of gene and miRNA signatures of diverse cHCECs made it possible to successfully develop an ELISA assay for distinguishing the quality of cHCECs. SASP was selected in parallel. An assay using secreted miRNA is published elsewhere. A method for the discrimination of whether cHCECs have undergone CST from culture supernatants is described herein for the first time.

This quality test assay allows providing high quality cHCECs for cell infusion therapy for tissue damage of HCECs due to Fuchs endothelial corneal dystrophy, trauma, or surgical intervention.

### (Example 9: Analysis of exosomes)

In this Example, exosomes were analyzed. In particular, CD63 and CD9 were analyzed.

### (Materials and Methods)

### Human corneal endothelial cell donors

The human tissue used in this Example was handled in accordance with the ethical tenets set forth in the Declaration of Helsinki. HCECs were obtained from 20 human cadaver corneas and were cultured before performing karyotype analysis. Human donor corneas were obtained from SightLife Inc. (Seattle, WA, USA). Informed written consent for eye donation for research was obtained from the next of kin of all deceased donors. All tissues were recovered under the tenets of the Uniform Anatomical Gift Act (UAGA) of the state in which the donor consent was obtained and the tissue was recovered.

The donor age ranged from 2 to 75 years (average 43.7 +/- 26.4). Both males and females were included. All donor corneas were preserved in Optisol GS (Chiron Vision, Irvine, CA, USA) and imported by airplane for research purposes. According to the donor information, all donor corneas were considered healthy without corneal disease and all donors had no past history of chromosomal abnormality.

### Cell Cultures of HCECs

Unless noted otherwise, the HCECs were cultured according to published protocols, with some modifications. Human donor corneas at distinct ages were used for the experiments. Briefly, the Descemet's membranes with CECs were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. A conditioned medium was prepared as previously described (Nakahara, M. et al. PLOS One (2013) 8, e69009). The HCECs were cultured using the conditioned medium at 37.degrees.C in a humidified atmosphere containing 5% CO₂. The culture medium was changed twice a week. The HCECs were subcultured at ratios of 1:3 using 10x TrypLE Select (Life Technologies) for 12 minutes at 37.degrees.C when they reached confluence. The HCECs at passages 2 through 5 were used for all experiments.

### Isolation of exosomes

Total exosome isolation from cell culture media (Invitrogen) was used to isolate exosomes from culture supernatant of each cell according to the production protocol.

### Western blot

Exosomes were isolated from culture supernatant of each cell or an antibody specific to a representative surface marker of exosomes (anti-CD63 antibody, anti-CD9 antibody, and anti-CD81 antibody) was used for Western blotting analysis.

Qubit Protein Assay kit (Q33211, Life Technologies) was used to measure the protein concentration of the aforementioned exosome solution. Samples were prepared under 10 .micro.g of protein per sample/nonreducing conditions based on the measured concentration. The samples were heated at 70.degrees.C for 10 minutes. The heated samples were loaded into Bolt 4-12% Bis-Tris Plus gel (NW04120BOX Invitrogen). The samples were subjected to electrophoresis at 165V for 35 minutes at 60 mA (1 mini gel) or 130 mA (2 mini gels). iBlot 2 Dry Blotting System (IB21001 Life Technologies) was used to transcribe proteins in pre-run-gel to the PVDF membrane (iBlot2 PVDF Regular Stacks IB24001 Life Technologies). The transcription conditions were 20 V for 1 minute, 23 V for 4 minutes, and 25 V for 2 minutes.

An iBind Western System (SLF1000S, Life Technologies) was used to carry out blocking and primary antibody and secondary antibody reactions. The reactions were carried out by using 2 ml of primary antibody specific to a target thereof at a final concentration of 10 ug/ml. HRP labeled anti-mouse antibodies were used as the secondary antibody at a 1000 to 2000-fold dilution. Antigen chemiluminescent detection using HRP reactions was carried out as a detection using a Novex ECL Chemiluminescent Substrates Reagent Kit (WP20005 Invitrogen)/ ImageQuant-LAS-3000 (Fujifilm) CCD camera.

### (Results)

A Western blot detection band was found for CD63 and CD9, allowing visual comparison of the size thereof (Fig. 64-A). Such a band could not be detected for CD81 (data not shown). Fig. 64-B further shows results of the detection of CD63 and/or CD9 markers in exosomal proteins in culture supernatant using Exoscreen.

### (Summary)

Thus, it was confirmed that exosomes secreted from HCECs can be detected at least by using CD63 or CD9 as a marker.

Exosomes may be associated with the mechanism of a human functional corneal endothelial cell capable of eliciting a human corneal endothelial functional property when infused into an anterior chamber of a human eye of interest in the present invention changing into an unintended state transitioned cell. For instance, state transition may occur when an energy metabolism system is biased towards anaerobic glycose metabolism from aerobic TCA cycle of the mitochondrial respiratory system due to various stresses. The possible involvement of miR expression of miR378 or the like in such bias in metabolism is suggested. Exosomes and secreted miR may be involved in the amplification of such miR.

### (Example 10: Clinical research on infusion of human corneal endothelial property possessing functional cells for bullous keratopathy)

In this Example, the objective for the clinical researches (hereinafter, referred to as the present trials) on infusion of the human corneal endothelial property possessing functional cells of the invention for bullous keratopathy patients (applicable target patients mentioned below are collectively called as such hereinafter) was to confirm the safety and efficacy of cultured human corneal endothelial cell infusion and the adequacy of the quality standard for infused cells while targeting patients with bullous keratopathy whose only conventional therapeutic method is corneal transplantation using an allo-donor cornea (allogeneic cornea).

The present trials were conducted by the following procedure.

### (*Compliance with guidelines)

The present clinical researches were conducted in accordance with the "Declaration of Helsinki", "Act on the Safety of Regenerative Medicine", "A study on ensuring the quality and safety of pharmaceuticals and medical devices derived from the processing of allogeneic human somatic stem cells", "Viral Safety Evaluation of Biotechnology Products Derived from Cell Lines of Human or Animal Origin", and "Standard for Biological Ingredients" after receiving an approval for " Clinical research on transplantation of cultured corneal endothelial cells for bullous keratopathy" from the examination committee for human stem cell clinical studies set under the supervision of Japanese Ministry of Health, Labour,and Welfare.

### (*Trial method)

### 1. Trial protocols and medical institution performing trials

After receiving a final approval of the Health Science Council of the Minister of Health, Labour and Welfare, this study targeted 15 cases diagnosed as requiring corneal transplantation due to bullous keratopathy at the University Hospital, Kyoto Prefectural University of Medicine.

### 2. Subject patients and diseases

Subject patients were bullous keratopathy patients diagnosed as requiring corneal transplantation surgery, who have given a written consent prior to the participation in the present trials and comply with all of the following criteria for determining eligibility: 1) best corrected visual acuity is less than 0.5, 2) a corneal endothelial cell cannot be observed with a corneal endothelial specular microscope or endothelial cell density is less than 500 cells/mm²; 3) corneal thickness of 630 .micro.m or greater and presence of corneal epithelial edema; 4) patients who are 20 years old or older and less than 90 years old as of giving the consent; and 5) patients who have given a written consent by themselves or by a legal representative; and who do not fall under any of the following exclusion criteria: 1) patients with an active corneal infection (bacteria, fungus, virus or the like); 2) patients who are or may be pregnant, or patients who are lactating; 3) patients with a hemorrhagic disease; 4) patients who were determined by the attending physician to be incapable of sufficient understanding or cooperation due to metal disability (including medium and severe dementia); 5) glaucoma patients with impaired intraocular pressure control; 6) diabetes patients with impaired blood sugar control; 7) patients with hypersensitivity to steroid agents; 8) patients with systemic autoimmune disease complications (SLE, Behcet's disease, or the like); 9) patients who are strongly suspected of having vision impairment due to another cause; 10) patients who have already undergone therapy under this protocol; 11) patients who use or is scheduled to use an anticancer agent; 12) patients with a history of vascular diseases (myocardial infarction, heart failure, arrhythmia with poor control, or the like), cerebrovascular disease (stroke) (and/or complication thereof of); and 13) other patients who are determined by the physician responsible for the study or attending physician for the study as having an impediment to participate in the present therapy, such as patients who are not considered suitable for conducting this therapy due to complications or the like .

### 3. Preparation method of cultured corneal endothelial cells for the trials of this Example and dosage and administration during infusion surgery

Cultured/prepared corneal endothelial cells were infused once at 1 × 10⁶ cells/300 .micro.L into the anterior chamber in accordance with the technique of corneal transplantation surgery according to the following procedures of 1)-3).
1) Raw material corneal tissue Corneas used as raw material for subculture were received from SightLife Inc., which is an eye bank in Seattle, USA. Human corneas were determined to be suitable for corneal transplantation by diagnosis of the eligibility of the cornea donor and safety test on the extracted cornea conducted by SightLife Inc.
2) Preparation of cultured corneal endothelial cells
   Cultured corneal endothelial cells were prepared according to the Standard Operating Procedure (SOP) of the Kyoto Prefectural University of Medicine Cell Processing Center (CPC) which conforms to GMP. Briefly, corneal endothelial cells were stripped with the Descemet's membrane from human corneas and treated with collagenase A, and then suspended in a cell culture medium. The cells were seeded on a culture dish for subculture. The cells were cultured using the conditions described in the preparation method of corneal endothelial property possessing functional cells of the invention described in Example 2 or 11 in the present specification as the specific conditions. After confirming that the cells are free of contamination or abnormality when providing the cells upon transplantation surgery, the cells were recovered by TrypLE enzyme treatment and washed with phenol red-free Opti-MEM I. The cells were dispensed into Proteosave such that the number of cultured corneal endothelial cells was 1.5 × 10⁶ cells/450 .micro.L in Opti-MEM I supplemented with Y-27632 ((R)-(+)-trans-(4-pyridyl)-4-(1-aminoethyl)-cyclohexanecarboxamide dihydrochloride monohydrate) with a final concentration of 100 .micro.M to prepare a sample for transplantation.
3) Infusion of cultured corneal endothelial cells

Surgeries were performed in principle using local anesthesia. After creating an approximately 2 mm incision on the corneal limbus, a silicone needle for corneal endothelium detachment (available from Inami or the like) was used to remove degenerated corneal endothelial cells or abnormal extracellular matrix of a recipient with a diameter of 5-10 mm. After removal, the cultured corneal endothelial cells suspended in Opti-MEM I (Life Technologies) containing Y-27632 with a final concentration of 100 .micro.M were infused into the anterior chamber at 1 × 10⁶/300 .micro.L by using a 26G needle. A steroid agent (see below) was injected under the conjunctiva. Immediately after the completion of surgery, patients were asked to lie face down for three hours or longer.

### (*Examined/observed items)

### 1) Examination items and examination period

Table 9 shows the trial schedule for inspected items and inspection period.

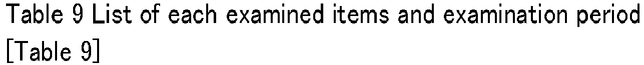

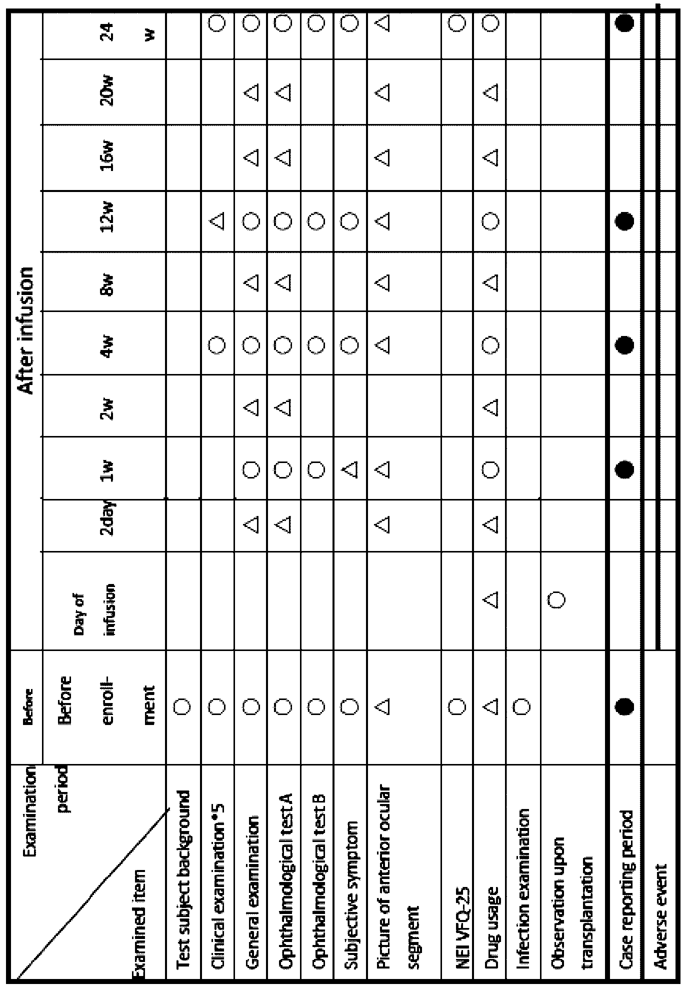

Before enrollment: any data within 4 weeks before enrollment can be used After infusion: 2 days +/- 1 day, 7 days +/- 2 days, and 14 days +/- 2 days are considered acceptable. Further, 4 weeks +/- 7 days, 8 weeks +/- 7 days, 12 weeks +/- 7 days, 16 weeks +/- 7 days, 20 weeks +/- 7 days, and 24 weeks +/- 14 days are considered acceptable. For each of 8 week, 16 week and 20 week examinations, it is acceptable to be examined by a partnering physician nearby if the patient is not able to make a long distance visit. 12 week (+/- 7 days) and 24 week (+/- 14 days) clinical examinations are conducted during continuous systemic administration of drugs. The data were also available in all 15 cases at 1 year and 8 cases at 2 years after cell infusion as a follow-up observation period.
Legend: open circle: item described in case report
triangle: item not required to be described in case report
filled circle: case reporting period, each of which is an item carried out in this Example.

It should be noted that the description of 4 weeks is synonymous with 1 month, description of 12 weeks is synonymous with three months, and description of 24 weeks is synonymous with 6 months in this Example and related diagrams.
<1> As the test subject background, circumstances of the original disease, history of corneal transplantation and ocular surgery, and presence of systemic disease/drug allergy were examined by interview and diagnosis.
<2> As clinical examination prior to surgery, the following were examined: (1) hematological examination: red blood cell count, white blood cell count, amount of hemoglobin, hematocrit value, platelet count, differential white blood cell count [INR (PT/APTT), fibrinogen]; (2) blood biochemical inspection: blood sugar, total cholesterol, triglyceride, total protein, albumin, creatinine, total bilirubin, GOT, GPT, gamma-GTP, LDH, and ALP; and (3) infection examination: presence of HBV, HCV, HIV, HTLV, syphilis infection, and active corneal infection (bacteria, fungus, virus, or the like) was examined.
<3> As the general ophthalmic exmaination, visual acuity was measured as decimal visual acuity. The corneal thickness was chronologically measured using a Pentacam. The corneal endothelial cell examination was conducted by using a non-contact or contact specular microscope, and the ocular pressure was measured using a non-contact or contact tonometer.
<4> Ophthalmological examination A was conducted by classifying and scoring observation of the cornea by a slit lamp microscope (epithelial edema, epithelial disorder, stromal edema, and stromal opacity), anterior chamber observation, and conjunctiva observation (conjunctival hyperemia, and conjunctival edema) into 4 levels, i.e., 0: none, 1: mild, 2: moderate, and 3: severe, and observation of the anterior ocular segment was recorded by taking pictures.
<5> For the ophthalmological observation B, slit lamp microscope examination or visual field examination and funduscopy was conducted with respect to iris observation, cataract, glaucoma, and retinal diseases that can affect vision for determination into 4 levels, i.e., 0: none, 1: mild, 2: moderate, and 3: severe.
<6> To evaluate the relationship between change in visual function before and after transplantation and QOL, a survey was conducted by NEI VFQ-25 (The 25-item National Eye Institute Visual Function Questionnaire).

### 2) Adverse events

All unfavorable or unintended indications, symptoms or diseases that occur during the follow up of the present trials are considered adverse events. Reactions whose causal relationship with transplantation technique, transplanted cells or overall therapy associated with the protocol therapy cannot be denied were considered side effects. In case of an adverse event, the name of the event, date of onset, judgment with respect to severe/non-severe related to death/hospitalization/irreversible damage, severity, confirmation date of outcome of an adverse event, and outcome were judged in 4 levels, i.e., eliminated, alleviated, no change, and exacerbated. In case an adverse event is found, a follow-up investigation was conducted until the outcome was definitive regardless of the presence of a causal relationship with the present infusion therapy. The causal relationship with the infusion technique, infused cells or associated therapy was recorded for adverse events whose causal relationship with the present therapy cannot be denied.

### 3) Combined drug and combined treatment

Combined therapy was performed by systemic and topical administration of adrenocortical steroid agent (methylprednisolone intravenous injection, betamethasone intravenous injection/oral administration, betamethasone eye drop, or fluorometholone eye drop) for suppressing rejection and controlling post-operation inflammation and antibiotics or synthetic antimicrobial agent (flomoxef sodium intravenous injection, cefcapene pivoxil oral administration, or gatifloxacin eye drop) as prophylaxis for post-operation infection, in accordance with drug dosing regimen in normal corneal transplantation. Anticancer agents for treating malignant tumor, administration of a drug in the vitreous body, or the like during the following-up period and invasive treatment/surgery such as cataract surgery on the transplanted eye was prohibited.

### (* Evaluation method and statistical approach)

All cases in which cultured corneal endothelial cells were infused were considered the population subjected to analysis. The proportion of number of cases with the corneal endothelial cell density 24 weeks post infusion surgery, used as a primary end point, of 500 cells/mm² and the 95% confidence interval thereof, change in the corneal thickness from before infusion to 24 weeks after infusion and the 95% confidence interval thereof, and the proportion of number of cases with corneal thickness 24 weeks after infusion of 650 .micro.m or less and the 95% confidence interval thereof were calculated.

As secondary endpoints, the proportion of cases achieving improvement in vision of 2 levels or more from before infusion to 24 weeks after infusion and the 95% confidence interval thereof, the proportion of cases where the sum of the scores for corneal stromal edema + opacity from before infusion to 24 weeks after infusion improved 1 point or more and the 95% confidence interval thereof, and the proportion of cases with improvement in the VFQ-25 score from before infusion to 24 weeks after infusion and the 95% confidence interval thereof were calculated.

In order to explore and examine the relationship between the specification of cultured endothelial cells and clinical effects, stratification analysis was performed between the proportion of E-ratios of endothelial cells used as infused cells and post-operation corneal endothelial cell density and corneal thickness thinning.

For adverse events, the proportion of adverse event cases that had occurred between the start of protocol therapy, through transplantation surgery, and 24 weeks after transplantation and the 95% confidence interval thereof were calculated.

### (* Basis for setting target number of cases)

The safety and efficacy of cultured corneal endothelial cell infusion surgery targeting bullous keratopathy are explored. 15 cases considered necessary for examining the validity of setting the standard for cultured corneal endothelial cells were determined.

### (Results) Table 10 shows the list of 15 cases where corneal endothelial cell infusion surgery was performed in this Examples with the results thereof.

Reported below are observation/evaluation up to two years post operation of the primary endpoints, change in the corneal endothelial cell density and corneal thickness and secondary endpoints, visual acuity and corneal observation/measurement in corneal endothelial cell infusion surgery cases subjecting a total of 15 cases (average 67.3 +/- 11.4 years old) consisting of 7 males and 8 females who were enrolled according to the inclusion/exclusion standards after receiving a written consent.

**[Table 10-1]**

| **List of cultured endothelial cell infusion surgery** | | | | | | | | |
|---|---|---|---|---|---|---|---|---|
| **Patient#** | **Age** | **Sex** | **Disease** | **Property of infused cell** | | | | |
| | | | | **E-ratio** | **corneal endothelial property possessing functional cell** | **CD44 intermediately positive** | **CD44 intermidiately positive** | **CD44strongly positive** |
| | | | | | | **CD24negative** | **CD24positive** | **CD24positive** |
| | | | | | | **CD26negative** | **CD26negative** | **CD26negative** |
| | | | | | **functional cell + Semi-functional cell** | **(intermediately differentiated corneal endothelial cell)** | | |
| **A** | **70s** | **Male** | **Pseudoexfoliation syndrome** | **14.2** | **86** | **71.8** | **0.5** | **0.9** |
| **B** | **40s** | **Male** | **Fuchs corneal dystrophy** | | | | | |
| **C** | **70s** | **Female** | **Laser iridotomy** | **55.5** | **96** | **40.5** | **1.4** | **0.9** |
| **D** | **70s** | **Female** | **Fuchs corneal dystrophy,** | | | | | |
| **E** | **50s** | **Female** | **Fuchs cortical dystrophy** | **77** | **97.4** | **20.4** | **2.3** | **0.3** |
| **F** | **60s** | **Male** | **After multiple surgeries** | | | | | |
| **G** | **80s** | **Female** | **Fuchs corneal dystrophy** | **40.4** | **96.2** | **55.8** | **0** | **0** |
| **H** | **50s** | **Female** | **Fuchs corneal dystrophy** | | | | | |
| **I** | **60s** | **Female** | **Fuchs corneal dystrophy** | **93.9** | **93.9** | **0** | **0.3** | **0** |
| **J** | **70s** | **Female** | **Laser iridotomy** | | | | | |
| **K** | **70s** | **Male** | **After corneal infusion surgery** | | | | | |
| **L** | **70s** | **Female** | **Laser iridotomy** | **99.2** | **99.3** | **0.1** | **0.4** | **0** |
| **M** | **70s** | **Male** | **After pseudophakic** | | | | | |
| **N** | **70s** | **Male** | **After corneal infusion surgery** | | | | | |
| **O** | **40s** | **Male** | **Congenital corneal endothelial** | | | | | |

**[Table 10-2]**

| **Cornjeal endothelial cell density** | | | **cells/mm²** | | | **Changes in corneal thickness** | | | **µm** | |
|---|---|---|---|---|---|---|---|---|---|---|
| **Week 4 (ECD)** | **Week 12 (ECD)** | **Week 24 (ECD)** | **1 year (ECD)** | **2 years (ECD)** | **Before infusion** | **Week 4** | **Week 12** | **Week 24** | **1 year** | **2 years** |
| **Unable to measure** | **1577** | **1271** | **1542** | **871** | **792** | **752** | **667** | **640** | **658** | **710** |
| **Unable to measure** | **1437** | **1134** | **1067** | **959** | **637** | **534** | **501** | **509** | **512** | **529** |
| **2268** | **2703** | **2232** | **2096** | **1447** | **775** | **522** | **517** | **505** | **526** | **525** |
| **Unable to measure** | **1942** | **2288** | **1757** | **1316** | **750** | **671** | **600** | **626** | **580** | **550** |
| **Unable to measure** | **3571** | **2833** | **2591** | **2188** | **657** | **627** | **475** | **489** | **490** | **503** |
| **Unable to measure** | **2463** | **1880** | **1961** | **1546** | **649** | **701** | **648** | **595** | **598** | **543** |
| **2326** | **2695** | **2141** | **2028** | **1600** | **741** | **521** | **545** | **539** | **542** | **523** |
| **Unable to measure** | **2247** | **2331** | **1996** | **1610** | **725** | **605** | **586** | **561** | **577** | **572** |
| **3484** | **2647** | **2857** | **2924** | | **681** | **571** | **561** | **544** | **556** | |
| **3155** | **3067** | **2710** | **2746** | | **824** | **564** | **548** | **541** | **529** | |
| **3333** | **3021** | **2950** | **3040** | | **774** | **532** | **553** | **519** | **527** | |
| **3584** | **3115** | **3546** | **3226** | | **780** | **557** | **535** | **544** | **557** | |
| **4854** | **4348** | **4149** | **4184** | | **792** | **569** | **554** | **556** | **550** | |
| **2804** | **3584** | **3788** | **2283** | | **806** | **584** | **584** | **543** | **549** | |
| **4016** | **3534** | **3115** | **2924** | | **792** | **637** | **666** | **643** | **647** | |

**[Table 10-3]**

| **Corenal thicness Δ% change** | | | | | **Change in corrected visual acuity (logMAR visual acuity)** | | | |
|---|---|---|---|---|---|---|---|---|
| **Week 4** | **Week 12** | **Week 24** | **1 year** | **2 years** | **Observation date** | | | |
| | | | | | **Before infusion** | **Week 4** | **Week 12** | **Week 24** |
| **-5.1** | **-15.8** | **-19.2** | **-16.9** | **-10.4** | **1.00** | **1.15** | **0.52** | **0.40** |
| **-16.2** | **-21.4** | **-20.1** | **-19.6** | **-17.0** | **0.40** | **1.00** | **0.40** | **0.00** |
| **-32.6** | **-33.3** | **-34.8** | **-32.1** | **-32.3** | **1.00** | **0.40** | **0.10** | **0.10** |
| **-10.5** | **-20.0** | **-16.5** | **-22.7** | **-26.7** | **0.52** | **0.52** | **0.52** | **0.52** |
| **-4.6** | **-27.7** | **-25.6** | **-25.4** | **-23.4** | **0.70** | **1.40** | **0.40** | **0.30** |
| **8.0** | **-0.2** | **-8.3** | **-7.9** | **-16.3** | **0.40** | **1.00** | **0.30** | **0.30** |
| **-29.7** | **-26.5** | **-27.3** | **-26.9** | **-29.4** | **0.70** | **0.22** | **0.10** | **0.10** |
| **-16.6** | **-19.2** | **-22.6** | **-20.4** | **-21.1** | **1.52** | **0.40** | **0.05** | **0.05** |
| **-16.2** | **-17.6** | **-20.1** | **-18.4** | | **0.40** | **0.15** | **0.10** | **0.05** |
| **-31.6** | **-33.5** | **-34.3** | **-35.8** | | **1.40** | **1.00** | **0.52** | **0.30** |
| **-31.3** | **-28.6** | **-32.9** | **-31.9** | | **1.40** | **0.70** | **1.10** | **1.00** |
| **-28.6** | **-31.4** | **-30.3** | **-28.6** | | **0.70** | **0.22** | **0.10** | **0.15** |
| **-28.2** | **-30.1** | **-29.8** | **-30.6** | | **2.00** | **1.22** | **0.52** | **0.52** |
| **-27.5** | **-27.5** | **-32.6** | **-31.9** | | **1.70** | **1.22** | **1.00** | **0.82** |
| **-19.6** | **-15.9** | **-18.8** | **-18.3** | | **0.70** | **0.52** | **0.70** | **0.30** |

**[Table 10-4]**

| **Corneal stromal edema score** | | | | **Corneal stroma opacity score** | |
|---|---|---|---|---|---|
| **Observation date** | | | | **Observation date** | |
| **Before infusion** | **Week 4** | **Week 12** | **Week 24** | **Before infusion** | **Week 24** |
| **2** | **2** | **1** | **0** | **2** | **0** |
| **2** | **1** | **0** | **0** | **2** | **0** |
| **2** | **0** | **0** | **0** | **2** | **0** |
| **2** | **2** | **1** | **1** | **1** | **1** |
| **2** | **2** | **0** | **0** | **2** | **0** |
| **2** | **1** | **0** | **0** | **2** | **0** |
| **2** | **0** | **0** | **0** | **1** | **0** |
| **2** | **1** | **0** | **0** | **2** | **0** |
| **2** | **1** | **0** | **0** | **1** | **0** |
| **2** | **0** | **0** | **0** | **0** | **0** |
| **3** | **1** | **0** | **0** | **1** | **0** |
| **2** | **0** | **0** | **0** | **0** | **0** |
| **3** | **0** | **0** | **0** | **0** | **0** |
| **3** | **1** | **0** | **0** | **0** | **0** |
| **3** | **1** | **0** | **0** | **1** | **1** |

Next, Table 11 shows an assessment item, the distribution of corneal endothelial cells.

**[Table 11]**

| **Distribution of corneal endothelial cell density after infusion surgery** | | | | | | | | |
|---|---|---|---|---|---|---|---|---|
| **Endothelium density** | | **Unable to measure** | < 1000 | 1000 ≦ | 2000 ≦ | 3000 ≦ | 4000 ≦ | **Total** |
| **4 weeks after surgery** | **Total** | **6** | | | **3** | **4** | **2** | **15** |
| | E-R < 90 | 6 | | | 2 | | | 8 |
| | E-R ≧ 90 | | | | 1 | 4 | 2 | 7 |
| **12 weeks after surgery** | **Total** | | | **3** | **5** | **6** | | **15** |
| | E-R < 90 | | | 3 | 4 | 1 | | 8 |
| | E-R ≧ 90 | | | | 1 | 5 | 1 | 7 |
| **24 weeks after surgery** | **Total** | | | **3** | **8** | **3** | **1** | **15** |
| | E-R < 90 | | | 3 | 5 | | | 8 |
| | E-R ≧ 90 | | | | 3 | 3 | 1 | 7 |
| **1 year after surgery** | **Total** | | | **5** | **7** | **2** | **1** | **15** |
| | E-R < 90 | | | 5 | 3 | | | 8 |
| | E-R ≧ 90 | | | | 4 | 2 | 1 | 7 |
| **2 years after surgery** | **Total** | | **2** | **5** | **1** | | | **8** |
| | E-R < 90 | | 2 | 5 | 1 | | | 8 |
| | E-R ≧ 90 | | | | | | | |

Follow up observation one year after surgery was completed for 15 cases (2 years for 8 cases), which resulted in endothelial cell densities of 1000 cells/mm² or greater in all 15 cases. The densities recovered to normal or Grade 1 (mild) under the severity classification of corneal endothelial discorders (Japanese Journal of Ophthalmology 118: 81-83, 2014). For the 15 cases subjected to examination in this Example, the corneal endothelial cell density pre-surgery could not be observed/measured with a specular microscope (hereinafter, specular) due to pre-surgery corneal stromal edema and opacity and the cases were diagnosed as bullous keratopathy with severity classification of Grade 4. These cases were confirmed to recover back to severity classification Grade 1 from 4 weeks to 24 weeks after surgery, and 12 out of 15 cases, i.e., 80.0% (95% confidence interval: 51.9-95.7%) maintained endothelial density of 2000 cells/mm² or greater, which is considered a normal cornea without any issue in maintaining endothelial function of a cornea.

Table 12 shows a primary endpoint, the change in cornea thickness.

For corneas with corneal stromal edema and opacity, the corneal thickness decreased to 650 .micro.m or less, which is a general standard for observation of an abnormality in the corneal thickness in the US, in 12 out of 15 cases, i.e., 80.0% (95% confidence interval: 51.9-95.7%), 4 weeks after infusion surgery or 13 out of 15 cases, i.e., 86.7% (95% confidence interval: 59.5-98.3%), 12 weeks after transplantation surgery. All 15 cases cleared this standard 24 weeks after surgery. The corneal thickness of 745 +/- 61 .micro.m before surgery exhibited statistically significant (p < 0.001) thinning to 596 +/- 69 .micro.m 4 weeks after surgery, and then to 569 +/- 57 .micro.m 12 weeks after surgery, then gradually decreased to 557 +/- 48 .micro.m 24 weeks after surgery, and finally to a stable corneal thickness that was almost normal. See Table 12 for further results (1 and 2 years).

**[Table 12]**

| **Change in corneal thickness before and after surgery** | | | | | | | | | | | |
|---|---|---|---|---|---|---|---|---|---|---|---|
| | | **Unable to measure** | < 500 | 500≦ | 550≦ | 600≦ | 650≦ | 700≦ | 750≦ | 800≦ | **Total** |
| **Before surgery** | **Total** | | | | | **2** | **2** | **2** | **7** | **2** | **15** |
| | E-R < 90 | | | | | 2 | 1 | 2 | 3 | | 8 |
| | E-R ≧ 90 | | | | | | 1 | | 4 | 2 | 7 |
| **4 weeks post surgery** | **Total** | | | **4** | **5** | **3** | **1** | **1** | **1** | | **15** |
| | E-R < 90 | | | 3 | | 2 | 1 | 1 | 1 | | 8 |
| | E-R ≧ 90 | | | 1 | 5 | 1 | | | | | 7 |
| **12 weeks post surgery** | **Total** | | 1 | 5 | 5 | 2 | 2 | | | | **15** |
| | E-R < 90 | | 1 | 3 | 1 | 2 | 1 | | | | 8 |
| | E-R ≧ 90 | | | 2 | 4 | | 1 | | | | 7 |
| **24 weeks post surgery** | **Total** | | **1** | **8** | **3** | **3** | | | | | **15** |
| | E-R < 90 | | 1 | 3 | 2 | 2 | | | | | 8 |
| | E-R ≧ 90 | | | 5 | 1 | 1 | | | | | 7 |
| **1 year post surgery** | **Total** | | **1** | **6** | **6** | **1** | **1** | | | | **15** |
| | E-R < 90 | | 1 | 3 | 3 | | 1 | | | | 8 |
| | E-R ≧ 90 | | | 3 | 3 | 1 | | | | | 7 |
| **2 years post surgery** | **Total** | | | **5** | **2** | | | **1** | | | **8** |
| | E-R < 90 | | | 5 | 2 | | | 1 | | | 8 |
| | E-R ≧ 90 | | | | | | | | | | |

Table 13 shows a secondary endpoint, the change in LogMAR visual acuity.

Follow up observation 24 weeks after surgery was completed for 15 cases, which showed significant (p < 0.001) improvement in vision 12 weeks or more after surgery, as the mean LogMAR vision was 0.43 +/- 0.33 after 12 weeks and 0.33 +/- 0.29 after 24 weeks to 0.97 +/- 0.52 before transplantation surgery. Improvement of 5.4 levels in average was observed after 12 weeks and 6.4 levels after 24 weeks (95% confidence interval: 4.2 to 8.7 levels) relative to the vision before surgery. 13 out of 15 cases, i.e., 86.7% (95% confidence interval: 59.5-98.3%), saw improvement in vision of 2 or more levels after 24 weeks. It is now possible to alleviate/thin out corneal edema with regeneration of corneal endothelial cells by infusion therapy. This is considered a result of such histological or morphological improvement being reflected in vision which is directly linked to the QOL of patients. Decimal vision is shown in the parentheses in the Table.

**[Table 13]**

| **Change in logMAR visual acuity ^{∗1)}** | | | | | | | | |
|---|---|---|---|---|---|---|---|---|
| LogMAR (Decimal visual acuity) | | ≦ 0.00 | ≦ 0.15 | ≦ 0.30 | ≦ 0.52 | ≦ 1.00 | 1.00 < | **Total** |
| | | (≧1.0) | (≧0.7) | (≧0.5) | (≧0.3) | (≧0.1) | (0.1>) | |
| **Before surgery** | **Total** | | | | **4** | **6** | **3** | **15** |
| | E-R<90 | | | | 3 | 4 | 1 | 8 |
| | E-R ≧ 90 | | | | 1 | 2 | 4 | 7 |
| **24 weeks after surgery** | **Total** | **1** | **5** | **4** | **3** | **2** | | **15** |
| | E-R<90 | 1 | 3 | 2 | 2 | | | 8 |
| | E-R ≧ 90 | | 2 | 2 | 1 | 2 | | 7 |

| | | | | | | | | |
|---|---|---|---|---|---|---|---|---|
| ^{∗1)} Decimal visual acuity is shown in parenthesis | | | | | | | | |

Table 14 shows the distribution of improvement points of the total score of corneal stromal opacity and stromal edema by a slit lamp microscope. Among the 15 cases with completed follow-up of 24 weeks after transplantation surgery, 13 out of 15 cases, i.e., 86.7% (95% confidence interval: 59.5-98.3%) saw notable improvement where the total score of corneal stroma observation was 0. All 15 cases exhibited significant improvement of 1 point or more in the sum of score totals, which is a secondary endpoint.

**[Table 14]**

| **Corneal stroma (opacity+edema)** | | | | **Change in score** | | | | |
|---|---|---|---|---|---|---|---|---|
| Opacity+edema score | | 5 | 4 | 3 | 2 | 1 | 0 | **Total** |
| **Score before surgery** | **Total** | | **8** | **5** | **2** | | | **15** |
| | E-R<90 | | 6 | 2 | | | | 8 |
| | E-R≧90 | | 2 | 3 | 2 | | | 7 |
| **Score 24 weeks post surgery** | **Total** | | | | **1** | **1** | **13** | **15** |
| | E-R<90 | | | | 1 | | 7 | 8 |
| | E-R≧90 | | | | | 1 | 6 | 7 |

In this Example, all specifications of corneal endothelial cells used in the 15 cases of corneal endothelial cell infusion surgery had a newly established ratio of functional cells to corneal endothelial property possessing functional cells combined with semi-functional cells of over 90%.

However, E-Ratios indicating the proportion of functional cells were widely distributed, from 10%=< to 90%=<. Thus, the effect of E-Ratios on corneal thickness and corneal endothelial cells was compared and studied by separating the cells into 2 groups consisting of E-Ratio >=90% group and E-Ratio < 90 group.

The E-Ratio < 90% group consists of cases A-H. The corneal thickness decreased to less than 600 .micro.m as of week 4 after surgery in patients of 3 out 8 cases, and the corneal thickness reached the standard of less than 650 .micro.m, which is an indicator and a primary endpoint, in patients in 5 cases. Meanwhile in cases I-O of the E-Ratio >=90% group, corneal thickness, the primary endpoint, reached the standard of less than 650 .micro.m 4 weeks after surgery in patients of all 7 cases. The corneal thickness further recovered to less than 600 .micro.m in 6 out of 7 cases.

Fig. 69 is a graph showing the change in corneal thickness before infusion, 4 weeks, 12 weeks, and 24 weeks after infusion of patients having a cell population with an E-Ratio of less than 90% infused in (patients A-H) and patients having a cell population with an E-ratio of 90% or greater infused in (patients I-O). It can be understood that thinning of corneas is achieved early by the corneal endothelial property possessing functional cells of the invention. Decrease in the corneal thickness is prominent in the group of patients I-O subjected to infusion surgery (with subpopulation selection, E-Ratio >=90%) by the present invention. As shown in Fig. 69, significant thinning in the corneal thickness was observed, 747 +/- 63 .micro.m before surgery to 596 +/-69 .micro.m at 4 weeks after surgery. It was confirmed that 6 out of 7 cases reached a level around 550 .micro.m after 24 weeks, which can be considered nearly normal corneal thickness.

Fig. 70 compares a conventional method (method without subpopulation selection) with cases of infusing cells prepared by subpopulation selection of the present invention into the anterior chamber. Fig. 70 shows results of infusion surgery (without subpopulation selection) by a conventional method on the top row, results of infusion surgery (with subpopulation selection, E-Ratio < 90%) by the present invention in the middle row, and results of infusion surgery (with subpopulation selection, E-Ratio >=90%) by the present invention in the bottom row. From the left side of each row, results of phase contrast microscope pictures during subculture, results of FACS analysis based on CD24, CD26, and CD44 of infused cells, and numerical values (cells/mm²) of corneal endothelial cell density and pictures from specular after surgery on the right side. When a cell population prepared by a conventional method is infused, some cases (albeit in small number of cases) are found where it is difficult to take pictures of corneal endothelial cells with a specular even 12 weeks after surgery due to the effect of corneal stromal opacity or edema. However, when infusion surgery (with subpopulation selection, E-Ratio < 90%) according to the present invention is used, endothelial cells could be observed with a specular from 3 months after surgery. As shown in the bottom row, when infusion surgery (with subpopulation selection, E-Ratio >=90%) is used, it was possible to take clear pictures and observe corneal endothelial cells with a specular at 4 weeks after surgery, In the E-R< 90 group, pictures could be taken with a specular 1 month after surgery in 2 out of 8 cases (25.0%), whereas the same was possible in all 7 infusing cases in the E-R >=90% group. Further, the average endothelial density of 3604 +/- 666 cells/mm² was attained in the E-R >=90% group at 4 weeks after surgery, which is histological regeneration to the same level as the endothelial cell density of young individuals. Pictures could be taken with a specular in all 8 cases in E-R<90% at 12 weeks after surgery. The mean endothelial cell density recovered to a normal endothelial density of 2329 +/- 696 cells/mm². Meanwhile in E-R >=90%, a more significant (p = 0.00899) corneal endothelial cell density than E-R <90% was also confirmed at 3331 +/- 551 cells/mm² as of 12 weeks after surgery. At 24 weeks after surgery which is the final observation time period, a more significant (p < 0.001) corneal endothelial cell density than E-R <90% was maintained, i.e., 2014 +/- 567 cells/mm² for E-R <90% to 3302 +/- 535 cells/mm² for E-R >=90%. It was revealed that a notable effect is attained earlier for cells prepared by subpopulation selection of the present invention relative to conventional methods. Fig. 71 shows post-surgery anterior ocular segment slit observation in the corneal endothelial cell infusing therapy of the present invention, DSAEK (conventional method) and PKP (penetrating keratoplasty, conventional method). After the endothelial cell infusion therapy of the present invention, the possibility of irregular astigmatism or notable distortion at the infusion sutures as in PKP is low. Further, it is considered a surgical method with few instances of steps or distortion at the corneal endothelial surface as in DSAEK. For a corneal endothelial specular 24 weeks after transplantation, a main assessment item corneal endothelial cell density exceeds the standard of 500 cells/mm² before 6 months, and all 15 cases achieved an endothelial cell density of 1000 cells/mm². In the stratification by E-Ratios, 5 out of 8 cases of patients A-H in the E-Ratio < 90% group recovered to the level of 2000 cells/mm² or greater, which is classified as normal corneal endothelium in the severity classification of corneal endothelial disorder, at 24 weeks after surgery. Further, the endothelial cell density of 2500 cells/mm² or greater was maintained in all patients in 7 cases as of 24 weeks after surgery for I-O in the patient group with an E-Ratio raised to 90% or higher. Furthermore, 4 out of 7 cases attained a very high level of corneal endothelial cell density of 3000 cell/mm² or greater as in a young individual to demonstrate a significant therapeutic outcome.

Further, conventional methods such as DSAEK or penetrating keratoplasty (PKP) cannot avoid warps in corneas. However, the method of this Example can be confirmed to be a therapeutic method with very minimal invasiveness to the cornea for reverting the curvature of the cornea after surgery to a value inherent for the organism as can be seen from the anterior ocular segment slit picture and anterior ocular segment OCT image from different surgical methods taken after surgery (Fig. 71). Further, cases K and N are patients who exhibited a rejection in corneal transplantation. Conventional techniques were considered to lack an effective therapeutic method against such cases. However, the infusion therapy with cells subjected to subpopulation selection of this Example induced immunological tolerance in the anterior chamber and shows smooth progress in recovery in corneal endothelial density and corneal thickness despite being cases exhibiting a rejection up to this point. It is thus proven that such therapy can be a revolutionary and safe and effective therapy for patients to replace conventional DSAEK, DMEK, or PKP.

Adverse events included one case of non-severe elevation in ocular pressure occurring in a 57 year old female as of 3 months after surgery, which is thought to be due to steroid eye drops in a combined therapy drug. The betamethasone eye drop was changed to a flumetholon eye drop and anti-glaucoma drug Xalacom was concomitantly used for the elevation in ocular pressure to end observation of progress in accordance with a 24 week protocol. No other adverse event though to be due to cultured corneal endothelial cells used in therapy such as an intraocular inflammation, infection or, iatrogenic event associated with a infusion technique was observed.

The above results demonstrate that the corneal endothelial property possessing functional cells and functional mature differentiated corneal endothelial cells of the present invention achieve an excellent therapeutic effect compared to conventional techniques. For cellular medicaments prepared based on the subpopulation classification of the present invention, it was found that the therapeutic results have improved overall relative to cases using a cell population considered to have a corneal endothelial function obtained by a conventional cell preparation method, which do not use subpopulation classification. In particular, a case was found as shown in Fig. 70 where corneal endothelial cells could not be observed due to the effects of corneal stromal opacity after three months when injection therapy was administered in cases without subpopulation classification, while corneal stroma edema/opacity was eliminated 3 months after surgery with medicaments using the corneal endothelial property possessing functional cells and/or functional mature differentiated corneal endothelial cells of the present invention that were prepared with subpopulation classification of the present invention, thus demonstrating the possibility of a high quality, revolutionary therapeutic method capable of restoring the transparency of a cornea early in 1 month after surgery by raising the E-Ratio.

In particular, subpopulation classification has enabled the identification of E-Ratio or the ratio of the corneal endothelial property possessing functional cells of the invention and the like. In this manner, the inventors have discovered in the present invention that therapeutic results can be improved by raising the E-Ratio or the ratio of the corneal endothelial property possessing functional cells of the invention. This was not revealed by conventional cell preparation techniques, or cell classification or selection approach. Thus, the effect as cells based on subpopulation classification, manufacturing method thereof, and cellular medicament according to the present invention were proven to reveal that they should be an indirect or direct indicator for quality control.

As discussed above, one case of non-severe elevation in intra-ocular pressure due to combined therapy occurred, but a severe adverse event was not observed in any case where corneal endothelial cell infusion surgery was administered. Further, mental pressure while waiting for the availability of a donor cornea and physical burden during surgery are alleviated. It is thus understood that QOL attained dramatically improved from the corneal endothelial therapy by the present invention.

### (Example 11: Manufacturing method of cells)

This Example summarizes below a manufacturing method of human cultured corneal endothelial cells aiming for the treatment of bullous keratopathy.

A single layer corneal endothelial layer was stripped from a cornea, which was imported from a US eye bank SightLife Inc. and in compliance with the safety standard of the FDA and Ministry of Health, Labour and Welfare. Corneal endothelial cells were separated overnight by collagenase treatment. The cells were cultured in a Nancy medium (Invest Ophthalmol Vis Sci 2004 1743/PLoS ONE 2012 e28310) (NGF, pituitary extract free) comprising 8% FBS while using Opti-MEM as the basal medium. P0 culture was started by seeding endothelial cells from left and right eyes of the same donor in a 2 well/6 well collagen I plate. 10 .micro.M of Y-27632 (ROCK inhibitor) was added to the culture to induce differentiation into mature differentiated cells. 10 .micro.M of SB203580 was added in order to suppress cell state transition during the culture period. After the cells had reached confluence after about 4-8 weeks, the cells were passaged. After reaching confluence, the same quality was retained over several weeks only by changing the medium. In passaging, the cells were detached from the plate with TrypLE and washed with the medium, and then seeded in a T25 collagen I flask at a cell density of 400 cells/mm² or greater. While clinical researches generally use up to P2 or P3 culture cells, up to P5-P6 culture cells can be used. The main organism derived raw material used in culture is FBS (made in Australia).

### Cultures of HCECs

HCECs were cultured according to published protocols, with some modifications (Nayak SK, Binder PS. Invest Ophthalmol Vis Sci. 1984; 25:1213-6). Corneas from a total of 30 human donors of different ages were used in the experiment. Briefly, the Descemet's membranes with the CECs were stripped from donor corneas and digested at 37.degrees.C with 1 mg/mL collagenase A (Roche Applied Science, Penzberg, Germany) for 2 hours. The HCECs obtained from a single donor cornea were seeded in one well of a Type I collagen-coated 6-well cell culture plate (Corning Inc., Corning, NY, USA). The culture medium was prepared according to published protocols. Briefly, basal medium was prepared with Opti-MEM-I (Life Technologies Corp., Carlsbad, CA, USA), 8% fetal bovine serum (FBS), 5 ng/mL epidermal growth factor (EGF; Life Technologies), 20 .micro.g/mL ascorbic acid (Sigma-Aldrich Corp.), 200 mg/L calcium chloride (Sigma-Aldrich Corp., St. Louis, MO, USA), 0.08% chondroitin sulfate (Wako Pure Chemical Industries, Ltd., Osaka, Japan), and 50 .micro.g/mL of gentamicin. Conditioned medium was prepared as previously described (Nakahara, M. et al. PLOS One (2013) 8, e69009). The HCECs were cultured using the conditioned medium at 37.degrees.C in a humidified atmosphere containing 5% CO₂. The culture medium was changed twice a week. The HCECs were subcultured at a cell density of 800 cells/mm² using 10x TrypLE Select (Life Technologies) for 12 minutes at 37.degrees.C when they reached confluence. The HCECs at passages 2 through 5 were used for all experiments.

### Measurement of cell count

A cell suspension was mixed with an equal amount of 0.4% trypan blue solution (Sigma, T8154) and the cell count was calculated with a hemocytometer.

### Cell sorting

For cell sorting experiments, HCECs were collected and stained with FITC-conjugated anti-human CD24 mAb and PE-Cy7-conjugated anti-human CD44 mAb (BD Biosciences) as described above. After washing with buffer, the cells were resuspended in FACS buffer. The CD24-negative/CD44-negative cells and CD24-negative/CD44-negative cells were sorted using a BD FACSJazz cell sorter (BD Biosciences) and seeded at a density of 4.2 × 10⁴ cells on a 24-well cell culture plate for subsequent analysis.

### Flow cytometry analysis of cultured HCECs

HCECs were collected from a culture dish by TrypLE Select treatment as described above and suspended at a concentration of 4 × 10⁶ cells/mL in FACS buffer (PBS containing 1% BSA and 0.05% NaN₃). An equal volume of antibody solution was added and incubated for 2 hours at 4.degrees.C. After washing with FACS buffer, the HCECs were analyzed with FACS Canto II (BD Biosciences).

### Isolation of HCEC subpopulations by MACS

The HCECs were detached with TrypLE Select as described above and CD44⁻HCEC subpopulation (effector subpopulation) was isolated using anti-human CD44 microbeads (Miltenyi Biotec, Bergisch Gladbach, Germany) and the program depl05 of an autoMACS Pro separator (Miltenyi Biotec). The purity of the isolated effector subpopulation was higher than 90% in all cases as demonstrated by flow cytometry (Figs. 65-67).

### (Example 12: Preparation of cell suspension administered to bullous keratopathy patient in clinical research: type of administered vehicles, ROCK inhibitor, stability of cells)

Preparation of cell suspension for infusion to a bullous keratopathy patient is summarized below.

After completion of culturing, cells in a flask are washed with PBS and treated with TrypLE. After collected the cells, the cells are washed twice with Opti-MEM, and components of the culture solution such as BSA are thoroughly removed. The cell suspension was adjusted by adding an infusion solution (vehicle) and 100 .micro.M of Y-27632 for use in infusion vehicle.

It was confirmed that there is no change in the survival rate up to 6 hours in ice and room temperature when using this infusion vehicle. A test for long-term stability in the infusion vehicle was also conducted. The cell survival rate was compared for over a maximum of 72 hours with an intraocular irrigating solution Opeguard (supplemented with 10% HSA) that is frequently used in the ophthalmic field in comparison with Opti-MEM.

As a result, both exhibited 80% or higher survival rate up to at least 24 hours. For cell stability in the infusion vehicle, a stability test of 24-48 hours after readjustment with CPC while expecting multi-facility trials shows stability for not only the survival rate, but also quality standard test items such as cell surface markers and products.

As described above, the present invention is exemplified by the use of its preferred Embodiments. However, it is understood that the scope of the present invention should be interpreted solely based on the claims. It is also understood that any patent, any patent application, and any references cited herein should be incorporated herein by reference in the same manner as the contents are specifically described herein. The present application claims priority to Japanese Patent Application No. 2016-26423, Japanese Patent Application No. 2016-26424, Japanese Patent Application No. 2016-26425, Japanese Patent Application No. 2016-26426, and Japanese Patent Application No. 2016-77450 filed on February 15, 2016 in Japan. The entire content of these applications is incorporated herein by reference.

### [Industrial Applicability]

The present therapeutic method gives rise to a paradigm shift in the corneal endothelium regenerative medicine, which has a potential expanded application to over a million patients worldwide as an internationally deployable, versatile medicine. Thus, applicability is found in the medical industry and its peripheral industries.

The invention is now described by reference to the following embodiments.

### [Embodiment 1]

A human functional corneal endothelial cell capable of eliciting a human corneal endothelial functional property when infused into an anterior chamber of a human eye.

### [Embodiment 2]

The cell of Embodiment 1, wherein the cell expresses cell surface antigens comprising CD166 positive and CD133 negative phenotypes.

### [Embodiment 3]

The cell of Embodiment 2, wherein the cell surface antigens comprise CD166 positive, CD133 negative, and CD44 negative to intermediately positive phenotypes.

### [Embodiment 4]

The cell of Embodiment 2, wherein the cell surface antigens comprise CD166 positive, CD133 negative, and CD44 negative to CD44 weakly positive phenotypes.

### [Embodiment 5]

The cell of Embodiment 2, wherein the cell surface antigens comprise CD166 positive, CD133 negative, and CD200 negative phenotypes.

### [Embodiment 6]

The cell of Embodiment 3, wherein the cell surface antigens comprise CD166 positive, CD133 negative, and CD44 negative to intermediately positive and CD90 negative phenotypes.

### [Embodiment 7]

The cell of any one of Embodiments 2-6, further comprising at least one surface antigen selected from the group consisting of CD105 negative to weakly positive, CD24 negative, CD26 negative, LGR5 negative, SSEA3 negative, MHC1 weakly positive, MHC2 negative, ZO-1 positive, and Na⁺/K⁺ATPase positive.

### [Embodiment 8]

The cell of any one of Embodiments 1-7, wherein a mean cell area of the cell is 250 .micro.m² or less.

### [Embodiment 9]

A cell population comprising the cells of any one of Embodiments 1-8.

### [Embodiment 10]

A medicament comprising a functional corneal endothelial cell capable of eliciting a human corneal functional property when infused into an anterior chamber of a human eye.

### [Embodiment 11]

The medicament of Embodiment 10, wherein the medicament is for treating a corneal endothelial dysfunction or disease.

### [Embodiment 12]

The medicament of Embodiment 11, wherein the corneal endothelial dysfunction or disease comprises at least one selected from the group consisting of corneal endothelial disorder Grade 3 and corneal endothelial disorder Grade 4 (bullous keratopathy) (e.g., Fuchs endothelial corneal dystrophy, PEX-BK (pseudoexfoliation bullous keratopathy; bullous keratopathy involving pseudoexfoliation syndrome), post-laser iridotomy bullous keratopathy, post-cataract surgery bullous keratopathy, post-glaucoma surgery bullous keratopathy (including pseudophakic or aphakic bullous keratopathy), and post-trauma bullous keratopathy, bullous keratopathy of unknown cause after multiple surgeries, post-corneal transplantation graft failure, congenital corneal endothelial dystrophy, and congenital anterior chamber angle hypoplasia syndrome, wherein the grade system used is based upon the severity classification of corneal endothelial disorders.

### [Embodiment 13]

The medicament of any one of Embodiments 10-12, wherein the cell is administered in conjunction with the additional agent comprises a ROCK inhibitor.

### [Embodiment 14]

The medicament of any one of Embodiments 10-13, wherein the medicament comprises the cell at a density of 5 × 10⁴ cells/300 .micro.L to 2 × 10⁶ cells/300 .micro.L.

### [Embodiment 15]

The medicament of any one of Embodiments 10-14, wherein the medicament further comprises cell infusion vehicle comprising at least one of ROCK inhibitor, albumin, ascorbic acid, and lactic acid.

### [Embodiment 16]

The medicament of Embodiment 15, wherein the cell infusion vehicle further comprises albumin, ascorbic acid, and lactic acid.

### [Embodiment 17]

The medicament of Embodiment 15 or 16, wherein the cell infusion vehicle comprises OPEGUARD-MA(R).

### [Embodiment 18]

The medicament of any one of Embodiments 10-17, wherein said corneal endothelial functional cell is the cell according to any one of Embodiments 1-8 or the cell population according to Embodiment 9.

### [Embodiment 19]

A method of manufacturing a human functional corneal endothelial cell capable of eliciting a human corneal endothelial functional property when infused into an anterior chamber of a human eye, comprising a step of growing, maturating and differentiating a human corneal endothelial tissue-derived cell or a corneal endothelial progenitor cell directly or indirectly via a step of dedifferentiation.

### [Embodiment 20]

A method of manufacturing a human functional corneal endothelial cell capable of eliciting a human corneal endothelial functional property when infused into an anterior chamber of a human eye, comprising a step of culturing to mature and differentiate a corneal endothelial tissue-derived cell or a corneal endothelial progenitor cell by a step comprising actin depolymerization.

### [Embodiment 21]

The method of manufacturing of Embodiment 19 or 20, wherein the actin depolymerization is accomplished by one or a plurality of agents selected from the group consisting of a ROCK inhibitor, HDAC inhibitor, actin depolymerization inhibitor, PPARgamma inhibitor, MMP2 inhibitor, p53 activator, and miRNA.

### [Embodiment 22]

The method of manufacturing of any one of Embodiments 19-21, further comprising a step of culturing the corneal endothelial tissue-derived cell or corneal endothelial progenitor cell under steps where a cell enter into epithelial-mesenchymal transition-like transformation, growth, maturation and differentiation.

### [Embodiment 23]

The method of manufacturing of Embodiment 22, wherein after the cultured cell reaches saturated cell density and then the differentiation and maturation of a cultured cell becomes complete with sufficient formation of tight junctions, culturing is further maintained for 1 week or more by only exchanging a medium to preserve the cultured cell.

### [Embodiment 24]

The method of manufacturing of Embodiment 23, further comprising a step of monitoring cell subpopulation composition during the culturing, wherein the monitoring comprises tracking at least one item selected from the group consisting of mitochondrial function, oxygen consumption and pH of a culture solution, amino acid composition, proteinaceous product, soluble miRNA, cell density with a noninvasive engineering approach, cell size, and cell homogeneity.

### [Embodiment 25]

The method of any one of Embodiments 19-24, comprising a step of culturing in the presence of a serum-free medium.

### [Embodiment 26]

A method of quality control or process control of a cultured human functional corneal endothelial cell capable of eliciting a human corneal endothelial functional property when infused into an anterior chamber of a human eye, comprising the step of measuring at least one cell function indicator selected from the group consisting of: a cell surface marker; a proteinaceous product and a related biological material of the product; a SASP related protein; intracellular and secreted miRNA; an exosome; a cellular metabolite comprising an amino acid and a related biological material of the metabolite; cell size; cell density and the presence of an autoantibody reactive cell.

### [Embodiment 27]

The method of any one of Embodiment 26, further comprising identifying a subpopulation of the cultured functional corneal endothelial cell by at least one of corneal functional properties according to any one of Embodiments 1-9.

### [Embodiment 28]

A method of detecting a corneal endothelial nonfunctional cell coexisting with a cultured human corneal endothelial cell comprising the step of measuring at least one cell function indicator selected from the group consisting of: a cell surface marker; a proteinaceous product and a related biological material of the product; a SASP related protein; intracellular and secreted miRNA; an exosome; a cellular metabolite comprising an amino acid and a related biological material of the metabolite; cell size; cell density and the presence of an autoantibody reactive cell.

### [Embodiment 29]

A method of quality control or process control of a cultured human functional corneal endothelial cell capable of eliciting human corneal functional phenotypes when infused into an anterior chamber of a human eye or a method of detecting a corneal endothelial nonfunctional cell coexisting with a cultured human corneal endothelial cell, comprising the step of examining one or a plurality of the following:
(1) purity test by culture supernatant ELISA
   TIMP-1: 500 ng/mL or less
   IL-8: 500 pg/mL or less
   PDGF-BB: 30 pg/mL or greater
   MCP-1: 3000 pg/mL or less
(2) purity test by cell FACS
   CD166 = 95% or greater
   CD133 = 5% or less
   CD105 negative-low positive = 95% or greater
   CD44 negative-low positive = 70% or greater
   CD44 medium-high positive = 15% or less
   CD24 = 5% or less
   CD26 positive = 5% or less
   CD200 = 5% or less
(3) barrier function (ZO-1) positive
(4) pump function (Na+/K+ ATPase) positive
(5) cell survival
   70% or greater with trypan blue stain
(6) cell form
   transformed cells cannot be found by visual inspection
(7) Claudin10 positive
(8) effector cell (E-ratio) > 50%
(9) non-intended cell
   non-intended cell A (CD44 strong positive cell) < 15%, non-intended cell B (CD26 positive cell) <5 %, non-intended cell C (CD24 positive cell) < 5% (10) karyotype abnormality negative.

### [Embodiment 30]

A method of quality control or process control of a cultured human functional corneal endothelial cell capable of eliciting human corneal functional phenotypes when infused into an anterior chamber of a human eye, comprising the step of determining one or a plurality of the following characteristics with respect to a target cell: (1) retention of endothelial pumping/barrier functions; (2) adhesion/attachment to a specific laminin; (3) secreted cytokine profile; (4) produced metabolite profile; (5) saturated cell density upon in vitro culture; (6) spatial size and distribution of a cell obtained in culturing; and (8) cell retention in case of cell infusion after freeze damage by cryo treatment by liquid nitrogen on mouse cornea.

## Claims

1. A method of manufacturing a human functional corneal endothelial cell capable of eliciting a human corneal endothelial functional property when infused into an anterior chamber of a human eye, comprising a step of growing, maturating and differentiating a human corneal endothelial tissue-derived cell or a corneal endothelial progenitor cell directly or indirectly via a step of dedifferentiation.

2. A method of manufacturing a human functional corneal endothelial cell capable of eliciting a human corneal endothelial functional property when infused into an anterior chamber of a human eye, comprising a step of culturing to mature and differentiate a corneal endothelial tissue-derived cell or a corneal endothelial progenitor cell by a step comprising actin depolymerization.

3. The method of manufacturing of Claim 1 or 2, wherein the actin depolymerization is accomplished by one or a plurality of agents selected from the group consisting of a ROCK inhibitor, HDAC inhibitor, actin depolymerization inhibitor, PPARgamma inhibitor, MMP2 inhibitor, p53 activator, and miRNA.

4. The method of manufacturing of any one of Claims 1-3, further comprising a step of culturing the corneal endothelial tissue-derived cell or corneal endothelial progenitor cell under steps where a cell enter into epithelial-mesenchymal transition-like transformation, growth, maturation and differentiation.

5. The method of manufacturing of Claim 4, wherein after the cultured cell reaches saturated cell density and then the differentiation and maturation of a cultured cell becomes complete with sufficient formation of tight junctions, culturing is further maintained for 1 week or more by only exchanging a medium to preserve the cultured cell.

6. The method of manufacturing of Claim 5, further comprising a step of monitoring cell subpopulation composition during the culturing, wherein the monitoring comprises tracking at least one item selected from the group consisting of mitochondrial function, oxygen consumption and pH of a culture solution, amino acid composition, proteinaceous product, soluble miRNA, cell density with a noninvasive engineering approach, cell size, and cell homogeneity.

7. The method of any one of Claims 1-6, comprising a step of culturing in the presence of a serum-free medium.

8. A method of quality control of process control of a cultured human functional corneal endothelial cell capable of eliciting a human corneal endothelial functional property when infused into an anterior chamber of a human eye, comprising the step of measuring at least one cell function indicator selected from the group consisting of: a cell surface marker; a proteinaceous product and a related biological material of the product; a SASP related protein; intracellular and secreted miRNA; an exosome; a cellular metabolite comprising an amino acid and a related biological material of the metabolite; cell size; cell density and the presence of an autoantibody reactive cell.

9. The method of any one of Claim 8, further comprising identifying a subpopulation of the cultured functional corneal endothelial cell by the human corneal endothelial functional property.

10. A method of detecting a corneal endothelial nonfunctional cell coexisting with a cultured human corneal endothelial cell comprising the step of measuring at least one cell function indicator selected from the group consisting of: a cell surface marker; a proteinaceous product and a related biological material of the product; a SASP related protein; intracellular and secreted miRNA; an exosome; a cellular metabolite comprising an amino acid and a related biological material of the metabolite; cell size; cell density and the presence of an autoantibody reactive cell.

11. A method of quality control of process control of a cultured human functional corneal endothelial cell capable of eliciting human corneal functional phenotypes when infused into an anterior chamber of a human eye or a method of detecting a corneal endothelial nonfunctional cell coexisting with a cultured human corneal endothelial cell, comprising the step of examining one or a plurality of the following:
(1) purity test by culture supernatant ELISA
TIMP-1: 500 ng/mL or less
IL-8: 500 pg/mL or less
PDGF-BB: 30 pg/mL or greater
MCP-1: 3000 pg/mL or less
(2) purity test by cell FACS
CD166 = 95% or greater
CD133 = 5% or less
CD105 negative-low positive = 95% or greater
CD44 negative-low positive = 70% or greater
CD44 medium-high positive = 15% or less
CD24 = 5% or less
CD26 positive = 5% or less
CD200 = 5% or less
(3) barrier function (ZO-1) positive
(4) pump function (Na+/K+ ATPase) positive
(5) cell survival
70% or greater with trypan blue stain
(6) cell form
transformed cells cannot be found by visual inspection
(7) Claudin10 positive
(8) effector cell (E-ratio) > 50%
(9) non-intended cell
non-intended cell A (CD44 strong positive cell) < 15%, non-intended cell B (CD26 positive cell) <5 %, non-intended cell C (CD24 positive cell) < 5% (10) karyotype abnormality negative.

12. A method of quality control or process control of a cultured human functional corneal endothelial cell capable of eliciting human corneal functional phenotypes when infused into an anterior chamber of a human eye, comprising the step of determining one or a plurality of the following characteristics with respect to a target cell: (1) retention of endothelial pumping/barrier functions; (2) adhesion/attachment to a specific laminin; (3) secreted cytokine profile; (4) produced metabolite profile; (5) saturated cell density upon in vitro culture; (6) spatial size and distribution of a cell obtained in culturing; and (8) cell retention in case of cell infusion after freeze damage by cryo treatment by liquid nitrogen on mouse cornea.
